# EUROPÄISCHE PATENTSCHRIFT

(11) **EP 2 539 326 B1**
(45) Veröffentlichungstag und Bekanntmachung des Hinweises auf die Patenterteilung: **03.05.2017**
(21) Anmeldenummer: 11704637.5
(22) Anmeldetag: 25.02.2011
(51) Int. Cl.: C07D 249/12, C07D 401/06, C07D 403/06, C07D 409/06, C07D 409/14, C07D 413/06, C07D 413/14, C07D 417/06, A61K 31/4196, A61P 9/00

(54) **BIS-ARYLVERKNÜPFTE ARYLTRIAZOLONE UND IHRE VERWENDUNG**
BISARYL-BONDED ARYLTRIAZOLONES AND USE THEREOF
ARYLTRIAZOLONE LIÉE À UN BIS-ARYLE ET SON UTILISATION

(30) Priorität: 27.02.2010 DE 102010009631
(43) Veröffentlichungstag der Anmeldung: 02.01.2013
(73) Patentinhaber: Bayer Intellectual Property GmbH, 40789 Monheim (DE)
(72) Erfinder: FÜRSTNER, Chantal, 45478 Mülheim/Ruhr (DE); KELDENICH, Jörg, 42113 Wuppertal (DE); DELBECK, Martina, 42579 Heiligenhaus (DE); KOLKHOF, Peter, 42113 Wuppertal (DE); KRETSCHMER, Axel, 42113 Wuppertal (DE); PLUSCHKELL, Ingo, 44879 Bochum (DE); POOK, Elisabeth, 42109 Bochum (DE); SCHMECK, Carsten, 45472 Mülheim/Ruhr (DE); TRÜBEL, Hubert, 42281 Wuppertal (DE)
(86) Internationale Anmeldenummer: PCT/EP2011/052781
(87) Internationale Veröffentlichungsnummer: WO 2011/104322

(56) Entgegenhaltungen:
- WO-A1-93/17681
- DE-A1-102006 024 024
- DOBOSZ MARIA ET AL: "Synthesis and some pharmacological properties of 3-(4-phenyl-5-oxo-1,2,4-triazolin-1-ylmeth yl)-1,2,4-triazolin-5-thione derivatives", ACTA POLONIAE PHARMACEUTICA - DRUG RESEARCH, POLISH PHARMACEUTICAL SOCIETY, WARZSAW, PL, Bd. 59, Nr. 4, 1. Juli 2002 (2002-07-01), Seiten 281-290, XP008135123, ISSN: 0001-6837
- CHANG L L ET AL: "Triazolinones as nonpeptide angiotensin II antagonists. 1. Synthesis and evaluation of potent 2,4,5-trisubstituted triazolinones", JOURNAL OF MEDICINAL CHEMISTRY 1993 US, Bd. 36, Nr. 17, 1993, Seiten 2558-2568, XP002633952, ISSN: 0022-2623

## Beschreibung

Die vorliegende Anmeldung betrifft neue, bis-arylverknüpfte 5-Aryl-1,2,4-triazolon-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefäßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. paraventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, V1b- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den V1b-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärharns. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G.S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar. Neben der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

Aufgabe der vorliegenden Erfindung war daher die Bereitstellung neuer Verbindungen, die als potente selektive oder duale V1a/V2-Rezeptor-Antagonisten wirken und als solche zur Behandlung und/oder Prävention von Krankheiten, insbesondere zur Behandlung und/oder Prävention von kardiovaskulären Erkrankungen geeignet sind.

In EP 0 412 594-A2, WO 92/20662-A1 und US 2001/0020100-A1 werden 4-(Biphenylalkyl)-1,2,4-triazolone mit Angiotensin II-antagonistischer Wirkung für die Behandlung kardiovaskulärer Erkrankungen beschrieben. In WO 99/31099-A1 werden verschiedenartig substituierte 1,2,4-Triazolone als therapeutisch nutzbare Integrin-Rezeptor-Antagonisten beansprucht. Die Verwendung von 5-Aryl-1,2,4-triazolonen als Arzneimittel mit neuroprotektiver Wirkung ist in WO 99/54315-A2 offenbart, und in WO 2006/117657-A1 werden 4,5-Diaryltriazolon-Derivate als anti-inflammatorische Agentien beschrieben. In WO 2006/078698-A1 werden verschiedene heterocyclische Verbindungen als Tyrosin-Phosphatase-Inhibitoren zur Behandlung von Diabetes beansprucht. In WO 2005/105779-A1 werden 3-Heterocyclyl-4-phenyltriazole als Inhibitoren des Vasopressin-V1A-Rezeptors offenbart, und in WO 2007/134862-A1 werden amidisch verknüpfte 5-Aryl-1,2,4-triazolone als duale Vasopressin-Antagonisten beschrieben. Aus WO 00/58306-A1 und WO 00/68227-A1 sind heterocyclisch substituierte Benzoylpyrazole und -isoxazole als Herbizide bekannt.
Offenbart sind Verbindungen der allgemeinen Formel (I) 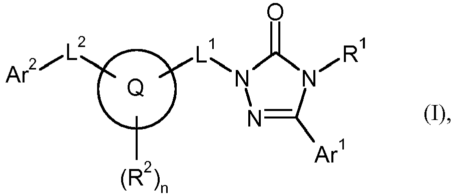 in welcher
- R¹: für (C₁-C₆)-Alkyl, (C₂-C₆)-Alkenyl oder (C₂-C₆)-Alkinyl steht, welche jeweils ein- bis dreifach, gleich oder verschieden, mit Resten ausgewählt aus der Reihe Fluor, Chlor, Cyano, Trifluormethyl, Oxo, Hydroxy, Difluormethoxy, Trifluormethoxy, (C₁-C₄)-Alkoxy, (C₃-C₇)-Cycloalkyl und Phenyl substituiert sein können,
wobei (C₃-C₇)-Cycloalkyl bis zu zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoxy substituiert sein kann
   und
wobei Phenyl bis zu dreifach, gleich oder verschieden, mit Resten ausgewählt aus der Reihe Halogen, Cyano, Nitro, Difluormethyl, Trifluormethyl, (C₁-C₄)-Alkyl, Hydroxy, Hydroxymethyl, Difluormethoxy, Trifluormethoxy, (C₁-C₄)-Alkoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
oder
für (C₃-C₇)-Cycloalkyl steht, welches ein- oder zweifach, gleich oder verschieden, mit Resten ausgewählt aus der Reihe Fluor, Trifluormethyl, (C₁-C₄)-Alkyl, Oxo, Hydroxy, Trifluormethoxy und (C₁-C₄)-Alkoxy substituiert sein kann,
- Ar¹: für Phenyl, Thienyl oder Furyl steht, welche jeweils ein- bis dreifach, gleich oder verschieden, mit Resten ausgewählt aus der Reihe Halogen, Cyano, Nitro, Difluormethyl, Trifluormethyl, (C₁-C₄)-Alkyl, Hydroxy, Trifluormethoxy und (C₁-C₄)-Alkoxy substituiert sein können,
- L¹: für die Gruppe -CH₂-, -C(=O)- oder -SO₂- steht,
- Q: für einen Phenyl-Ring, einen 5-gliedrigen Heteroaryl-Ring mit bis zu drei Ring-Heteroatomen aus der Reihe N, O und/oder S oder einen 6-gliedrigen Heteroaryl-Ring mit bis zu drei Ring-Stickstoffatomen steht,
- R²: für einen Substituenten ausgewählt aus der Reihe Fluor, Chlor, Brom, Cyano, Nitro, (C₁-C₄)-Alkyl, (C₃-C₆)-Cycloalkyl, Phenyl, Hydroxy, (C₁-C₄)-Alkoxy, Amino, Aminocarbonylamino, (C₁-C₄)-Alkylcarbonylamino, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl steht,
wobei der (C₁-C₄)-Alkyl-Substituent seinerseits mit Hydroxy, (C₁-C₄)-Alkoxy, Carbamoyloxy, Hydroxycarbonyl, (C₁-C₄)-Alkoxycarbonyl, Aminocarbonyl, Mono-(C₁-C₄)-alkylaminocarbonyl oder Di-(C₁-C₄)-alkylaminocarbonyl oder bis zu dreifach mit Fluor substituiert sein kann
   und
wobei der Phenyl-Substituent seinerseits mit Fluor, Chlor, Cyano, Methyl, Trifluormethyl oder Methoxy substituiert sein kann,
- n: für die Zahl 0, 1 oder 2 steht,
wobei im Fall, dass der Substituent R² zweifach auftritt, seine Bedeutungen gleich oder verschieden sein können,
- L²: für eine Bindung, für -O- oder für eine Gruppe der Formel -(CR^{3A}R^{3B})ₚ- steht, worin
R^{3A} Wasserstoff, Fluor oder Methyl bedeutet,
R^{3B} Wasserstoff, Fluor, (C₁-C₄)-Alkyl, Hydroxycarbonyl, (C₁-C₄)-Alkoxycarbonyl oder Aminocarbonyl bedeutet,
wobei (C₁-C₄)-Alkyl mit Hydroxy oder Carbamoyloxy oder bis zu dreifach mit Fluor substituiert sein kann,
oder
R^{3A} und R^{3B} miteinander verknüpft sind und zusammen eine -(CH₂)ᵣ-Brücke bilden, worin
r die Zahl 2, 3, 4 oder 5 bedeutet
   und eine CH₂-Gruppe dieser Brücke gegen -O- ausgetauscht sein kann,
und
- p: die Zahl 1 oder 2 bedeutet,
wobei im Falle, dass die Gruppe -CR^{3A}R^{3B}- zweifach auftritt, die einzelnen Bedeutungen von R^{3A} und R^{3B} jeweils gleich oder verschieden sein können,
und
- Ar²: für Phenyl, Naphthyl oder 5- bis 10-gliedriges Heteroaryl mit bis zu drei Ring-Heteroatomen aus der Reihe N, O und/oder S steht, welche jeweils ein- bis dreifach, gleich oder verschieden, mit Resten ausgewählt aus der Reihe Halogen, Cyano, Nitro, Difluormethyl, Trifluormethyl, (C₁-C₄)-Alkyl, Hydroxy, Difluormethoxy, Trifluormethoxy und (C₁-C₄)-Alkoxy substituiert sein können,
sowie ihre Salze, Solvate und Solvate der Salze.

Alle nachfolgenden Gegenstände und Aspekte, die nicht unter die Ansprüche fallen, sind lediglich offenbart, aber nicht Teil der Erfindung, auch wenn sie als erfindungsgemäß bezeichnet sind.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N,N*-Diisopropylethylamin, Monoethanolamin, Diethanolamin, Triethanolamin, Dimethylaminoethanol, Diethylaminoethanol, Procain, Dicyclohexylamin, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec.*-Butyl, *tert*.-Butyl, *n*-Pentyl, 2-Pentyl, 3-Pentyl, Neopentyl, *n*-Hexyl, 2-Hexyl und 3-Hexyl.

(C₁-C₄)-Alkylcarbonyl steht im Rahmen der Erfindung für einen geradkettigen oder verzweigten Alkylrest mit 1 bis 4 Kohlenstoffatomen, der über eine Carbonyl-Gruppe [-C(=O)-] mit dem Rest des Moleküls verknüpft ist. Beispielhaft und vorzugsweise seien genannt: Acetyl, Propionyl, *n*-Butyryl, *iso*-Butyryl, *n*-Pentanoyl und Pivaloyl.

(C₂-C₆)-Alkenyl steht im Rahmen der Erfindung für einen geradkettigen oder verzweigten Alkenylrest mit 2 bis 6 Kohlenstoffatomen und einer Doppelbindung. Bevorzugt ist ein geradkettiger oder verzweigter Alkenylrest mit 3 bis 6 Kohlenstoffatomen. Beispielhaft und vorzugsweise seien genannt: Vinyl, *n*-Prop-1-en-1-yl, Allyl, Isopropenyl, 2-Methyl-2-propen-1-yl, *n*-But-1-en-1-yl, *n*-But-2-en-1-yl, *n*-But-3-en-1-yl, *n*-Pent-2-en-1-yl, *n*-Pent-3-en-1-yl, *n*-Pent-4-en-1-yl, 3-Methylbut-2-en-1-yl und 4-Methylpent-3-en-1-yl.

(C₂-C₆)-Alkinyl steht im Rahmen der Erfindung für einen geradkettigen oder verzweigten Alkinylrest mit 2 bis 6 Kohlenstoffatomen und einer Dreifachbindung. Bevorzugt ist ein geradkettiger oder verzweigter Alkinylrest mit 3 bis 6 Kohlenstoffatomen. Beispielhaft und vorzugsweise seien genannt: Ethinyl, *n*-Prop-1-in-1-yl, *n*-Prop-2-in-1-yl, *n*-But-2-in-1-yl, *n*-But-3-in-1-yl, *n*-Pent-2-in-1-yl, *n-*Pent-3-in-1-yl und *n*-Pent-4-in-1-yl.

(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n-*Propoxy, Isopropoxy, *n*-Butoxy, *iso*-Butoxy, *sec.*-Butoxy und *tert*.-Butoxy.

(C₁-C₄)-Alkoxymethyl steht im Rahmen der Erfindung für einen geradkettigen oder verzweigten Alkoxyrest mit 1 bis 4 Kohlenstoffatomen, der über eine an das O-Atom gebundene MethylenGruppe [-CH₂-] mit dem Rest des Moleküls verknüpft ist. Beispielhaft und vorzugsweise seien genannt: Methoxymethyl, Ethoxymethyl, *n*-Propoxymethyl, Isopropoxymethyl, *n*-Butoxymethyl und *tert*.-Butoxymethyl.

(C₁-C₄)-Alkoxycarbornyl steht im Rahmen der Erfindung für einen geradkettigen oder verzweigten Alkoxyrest mit 1 bis 4 Kohlenstoffatomen, der über eine an das O-Atom gebundene Carbonyl-Gruppe [-C(=O)-] mit dem Rest des Moleküls verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und tert.-Butoxycarbonyl.

Mono-(C₁-C₄)-akylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n-*Butylamino und *tert.*-Butylamino.

Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N-*Diethylamino, *N-*Ethyl-*N-*methylamino, *N-*Methyl-*N-n*-propylamino, *N-*Isopropyl-*N-*methylamino, *N-*Isopropyl-*N-n*-propylamino, *N*,*N*-Diisopropylamino, *N-n*-Butyl-*N-N*-methylamino, *N,N-*Di-*n*-butyl-amino und *N-tert.*-Butyl*-N-*methylamino.

Mono- bzw. Di-(C₁-C₄)-alkylaminocarbonyl steht im Rahmen der Erfindung für eine Amino-Gruppe, die über eine Carbonyl-Gruppe [-C(=O)-] mit dem Rest des Moleküls verknüpft ist und die einen geradkettigen oder verzweigten bzw. zwei gleiche oder verschiedene geradkettige oder verzweigte *N-*Alkylsubstituenten mit jeweils 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N,N-*Dimethylaminocarbonyl, *N,N-*Diethylaminocarbonyl, *N-*Ethyl-*N-*methylaminocarbonyl, *N-*Methyl-*N-n*-propylaminocarbonyl, *N,N-*Diisopropylaminocarbonyl, *N-n*-Butyl-*N*-methylaminocarbonyl und *N-tert.-*Butyl*-N-*methylaminocarbonyl.

(C₁-C₄)-Alkylcarbonylamino steht im Rahmen der Erfindung für eine Amino-Gruppe mit einem geradkettigen oder verzweigten Alkylcarbonyl-Substituenten, der 1 bis 4 Kohlenstoffatome im Alkylrest aufweist und über die Carbonylgruppe mit dem N-Atom verknüpft ist. Beispielhaft und vorzugsweise seien genannt: Acetylamino, Propionylamino, *n*-Butyrylamino, *iso*-Butyrylamino, *n*-Pentanoylamino und Pivaloylamino.

(C₃-C₇)-Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

5-gliedriges Heteroaryl in der Definition des Ringes Q steht für einen aromatischen Heterocyclus (Heteroaromaten) mit insgesamt 5 Ringatomen, der bis zu drei gleiche oder verschiedene Ring-Heteroatome aus der Reihe N, O und/oder S enthält und über Ring-Kohlenstoffatome oder gegebenenfalls ein Ring-Stickstoffatom verknüpft ist. Beispielhaft seien genannt: Furyl, Pyrrolyl, Thienyl, Pyrazolyl, Imidazolyl, 1,2-Oxazolyl (Isoxazolyl), 1,3-Oxazolyl, 1,2-Thiazolyl (Isothiazolyl), 1,3-Thiazolyl, 1,2,3-Triazolyl, 1,2,4-Triazolyl, 1,2,4-Oxadiazolyl, 1,3,4-Oxadiazolyl, 1,2,4-Thiadiazolyl und 1,3,4-Thiadiazolyl.

6-gliedriges Heteroaryl in der Definition des Ringes Q steht für einen aromatischen Heterocyclus (Heteroaromaten) mit insgesamt 6 Ringatomen, der ein, zwei oder drei Ring-Stickstoffatome enthält und über Ring-Kohlenstoffatome verknüpft ist. Beispielhaft seien genannt: Pyridyl, Pyrimidinyl, Pyridazinyl, Pyrazinyl, 1,2,4-Triazinyl und 1,3,5-Triazinyl. Bevorzugt ist 6-gliedriges Heteroaryl mit ein oder zwei Ring-Stickstoffatomen, wie Pyridyl, Pyrimidinyl, Pyridazinyl und Pyrazinyl.

5- bis 10-gliedriges Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind mono- oder gegebenenfalls bicyclische 5- bis 10-gliedrige Heteroaryl-Reste mit bis zu zwei Heteroatomen aus der Reihe N, O und/oder S. Besonders bevorzugt sind monocyclische 5- oder 6-gliedrige Heteroaryl-Reste mit bis zu zwei Heteroatomen aus der Reihe N, O und/oder S, wie beispielsweise Furyl, Thienyl, Thiazolyl, Oxazolyl, Isothiazolyl, Isoxazolyl, Pyrazolyl, Imidazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Chlor, Fluor oder Brom, besonders bevorzugt Fluor oder Chlor.

Ein Oxo-Substituent steht im Rahmen der Erfindung für ein Sauerstoffatom, das über eine Doppelbindung an ein Kohlenstoffatom gebunden ist.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei oder drei gleichen oder verschiedenen Substituenten ist bevorzugt. Besonders bevorzugt ist die Substitution mit einem oder mit zwei gleichen oder verschiedenen Substituenten. Ganz besonders bevorzugt ist die Substitution mit einem Substituenten.

Bevorzugt im Rahmen der vorliegenden Erfindung sind Verbindungen der Formel (I), in welcher
- R¹: für (C₁-C₆)-Alkyl steht, welches ein- bis dreifach, gleich oder verschieden, mit Resten ausgewählt aus der Reihe Fluor, Trifluormethyl, Oxo, Hydroxy, Methoxy, Ethoxy, (C₃-C₆)-Cycloalkyl und Phenyl substituiert sein kann,
wobei (C₃-C₆)-Cycloalkyl bis zu zweifach, gleich oder verschieden, mit Resten ausgewählt aus der Reihe Fluor, Methyl, Trifluormethyl, Ethyl und Hydroxy substituiert sein kann
   und
wobei Phenyl bis zu zweifach, gleich oder verschieden, mit Resten ausgewählt aus der Reihe Fluor, Chlor, Cyano, Methyl, Difluormethyl, Trifluormethyl, Ethyl, Hydroxy, Methoxy, Trifluormethoxy, Ethoxy, Hydroxycarbonyl, Methoxycarbonyl, Ethoxycarbonyl und Aminocarbonyl substituiert sein kann,
oder
für (C₂-C₆)-Alkenyl steht
oder
für (C₃-C₆)-Cycloalkyl steht, welches ein- oder zweifach, gleich oder verschieden, mit Resten ausgewählt aus der Reihe Fluor, Methyl, Trifluormethyl, Ethyl und Hydroxy substituiert sein kann,
- Ar¹: für Phenyl oder Thienyl steht, welche jeweils ein- oder zweifach, gleich oder verschieden, mit Resten ausgewählt aus der Reihe Fluor, Chlor, Cyano, Methyl, Trifluormethyl, Ethyl, Hydroxy, Methoxy, Trifluormethoxy und Ethoxy substituiert sein können,
- L¹: für die Gruppe -CH₂- oder -SO₂- steht,
- Q: für einen 5-gliedrigen Heteroaryl-Ring mit bis zu drei Ring-Heteroatomen aus der Reihe N, O und/oder S oder einen 6-gliedrigen Heteroaryl-Ring mit bis zu zwei Ring-Stickstoffatomen steht, oder
für einen gegebenenfalls substituierten Phenyl-Ring der Formel 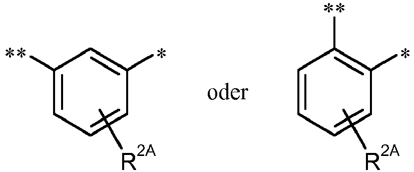 steht, worin
* die Verknüpfungsstelle mit der Gruppe L¹
und
** die Verknüpfungsstelle mit der Gruppe L² kennzeichnen,
und
R^{2A} Wasserstoff, Fluor, Chlor, Brom, Methyl, Trifluormethyl, Hydroxymethyl, Carbamoyloxymethyl, Hydroxycarbonyl, Methoxycarbonyl, Ethoxycarbonyl, Aminocarbonyl, Methylaminocarbonyl oder tert.-Butylaminocarbonyl bedeutet,
- R²: für einen Substituenten ausgewählt aus der Reihe Fluor, Chlor, Brom, (C₁-C₄)-Alkyl, (C₃-C₆)-Cycloalkyl, Phenyl, (C₁-C₄)-Alkoxy, Hydroxycarbonyl, (C₁-C₄)-Alkoxycarbonyl, Aminocarbonyl und Mono-(C₁-C₄)-alkylaminocarbonyl steht,
wobei der (C₁-C₄)-Alkyl-Substituent seinerseits mit Hydroxy, (C₁-C₄)-Alkoxy, Carbamoyloxy, Hydroxycarbonyl, (C₁-C₄)-Alkoxycarbonyl oder Aminocarbonyl oder bis zu dreifach mit Fluor substituiert sein kann
und
wobei der Phenyl-Substituent seinerseits mit Fluor, Chlor, Methyl oder Trifluormethyl substituiert sein kann,
- n: für die Zahl 0 oder 1 steht,
- L²: für eine Bindung oder für eine Gruppe der Formel -(CR^{3A}R^{3B})ₚ- steht, worin
R^{3A} Wasserstoff oder Methyl bedeutet,
R^{3B} Wasserstoff, (C₁-C₄)-Alkyl, Hydroxycarbonyl, (C₁-C₄)-Alkoxycarbonyl oder Aminocarbonyl bedeutet,
wobei (C₁-C₄)-Alkyl mit Hydroxy oder Carbamoyloxy substituiert sein kann,
und
p die Zahl 1 oder 2 bedeutet,
wobei im Falle, dass die Gruppe -CR^{3A}R^{3B}- zweifach auftritt, die einzelnen Bedeutungen von R^{3A} und R^{3B} jeweils gleich oder verschieden sein können, und
- Ar²: für Phenyl steht, welches ein- oder zweifach, gleich oder verschieden, mit Resten ausgewählt aus der Reihe Fluor, Chlor, Cyano, Difluormethyl, Trifluormethyl, (C₁-C₄)-Alkyl, Methoxy, Difluormethoxy, Trifluormethoxy und Ethoxy substituiert sein kann,
sowie ihre Salze, Solvate und Solvate der Salze.

Eine besondere Ausführungsform der vorliegenden Erfindung umfasst Verbindungen der Formel (I), in welcher
- R¹: für (C₁-C₄)-Alkyl, welches ein- oder zweifach, gleich oder verschieden, mit Resten ausgewählt aus der Reihe Fluor, Trifluormethyl, Oxo und Hydroxy substituiert sein kann, oder für Allyl oder Cyclopropyl steht,
sowie ihre Salze, Solvate und Solvate der Salze.

Eine weitere besondere Ausführungsform der vorliegenden Erfindung umfasst Verbindungen der Formel (I), in welcher
- Ar¹: für Phenyl oder Thienyl steht, welche jeweils mit einem Rest ausgewählt aus der Reihe Fluor, Chlor, Cyano, Methyl, Trifluormethyl, Ethyl, Hydroxy, Methoxy, Trifluormethoxy und Ethoxy substituiert sind,
sowie ihre Salze, Solvate und Solvate der Salze.

Eine weitere besondere Ausführungsform der vorliegenden Erfindung umfasst Verbindungen der Formel (I), in welcher
- Ar²: für Phenyl steht, welches ein- oder zweifach, gleich oder verschieden, mit Resten ausgewählt aus der Reihe Fluor, Chlor, Cyano, Difluormethyl, Trifluormethyl, (C₁-C₄)-Alkyl, Methoxy, Difluormethoxy, Trifluormethoxy und Ethoxy substituiert ist,
sowie ihre Salze, Solvate und Solvate der Salze.

Eine weitere besondere Ausführungsform der vorliegenden Erfindung umfasst Verbindungen der Formel (I), in welcher
- Q: für einen gegebenenfalls substituierten Phenyl-Ring der Formel 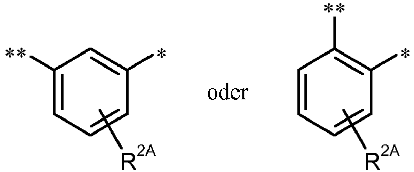 steht, worin
* die Verknüpfungsstelle mit der Gruppe L¹
und
** die Verknüpfungsstelle mit der Gruppe L² kennzeichnen, und
R^{2A} Wasserstoff, Fluor, Chlor, Brom, Methyl, Trifluormethyl, Hydroxymethyl, Carbamoyloxymethyl, Hydroxycarbonyl, Methoxycarbonyl, Ethoxycarbonyl, Aminocarbonyl, Methylaminocarbonyl oder *tert.*-Butylaminocarbonyl bedeutet,
sowie ihre Salze, Solvate und Solvate der Salze.

Eine weitere besondere Ausführungsform der vorliegenden Erfindung umfasst Verbindungen der Formel (I), in welcher
- Q: für einen Pyridyl- oder Pyrimidinyl-Ring der Formel 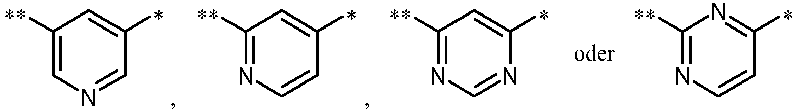 steht, worin
* die Verknüpfungsstelle mit der Gruppe L¹ und
** die Verknüpfungsstelle mit der Gruppe L² kennzeichnen,
sowie ihre Salze, Solvate und Solvate der Salze.

Eine weitere besondere Ausführungsform der vorliegenden Erfindung umfasst Verbindungen der Formel (I), in welcher
- Q: für einen gegebenenfalls substituierten 5-gliedrigen Heteroaryl-Ring der Formel 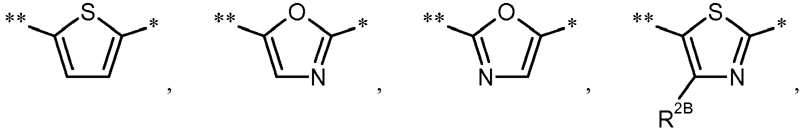 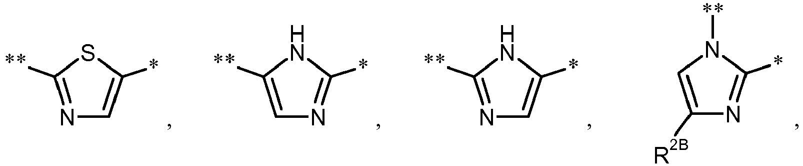 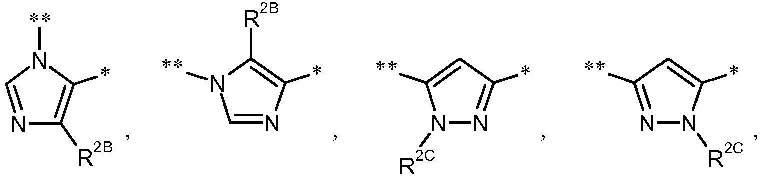 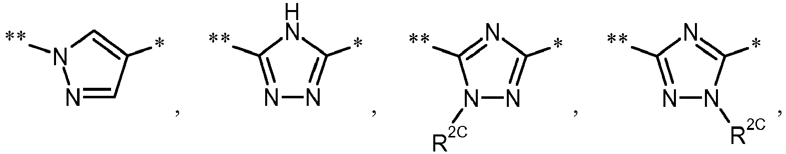 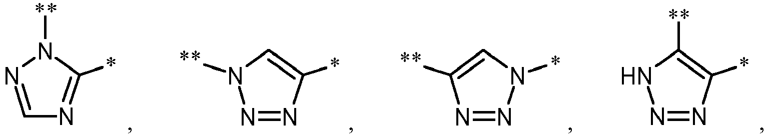 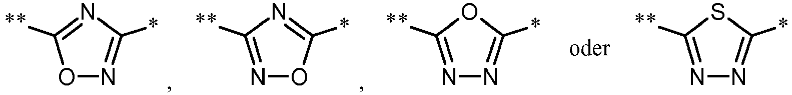 steht, worin
* die Verknüpfungsstelle mit der Gruppe L¹ und
** die Verknüpfungsstelle mit der Gruppe L² kennzeichnen,
R^{2B} Wasserstoff, Methyl oder Trifluormethyl bedeutet und
R^{2C} Wasserstoff oder Methyl, welches mit Hydroxycarbonyl, Methoxycarbonyl oder Aminocarbonyl substituiert sein kann, bedeutet,
sowie ihre Salze, Solvate und Solvate der Salze.

Besonders bevorzugt im Rahmen der vorliegenden Erfindung sind Verbindungen der Formel (I), in welcher
- R¹: für (C₁-C₄)-Alkyl steht, welches ein- oder zweifach, gleich oder verschieden, mit Resten ausgewählt aus der Reihe Fluor, Trifluormethyl, Oxo, Hydroxy und Phenyl substituiert sein kann,
wobei Phenyl seinerseits mit einem Rest ausgewählt aus der Reihe Fluor, Chlor, Methyl, Trifluormethyl, Methoxy, Hydroxycarbonyl und Methoxycarbonyl substituiert sein kann,
oder
für Allyl oder Cyclopropyl steht,
- Ar¹: für Phenyl oder Thienyl steht, welche jeweils mit einem Rest ausgewählt aus der Reihe Fluor und Chlor substituiert sind,
- L¹: für die Gruppe -CH₂- steht,
- Q: für einen Pyridyl-, Pyrimidinyl- oder gegebenenfalls substituierten Phenyl-Ring der Formel 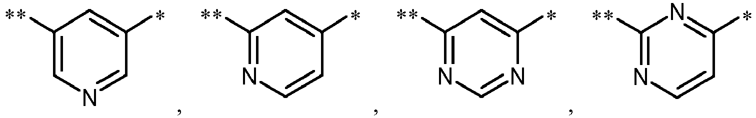 oder 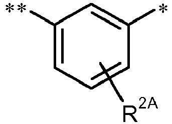 oder
für einen gegebenenfalls substituierten 5-gliedrigen Heteroaryl-Ring der Formel 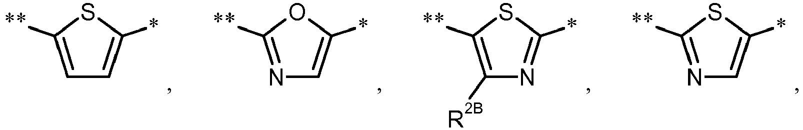 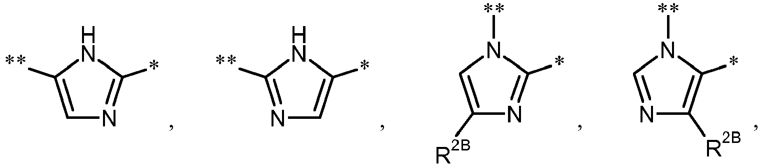 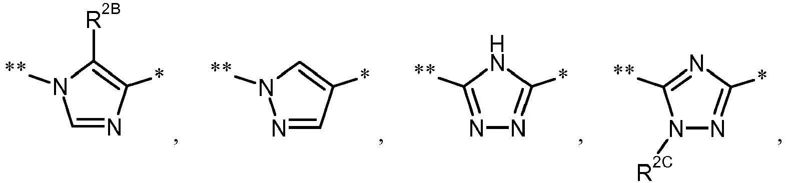 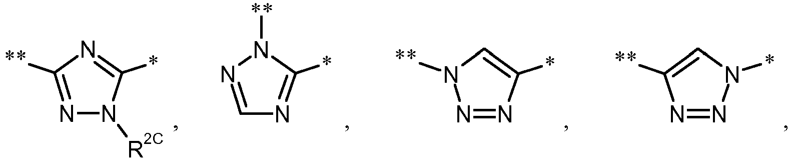 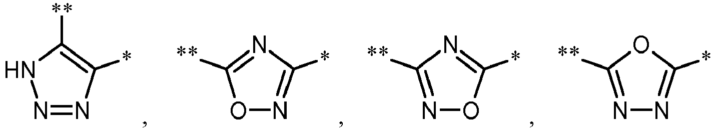 oder 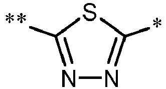 steht, worin
- *: die Verknüpfungsstelle mit der Gruppe L¹
und
- **: die Verknüpfungsstelle mit der Gruppe L² kennzeichnen,
- R^{2A}: Wasserstoff, Fluor, Chlor, Brom, Methyl, Trifluormethyl, Hydroxymethyl, Carbamoyloxymethyl, Hydroxycarbonyl, Methoxycarbonyl, Ethoxycarbonyl, Aminocarbonyl, Methylaminocarbonyl oder *tert*.-Butylaminocarbonyl bedeutet,
- R^{2B}: Wasserstoff, Methyl oder Trifluormethyl bedeutet
und
- R^{2C}: Wasserstoff oder Methyl, welches mit Hydroxycarbonyl, Methoxycarbonyl oder Aminocarbonyl substituiert sein kann, bedeutet,
- L²: für eine Bindung oder die Gruppe -CH₂- steht
und
- Ar²: für Phenyl steht, welches ein- oder zweifach, gleich oder verschieden, mit Resten ausgewählt aus der Reihe Fluor, Chlor, Methyl, Trifluormethyl, Methoxy und Trifluormethoxy substituiert ist,
sowie ihre Salze, Solvate und Solvate der Salze.

Ganz besonders bevorzugt im Rahmen der vorliegenden Erfindung sind Verbindungen der Formel (I), in welcher
- R¹: für (C₁-C₄)-Alkyl, welches ein- oder zweifach, gleich oder verschieden, mit Resten ausgewählt aus der Reihe Fluor, Trifluormethyl und Hydroxy substituiert sein kann, oder für Cyclopropyl steht,
- Ar¹: für *p*-Chlorphenyl steht,
- L¹: für die Gruppe -CH₂- steht,
- Q: für einen Pyrimidinyl-Ring der Formel 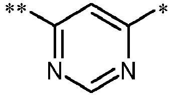 oder 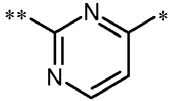 oder
für einen gegebenenfalls substituierten 5-gliedrigen Heteroaryl-Ring der Formel 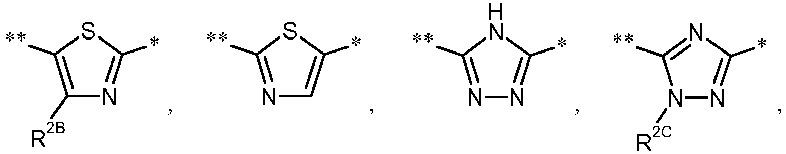 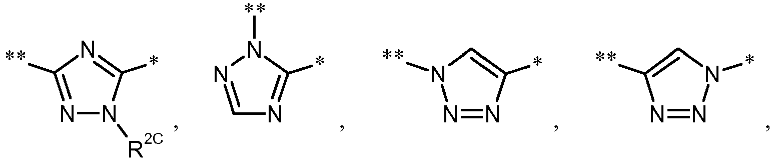 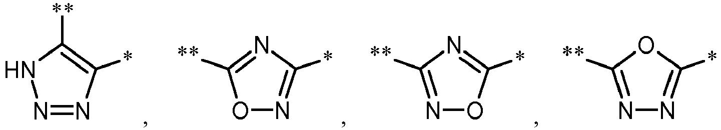 oder 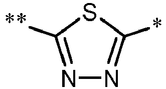 steht, worin
* die Verknüpfungsstelle mit der Gruppe L¹ und
** die Verknüpfungsstelle mit der Gruppe L² kennzeichnen,
R^{2B} Wasserstoff, Methyl oder Trifluormethyl bedeutet
und
R^{2C} Wasserstoff oder Methyl, welches mit Hydroxycarbonyl, Methoxycarbonyl oder Aminocarbonyl substituiert sein kann, bedeutet,
- L²: für eine Bindung oder die Gruppe -CH₂- steht und
- Ar²: für Phenyl steht, welches ein- oder zweifach, gleich oder verschieden, mit Resten ausgewählt aus der Reihe Fluor, Chlor, Methyl, Trifluormethyl, Methoxy und Trifluormethoxy substituiert ist,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man ein 5-Aryl-1,2,4-triazolon-Derivat der Formel (II) 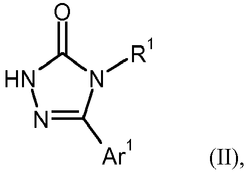 in welcher Ar¹ und R¹ die oben angegebenen Bedeutungen haben,
in Gegenwart einer Base entweder
[A] mit einer Verbindung der Formel (III) 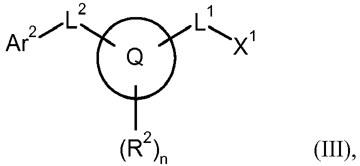 in welcher Ar², L¹, L², Q, R² und n die oben angegebenen Bedeutungen haben und
   - X¹: für eine Abgangsgruppe wie Chlor, Brom, Iod, Mesylat oder Tosylat steht, zu einer Verbindung der Formel (I) umsetzt
   oder
[B] in einer Alternative für den Fall, dass L² in Formel (I) für eine Bindung steht und die Gruppe Ar² an ein Kohlenstoffatom des Ringes Q gebunden ist,
   mit einer Verbindung der Formel (IV) 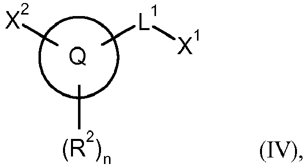 in welcher L¹, Q, R² und n die oben angegebenen Bedeutungen haben,
   - X¹: für eine Abgangsgruppe wie Chlor, Brom, Iod, Mesylat oder Tosylat steht
   und
   - X²: für eine an ein Kohlenstoffatom des Ringes Q gebundene Abgangsgruppe wie Chlor, Brom, Iod, Mesylat oder Triflat steht,
   zu einem Intermediat der Formel (V) 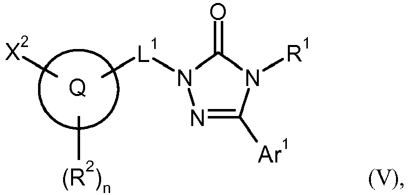 in welcher Ar¹, L¹, Q, R¹, R², X² und n die oben angegebenen Bedeutungen haben,
   umsetzt und dieses dann in Gegenwart eines geeigneten Übergangsmetall-Katalysators mit einer Verbindung der Formel (VI)

   Ar²-M (VI),

   in welcher Ar² die oben angegebene Bedeutung hat und
   - M: für eine Gruppe der Formel -B(OR⁴)₂, -MgHal, -ZnHal oder -Sn(R⁵)₃ steht, worin
   Hal Halogen, insbesondere Chlor, Brom oder Iod bedeutet,
   R⁴ Wasserstoff oder (C₁-C₄)-Alkyl bedeutet oder beide Reste R⁴ miteinander verknüpft sind und zusammen eine -(CH₂)₂-, -C(CH₃)₂-C(CH₃)₂-, -(CH₂)₃- oder -CH₂-C(CH₃)₂-CH₂-Brücke bilden
   und
   R⁵ (C₁-C₄)-Alkyl bedeutet,
   zu einer Verbindung der Formel (I-A) 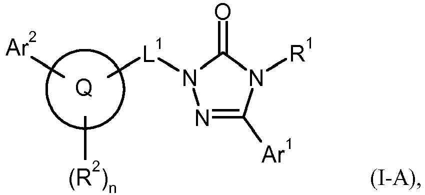 in welcher Ar¹, Ar², L¹, Q, R¹, R² und n die oben angegebenen Bedeutungen haben,
   kuppelt
   oder
[C] in einer Alternative für den Fall, dass L² in Formel (I) für die Gruppe -(CR^{3A}R^{3B})ₚ-, wie oben definiert, steht und an ein Stickstoffatom des Ringes Q gebunden ist,
   mit einer Verbindung der Formel (VII) 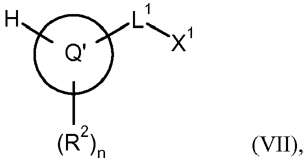 in welcher L¹, R² und n die oben angegebenen Bedeutungen haben,
   - Q': für einen 5-gliedrigen Heteroaryl-Ring, wie oben unter Q definiert, steht, welcher ein mit dem angezeigten Wasserstoffatom verbundenes, trivalentes Ring-Stickstoffatom enthält,
   und
   - X¹: für eine Abgangsgruppe wie Chlor, Brom, Iod, Mesylat oder Tosylat steht,
   zu einem Intermediat der Formel (VIII) 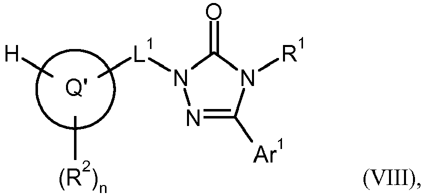 in welcher Ar¹, L¹, Q', R¹, R² und n die oben angegebenen Bedeutungen haben,
   umsetzt und dieses dann in Gegenwart einer Base mit einer Verbindung der Formel (IX) 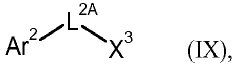 in welcher Ar² die oben angegebene Bedeutung hat,
   - L^{2A}: für die Gruppe -(CR^{3A}R^{3B})ₚ-, wie oben definiert, steht
   und
   - X³: für eine Abgangsgruppe wie Chlor, Brom, Iod, Mesylat oder Tosylat steht,
   zu einer Verbindung der Formel (I-B) 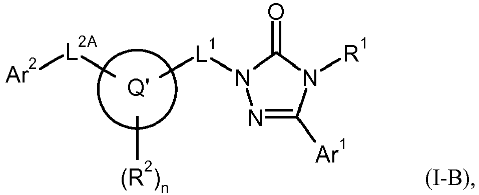 in welcher Ar¹, Ar², L¹, L^{2A}, Q', R¹, R² und n die oben angegebenen Bedeutungen haben, N-alkyliert
   und die so erhaltenen Verbindungen der Formel (I), (I-A) bzw. (I-B) gegebenenfalls in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Inerte Lösungsmittel für die Verfahrensschritte (II) + (III) → (I), (II) + (IV) → (V), (II) + (VII) → (VIII) und (VIII) + (IX) → (I-B) sind beispielsweise Halogenkohlenwasserstoffe wie Dichlormethan, Trichlormethan, Tetrachlormethan, Trichlorethylen oder Chlorbenzol, Ether wie Diethylether, Diisopropylether, Methyl-*tert*.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Cyclohexan oder Erdölfraktionen, oder dipolar-aprotische Lösungsmittel wie Aceton, Methylethylketon, Ethylacetat, Acetonitril, *N,N-*Dimethylformamid (DMF), *N,N-*Dimethylacetamid (DMA), Dimethylsulfoxid (DMSO), *N,N*'-Dimethylpropylenharnstoff (DMPU), *N-*Methylpyrrolidinon (NMP) oder Pyridin. Ebenso ist es möglich, Gemische der genannten Lösungsmittel einzusetzen. Bevorzugt wird Tetrahydrofuran, Acetonitril, Aceton oder Dimethylformamid verwendet.

Als Basen für die Verfahrensschritte (II) + (III) → (I), (II) + (IV) → (V), (II) + (VII) → (VIII) und (VIII) + (IX) → (I-B) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N-*Methylmorpholin, *N-*Methylpiperidin, *N,N-*Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kalium- oder Cäsiumcarbonat oder Natriumhydrid verwendet.

Die Base wird hierbei in einer Menge von 1 bis 5 Mol, bevorzugt in einer Menge von 1 bis 2.5 Mol, bezogen auf 1 Mol der Verbindung der Formel (II) bzw. (VIII), eingesetzt. Gegebenenfalls können diese Verfahrensschritte vorteilhaft unter Zusatz von Alkylierungskatalysatoren, wie beispielsweise Lithiumbromid, Natriumiodid, Tetra-*n*-butylammoniumbromid oder Benzyltriethylammoniumchlorid, durchgeführt werden. Die Reaktionen erfolgen im Allgemeinen in einem Temperaturbereich von -20°C bis +150°C, bevorzugt bei 0°C bis +80°C. Die Umsetzungen können bei normalem, bei erhöhtem oder bei erniedrigtem Druck durchgeführt werden (z.B. von 0.5 bis 5 bar); in der Regel arbeitet man bei Normaldruck.

Als inerte Lösungsmittel für den Verfahrensschritt (V) + (VI) → (I-A) eignen sich beispielsweise aromatische Kohlenwasserstoffe wie Benzol, Toluol oder Xylol, Ether wie Diethylether, Diisopropylether, Methyl-*tert*.-butylether, 1,2-Dimethoxyethan, Bis-(2-methoxyethyl)-ether, Tetrahydrofuran oder 1,4-Dioxan, oder dipolar-aprotische Lösungsmittel wie Acetonitril, *N,N-*Dimethylformamid (DMF), *N,N*-Dimethylacetamid (DMA), Dimethylsulfoxid (DMSO), *N,N'-*Dimethylpropylenharnstoff (DMPU), *N-*Methylpyrrolidinon (NMP) oder Pyridin. Ebenso ist es möglich, Gemische solcher Lösungsmittel zu verwenden. Bevorzugt wird Toluol, Tetrahydrofuran, 1,4-Dioxan oder Dimethylformamid eingesetzt.

Die Kupplungsreaktion (V) + (VI) → (I-A) wird im Allgemeinen mit Hilfe eines Übergangsmetall-Katalysators ausgeführt. Hierfür eignen sich Kupfer-Katalysatoren, wie beispielsweise Kupfer(I)-iodid, und insbesondere Palladium-Katalysatoren, wie beispielsweise Palladium auf Aktivkohle, Palladium(II)acetat, Bis(triphenylphosphino)palladium(II)chlorid, Bis(acetonitril)palladium(II)-chlorid, [1,1'-Bis(diphenylphosphino)ferrocen]palladium(II)chlorid, Bis(dibenzylidenaceton)palladium(0), Tris(dibenzylidenaceton)dipalladium(0) oder Tetrakis(triphenylphosphino)palladium(0), gegebenenfalls in Kombination mit zusätzlichen Phosphanliganden wie Tri-*tert*.-butylphosphin, 2-Dicyclohexylphosphino-2'-(*N,N-*dimethylamino)biphenyl, Dicyclohexyl[2',4',6'-tris(1-methylethyl)-biphenyl-2-yl]phosphan (XPHOS) oder 4,5-Bis(diphenylphosphino)-9,9-dimethylxanthen (Xantphos) [vgl. z.B. J. Hassan et al., Chem. Rev. 102, 1359-1469 (2002); V. Farina, V. Krishnamurthy und W.J. Scott, in: The Stille Reaction, Wiley, New York, 1998].

Die Kupplung mit einem Arylboronat [M = B(OR⁴)₂; "Suzuki-Kupplung"] wird in der Regel unter Zusatz einer anorganischen Base durchgeführt. Hierfür eignen sich insbesondere Alkalicarbonate, -hydrogencarbonate, -phosphate, -hydrogenphosphate, -acetate oder -fluoride wie Natriumcarbonat, Kaliumcarbonat, Natriumhydrogencarbonat, Kaliumhydrogencarbonat, Trikaliumphosphat, Dinatriumhydrogenphosphat, Dikaliumhydrogenphosphat, Natriumacetat, Kaliumacetat, Kaliumfluorid und Cäsiumfluorid. Solche Basen können auch in Form ihrer wässrigen Lösungen eingesetzt werden. Bevorzugt wird Natrium- oder Kaliumcarbonat oder Trikaliumphosphat verwendet.

Der Verfahrensschritt (V) + (VI) → (I-A) erfolgt im Allgemeinen in einem Temperaturbereich von +20°C bis +200°C, bevorzugt bei +60°C bis +150°C, bei Normaldruck. Es ist jedoch auch möglich, die Reaktion bei vermindertem oder bei erhöhtem Druck durchzuführen (z.B. von 0.5 bis 5 bar). Gegebenenfalls kann es von Vorteil sein, die Umsetzung unter Mikrowellen-Bestrahlung vorzunehmen.

In einer besonderen Variante des zuvor beschriebenen Verfahrens [A] können erfindungsgemäße Verbindungen der Formel (I) gegebenenfalls auch dadurch hergestellt werden, dass anstelle der Verbindung (II) zunächst ein temporär geschütztes 1,2,4-Triazolon-Derivat der Formel (X) 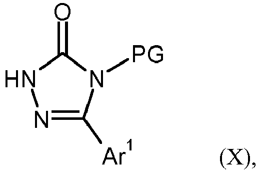 in welcher Ar¹ die oben angegebene Bedeutung hat
und
- PG: für eine geeignete Schutzgruppe, wie beispielsweise Allyl oder *p*-Methoxybenzyl, steht,
mit einer Verbindung der Formel (III) umgesetzt wird; das hierbei resultierende Produkt der Formel (XI) 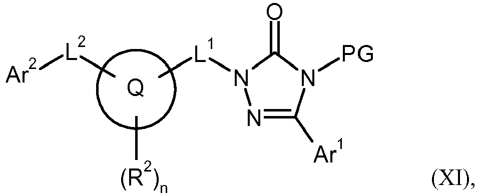 in welcher Ar¹, Ar², L¹, L², PG, Q, R² und n die oben angegebenen Bedeutungen haben,
kann nach Abspaltung der Schutzgruppe zur Verbindung der Formel (XII) 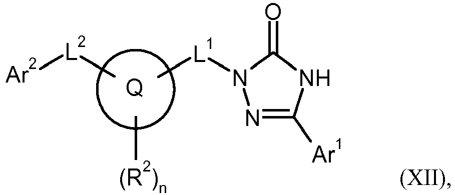 in welcher Ar¹, Ar² , L¹, L², Q, R² und n die oben angegebenen Bedeutungen haben,
dann durch baseninduzierte Reaktion mit einer Verbindung der Formel (XIII)

R¹-X⁴ (XIII),

in welcher R¹ die oben angegebene Bedeutung hat und
- X⁴: für eine Abgangsgruppe wie Chlor, Brom, Iod, Mesylat oder Tosylat steht,
in entsprechende Verbindungen der Formel (I) umgewandelt werden.

Eine analoge Transformation PG → R¹ kann gegebenenfalls auch im Zuge der oben beschriebenen Verfahrensvarianten [B] und [C], jeweils ausgehend von dem geschützten Aryltriazolon (X), erfolgen.

Einige der solcherart PG-geschützten Verbindungen der Formel (XI) weisen ebenfalls eine signifikante Vasopressin-antagonistische Wirkung auf und sind insofern auch vom Bedeutungsumfang der vorliegenden Erfindung, d.h. den Verbindungen der Formel (I) umfasst.

Die Einführung und Abspaltung der Schutzgruppe PG erfolgt nach literaturüblichen Methoden [siehe z.B. T.W. Greene und P.G.M. Wuts, Protective Groups in Organic Synthesis, Wiley, New York, 1999]. So wird die Allyl-Gruppe vorzugsweise mit Hilfe von Ameisensäure in Gegenwart des Tetrakis(triphenylphosphin)palladium(0)-Katalysators und einer Amin-Base wie Triethylamin entfernt. Die Abspaltung der *p*-Methoxybenzyl-Schutzgruppe erfolgt bevorzugt mit Hilfe starker Säuren, wie beispielsweise Trifluoressigsäure, oder auf oxidativem Wege, z.B. durch Behandlung mit 2,3-Dichlor-5,6-dicyano-1,4-benzochinon (DDQ) oder Ammoniumcer(IV)nitrat.

Die nachfolgende Umsetzung (XII) + (XIII) → (I) wird analog zum zuvor beschriebenen Verfahrensschritt (II) + (III) → (I) durchgeführt. Als inerte Lösungsmittel kommen hier vorzugsweise Aceton, Acetonitril, Dimethylformamid, Dimethylsulfoxid, Toluol, Tetrahydrofuran, Glykoldimethylether und deren Gemische in Betracht. Als Base wird bevorzugt Natriumhydrid oder Kalium- oder Cäsiumcarbonat eingesetzt. Die Umsetzung erfolgt im Allgemeinen bei Normaldruck in einem Temperaturbereich von 0°C bis +150°C, bevorzugt bei +20°C bis +80°C.

Die 1,2,4-Triazolon-Derivate der Formel (II) können ausgehend von Carbonsäurehydraziden der Formel (XIV) durch Umsetzung mit Isocyanaten der Formel (XV) oder Nitrophenylcarbamaten der Formel (XVI) und nachfolgende baseninduzierte Cyclisierung der intermediären Hydrazincarboxamide (XVII) hergestellt werden (siehe Schema 1):

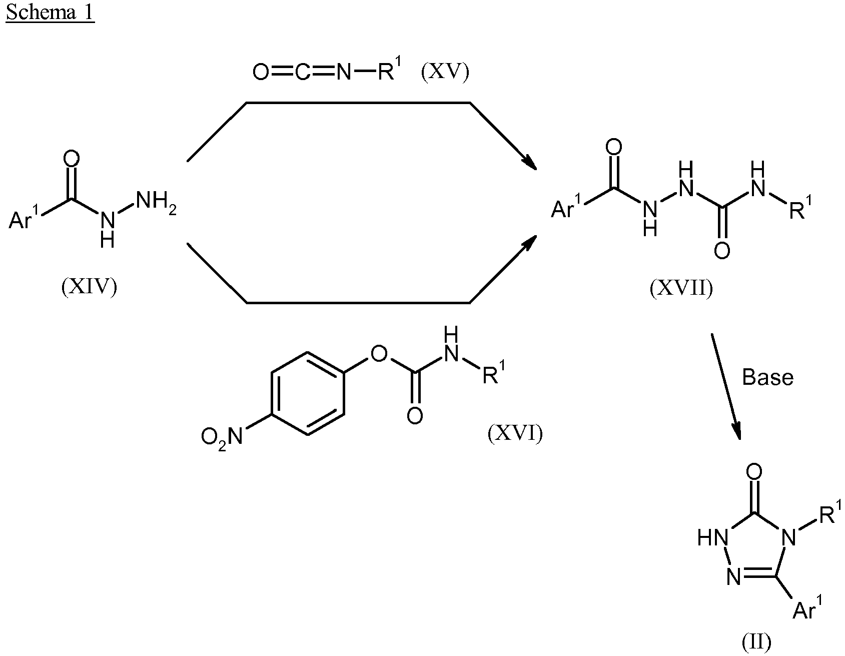

Auf analoge Weise lassen sich auch temporär geschützte 1,2,4-Triazolon-Derivate der Formel (X) erhalten, insbesondere solche, in denen PG für Allyl oder *p*-Methoxybenzyl steht.

Verbindungen der Formel (III), in welcher L¹ für -CH₂- und L² für eine Bindung steht, können beispielsweise in Analogie zum oben beschriebenen Verfahren [B] durch Übergangsmetall-katalysierte Kupplung der Verbindung der Formel (VI) mit einer Verbindung der Formel (XVIII) und nachfolgende radikalische Halogenierung des Intermediats (XIX) hergestellt werden (Schema 2):

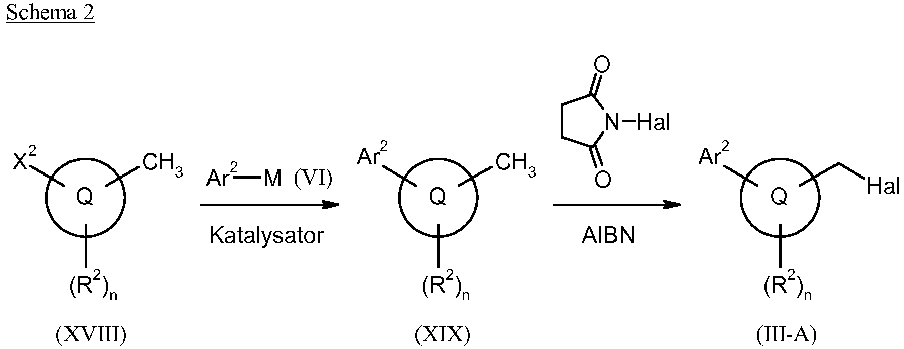

In einer Variante dieses Verfahrens erfolgt die Kupplung mit einem Ester-Derivat der Formel (XX); nachfolgende Reduktion zum primären Alkohol der Formel (XXII) und literaturübliche Umwandlung der Hydroxyfunktion in eine Abgangsgruppe ergibt die korrespondierende Verbindung der Formel (III-B) (Schema 3):

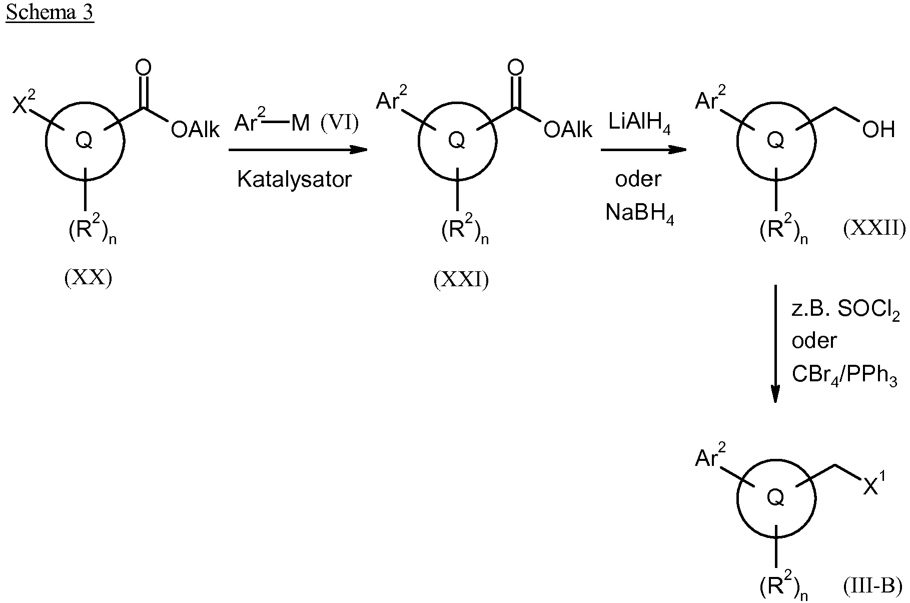

Auf ähnliche Weise können Verbindungen der Formel (III), in welcher L¹ für -CH₂- und L² für die an ein Stickstoffatom des Ringes Q gebundene Gruppe -(CR^{3A}R^{3B})ₚ- steht, aus entsprechenden Carbonsäureestern der Formel (XXIV) hergestellt werden, welche ihrerseits analog zum oben beschriebenen Verfahren [C] zugänglich sind (siehe Schema 4):

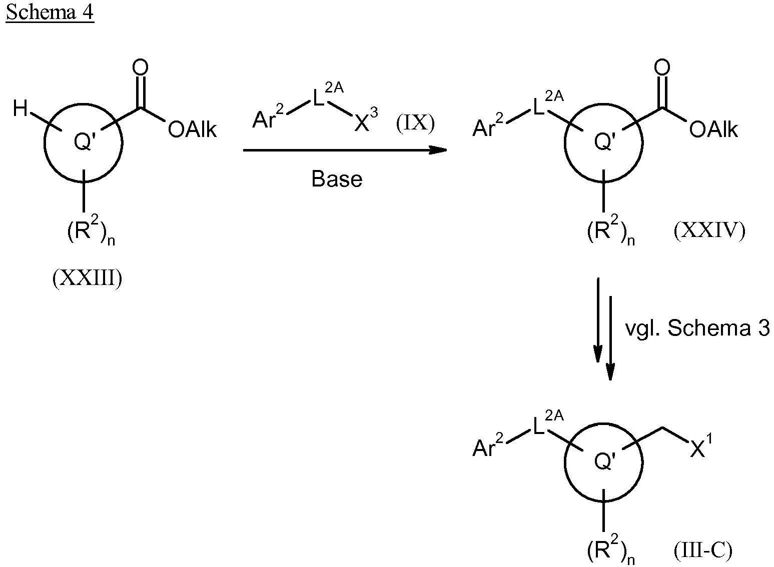

Erfindungsgemäße Verbindungen der Formel (I), in denen der Ring Q für einen 5-gliedrigen Heteroaryl-Ring steht, können gegebenenfalls auch in Anlehnung an literaturbekannte Methoden über eine *de novo*-Synthese des betreffenden Heteroaryl-Systems hergestellt werden. Solche Verfahrenswege können durch die folgenden Reaktionsschemata 5-10 beispielhaft veranschaulicht werden:

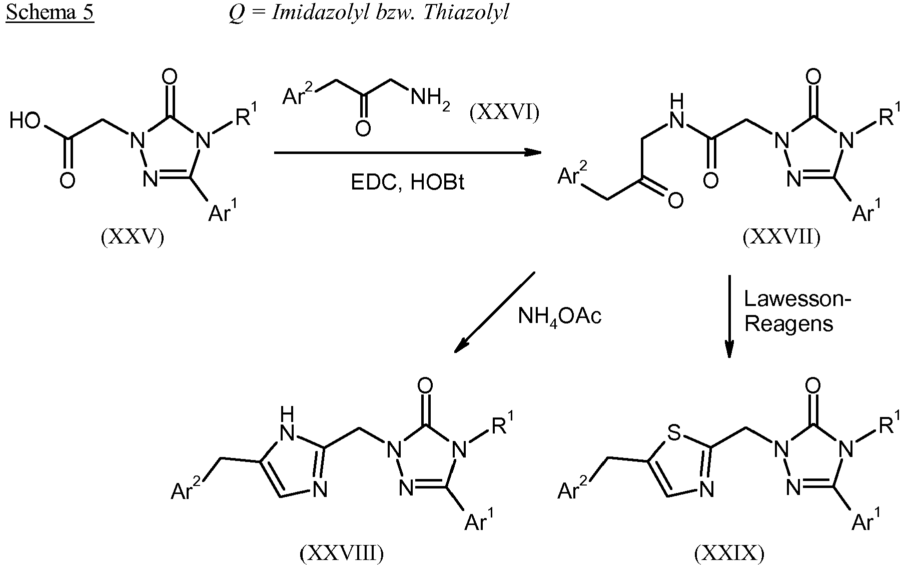

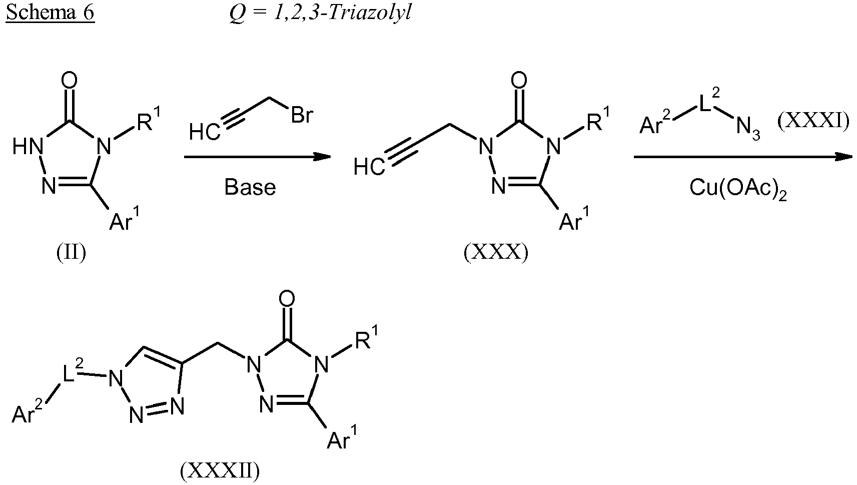

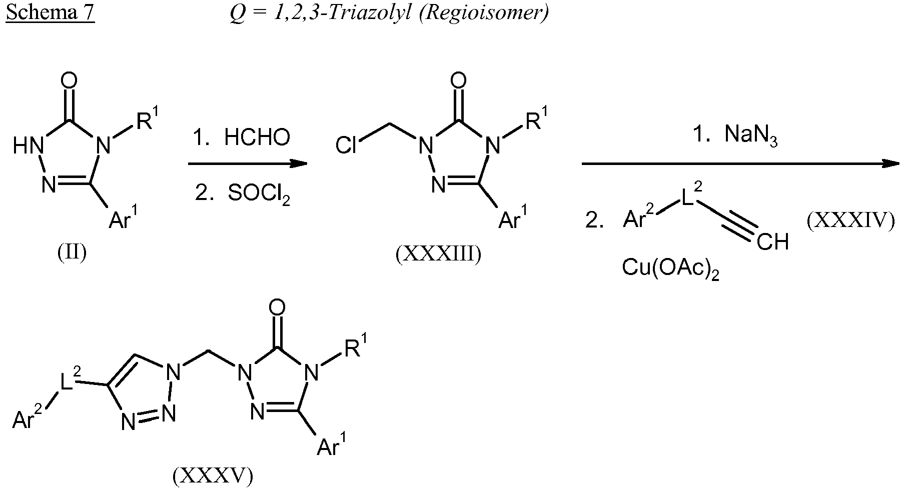

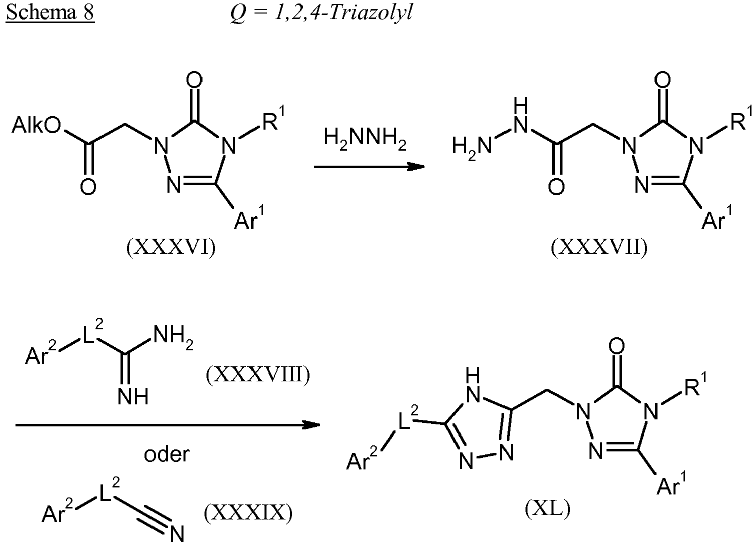

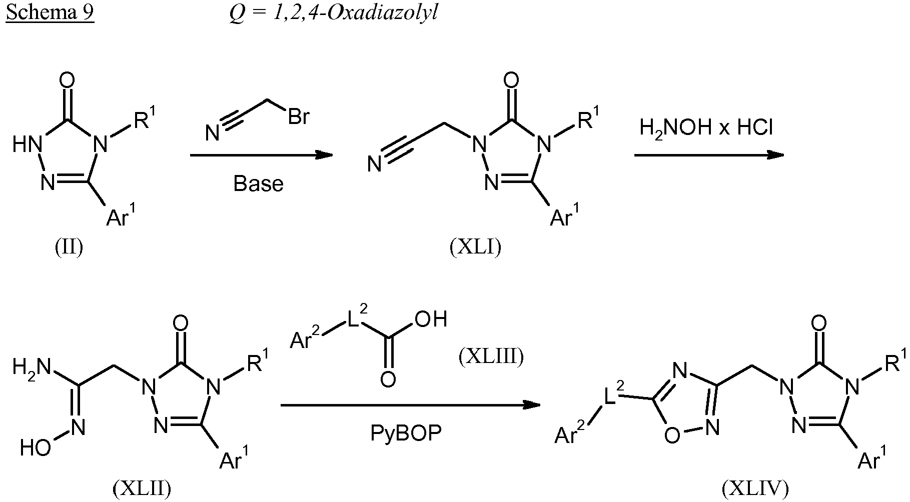

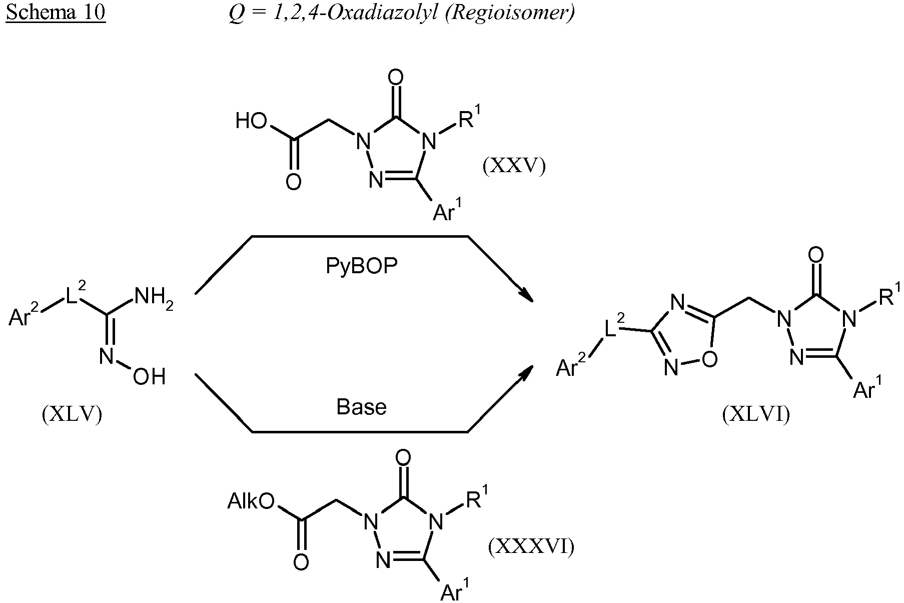

Weitere erfindungsgemäße Verbindungen der Formel (I) können, falls zweckmäßig, auch durch Umwandlungen von funktionellen Gruppen einzelner Reste und Substituenten, insbesondere den unter R¹, R², Ar¹ und Ar² aufgeführten, hergestellt werden, wobei von anderen, nach obigen Verfahren erhaltenen Verbindungen der Formel (I) ausgegangen wird. Diese Umwandlungen werden nach üblichen, dem Fachmann geläufigen Methoden durchgeführt und umfassen beispielsweise Reaktionen wie nukleophile oder elektrophile Substitutionsreaktionen, Übergangsmetall-vermittelte Kupplungsreaktionen (z.B. Suzuki- oder Heck-Reaktion), Oxidation, Reduktion, Hydrierung, Alkylierung, Acylierung, Aminierung, Hydroxylierung, Veretherung, Veresterung, Esterspaltung und -hydrolyse, Bildung von Nitrilen, Carbonamiden, Sulfonamiden, Carbamaten und Harnstoffen, sowie die Einführung und Entfernung temporärer Schutzgruppen [vgl. auch die im nachfolgenden Experimentellen Teil detailliert beschriebene Herstellung der Ausführungsbeispiele].

Die Intermediate der Formel (XXXVI) können auf einfache Weise durch baseninduzierte Alkylierung der 5-Aryl-1,2,4-triazol-3-one der Formel (II) mit Halogenoessigestern der Formel (XLVII) hergestellt werden; über nachfolgende Ester-Hydrolyse sind die entsprechenden Carbonsäuren der Formel (XXV) zugänglich (siehe Schema 11):

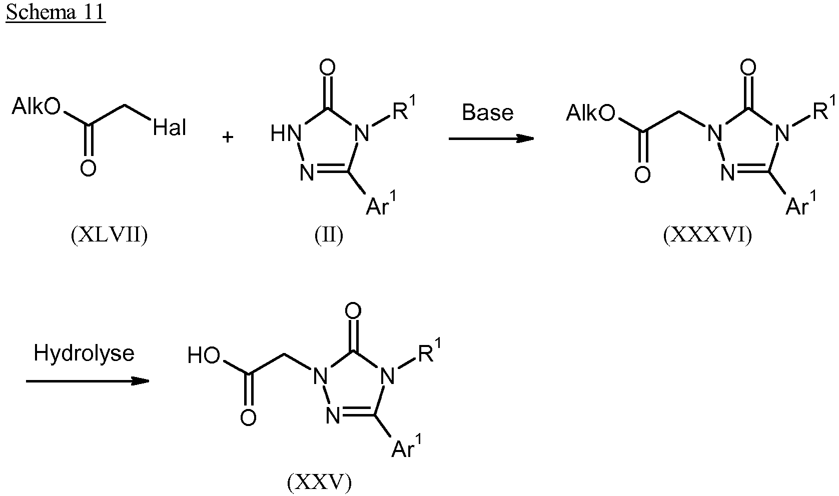

Die Verbindungen der Formel (XXXVI) können alternativ auch aus literaturbekannten *N-*(Alkoxycarbonyl)arylthioamiden der Formel (XLIX) [siehe z.B. M. Arnswald, W.P. Neumann, J. Org. Chem. 58 (25), 7022-7028 (1993); E.P. Papadopoulos, J. Org. Chem. 41 (6), 962-965 (1976)] durch Reaktion mit Hydrazinoessigestern der Formel (XLVIII) und nachfolgende Alkylierung an N-4 des Triazolons (L) hergestellt werden (Schema 12):

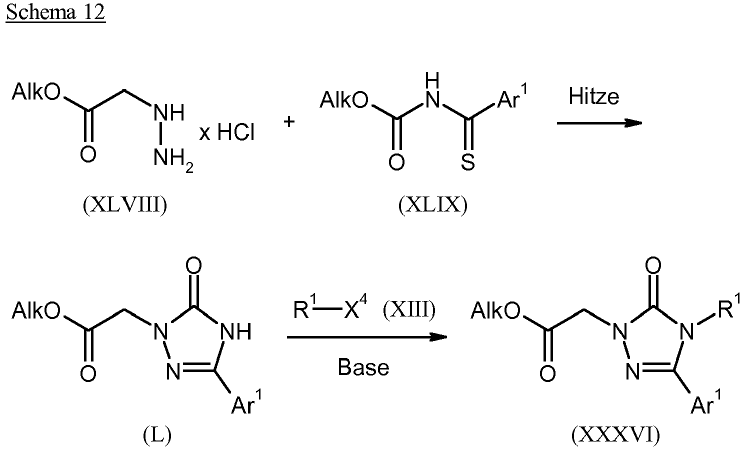

Eine Trennung der erfindungsgemäßen Verbindungen in die entsprechenden Enantiomere und/ oder Diastereomere kann, je nach Zweckmäßigkeit, auch bereits auf der Stufe einzelner Intermediate, wie oben aufgeführt, erfolgen, wobei diese Intermediate dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an achiraler bzw. chiraler Phase, verwendet.

Die Verbindungen der Formeln (IV), (VI), (VII), (IX), (XIII), (XIV), (XV), (XVI), (XVIII), (XX), (XXIII), (XXVI), (XXXI), (XXXIV), (XXXVIII), (XXXIX), (XLIII), (XLV), (XLVII), (XLVIII) und (XLIX) sind entweder kommerziell erhältlich oder als solche in der Literatur beschrieben, oder sie können auf für den Fachmann offenkundigem Wege in Analogie zu in der Literatur publizierten Methoden hergestellt werden. Zahlreiche detaillierte Vorschriften sowie Literaturangaben zur Herstellung der Ausgangsmaterialien befinden sich auch im Experimentellen Teil im Abschnitt zur Herstellung der Ausgangsverbindungen und Intermediate.

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen bei Menschen und Tieren verwendet werden.

Die erfindungsgemäßen Verbindungen stellen potente selektive V1a-, V2- oder duale V1a/V2-Rezeptor-Antagonisten dar, die die Vasopressin-Aktivität *in vitro* und *in vivo* inhibieren. Darüber hinaus wirken die erfindungsgemäßen Verbindungen auch als Antagonisten am verwandten Oxytozin-Rezeptor.

Die erfindungsgemäßen Verbindungen sind insbesondere für die Prävention und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienzbedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prävention und/oder Behandlung der polyzystischen Nierenkrankheit (PCKD) und des Syndroms der inadäquaten ADH-Sekretion (SIADH).

Außerdem können die erfindungsgemäßen Verbindungen für die Prävention und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prävention und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prävention und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, von Menstruationsstörungen wie zum Beispiel Dysmenorrhöen oder von Endometriose eingesetzt werden.

Aufgrund ihres Wirkprofils eignen sich die erfindungsgemäßen Verbindungen insbesondere zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prävention der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugs-weise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP), wie beispielhaft und vorzugsweise Omapatrilat oder AVE-7688, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib, Dalcetrapib, Anacetrapib, BAY 60-5521 oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- Ac: Acetyl
- AIBN: 2,2'-Azobis-2-methylpropannitril
- Alk: Alkyl
- Boc: *tert*.-Butoxycarbonyl
- Bsp.: Beispiel
- CI: chemische Ionisation (bei MS)
- DCI: direkte chemische Ionisation (bei MS)
- DME: 1,2-Dimethoxyethan
- DMF: *N,N-*Dimethylformamid
- DMSO: Dimethylsulfoxid
- d. Th.: der Theorie (bei Ausbeute)
- EDC: *N'*-(3-Dimethylaminopropyl)-*N-*ethylcarbodiimid-Hydrochlorid
- ee: Enantiomerenüberschuss
- EE: Essigsäureethylester
- eq.: Äquivalent(e)
- ESI: Elektrospray-Ionisation (bei MS)
- GC/MS: Gaschromatographie-gekoppelte Massenspektrometrie
- ges.: gesättigt
- h: Stunde(n)
- Hal: Halogen
- HOBt: 1-Hydroxy-1*H*-benzotriazol-Hydrat
- HPLC: Hochdruck-, Hochleistungsflüssigchromatographie
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- MTBE: Methyl-*tert*.-butylether
- NMR: Kernresonanzspektrometrie
- OAc: Acetat
- *p*: para
- Ph: Phenyl
- PyBOP: Benzotriazol-1-yloxy-tris(pyrrolidino)phosphonium-hexafluorophosphat
- quant.: quantitativ (bei Ausbeute)
- rac: racemisch / Racemat
- RT: Raumtemperatur
- Rₜ: Retentionszeit (bei HPLC)
- THF: Tetrahydrofuran
- TMOF: Trimethylorthoformiat
- UV: Ultraviolett-Spektrometrie
- v/v: Volumen zu Volumen-Verhältnis (einer Lösung)

### LC/MS-, GC/MS- und HPLC-Methoden:

### Methode 1 (LC/MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 2 (LC/MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 3 (LC/MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 4 (LC/MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 5 (LC/MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 6 (LC/MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 7 (LC/MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8 (LC/MS):

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Fluss: 0.8 ml/min; Ofen: 50°C; UV-Detektion: 210 nm.

### Methode 9 (LC/MS):

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 10 (LC/MS):

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 11 (chirale analytische HPLC):

Stationäre Phase: Daicel Chiralcel OD-H; Säule: 250 mm x 4 mm; Fluss: 1 ml/min; Temperatur: RT; UV-Detektion: 230 nm; Elutionsmittel:
Methode 11a: Isohexan/Isopropanol 50:50 (v/v);
Methode 11b: Isohexan/Methanol/Ethanol 70:15:15 (v/v/v);
Methode 11c: Isohexan/Isopropanol 75:25 (v/v).

### Methode 12 (chirale präparative HPLC):

Stationäre Phase: Daicel Chiralpak AD-H, 5 µm; Säule: 250 mm x 20 mm; Temperatur: 40°C; UV-Detektion: 220 nm; Elutionsmittel: Isohexan/Isopropanol 80:20 (v/v); Fluss: 15 ml/min.

### Methode 13 (chirale analytische HPLC):

Stationäre Phase: Daicel Chiralpak AD-H, 5 µm; Säule: 250 mm x 4.6 mm; Temperatur: 40°C; UV-Detektion: 220 nm; Elutionsmittel: Isohexan/Isopropanol 80:20 (v/v); Fluss: 1.0 ml/min.

### Methode 14 (chirale analytische HPLC):

Stationäre Phase: Daicel Chiralpak AD-H, 5 µm; Säule: 250 mm x 4.6 mm; Temperatur: 30°C; UV-Detektion: 220 nm; Elutionsmittel: Isohexan/Isopropanol/20%-ige Trifluoressigsäure 75:24:1 (v/v/v); Fluss: 1.0 ml/min.

### Methode 15 (präparative HPLC):

Instrument: Abimed Gilson Pump 305/306, Manometric Module 806; Säule: Grom-Sil 120 ODS-4HE, 10 µm, 250 mm x 30 mm; Eluent A: Wasser, Eluent B: Acetonitril; Gradient: 0.0 min 30% B → 3 min 30% B → 30 min 95% B → 42 min 95% B → 42.01 min 10% B → 45 min 10% B; Fluss: 50 ml/min; Säulentemperatur: RT; UV-Detektion: 210 nm.

### Methode 16 (präparative HPLC):

Säule: Kromasil 100 C18, 5 µm, 250 mm x 20 mm; Eluent A: 0.2%-ige Trifluoressigsäure, Eluent B: Acetonitril; isokratisch 55% A, 45% B; Fluss: 25 ml/min; Säulentemperatur: 30°C; UV-Detektion: 210 nm.

### Methode 17 (chirale präparative HPLC):

Stationäre Phase: Daicel Chiralpak AD-H, 5 µm; Säule: 250 mm x 20 mm; Temperatur: 40°C; UV-Detektion: 220 nm; Elutionsmittel: Isohexan/Ethanol 80:20 (v/v); Fluss: 15 ml/min.

### Methode 18 (chirale analytische HPLC):

Stationäre Phase: Daicel Chiralpak AS-H, 5 µm; Säule: 250 mm x 4.6 mm; Temperatur: 40°C; UV-Detektion: 220 nm; Elutionsmittel: Isohexan/Ethanol 80:20 (v/v); Fluss: 1 ml/min.

### Methode 19 (präparative HPLC):

Säule: Grom-Sil 120 ODS-4HE, 10 µm, 250 mm x 30 mm; Eluent A: Wasser, Eluent B: Acetonitril; Gradient: 0.0 min 10% B → 3 min 10% B → 30 min 95% B → 42 min 95% B → 42.01 min 10% B → 45 min 10% B; Fluss: 50 ml/min; Säulentemperatur: RT; UV-Detektion: 210 nm.

### Methode 20 (GC/MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Ausgangsverbindungen und Intermediate:

### Beispiel 1A

### Ethyl-N-({2-[(4-chlorphenyl)carbonyl]hydrazinyl}carbonyl)glycinat

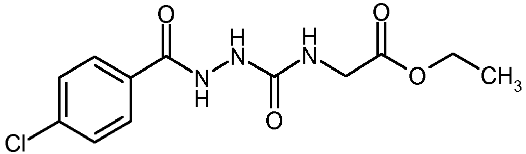

Eine Suspension von 12.95 g (75.9 mmol) 4-Chlorbenzohydrazid in 50 ml trockenem THF wurde bei 50°C vorgelegt und tropfenweise mit einer Lösung von 10.0 g (77.5 mmol) Ethyl-2-isocyanatoacetat in 100 ml trockenem THF versetzt. Zunächst bildete sich eine Lösung, dann fiel ein Niederschlag aus. Nach Ende der Zugabe wurde die Mischung noch 2 h bei 50°C weiter gerührt und dann über Nacht bei RT stehen gelassen. Die Kristalle wurden durch Filtration isoliert, mit wenig Diethylether gewaschen und im Hochvakuum getrocknet. Es wurden 21.43 g (89% d. Th) der Titelverbindung erhalten.
LC/MS [Methode 1]: Rₜ = 1.13 min; m/z = 300 (M+H)⁺
¹H-NMR (DMSO-d₆, 400 MHz): δ = 1.19 (t, 3H), 3.77 (d, 2H), 4.09 (q, 2H), 6.88 (br. s, 1H), 7.57 (d, 2H), 7.91 (d, 2H), 8.21 (s, 1H), 10.29 (s, 1H).

### Beispiel 2A

### [3-(4-Chlorphenyl)-5-oxo-1,5-dihydro-4H-1,2,4-triazol-4-yl]essigsäure

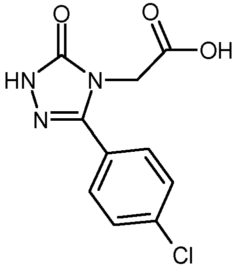

21.43 g (67.9 mmol) der Verbindung aus Beispiel 1A wurden mit 91 ml 3 N Natronlauge versetzt und über Nacht unter Rückfluss erhitzt. Nach Abkühlen auf RT wurde die Mischung durch langsame Zugabe von ca. 20%-iger Salzsäure auf pH 1 eingestellt. Der ausgefallene Feststoff wurde durch Filtration isoliert, mit Wasser gewaschen und bei 60°C im Vakuum getrocknet. Es wurden 17.55 g der Titelverbindung in ca. 88% Reinheit (90% d. Th.) erhalten.
LC/MS [Methode 1]: Rₜ = 0.94 min; m/z = 254 (M+H)⁺
¹H-NMR (DMSO-d₆, 400 MHz): δ = 4.45 (s, 2H), 7.65-7.56 (m, 4H), 12.09 (s, 1H), 13.25 (br. s, 1 H).

### Beispiel 3A

### 5-(4-Chlorphenyl)-4-(3,3,3-trifluor-2-oxopropyl)-2,4-dihydro-3H-1,2,4-triazol-3-on (Keton-Form) bzw. 5-(4-Chlorphenyl)-4-(3,3,3-trifluor-2,2-dihydroxypropyl)-2,4-dihydro-3H-1,2,4-triazol-3-on (Hydrat-Form)

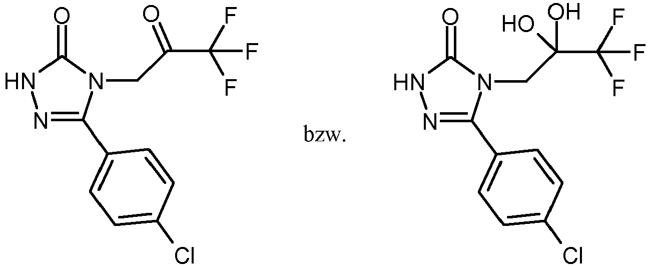

5.0 g (16.36 mmol) der Verbindung aus Beispiel 2A wurden unter Argon in 200 ml Pyridin gelöst und mit 17.18 g (81.8 mmol) Trifluoressigsäureanhydrid versetzt. Dabei stieg die Temperatur auf ca. 35°C an. Nach 30 min wurde das Pyridin am Rotationsverdampfer entfernt und der Rückstand mit 1.5 Liter 0.5 N Salzsäure versetzt. Diese Mischung wurde auf 70°C erwärmt und dann in der Wärme filtriert. Der Feststoff wurde mit etwas Wasser gewaschen. Das gesamte Filtrat wurde dreimal mit Ethylacetat extrahiert. Die vereinigten organischen Phasen wurden nacheinander mit Wasser, ges. Natriumhydrogencarbonat-Lösung und ges. Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.56 g (68% d. Th.) der Titelverbindung in der Hydrat-Form erhalten.
LC/MS [Methode 1]: Rₜ = 1.51 min; m/z = 306 (M+H)⁺ und 324 (M+H)⁺ (Keton- bzw. Hydrat-Form)
¹H-NMR (DMSO-d₆, 400 MHz): δ = 3.98 (s, 2H), 7.61 (d, 2H), 7.68 (br. s, 2H), 7.72 (d, 2H), 12.44 (s, 1H).

### Beispiel 4A

### 5-(4-Chlorphenyl)-4-(3,3,3-trifluor-2-hydroxypropyl)-2,4-dihydro-3H-1,2,4-triazol-3-on

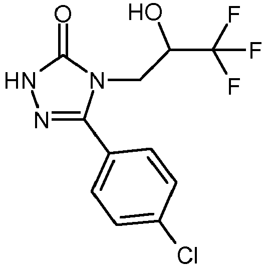

3.56 g (11.0 mmol) der Verbindung aus Beispiel 3A wurden in 100 ml Methanol gelöst und unter Eiskühlung mit 3.75 g (99.5 mmol) Natriumborhydrid versetzt. Nach 1.5 h wurden langsam 200 ml 1 M Salzsäure zugegeben. Das Methanol wurde am Rotationsverdampfer entfernt, der Rückstand mit 500 ml Wasser verdünnt und dreimal mit Ethylacetat extrahiert. Die vereinigten organischen Phasen wurden mit ges. Natriumhydrogencarbonat-Lösung und dann mit ges. Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.04 g (90% d. Th.) der Titelverbindung erhalten.
LC/MS [Methode 2]: Rₜ = 1.80 min; m/z = 308 (M+H)⁺
¹H-NMR (DMSO-d₆, 400 MHz): δ = 3.77 (dd, 1H), 3.92 (dd, 1H), 4.34-4.23 (m, 1H), 6.85 (d, 1H), 7.62 (d, 2H), 7.75 (d, 2H), 12.11 (s, 1H).

### Beispiel 5A

### 5-(4-Chlorphenyl)-4-[(2S)-3,3,3-trifluor-2-hydroxypropyl]-2,4-dihydro-3H-1,2,4-triazol-3-on

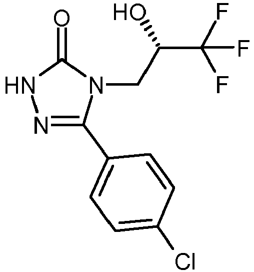

1.08 g (3.3 mmol) der Verbindung aus Beispiel 3A wurden in 11 ml *N,N-*Dimethylacetamid gelöst. Die Lösung wurde mittels Vakuum von Luftsauerstoff befreit und mit Argon gesättigt. In diese Lösung gab man unter Argon 21 mg (0.033 mmol) (*N*-[(1*S*,2*S*)-(+)-2-Amino-1,2-diphenylethyl]-(4-toluolsulfonyl)amido)(p-cymol)ruthenium(II)chlorid [CAS Reg.-Nr. 192139-90-5]. Anschließend versetzte man mit einem Gemisch aus 0.63 ml (16.6 mmol) Ameisensäure und 0.27 ml (1.91 mmol) Triethylamin und rührte 48 h unter Luftausschluss bei RT. Zur Aufarbeitung gab man das Gemisch in 10 ml 0.1 N Salzsäure und extrahierte zweimal mit je 20 ml Ethylacetat. Die vereinigten organischen Phasen wurden mit ges. Natriumhydrogencarbonat-Lösung gewaschen, über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 3:1, dann 1:1). Es wurden 830 mg (81% d. Th.) der Zielverbindung erhalten.

Der Enantiomerenüberschuss (ee-Wert) wurde chromatographisch nach Methode 14 zu 96% bestimmt: *S*-Enantiomer Rₜ = 5.73 min, *R*-Enantiomer Rₜ = 6.82 min.

### Beispiel 6A

### {3-(4-Chlorphenyl)-5-oxo-4-(3,3,3-trifluor-2-hydroxypropyl)-4,5-dihydro-1H-1,2,4-triazol-1-yl}-essigsäuremethylester (Racemat)

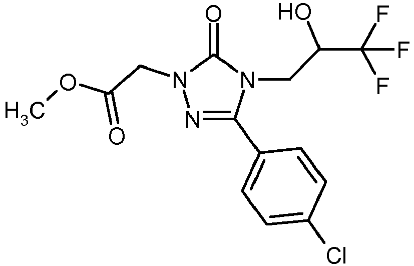

3.04 g (9.9 mmol) der Verbindung aus Beispiel 4A wurden in 100 ml Acetonitril gelöst und mit 1.07 g (9.9 mmol) Chloressigsäuremethylester, 2.73 g (19.8 mmol) Kaliumcarbonat sowie einer kleinen Spatelspitze Kaliumiodid versetzt. Die Reaktionsmischung wurde 1 h unter Rückfluss erhitzt, dann auf RT abkühlen gelassen und filtriert. Das Filtrat wurde am Rotationsverdampfer von den flüchtigen Komponenten befreit und der Rückstand im Hochvakuum getrocknet. Es wurden 3.70 g der Titelverbindung in ca. 90% Reinheit (89% d. Th.) erhalten.
LC/MS [Methode 3]: Rₜ = 1.10 min; m/z = 380 (M+H)^{+ 1}H-NMR (DMSO-d₆, 400 MHz): δ = 3.70 (s, 3H), 3.84 (dd, 1H), 3.99 (dd, 1H), 4.16-4.35 (m, 1H), 4.72 (s, 2H), 6.91 (d, 1H), 7.64 (d, 2H), 7.78 (d, 2H).

Die racemische Verbindung aus Beispiel 6A wurde durch präparative HPLC an chiraler Phase in die Enantiomere aufgetrennt [Probenvorbereitung: 3.6 g Racemat gelöst in 54 ml Ethylacetat/Isohexan (1:1 v/v), in drei Portionen über die Säule getrennt; Säule: chirale Kieselgel-Phase basierend auf dem Selektor Poly(*N*-methacryloyl-L-isoleucin-3-pentylamid), 430 mm x 40 mm; Eluent: Stufengradient Isohexan/Ethylacetat 1:1 1 → Ethylacetat → Isohexan/Ethylacetat 1:1; Fluss: 50 ml/ min; Temperatur: 24°C; UV-Detektion: 260 nm]. Auf diese Weise wurden 1.6 g des zuerst eluierenden Enantiomers 1 (Beispiel 7A) sowie 1.6 g des später eluierenden Enantiomers 2 (Beispiel 8A) erhalten:

### Beispiel 7A

### {3-(4-Chlorphenyl)-5-oxo-4-[(2S)-3,3,3-trifluor-2-hydroxypropyl]-4,5-dihydro-1H-1,2,4-triazol-1-yl} essigsäuremethylester (Enantiomer 1)

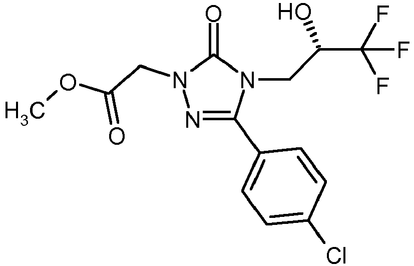

Zuerst eluierendes Enantiomer aus der Racemat-Trennung von Beispiel 6A.

Rₜ = 3.21 min [Säule: chirale Kieselgel-Phase basierend auf dem Selektor Poly(*N*-methacryloyl-L-isoleucin-3-pentylamid), 250 mm x 4.6 mm; Eluent: Isohexan/Ethylacetat 1:1; Fluss: 1 ml/min; UV-Detektion: 260 nm].

### Beispiel 8A

### {3-(4-Chlorphenyl)-5-oxo-4-[(2R)-3,3,3-trifluor-2-hydroxypropyl]-4,5-dihydro-1H-1,2,4-triazol-1-yl} essigsäuremethylester (Enantiomer 2)

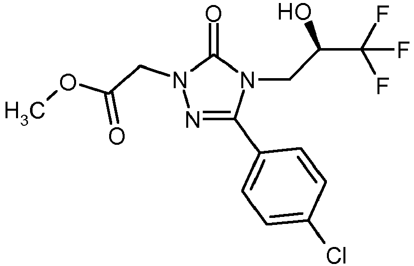

Zuletzt eluierendes Enantiomer aus der Racemat-Trennung von Beispiel 6A.

Rₜ = 4.48 min [Säule: chirale Kieselgel-Phase basierend auf dem Selektor Poly(*N*-methacryloyl-L-isoleucin-3-pentylamid), 250 mm x 4.6 mm; Eluent: Isohexan/Ethylacetat 1:1; Fluss: 1 ml/min; UV-Detektion: 260 nm].

### Beispiel 9A

### Methyl-{3-(4-chlorphenyl)-5-oxo-4-[(1E)-3,3,3-trifluorprop-1-en-1-yl]-4,5-dihydro-1H-1,2,4-triazol-1-yl} acetat

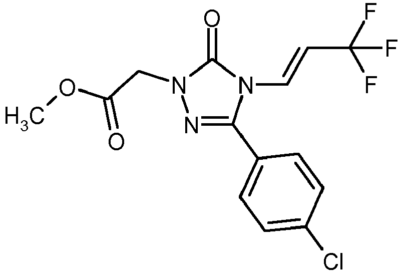

5.0 g (13.12 mmol) der Verbindung aus Beispiel 8A wurden bei RT zusammen mit 1.93 g (15.8 mmol) 4-*N*,*N*-Dimethylaminopyridin in 70 ml Pyridin vorgelegt, portionsweise mit 5.54 ml (32.92 mmol) Trifluormethansulfonsäureanhydrid versetzt und 18 h gerührt. Zur Aufarbeitung wurde mit 5 ml 1 N Salzsäure versetzt und das Pyridin am Rotationsverdampfer entfernt. Der Rückstand wurde in 50 ml Ethylacetat aufgenommen und mit 25 ml Wasser gewaschen. Die wässrige Phase wurde zweimal mit je 25 ml Ethylacetat rückextrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 10:1, dann 4:1). Es wurden 3.50 g (73% d. Th.) der Titelverbindung erhalten.
LC/MS [Methode 4]: Rₜ = 1.14 min; m/z = 362 (M+H)⁺
¹H-NMR (400 MHz, DMSO-d₆): δ = 7.68 (s, 4H), 7.18 (d, 1H), 6.85 (dd, 1H), 4.78 (s, 2H), 3.72 (s, 3H).

### Beispiel 10A

### Methyl-[3-(4-chlorphenyl)-5-oxo-4-(3,3,3-trifluorpropyl)-4,5-dihydro-1H-1,2,4-triazol-1-yl]acetat

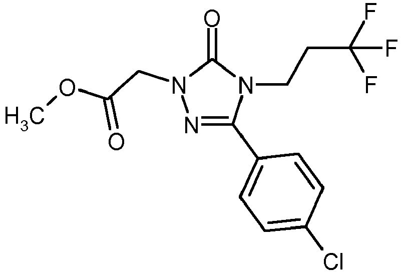

1.3 g (3.59 mmol) der Verbindung aus Beispiel 9A und 150 mg Platin auf Kohle (5%) wurden in 150 ml Methanol gelöst und 18 h bei Normaldruck hydriert. Zur Aufarbeitung wurde der Katalysator über Kieselgur abfiltriert und das Filtrat am Rotationsverdampfer eingeengt. Nach Trocknen des Rückstands im Hochvakuum erhielt man 1.26 g (89% d. Th.) der Titelverbindung mit einer Reinheit von 92%.
LC/MS [Methode 4]: Rₜ = 1.00 min; m/z = 364 (M+H)⁺
¹H-NMR (400 MHz, DMSO-d₆): δ = 2.55-2.68 (m, 2H), 3.69 (s, 3H), 4.01 (t, 2H), 4.70 (s, 2H), 7.61-7.72 (m, 4H).

### Beispiel 11A

### 2-[(4-Chlorphenyl)carbonyl]-N-(prop-2-en-1-yl)hydrazincarboxamid

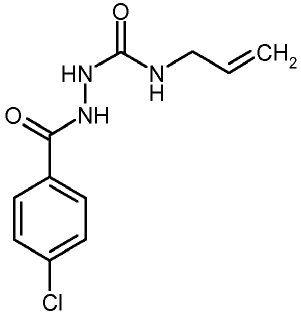

5.00 g (29.3 mmol) 4-Chlorbenzohydrazid wurden in 150 ml trockenem THF bei 50°C suspendiert. Anschließend tropfte man 2.63 ml (29.9 mmol) Allylisocyanat, gelöst in 110 ml trockenem THF, hinzu. Vorübergehend ging das Ausgangsmaterial vollständig in Lösung, dann fiel ein feiner Niederschlag aus. Die Mischung wurde 2 h bei 50°C gerührt. Nach Abkühlen auf Raumtemperatur wurde mit Diethylether versetzt. Der farblose Feststoff wurde abgesaugt, mit Diethylether gewaschen und im Hochvakuum getrocknet. Es wurden 7.42 g (100% d. Th.) der Zielverbindung erhalten.
LC/MS [Methode 5]: Rₜ = 1.51 min; MS [ESIpos]: m/z = 254 (M+H)⁺
¹H-NMR (400 MHz, DMSO-d₆): δ = 3.60-3.70 (m, 2H), 5.01 (dd, 1H), 5.14 (dd, 1H), 5.72-5.86 (m, 1H), 6.70 (s, 1H), 7.56 (d, 2H), 7.85-7.95 (m, 3H), 10.21 (s, 1H).

### Beispiel 12A

### 5-(4-Chlorphenyl)-4-(prop-2-en-1-yl)-2,4-dihydro-3H-1,2,4-triazol-3-on

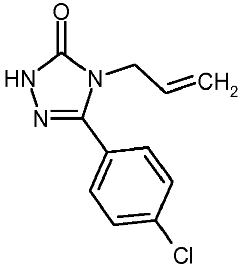

26.8 g (106 mmol) 2-[(4-Chlorphenyl)carbonyl]-*N*-(prop-2-en-1-yl)hydrazincarboxamid aus Beispiel 11A wurden in 210 ml 3 M Natronlauge suspendiert und 20 h unter Rückfluss erhitzt. Nach dem Abkühlen stellte man mit halbkonzentrierter Salzsäure auf pH 10 ein. Der ausgefallene farblose Feststoff wurde abgesaugt, mit Wasser neutral gewaschen und anschließend in Methanol verrührt. Die Mischung wurde durch Filtration von unlöslichen Bestandteilen befreit, das Filtrat am Rotationsverdampfer unter vermindertem Druck eingedampft und der Rückstand im Hochvakuum getrocknet. Man erhielt so 21.5 g (86.4% d. Th.) der Titelverbindung als farblosen Feststoff.
LC/MS [Methode 5]: Rₜ = 1.79 min; MS [ESIpos]: m/z = 236 (M+H)⁺
¹H-NMR (400 MHz, DMSO-d₆): δ = 4.30-4.35 (m, 2H), 4.91 (dd, 1H), 5.11 (dd, 1H), 5.76-5.90 (m, 1H), 7.58 (d, 2H), 7.65 (d, 2H), 12.05 (s, 1H).

### Beispiel 13A

### 5-(4-Chlorphenyl)-2-(prop-2-in-1-yl)-4-(3,3,3-trifluor-2-hydroxypropyl)-2,4-dihydro-3H-1,2,4-triazol-3-on

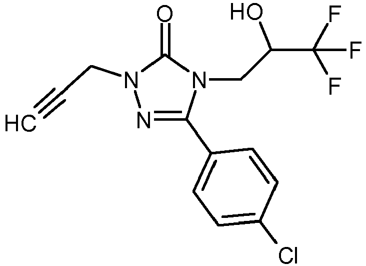

300 mg (0.98 mmol) der Verbindung aus Beispiel 4A wurden in 10 ml Acetonitril gelöst und mit 122 mg (1.02 mmol) 3-Brom-1-propin sowie 270 mg (1.95 mmol) Kaliumcarbonat versetzt. Die Mischung wurde für 1 h auf Rückfluss erhitzt. Zur Aufarbeitung ließ man auf RT abkühlen und versetzte mit ca. 10 ml Wasser. Es wurde zweimal mit je 15 ml Ethylacetat extrahiert. Die vereinigten organischen Phasen wurden über Natriumsulfat getrocknet, filtriert und im Vakuum eingeengt. Das Rohprodukt wurde chromatograpisch gereinigt [Methode 19]. Es wurden 166 mg (49% d. Th.) der Zielverbindung erhalten.
LC/MS [Methode 4]: Rₜ = 0.97 min; m/z = 346 (M+H)⁺
¹H-NMR (CDCl₃, 400 MHz): δ = 2.38 (t, 1H), 3.94-4.09 (m, 1H), 4.43-4.54 (m, 1H), 4.68 (d, 2H), 4.73-4.78 (m, 1H), 7.50 (d, 2H), 7.57 (d, 2H).

### Beispiel 14A

### 5-(4-Chlorphenyl)-2-(prop-2-in-1-yl)-4-[(2S)-3,3,3-trifluor-2-hydroxypropyl]-2,4-dihydro-3H-1,2,4-triazol-3-on

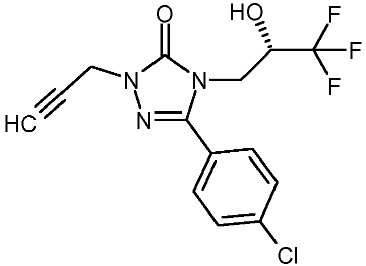

1110 mg (3.61 mmol) der Verbindung aus Beispiel 5A wurden in 30 ml Acetonitril gelöst und mit 451 mg (3.79 mmol) 3-Brom-1-propin sowie 2.35 g (7.22 mmol) Cäsiumcarbonat versetzt. Die Mischung wurde für 1 h unter Rückfluss erhitzt. Zur Aufarbeitung ließ man auf RT abkühlen und versetzte mit ca. 30 ml Wasser. Es wurde zweimal mit je 30 ml Ethylacetat extrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 10:1, dann 3:1). Es wurden 203 mg (16% d. Th.) der Titelverbindung erhalten.
LC/MS [Methode 3]: Rₜ = 1.11 min; m/z = 346 (M+H)⁺
¹H-NMR (CDCl₃, 400 MHz): δ = 2.38 (t, 1H), 3.94-4.09 (m, 1H), 4.43-4.54 (m, 1H), 4.68 (d, 2H), 4.73-4.78 (m, 1H), 7.50 (d, 2H), 7.57 (d, 2H).

### Beispiel 15A

### Methyl-3-{[3-(4-chlorphenyl)-1-(2-methoxy-2-oxoethyl)-5-oxo-1,5-dihydro-4H-1,2,4-triazol-4-yl]-methyl}benzolcarboxylat

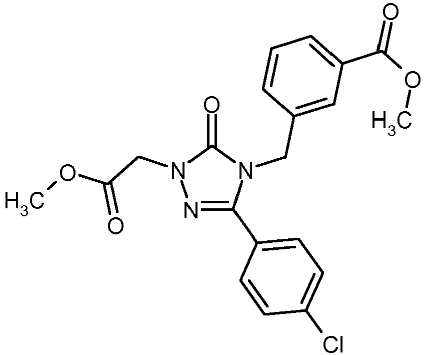

200 mg (0.75 mmol) Methyl-[3-(4-chlorphenyl)-5-oxo-4,5-dihydro-1*H*-1,2,4-triazol-1-yl]acetat [Herstellung gemäß WO 2007/134862 Beispiel 222A] wurden in 7.5 ml Aceton suspendiert, mit 365 mg (1.12 mmol) Cäsiumcarbonat sowie 223 mg (0.97 mmol) Methyl-3-brommethylbenzoat versetzt und 1 h zum Sieden erhitzt. Nach dem Abkühlen wurde über Extrelut filtriert und mit Aceton nachgewaschen. Das Filtrat wurde im Vakuum eingeengt und der Rückstand durch Chromatographie an Kieselgel gereinigt (Eluent: Cyclohexan/Ethylacetat 4:1 → 1:1). Die Zielverbindung wurde als farbloser Schaum erhalten (165 mg, 53% d. Th.).
MS [DCI]: m/z = 433 (M+NH₄)⁺, 416 (M+H)⁺
¹H-NMR (400 MHz, DMSO-d₆): δ = 3.71 (s, 3H), 3.82 (s, 3H), 4.75 (s, 2H), 5.08 (s, 2H), 7.32 (d, 1H), 7.46 (t, 1H), 7.55 (s, 4H), 7.66 (s, 1H), 7.82 (d, 1H).

### Beispiel 16A

### Methyl-3-{[3-(4-chlorphenyl)-1-(2-hydrazino-2-oxoethyl)-5-oxo-1,5-dihydro-4H-1,2,4-triazol-4-yl]-methyl}benzolcarboxylat

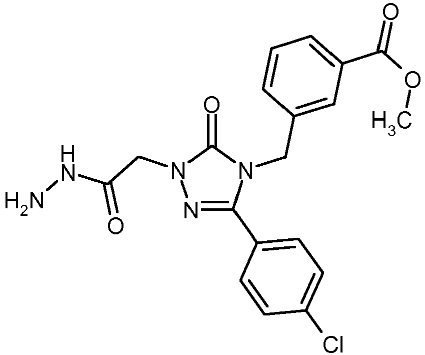

172 mg (0.41 mmol) der Verbindung aus Beispiel 15A wurden in 1 ml Ethanol suspendiert und mit 40 µl (0.83 mmol) Hydrazinhydrat versetzt. Die Mischung wurde 4 h unter Rückfluss erhitzt und dann 18 h bei Raumtemperatur stehen gelassen. Der Ansatz wurde am Rotationsverdampfer unter vermindertem Druck eingeengt und der Rückstand im Hochvakuum getrocknet. Man erhielt so 161 mg (94% d. Th.) der Zielverbindung als farblosen Feststoff.
MS [DCI]: m/z = 433 (M+NH₄)⁺, 416 (M+H)⁺
¹H-NMR (400 MHz, DMSO-d₆): δ = 3.82 (s, 3H), 4.31 (d, 2H), 4.43 (s, 2H), 5.05 (s, 2H), 7.37 (d, 1H), 7.42-7.57 (m, 5H), 7.71 (s, 1H), 7.83 (d, 1H), 9.30 (t, 1H).

In analoger Weise wurden die folgenden Verbindungen erhalten:

| **Beispiel** | **Name** | **Struktur** | **Edukt** | **Analytische Daten** |
|---|---|---|---|---|
| **17A** | 2-[3-(4-Chlorphenyl)-4-(4-methoxybenzyl)-5-oxo-4,5-dihydro-1*H-*1,2,4-triazol-1-yl]-acetohydrazid | 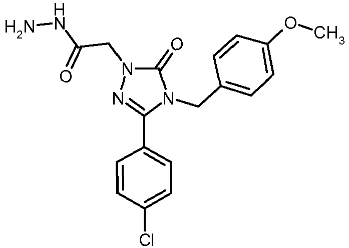 | WO 2007/ 134862 Bsp. 78A | LC/MS [Methode 5]: Rₜ = 1.88 min; MS [ESIpos]: m/z = 388 (M+H)⁺ |
| **18A** | 2-[3-(4-Chlorphenyl)-5-oxo-4-(3,3,3-trifluor-2-hydroxypropyl)-4,5-dihydro-1*H-*1,2,4-triazol-1-yl]acetohydrazid | 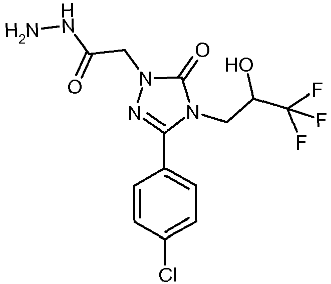 | Bsp. 6A | LC/MS [Methode 5]: Rₜ = 1.83 min; MS [ESIpos]: m/z = 380 (M+Na)⁺ |
| **19A** | 2-{3-(4-Chlorphenyl)-5-oxo-4-[(2*S*)-3,3,3-trifluor-2-hydroxypropyl]-4,5-dihydro-1*H-*1,2,4-triazol-1-yl}acetohydrazid *(Enantiomer I)* | 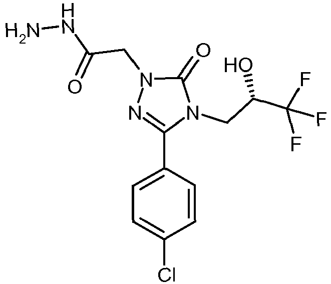 | Bsp. 7A | ¹H-NMR (400 MHz, DMSO-d₆): δ = 3.33 (br. s, 2H), 3.77-3.86 (m, 1H), 3.91-3.99 (m, 1H), 4.33-4.43 (m, 2H), 6.93 (br. s, 1H), 7.61-7.66 (m, 2H), 7.72-7.79 (m, 2H), 9.21-9.30 (br. s, 1H). |
| **20A** | 2-{3-(4-Chlorphenyl)-5-oxo-4-[(2R)-3,3,3-trifluor-2-hydroxypropyl]-4,5-dihydro-1*H-*1,2,4-triazol-1-yl}acetohydrazid *(Enantiomer II)* | 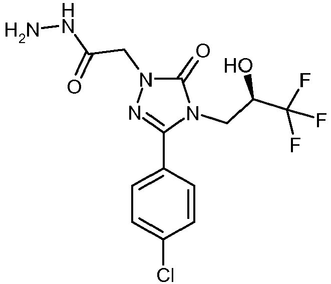 | Bsp. 8A | LC/MS [Methode 3]: Rₜ = 0.90 min; MS [ESIpos]: m/z = 380 (M+H)⁺ |

### Beispiel 21A

### 2-[3-(4-Chlorphenyl)-4-cyclopropyl-5-oxo-4,5-dihydro-1H-1,2,4-triazol-1-yl]acetohydrazid

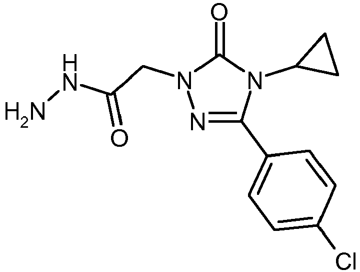

50 mg (0.17 mmol) [3-(4-Chlorphenyl)-4-cyclopropyl-5-oxo-4,5-dihydro-1*H*-1,2,4-triazol-1-yl]-essigsäure [Herstellung gemäß WO 2007/134862 Beispiel 88A] wurden in 0.4 ml Methanol gelöst, mit 0.6 ml Toluol verdünnt und dann tropfenweise mit 128 µl (0.26 mmol) Trimethylsilyldiazomethan-Lösung (2 M in Hexan-Gemisch) versetzt, bis eine leichte Gelbfärbung bestehen blieb. Der Ansatz wurde 1 h gerührt und dann bis zur Trockene eingeengt. Der Rückstand wurde in 1 ml Ethanol aufgenommen, mit 43 mg (0.85 mmol) Hydrazinhydrat versetzt und 2.5 h unter Rückfluss gerührt. Nach dem Abkühlen wurde die Lösung im Vakuum eingeengt und der Rückstand im Hochvakuum getrocknet. Man erhielt so 54 mg (96% d. Th.) der Titelverbindung in 93% Reinheit als farblosen Feststoff.
LC/MS [Methode 5]: Rₜ = 1.65 min; MS [ESIpos]: m/z = 308 (M+H)⁺.

In analoger Weise wurden die beiden folgenden Verbindungen erhalten:

| **Beispiel** | **Name** | **Struktur** | **Edukt** | **Analytische Daten** |
|---|---|---|---|---|
| **22A** | 2-[3-(5-Chlorthiophen-2-yl)-4-(2-fluorbenzyl)-5-oxo-4,5-dihydro-1*H-*1,2,4-triazol-1-yl]-acetohydrazid | 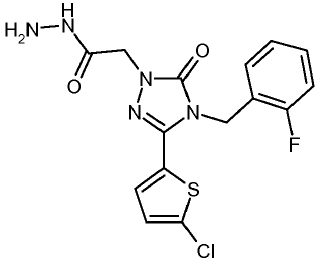 | WO 2007/ 134862 Bsp. 154A | LC/MS [Methode 7]: Rₜ = 1.73 min; MS [ESIpos]: m/z = 382 (M+H)⁺ |
| **23A** | 2-[3-(4-Chlorphenyl)-5-oxo-4-(3,3,3-trifluorpropyl)-4,5-dihydro-1*H-*1,2,4-triazol-1-yl]-acetohydrazid | 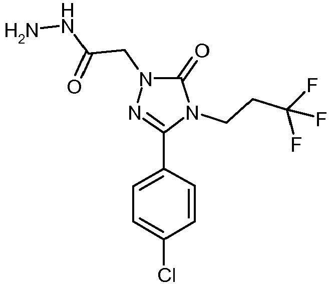 | 10A | LC/MS [Methode 6]: Rₜ = 1.75 min; MS [ESIpos]: m/z = 384 (M+H)⁺ |

### Beispiel 24A

### [3-(5-Chlorthiophen-2-yl)-4-(2-fluorbenzyl)-5-oxo-4,5-dihydro-1H-1,2,4-triazol-1-yl]acetonitril

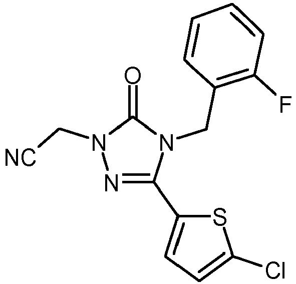

Eine Mischung aus 250 mg (0.81 mmol) 5-(5-Chlor-2-thienyl)-4-(2-fluorbenzyl)-2,4-dihydro-3*H-*1,2,4-triazol-3-on [Herstellung gemäß WO 2007/134862 Beispiel 154A], 67 µl (0.97 mmol) Bromacetonitril sowie 223 mg (1.61 mmol) Kaliumcarbonat wurde in 8 ml trockenem DMF 1 h bei 100°C Ölbadtemperatur gerührt. Nach dem Abkühlen wurde über Kieselgur filtriert, das Filtrat unter vermindertem Druck eingeengt und der Rückstand zwischen MTBE und Wasser verteilt. Die organische Phase wurde nacheinander mit 10 ml Wasser und 10 ml gesättigter Natriumchlorid-Lösung gewaschen, über Natriumsulfat getrocknet, filtriert und im Vakuum eingeengt. Der Rückstand wurde an Kieselgel chromatographisch gereinigt (Eluent: Cyclohexan/Ethylacetat 4:1). Es wurden 258 mg (92% d. Th.) der Zielverbindung als leicht gelblicher Feststoff erhalten.
MS [DCI]: m/z = 366 (M+NH₄)⁺, 349 (M+H)⁺
¹H-NMR (400 MHz, CDCl₃): δ = 4.81 (s, 2H), 5.11 (s, 2H), 6.87 (d, 1H), 6.97 (s, 1H), 7.05-7.16 (m, 3H), 7.28-7.36 (m, 1H).

### Beispiel 25A

### (1Z)-2-[3-(5-Chlorthiophen-2-yl)-4-(2-fluorbenzyl)-5-oxo-4,5-dihydro-1H-1,2,4-triazol-1-yl]-N'-hydroxyethanimidamid

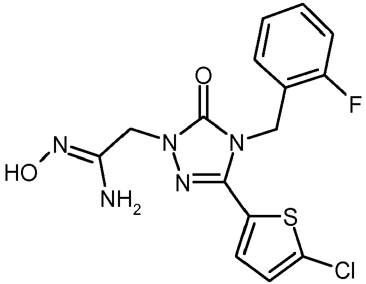

250 mg (0.66 mmol) der Verbindung aus Beispiel 24A und 92 mg (1.32 mmol) Hydroxylamin-Hydrochlorid wurden in 3.3 ml Ethanol suspendiert und zum Sieden erhitzt. Zu der heißen Lösung gab man 193 µl (1.39 mmol) Triethylamin und erhitzte weiter für 1 h unter Rückfluss. Beim Erkalten des Ansatzes kristallisierte ein farbloser Feststoff aus, der abgesaugt und mit wenig Ethanol gewaschen wurde. Man erhielt so 174 mg (69% d. Th.) der Zielverbindung als farblosen Feststoff. Die Mutterlauge wurde am Rotationsverdampfer unter vermindertem Druck eingedampft und der Rückstand zwischen Wasser und Ethylacetat verteilt. Die organische Phase wurde nacheinander mit 10 ml Wasser und 10 ml gesättigter Natriumchlorid-Lösung gewaschen, über Natriumsulfat getrocknet, filtriert und im Vakuum eingeengt. Man erhielt so weitere 69 mg (24% d. Th.) der Zielverbindung in 87% Reinheit als leicht gelblichen Feststoff.
MS [ESIpos]: m/z = 382 (M+H)⁺
¹H-NMR (400 MHz, DMSO-d₆): δ = 4.36 (s, 2H), 5.11 (s, 2H), 5.46 (s, 2H), 7.04-7.12 (m, 1H), 7.12-7.28 (m, 4H), 7.30-7.41 (m, 1H), 9.27 (s, 1H).

### Beispiel 26A

### 1-Amino-3-[3-(trifluormethyl)phenyl]aceton-Hydrochlorid

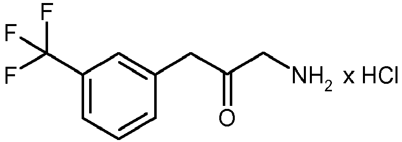

Zu einer Lösung von 2.67 g (11.2 mmol) 1-Chlor-3-[3-(trifluormethyl)phenyl]propan-2-on in 11 ml DMF gab man unter Argon und Eiskühlung portionsweise 1.13 g (11.8 mmol) Diformylamid-Natriumsalz. Das Gemisch wurde 1 h im Eisbad und dann über Nacht bei RT gerührt. Danach wurde mit 25 ml Ethylacetat verdünnt und nacheinander mit je 15 ml 0.5 N Salzsäure, Wasser, gesättigter Natriumhydrogencarbonat-Lösung und erneut Wasser gewaschen. Die organische Phase wurde über Natriumsulfat getrocknet, filtriert und im Vakuum eingeengt. Der dunkle, ölige Rückstand wurde über eine kurze Kieselgel-Säule vorgereinigt (Eluent: Dichlormethan/Ethanol 95:5). Man erhielt so 2.0 g eines dunkelbraunen Feststoffs. Dieser wurde in 25 ml einer 7 N Lösung von Chlorwasserstoff in Isopropanol aufgenommen und über Nacht gerührt. Das Solvens wurde am Rotationsverdampfer unter vermindertem Druck entfernt und der Rückstand in ca. 15 ml Methanol gelöst. In diese Lösung rührte man 100 ml Diethylether ein und isolierte den ausgefallenen Feststoff durch Filtration. Der Filterkuchen wird mit ca. 10 ml Diisopropylether gewaschen und im Hochvakuum getrocknet. Es wurden so 0.79 g (28% d. Th.) der Zielverbindung als brauner Feststoff erhalten.
LC/MS [Methode 8]: Rₜ = 2.30 min; MS [ESIpos]: m/z = 218 (M-HCl)⁺
¹H-NMR (400 MHz, DMSO-d₆): δ = 4.00-4.11 (m, 4H), 7.49-7.70 (m, 4H), 8.17 (br. d, 1H).

### Beispiel 27A

### 2-[3-(4-Chlorphenyl)-4-cyclopropyl-5-oxo-4,5-dihydro-1H-1,2,4-triazol-1-yl]-N-{2-oxo-3-[3-(trifluormethyl)phenyl]propyl}acetamid

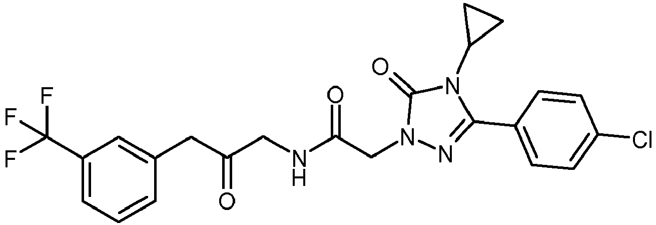

Zu einer Lösung von 654 mg (2.23 mmol) [3-(4-Chlorphenyl)-4-cyclopropyl-5-oxo-4,5-dihydro-1*H-*1,2,4-triazol-1-yl]essigsäure [Herstellung gemäß WO 2007/134862 Beispiel 88A] und 650 mg (2.56 mmol) der Verbindung aus Beispiel 26A in 4.4 ml trockenem DMF gab man 391 mg (2.90 mmol) HOBt sowie 513 mg (2.67 mmol) EDC. Anschließend wurden 465 µl (2.67 mmol) *N,N-*Diisopropylethylamin zugesetzt und der Ansatz über Nacht gerührt. Zur Aufarbeitung wurde mit 20 ml Ethylacetat verdünnt und zweimal mit je 15 ml Wasser extrahiert. Die organische Phase wurde über Natriumsulfat getrocknet, filtriert und im Vakuum eingeengt. Der verbliebene Rückstand wurde aus Acetonitril umkristallisiert. Man erhielt 610 mg (56% d. Th.) der Zielverbindung als sehr feine, farblose Kristalle. Weitere 84 mg (8% d. Th.) der Zielverbindung wurden aus einer zweiten Kristallisation der eingeengten Mutterlauge in Form von leicht gelblichen Kristallen erhalten.
MS [ESIpos]: m/z = 493 (M+H)⁺
¹H-NMR (400 MHz, CDCl₃): δ = 0.73-0.83 (m, 2H), 0.97-1.09 (m, 2H), 2.94-3.04 (m, 1H), 3.80 (s, 2H), 4.25 (d, 2H), 4.53 (s, 2H), 6.91-7.02 (m, 1H), 7.39 (d, 1H), 7.43-7.51 (m, 4H), 7.57 (d, 1H), 7.71 (d, 2H).

### Beispiel 28A

### 2-[(5-Brompyridin-3-yl)methyl]-5-(4-chlorphenyl)-4-(3,3,3-trifluor-2-hydroxypropyl)-2,4-dihydro-3H-1,2,4-triazol-3-on

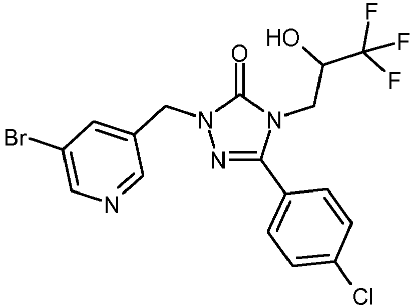

300 mg (0.98 mmol) der Verbindung aus Beispiel 4A und 953 mg (2.93 mmol) Cäsiumcarbonat wurden in 4 ml DMF gelöst und mit 261 mg (1.10 mmol) 3-Brom-5-(chlormethyl)pyridin-Hydrochlorid versetzt. Es wurde zunächst 20 h bei 40°C und anschließend 24 h bei 70°C gerührt. Zur Vervollständigung der Reaktion wurden danach weitere 130 mg (0.55 mmol) 3-Brom-5-(chlormethyl)pyridin-Hydrochlorid sowie 450 mg (1.38 mmol) Cäsiumcarbonat hinzugefügt und der Ansatz erneut 20 h bei 70°C gerührt. Nach Abkühlen auf RT wurde das Gemisch mit 10 ml Wasser versetzt und dreimal mit je 10 ml Ethylacetat extrahiert. Die vereinigten organischen Phasen wurden über Magnesiumsulfat getrocknet, filtriert und im Vakuum eingeengt. Das Rohprodukt wurde durch präparative HPLC gereinigt [Methode 19]. Es wurden 263 mg (56% d. Th.) der Zielverbindung erhalten.
LC/MS [Methode 4]: Rₜ = 1.07 min; MS [ESIpos]: m/z = 477 und 479 (M+H)⁺.

### Beispiel 29A

### 2-[(5-Brompyridin-3-yl)methyl]-5-(4-chlorphenyl)-4-[(2S)-3,3,3-trifluor-2-hydroxypropyl]-2,4-dihydro-3H-1,2,4-triazol-3-on

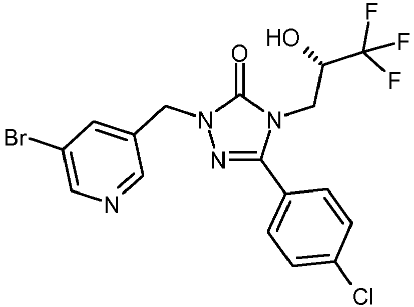

171 mg (0.56 mmol) der Verbindung aus Beispiel 5A und 543 mg (1.67 mmol) Cäsiumcarbonat wurden in 11 ml Acetonitril gelöst und mit 135 mg (0.56 mmol) 3-Brom-5-(chlormethyl)pyridinHydrochlorid versetzt. Es wurde 5 h bei 65°C gerührt. Nach Abkühlen auf RT wurde das Gemisch mit 10 ml Wasser versetzt und dreimal mit je 10 ml Ethylacetat extrahiert. Die vereinigten organischen Phasen wurden über Natriumsulfat getrocknet, filtriert und im Vakuum eingeengt. Das Rohprodukt wurde durch präparative HPLC gereinigt [Methode 19]. Es wurden 102 mg (38% d. Th.) der Zielverbindung erhalten.
LC/MS [Methode 4]: Rₜ = 1.05 min; MS [ESIpos]: m/z = 477 und 479 (M+H)⁺.

### Beispiel 30A

### 5-(4-Chlorphenyl)-2-[(5-chlor-2-thienyl)methyl]-4-[(2S)-3,3,3-trifluor-2-hydroxypropyl]-2,4-dihydro-3H-1,2,4-triazol-3-on

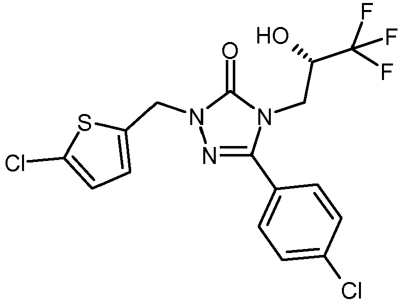

119 mg (0.39 mmol) der Verbindung aus Beispiel 5A und 107 mg (0.77 mmol) Kaliumcarbonat wurden in 5 ml Acetonitril gelöst und mit 65 mg (0.39 mmol) 2-Chlor-5-(chlormethyl)thiophen versetzt. Es wurde 2 h unter Rückfluss gerührt. Nach Abkühlen auf RT wurde das Gemisch mit 10 ml Wasser versetzt und dreimal mit je 10 ml Ethylacetat extrahiert. Die vereinigten organischen Phasen wurden mit 10 ml gesättigter Natriumchlorid-Lösung gewaschen, über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 10:1 → 8:1 → 5:1 → 1:1). Es wurden 114 mg (65% d. Th.) der Zielverbindung erhalten.
LC/MS [Methode 4]: Rₜ = 1.20 min; MS [ESIpos]: m/z = 438 und 440 (M+H)⁺.

### Beispiel 31A

### 5-(4-Chlorphenyl)-2-[(2-chlor-1,3-thiazol-5-yl)methyl]-4-[(2S)-3,3,3-trifluor-2-hydroxypropyl]-2,4-dihydro-3H-1,2,4-triazol-3-on

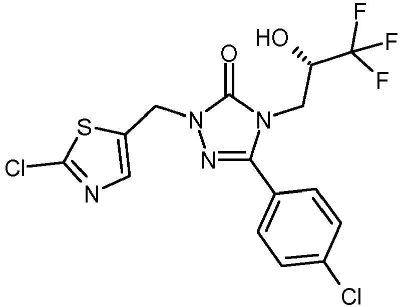

500 mg (1.63 mmol) der Verbindung aus Beispiel 5A und 449 mg (3.25 mmol) Kaliumcarbonat wurden in 4 ml Acetonitril gelöst und mit 287 mg (1.71 mmol) 2-Chlor-5-(chlormethyl)-1,3-thiazol versetzt. Es wurde 1.5 h bei 80°C gerührt. Nach Abkühlen auf RT wurde das Gemisch mit 10 ml Wasser versetzt und dreimal mit je 10 ml Ethylacetat extrahiert. Die vereinigten organischen Phasen wurden mit 10 ml gesättigter Natriumchlorid-Lösung gewaschen, über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 7:1, dann 1:1). Es wurden 619 mg (87% d. Th.) der Zielverbindung erhalten.
LC/MS [Methode 3]: Rₜ = 1.24 min; MS [ESIpos]: m/z = 439 und 441 (M+H)⁺.

### Beispiel 32A

### Ethyl-1-(2,6-dichlorbcnzyl)-1H-imidazol-5-carboxylat

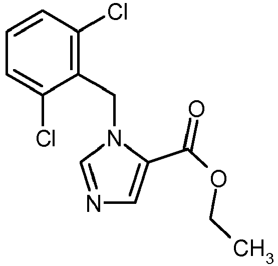

258 mg (1.84 mmol) Ethyl-1*H*-imidazol-4-carboxylat wurden zusammen mit 486 mg (2.03 mmol) 2,6-Dichlorbenzylbromid in 7 ml DMF gelöst und mit 720 mg (2.21 mmol) Cäsiumcarbonat versetzt. Es wurde 16 h bei 80°C gerührt. Nach Abkühlen auf RT wurde mit 10 ml Wasser versetzt und zweimal mit je 10 ml Ethylacetat extrahiert. Die vereinigten organischen Phasen wurden über Natriumsulfat getrocknet und am Rotationsverdampfer vom Lösungsmittel befreit. Das Rohprodukt wurde chromatographisch gereinigt [Methode 19]. Man erhielt 220 mg (40% d. Th.) der Zielverbindung.
LC/MS [Methode 3]: Rₜ = 1.03 min; MS [ESIpos]: m/z = 299 und 301 (M+H)⁺
¹H-NMR (400 MHz, CDCl₃): δ = 1.37 (t, 3H), 4.34 (q, 2H), 5.44 (s, 2H), 7.27-7.33 (m, 1H), 7.37-7.43 (m, 2H), 7.68 (d, 2H).

In einer weiteren Fraktion erhielt man 110 mg (20% d. Th.) des Regioisomeren Ethyl-1-(2,6-dichlorbenzyl)-1*H*-imidazol-4-carboxylat:
LC/MS [Methode 3]: Rₜ = 1.12 min; MS [ESIpos]: m/z = 299 und 301 (M+H)⁺.

### Beispiel 33A

### Ethyl-1-(2,6-dichlorbenzyl)-4-nitro-1H-imidazol-2-carboxylat

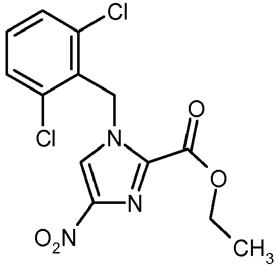

1000 mg (5.4 mmol) Ethyl-4-nitro-1*H*-imidazol-2-carboxylat wurden zusammen mit 1426 mg (5.94 mmol) 2,6-Dichlorbenzylbromid in 37 ml DMF gelöst und mit 2112 mg (6.48 mmol) Cäsiumcarbonat versetzt. Es wurde 4 h bei 75°C gerührt. Nach Abkühlen auf RT wurde das Gemisch auf 100 ml Eiswasser gegeben. Das ausgefallene Produkt wurde abfiltriert und mit Wasser gewaschen. Der beigefarbene Feststoff wurde im Hochvakuum getrocknet. Man erhielt 1500 mg (81% d. Th.) der Zielverbindung.
LC/MS [Methode 1]: Rₜ = 2.05 min; MS [ESIpos]: m/z = 344 und 346 (M+H)⁺
¹H-NMR (400 MHz, DMSO-d₆): δ = 1.35 (t, 3H), 4.41 (q, 2H), 5.96 (s, 2H), 7.53 (dd, 1H), 7.59-7.64 (m, 2H), 7.96 (s, 1H).

### Beispiel 34A

### Methyl-1-(2-chlorphenyl)-1H-imidazol-4-carboxylat

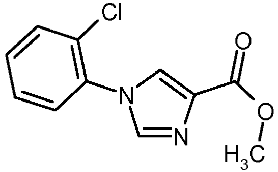

200 mg (1.59 mmol) Methyl-1*H*-imidazol-4-carboxylat, 496 mg (3.17 mmol) 2-Chlorphenylboronsäure, 100 mg 3Å-Molekularsieb sowie 432 mg (2.28 mmol) Kupfer(II)acetat wurden in 2 ml Dichlormethan vorgelegt und mit 256 µl (3.17 mmol) Pyridin versetzt. Das Gemisch wurde 20 h bei RT gerührt. Zur Aufarbeitung wurde über etwas Kieselgur filtriert, der Filter-Rückstand mit ca. 15 ml Ethylacetat nachgewaschen und die vereinigten Filtrate mit 5 ml Wasser gewaschen. Die organische Phase wurde über Natriumsulfat getrocknet, filtriert und im Vakuum eingeengt. Das Rohprodukt wurde durch Chromatographie an Kieselgel gereinigt (Elutionsmittel: Cyclohexan/ Ethylacetat 3:1 → 1:1 → 1:3). Es wurden 55 mg (13% d. Th.) der Zielverbindung erhalten.
LC/MS [Methode 3]: Rₜ = 0.90 min; MS [ESIpos]: m/z = 237 (M+H)⁺
¹H-NMR (400 MHz, DMSO-d₆): δ = 3.79 (s, 3H), 7.53-7.58 (m, 2H), 7.64 (dd, 1H), 7.70-7.77 (m, 1H), 8.05 (d, 1H), 8.20 (d, 1H).

### Beispiel 35A

### Ethyl-1-(2-chlorphenyl)-1H-pyrazol-4-carboxylat

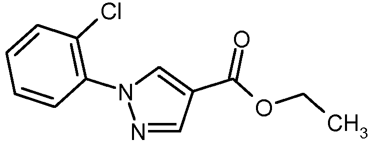

400 mg (2.85 mmol) Ethyl-1*H*-pyrazol-4-carboxylat, 893 mg (5.71 mmol) 2-Chlorphenylboronsäure, 100 mg 3Å-Molekularsieb sowie 778 mg (4.28 mmol) Kupfer(II)acetat wurden in 2 ml Dichlormethan vorgelegt und mit 461 µl (5.71 mmol) Pyridin versetzt. Das Gemisch wurde 20 h bei RT gerührt. Zur Aufarbeitung wurde über etwas Kieselgur filtriert, der Filter-Rückstand mit ca. 10 ml Dichlormethan nachgewaschen und die vereinigten Filtrate im Vakuum eingeengt. Das Rohprodukt wurde durch Chromatographie an Kieselgel gereinigt (Elutionsmittel: Cyclohexan/Ethylacetat 10:1, dann 5:1). Es wurden 71 mg (10% d. Th.) der Zielverbindung erhalten.
LC/MS [Methode 3]: Rₜ = 1.15 min; MS [ESIpos]: m/z = 251 (M+H)⁺.

### Beispiel 36A

### [1-(2,6-Dichlorbenzyl)-1H-imidazol-5-yl]methanol

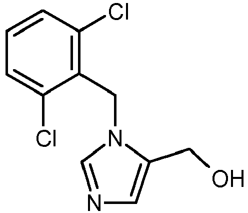

220 mg (0.74 mmol) der Verbindung aus Beispiel 32A wurden in 3 ml THF gelöst und bei 0°C tropfenweise mit 0.74 ml (0.74 mmol) einer 1 M Lösung von Lithiumaluminiumhydrid in THF versetzt. Es wurde 1 h bei RT gerührt. Zur Aufarbeitung wurde unter Eiskühlung mit 5 ml einer ges. Kaliumnatriumtartrat-Lösung versetzt, mit 10 ml Ethylacetat verdünnt und der ausgefallene Feststoff abfiltriert. Das Rohprodukt wurde chromatographisch gereinigt [Methode 19]. Man erhielt 188 mg (99% d. Th.) der Zielverbindung.
LC/MS [Methode 3]: Rₜ = 0.51 min; MS [ESIpos]: m/z = 257 und 259 (M+H)⁺.

### Beispiel 37A

### [1-(2,6-Dichlorbenzyl)-4-nitro-1H-imidazol-2-yl]methanol

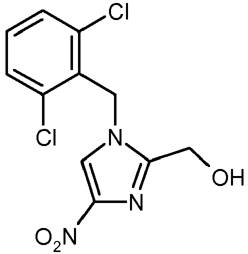

1200 mg (3.5 mmol) der Verbindung aus Beispiel 33A wurden zusammen mit 15 mg (0.35 mmol) Lithiumchlorid in 53 ml 1,2-Dimethoxyethan gelöst und bei 0°C mit 198 mg (5.23 mmol) Natriumborhydrid versetzt. Es wurde 1 h bei RT gerührt. Zur Aufarbeitung wurde unter Eiskühlung mit 25 ml einer ges. Kaliumnatriumtartrat-Lösung versetzt und mit 50 ml Ethylacetat extrahiert. Die organische Phase wurde über Natriumsulfat getrocknet, filtriert und im Vakuum eingeengt. Das Rohprodukt wurde durch Chromatographie an Kieselgel gereinigt (Eluent: Ethylacetat/Cyclohexan 1:1). Man erhielt 800 mg (76% d. Th.) der Zielverbindung.
LC/MS [Methode 5]: Rₜ = 1.82 min; MS [ESIpos]: m/z = 302 und 304 (M+H)⁺.

### Beispiel 38A

### [1-(2-Chlorphenyl)-1H-imidazol-4-yl]methanol

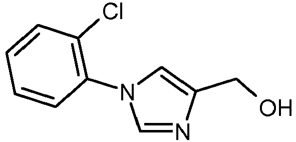

50 mg (0.21 mmol) der Verbindung aus Beispiel 34A wurden in 1 ml THF gelöst und bei -10°C mit 222 µl (0.22 mmol) einer 1 N Lösung von Lithiumaluminiumhydrid in THF versetzt. Anschließend wurde das Gemisch innerhalb einer Stunde auf RT erwärmt. Zur Aufarbeitung wurde mit 2 ml Wasser sowie 5 ml gesättigter Kaliumnatriumtartrat-Lösung versetzt und zweimal mit je 10 ml Ethylacetat extrahiert. Die vereinigten organischen Phasen wurden über Natriumsulfat getrocknet, filtriert und im Vakuum eingeengt. Man erhielt 34 mg (56% d. Th.) der Zielverbindung in 73%-iger Reinheit.
LC/MS [Methode 3]: Rₜ = 0.34 min; MS [ESIpos]: m/z = 209 (M+H)⁺.

### Beispiel 39A

### [1-(2-Chlorphenyl)-1H-pyrazol-4-yl]methanol

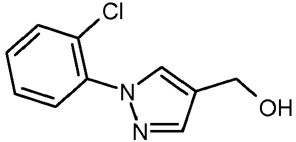

80 mg (0.32 mmol) der Verbindung aus Beispiel 35A wurden in 2 ml THF gelöst und bei -10°C mit 335 µl (0.34 mmol) einer 1 N Lösung von Lithiumaluminiumhydrid in THF versetzt. Anschließend wurde das Gemisch innerhalb einer Stunde auf RT erwärmt. Zur Aufarbeitung wurde mit 2 ml Wasser sowie 5 ml gesättigter Kaliumnatriumtartrat-Lösung versetzt und zweimal mit je 10 ml Ethylacetat extrahiert. Die vereinigten organischen Phasen wurden über Natriumsulfat getrocknet, filtriert und im Vakuum eingeengt. Man erhielt 61 mg (83% d. Th.) der Zielverbindung in 91%-iger Reinheit.
LC/MS [Methode 3]: Rₜ = 0.76 min; MS [ESIpos]: m/z = 209 (M+H)⁺.

### Beispiel 40A

### 5-(Chlormethyl)-1-(2,6-dichlorbenzyl)-1H-imidazol

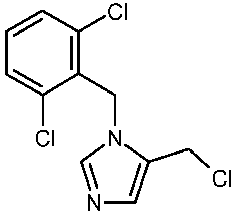

45 mg (0.18 mmol) der Verbindung aus Beispiel 36A wurden zusammen mit 27 mg (0.26 mmol) Triethylamin in 1 ml Toluol gelöst und bei RT tropfenweise mit 25 mg (0.21 mmol) Thionylchlorid versetzt. Es wurde 1 h bei RT gerührt. Das Reaktionsgemisch wurde im Vakuum von allen flüchtigen Bestandteilen befreit. Man erhielt 48 mg (99% d. Th.) der Zielverbindung, welche ohne weitere Aufreinigung weiter umgesetzt wurde.

### Beispiel 41A

### 2-(Chlormethyl)-1-(2,6-dichlorbenzyl)-4-nitro-1H-imidazol

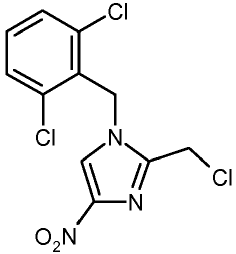

210 mg (0.70 mmol) der Verbindung aus Beispiel 37A wurden in 10 ml Dichlormethan gelöst und bei 0°C mit 145 µl (1.04 mmol) Triethylamin sowie 61 µl (0.83 mmol) Thionylchlorid versetzt. Es wurde 24 h bei RT gerührt. Anschließend wurden weitere 200 µl (2.72 mmol) Thionylchlorid hinzugefügt und das Gemisch 15 min unter Rückfluss gerührt. Danach wurde das Reaktionsgemisch im Vakuum von allen flüchtigen Bestandteilen befreit. Man erhielt in 88% Reinheit 200 mg (80% d. Th.) der Zielverbindung, welche ohne weitere Aufreinigung weiter umgesetzt wurde.
LC/MS [Methode 3]: Rₜ = 1.16 min; MS [ESIpos]: m/z = 320 und 322 (M+H)⁺
¹H-NMR (400 MHz, DMSO-d₆): δ = 5.03 (s, 2H), 5.61 (s, 2H), 7.52-7.57 (m, 1H), 7.62-7.65 (m, 2H), 7.86-7.88 (m, 1H).

### Beispiel 42A

### 4-(Brommethyl)-1-(2-chlorphenyl)-1H-imidazol

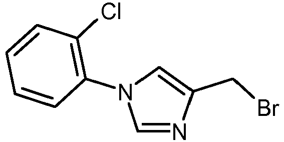

48 mg (0.16 mmol) der Verbindung aus Beispiel 38A und 63 mg (0.24 mmol) Triphenylphosphin wurden in 1.6 ml THF gelöst und bei RT mit 80 mg (0.24 mmol) Tetrabromkohlenstoff versetzt. Anschließend wurde das Gemisch 16 h bei RT gerührt. Zur Aufarbeitung wurde über 20 g Kieselgur filtriert, der Filter-Rückstand mit Ethylacetat nachgewaschen und das Filtrat im Vakuum eingeengt. Der Rückstand wurde chromatographisch an Kieselgel gereinigt (Elutionsmittel: Cyclohexan/Ethylacetat 5:1, dann 1:1). Es wurden 17 mg (39% d. Th.) der Zielverbindung erhalten, welche sofort weiter umgesetzt wurde.
LC/MS [Methode 4]: Rₜ = 0.34 min; MS [ESIpos]: m/z = 209 (M-Br+OH+H)⁺.

### Beispiel 43A

### 4-(Brommethyl)-1-(2-chlorphenyl)-1H-pyrazol

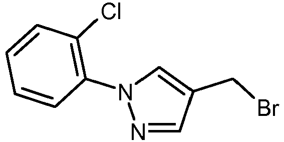

61 mg (0.27 mmol) der Verbindung aus Beispiel 39A und 105 mg (0.40 mmol) Triphenylphosphin wurden in 1.6 ml THF gelöst und bei RT mit 132 mg (0.40 mmol) Tetrabromkohlenstoff versetzt. Anschließend wurde das Gemisch 16 h bei RT gerührt. Zur Aufarbeitung wurde über 20 g Kieselgur filtriert, der Filter-Rückstand mit Ethylacetat nachgewaschen und das Filtrat im Vakuum eingeengt. Der Rückstand wurde chromatographisch an Kieselgel gereinigt (Elutionsmittel: Cyclohexan/Ethylacetat 10:1, dann 5:1). Es wurden 30 mg (42% d. Th.) der Zielverbindung erhalten, welche sofort weiter umgesetzt wurde.
LC/MS [Methode 4]: Rₜ = 0.67 min; MS [ESIpos]: m/z = 209 (M-Br+OH+H)⁺.

### Beispiel 44A

### Ethyl-2-(2-chlorphenyl)-1,3-oxazol-5-carboxylat

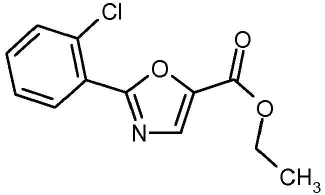

Unter Argon wurden 300 mg (1.71 mmol) Ethyl-2-brom-1,3-oxazol-5-carboxylat zusammen mit 422 mg (2.56 mmol) 2-Chlorphenylboronsäure in 7 ml Toluol gelöst und nacheinander mit 67 mg (0.17 mmol) 2-Dicyclohexylphosphino-2'-(*N,N*-dimethylamino)biphenyl, 78 mg (0.085 mmol) Tris-(dibenzylidenaceton)dipalladium sowie 725 mg (3.42 mmol) Kaliumphosphat versetzt. Das Gemisch wurde auf 110°C erhitzt und 20 h bei dieser Temperatur gerührt. Zur Aufarbeitung ließ man auf RT abkühlen und verdünnte das Reaktionsgemisch mit 20 ml Ethylacetat und 20 ml Wasser. Nach Trennen der Phasen wurde die wässrige Phase noch zweimal mit je 20 ml Ethylacetat extrahiert. Die vereinigten organischen Phasen wurden über Magnesiumsulfat getrocknet, filtriert und im Vakuum eingeengt. Der Rückstand wurde durch präparative HPLC gereinigt [Methode 19]. Es wurden 217 mg (50% d. Th.) der Zielverbindung erhalten.
LC/MS [Methode 3]: Rₜ = 1.21 min; MS [ESIpos]: m/z = 252 (M+H)⁺.

### Beispiel 45A

### Ethyl-2-(2,3-dichlorphenyl)-1,3-oxazol-5-carboxylat

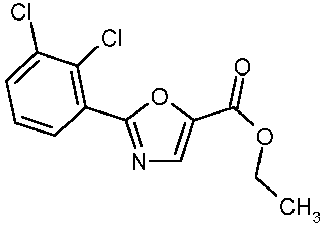

Analog zum Verfahren in Beispiel 44A wurden 300 mg (1.71 mmol) Ethyl-2-brom-1,3-oxazol-5-carboxylat umgesetzt. Es wurden 174 mg (31% d. Th.) der Zielverbindung in 87%-iger Reinheit erhalten.
LC/MS [Methode 4]: Rₜ = 1.19 min; MS [ESIpos]: m/z = 286 und 288 (M+H)⁺.

### Beispiel 46A

### Methyl-5-(2-chlorphenyl)thiophen-2-carboxylat

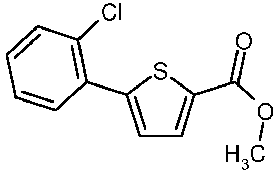

Unter Argon wurden 310 mg (1.40 mmol) Methyl-5-bromthiophen-2-carboxylat zusammen mit 328 mg (2.10 mmol) 2-Chlorphenylboronsäure in 10 ml Dioxan gelöst und mit 81 mg (0.07 mmol) Tetrakis(triphenylphosphin)palladium(0) versetzt. Das Gemisch wurde auf 110°C erhitzt, mit 1.4 ml (2.80 mmol) 2 M wässriger Natriumcarbonat-Lösung versetzt und 20 h bei dieser Temperatur gerührt. Zur Aufarbeitung ließ man auf RT abkühlen und verdünnte das Reaktionsgemisch mit 20 ml Ethylacetat und 20 ml Wasser. Nach Trennen der Phasen wurde die wässrige Phase noch zweimal mit je 20 ml Ethylacetat extrahiert. Die vereinigten organischen Phasen wurden über Natriumsulfat getrocknet, filtriert und im Vakuum eingeengt. Der Rückstand wurde chromatographisch an Kieselgel gereinigt (Elutionsmittel: Cyclohexan/Ethylacetat 15:1, dann 10:1). Es wurden 289 mg (61% d. Th.) der Zielverbindung in 75%-iger Reinheit erhalten.
LC/MS [Methode 2]: Rₜ = 2.58 min; MS [ESIpos]: m/z = 253 (M+H)⁺
¹H-NMR (400 MHz, DMSO-d₆): δ = 3.85 (s, 3H), 7.45-7.48 (m, 2H), 7.53 (d, 1H), 7.64 (dd, 1H), 7.73 (dd, 1H), 7.85 (d, 1H).

### Beispiel 47A

### Methyl-5-(2,3-dichlorphenyl)thiophen-2-carboxylat

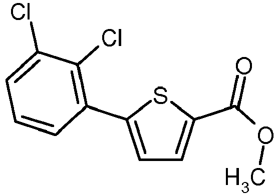

Analog zum Verfahren in Beispiel 46A wurden 300 mg (1.36 mmol) Methyl-5-bromthiophen-2-carboxylat umgesetzt. Es wurden 273 mg (58% d. Th.) der Zielverbindung in 83%-iger Reinheit erhalten.
LC/MS [Methode 4]: Rₜ = 1.30 min; MS [ESIpos]: m/z = 287 (M+H)⁺.

### Beispiel 48A

### 5-(2-Chlorphenyl)thiophen-2-carbonsäure

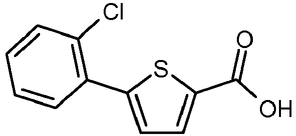

289 mg (1.14 mmol) der Verbindung aus Beispiel 46A wurden in 2 ml THF/ Methanol (1:1) gelöst und mit 1.14 ml (2.29 mmol) 2 M Natronlauge versetzt. Es wurde 2 h bei 80°C gerührt. Nach Abkühlen auf RT wurde das Solvens am Rotationsverdampfer entfernt, der Rückstand mit 5 ml Wasser aufgenommen und mit 5 ml Ethylacetat gewaschen. Die wässrige Phase wurde mit 1 N Salzsäure angesäuert und zweimal mit je 5 ml Ethylacetat extrahiert. Die vereinigten organischen Phasen wurden über Natriumsulfat getrocknet, filtriert und im Vakuum eingeengt. Es wurden in 91 % Reinheit 218 mg (73% d. Th.) der Zielverbindung erhalten.
LC/MS [Methode 2]: Rₜ = 2.16 min; MS [ESIpos]: m/z = 239 (M+H)⁺.

### Beispiel 49A

### 5-(2,3-Dichlorphenyl)thiophen-2-carbonsäure

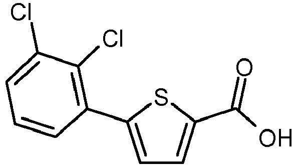

Analog zum Verfahren in Beispiel 48A wurden 273 mg (0.79 mmol) der Verbindung aus Beispiel 47A umgesetzt. Es wurden in 92% Reinheit 228 mg (97% d. Th.) der Zielverbindung erhalten.
LC/MS [Methode 4]: Rₜ = 1.07 min; MS [ESIpos]: m/z = 271 und 273 (M-H)⁻.

### Beispiel 50A

### [2-(2-Chlorphenyl)-1,3-oxazol-5-yl]methanol

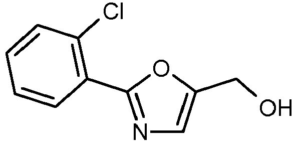

Analog zum Verfahren in Beispiel 39A wurden 217 mg (0.86 mmol) der Verbindung aus Beispiel 44A umgesetzt. Es wurden in 89% Reinheit 181 mg (89% d. Th.) der Zielverbindung erhalten.
LC/MS [Methode 4]: Rₜ = 0.75 min; MS [ESIpos]: m/z = 210 (M+H)⁺.

### Beispiel 51A

### [2-(2,3-Dichlorphenyl)-1,3-oxazol-5-yl]methanol

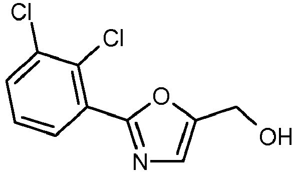

Analog zum Verfahren in Beispiel 39A wurden 186 mg (0.65 mmol) der Verbindung aus Beispiel 45A umgesetzt. Es wurden in 86% Reinheit 89 mg (48% d. Th.) der Zielverbindung erhalten.
LC/MS [Methode 4]: Rₜ = 0.87 min; MS [ESIpos]: m/z = 244 und 246 (M+H)⁺.

### Beispiel 52A

### [5-(2-Chlorphenyl)-2-thienyl]methanol

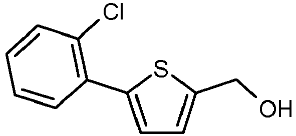

350 mg (1.47 mmol) der Verbindung aus Beispiel 48A wurden in 5 ml THF gelöst, auf 0°C abgekühlt und mit 0.20 ml (1.47 mmol) Triethylamin sowie 0.21 ml (1.61 mmol) Chlorameisensäureisobutylester versetzt. Es wurde 1 h bei 0°C gerührt. Anschließend wurde die Suspension über eine Seitz-Fritte in einen auf 0°C gekühlten Kolben filtriert und der Rückstand mit ca. 2 ml THF nachgewaschen. Das erhaltene Filtrat wurde dann unter kräftigem Rühren zu einer auf 0°C gekühlten Lösung von 166 mg (4.40 mmol) Natriumborhydrid in 2 ml Wasser gegeben. Nach 1 h wurde mit 5 ml gesättigter Natriumhydrogencarbonat-Lösung versetzt und das Gemisch auf RT erwärmt. Es wurde mit 15 ml Ethylacetat extrahiert. Die organische Phase wurde nacheinander mit je 5 ml ges. Natriumhydrogencarbonat-Lösung und ges. Natriumchlorid-Lösung gewaschen. Nach Trocknen über Natriumsulfat wurde filtriert und im Vakuum eingeengt. Das Rohprodukt wurde durch Chromatographie an Kieselgel gereinigt (Elutionsmittel: Cyclohexan/Ethylacetat 10:1, dann 5:1). Es wurden 252 mg (63% d. Th.) der Titelverbindung in 83%-iger Reinheit erhalten.
LC/MS [Methode 3]: Rₜ = 1.12 min; MS [ESIpos]: m/z = 206 (M-H₂O+H)⁺
¹H-NMR (400 MHz, DMSO-d₆): δ = 4.66 (d, 2H), 5.54 (t, 1H), 6.99 (d, 1H), 7.27 (d, 1H), 7.33-7.43 (m, 2H), 7.55-7.62 (m, 2H).

### Beispiel 53A

### [5-(2,3-Dichlorphenyl)-2-thienyl]methanol

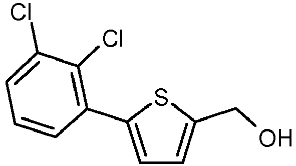

Analog zum Verfahren in Beispiel 52A wurden 268 mg (0.98 mmol) der Verbindung aus Beispiel 49A umgesetzt. Es wurden in 87% Reinheit 184 mg (63% d. Th.) der Zielverbindung erhalten.
LC/MS [Methode 4]: Rₜ = 1.10 min; MS [ESIpos]: m/z = 241 und 243 (M-H₂O+H)⁺
¹H-NMR (400 MHz, DMSO-d₆): δ = 4.67 (d, 2H), 5.58 (t, 1H), 7.01 (d, 1H), 7.29 (d, 1H), 7.39-7.44 (m, 1H), 7.55-7.58 (m, 1H), 7.62-7.66 (m, 1H).

### Beispiel 54A

### 5-(Brommethyl)-2-(2-chlorphenyl)-1,3-oxazol

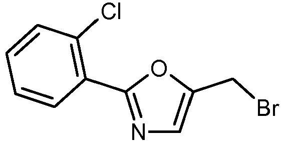

181 mg (0.77 mmol) der Verbindung aus Beispiel 50A und 242 mg (0.92 mmol) Triphenylphosphin wurden in 4 ml THF gelöst und bei RT mit 306 mg (0.92 mmol) Tetrabromkohlenstoff versetzt. Anschließend wurde das Gemisch 16 h bei RT gerührt. Zur Aufarbeitung wurde über 20 g Kieselgur filtriert, der Filter-Rückstand mit Ethylacetat nachgewaschen und das Filtrat im Vakuum eingeengt. Der Rückstand wurde durch präparative HPLC gereinigt [Methode 19]. Es wurden 112 mg (42% d. Th.) der Zielverbindung in 80%-iger Reinheit erhalten, welche sofort weiter umgesetzt wurden.
LC/MS [Methode 4]: Rₜ = 1.09 min; MS [ESIpos]: m/z = 272 und 274 (M+H)⁺.

### Beispiel 55A

### 5-(Brommethyl)-2-(2,3-dichlorphenyl)-1,3-oxazol

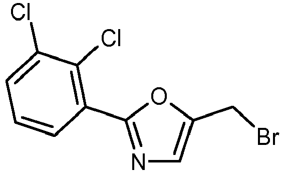

Analog zum Verfahren in Beispiel 54A wurden 89 mg (0.31 mmol) der Verbindung aus Beispiel 51A umgesetzt. Es wurden in 87% Reinheit 50 mg (52% d. Th.) der Zielverbindung erhalten.
LC/MS [Methode 4]: Rₜ = 1.15 min; MS [ESIpos]: m/z = 308 (M+H)⁺.

### Beispiel 56A

### 2-(Brommethyl)-5-(2-chlorphenyl)thiophen

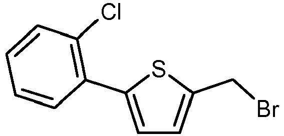

200 mg (0.74 mmol) der Verbindung aus Beispiel 52A und 291 mg (1.11 mmol) Triphenylphosphin wurden in 8 ml THF gelöst und bei RT mit 367 mg (1.11 mmol) Tetrabromkohlenstoff versetzt. Anschließend wurde das Gemisch 16 h bei RT gerührt. Zur Aufarbeitung wurde über 20 g Kieselgur filtriert, der Filter-Rückstand mit Ethylacetat nachgewaschen und das Filtrat im Vakuum eingeengt. Der Rückstand wurde chromatographisch an Kieselgel gereinigt (Elutionsmittel: Cyclohexan/Ethylacetat 10:1, dann 5:1). Es wurden 113 mg an verunreinigtem Zielprodukt isoliert (32% Reinheit, 17% d. Th.), welche sofort weiter umgesetzt wurden.
LC/MS [Methode 4]: Rₜ = 1.37 min; MS [ESIpos]: m/z = 207 (M-HBr)⁺.

### Beispiel 57A

### 2-(Brommethyl)-5-(2,3-dichlorphenyl)thiophen

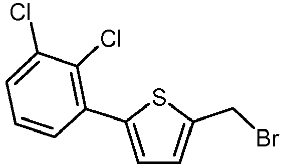

Analog zum Verfahren in Beispiel 56A wurden 89 mg (0.31 mmol) der Verbindung aus Beispiel 53A umgesetzt. Es wurden in 86% Reinheit 70 mg (30% d. Th.) der Zielverbindung erhalten.
LC/MS [Methode 4]: Rₜ = 1.08 min; MS [ESIpos]: m/z = 241 und 243 (M-HBr)⁺.

### Beispiel 58A

### Methyl-2-[2-(trifluormethyl)phenyl]isonicotinat

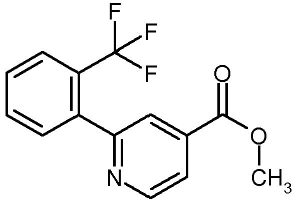

500 mg (2.31 mmol) Methyl-2-bromisonicotinat sowie 694 mg (3.47 mmol) 2-(Trifluormethyl)-phenylboronsäure wurden unter einer Argonatmosphäre in 10 ml Toluol gelöst. Anschließend fügte man 106 mg (0.12 mmol) Tris(dibenzylidenaceton)dipalladium, 91 mg (0.23 mmol) *Tri-tert.-*butylphosphin sowie 982 mg (4.63 mmol) Kaliumphosphat hinzu und erhitzte das Gemisch unter Argon für 20 h auf 110°C. Zur Aufarbeitung wurde bei RT mit 15 ml Ethylacetat und 15 ml Wasser verdünnt, die organische Phase abgetrennt und die wässrige Phase noch zweimal mit je 15 ml Ethylacetat extrahiert. Die vereinigten organischen Phasen wurden über Magnesiumsulfat getrocknet, filtriert und im Vakuum eingeengt. Das Rohprodukt wurde durch Chromatographie an Kieselgel gereinigt (Elutionsmittel: Cyclohexan/Ethylacetat 20:1, dann 10:1). Es wurden 498 mg (59% d. Th.) der Zielverbindung mit 77% Reinheit erhalten. Eine zweite Produktfraktion mit geringerer Reinheit wurde nach Methode 19 weiter aufgereinigt. Man erhielt so weitere 54 mg (8% d. Th.) der Zielverbindung.
LC/MS [Methode 2]: Rₜ = 2.21 min; MS [ESIpos]: m/z = 282 (M+H)⁺.

### Beispiel 59A

### Methyl-2-(2-chlorphenyl)isonicotinat

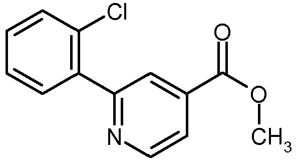

Analog zum Verfahren in Beispiel 58A wurden 500 mg (2.31 mmol) Methyl-2-bromisonicotinat und 597 mg (3.47 mmol) 2-Chlorphenylboronsäure miteinander umgesetzt. Es wurden 323 mg (56% d. Th.) der Zielverbindung erhalten.
LC/MS [Methode 3]: Rₜ = 1.15 min; MS [ESIpos]: m/z = 248 (M+H)⁺.

### Beispiel 60A

### Methyl-2-(2,3-dichlorphenyl)isonicotinat

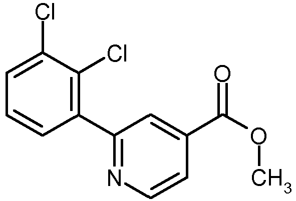

250 mg (1.16 mmol) Methyl-2-bromisonicotinat sowie 331 mg (1.74 mmol) 2,3-Dichlorphenylboronsäure wurden unter einer Argonatmosphäre in 5 ml Toluol gelöst. Anschließend fügte man 53 mg (0.06 mmol) Tris(dibenzylidenaceton)dipalladium, 46 mg (0.12 mmol) 2-Dicyclohexyl-phosphino-2'-(*N,N*-dimethylamino)biphenyl sowie 491 mg (2.31 mmol) Kaliumphosphat hinzu und erhitzte das Gemisch unter Argon für 20 h auf 110°C. Zur Aufarbeitung wurde bei RT mit 15 ml Ethylacetat und 15 ml Wasser verdünnt, die organische Phase abgetrennt und die wässrige Phase noch zweimal mit je 15 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 138 mg (42% d. Th.) der Zielverbindung mit 87% Reinheit erhalten.
LC/MS [Methode 4]: Rₜ = 1.13 min; MS [ESIpos]: m/z = 282 und 284 (M+H)⁺.

### Beispiel 61A

### {2-[2-(Trifluormethyl)phenyl]pyridin-4-yl}methanol

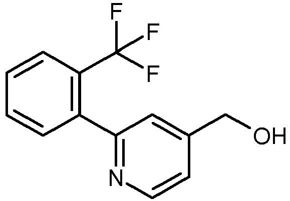

432 mg (1.54 mmol) der Verbindung aus Beispiel 58A wurden in 10 ml THF gelöst und bei -10°C mit 1.08 ml (1.08 mmol) einer 1 M Lithiumaluminiumhydrid-Lösung in THF versetzt. Nach vollständiger Zugabe wurde 2 h bei RT gerührt. Zur Aufarbeitung wurde bei RT mit 4 ml einer ges. Natriumkaliumtartrat-Lösung versetzt und mit 15 ml Ethylacetat extrahiert. Die organische Phase wurde einmal mit 10 ml ges. Natriumkaliumtartrat-Lösung gewaschen, über Magnesiumsulfat getrocknet, filtriert und im Vakuum eingeengt. Es wurden 497 mg (>100% d. Th.) der Zielverbindung erhalten, welche ohne weitere Aufreinigung weiter umgesetzt wurden.
LC/MS [Methode 2]: Rₜ = 1.40 min; MS [ESIpos]: m/z = 254 (M+H)⁺.

### Beispiel 62A

### [2-(2-Chlorphenyl)pyridin-4-yl]methanol

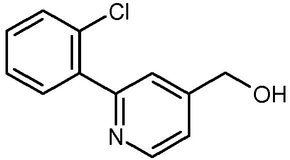

Analog zum Verfahren in Beispiel 61A wurden 323 mg (1.24 mmol) der Verbindung aus Beispiel 59A umgesetzt. Es wurden 303 mg (>100% d. Th.) der Zielverbindung erhalten, welche ohne weitere Aufreinigung weiter umgesetzt wurden.
¹H-NMR (400 MHz, DMSO-d₆): δ = 4.61 (d, 2H), 5.47 (t, 1H), 7.33-7.37 (m, 1H), 7.42-7.48 (m, 2H), 7.53-7.60 (m, 3H), 8.61 (d, 1H).

### Beispiel 63A

### [2-(2,3-Dichlorphenyl)pyridin-4-yl]methanol

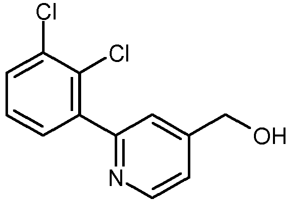

Analog zum Verfahren in Beispiel 61A wurden 138 mg (0.49 mmol) der Verbindung aus Beispiel 60A umgesetzt. Es wurden 132 mg (90% d. Th.) der Zielverbindung in 84%-iger Reinheit erhalten, welche ohne weitere Aufreinigung weiter umgesetzt wurden.
LC/MS [Methode 2]: Rₜ = 0.78 min; MS [ESIpos]: m/z = 254 und 256 (M+H)⁺.

### Beispiel 64A

### 4-(Brommethyl)-2-[2-(trifluormethyl)phenyl]pyridin

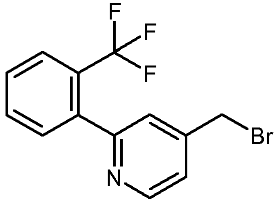

495 mg (1.96 mmol) der Verbindung aus Beispiel 61A und 615 mg (2.35 mmol) Triphenylphosphin wurden in 15 ml THF gelöst und bei RT mit 778 mg (2.35 mmol) Tetrabromkohlenstoff versetzt. Nach vollständiger Zugabe wurde 16 h bei RT gerührt. Zur Aufarbeitung wurde über 20 g Kieselgur filtriert und das Filtrat im Vakuum eingeengt. Der Rückstand wurde chromatographisch an Kieselgel gereinigt (Elutionsmittel: zunächst Cyclohexan/Ethylacetat 70:30, dann Ethylacetat). Es wurden in 90% Reinheit 149 mg (22% d. Th.) der Zielverbindung erhalten, welche sofort weiter umgesetzt wurden.
LC/MS [Methode 3]: Rₜ = 1.23 min; MS [ESIpos]: m/z = 318 (M+H)⁺.

### Beispiel 65A

### 4-(Brommethyl)-2-(2-chlorphenyl)pyridin

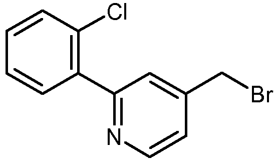

Analog zum Verfahren in Beispiel 64A wurden 294 mg (1.34 mmol) der Verbindung aus Beispiel 62A umgesetzt. Es wurden in 89% Reinheit 242 mg (57% d. Th.) der Zielverbindung erhalten, welche sofort weiter umgesetzt wurden.
LC/MS [Methode 4]: Rₜ = 1.08 min; MS [ESIpos]: m/z = 282 und 284 (M+H)⁺.

### Beispiel 66A

### 4-(Brommethyl)-2-(2,3-dichlorphenyl)pyridin

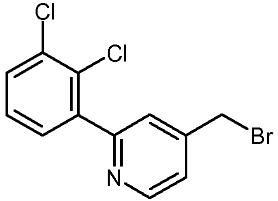

Analog zum Verfahren in Beispiel 54A wurden 131 mg (0.52 mmol) der Verbindung aus Beispiel 63A umgesetzt. Es wurden 81 mg (50% d. Th.) der Zielverbindung erhalten, welche sofort weiter umgesetzt wurden.
LC/MS [Methode 4]: Rₜ = 1.08 min; MS [ESIpos]: m/z = 316, 318 und 320 (M+H)⁺.

### Beispiel 67A

### 4-Methyl-2-[2-(trifluormethyl)phenyl]pyrimidin

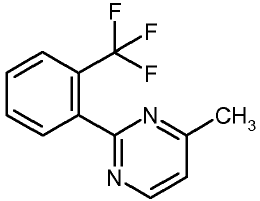

250 mg (1.95 mmol) 2-Chlor-4-methylpyrimidin sowie 583 mg (2.92 mmol) 2-(Trifluormethyl)-phenylboronsäure wurden unter einer Argonatmosphäre in 8 ml Toluol gelöst. Anschließend fügte man 89 mg (0.10 mmol) Tris(dibenzylidenaceton)dipalladium, 77 mg (0.19 mmol) 2-Dicyclohexyl-phosphino-2'-(*N,N*-dimethylamino)biphenyl sowie 826 mg (3.89 mmol) Kaliumphosphat hinzu und erhitzte das Gemisch unter Argon für 20 h auf 110°C. Zur Aufarbeitung wurde bei RT mit 15 ml Ethylacetat und 15 ml Wasser verdünnt, die organische Phase abgetrennt und die wässrige Phase noch zweimal mit je 15 ml Ethylacetat extrahiert. Die vereinigten organischen Phasen wurden über Magnesiumsulfat getrocknet, filtriert und im Vakuum eingeengt. Das Rohprodukt wurde chromatographisch an Kieselgel gereinigt (Elutionsmittel: Cyclohexan/Ethylacetat 9:1, dann 4:1). Es wurden 249 mg (54% d. Th.) der Zielverbindung mit 87% Reinheit erhalten.
LC/MS [Methode 3]: Rₜ = 1.02 min; MS [ESIpos]: m/z = 239 (M+H)⁺.

### Beispiel 68A

### 2-(2-Chlorphenyl)-4-methylpyrimidin

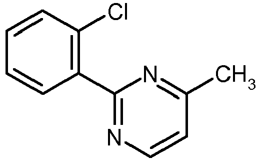

Analog zum Verfahren in Beispiel 67A wurden 250 mg (1.95 mmol) 2-Chlor-4-methylpyrimidin umgesetzt. Es wurden 202 mg (34% d. Th.) der Zielverbindung in 66%-iger Reinheit erhalten.
LC/MS [Methode 3]: Rₜ = 0.90 min; MS [ESIpos]: m/z = 205 (M+H)⁺.

### Beispiel 69A

### 4-Methyl-6-[2-(trifluormethyl)phenyl]pyrimidin

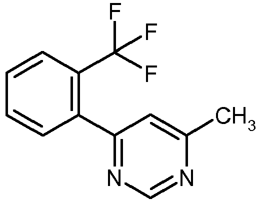

Analog zum Verfahren in Beispiel 67A wurden 250 mg (1.95 mmol) 4-Chlor-6-methylpyrimidin umgesetzt. Es wurden 332 mg (70% d. Th.) der Zielverbindung erhalten.
LC/MS [Methode 3]: Rₜ = 1.04 min; MS [ESIpos]: m/z = 239 (M+H)⁺.

### Beispiel 70A

### 4-Methyl-6-[2-chlorphenyl]pyrimidin

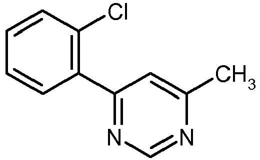

Analog zum Verfahren in Beispiel 67A wurden 250 mg (1.95 mmol) 4-Chlor-6-methylpyrimidin umgesetzt. Es wurden 191 mg (43% d. Th.) der Zielverbindung erhalten.
LC/MS [Methode 3]: Rₜ = 0.99 min; MS [ESIpos]: m/z = 205 (M+H)⁺.

### Beispiel 71A

### 4-(2,3-Dichlorphenyl)-6-methylpyrimidin

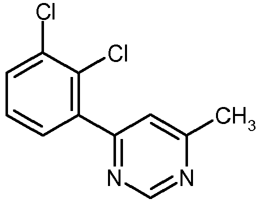

Analog zum Verfahren in Beispiel 67A wurden 250 mg (1.95 mmol) 4-Chlor-6-methylpyrimidin umgesetzt. Es wurden 185 mg (40% d. Th.) der Zielverbindung erhalten.
LC/MS [Methode 6]: Rₜ = 1.93 min; MS [ESIpos]: m/z = 239 und 241 (M+H)⁺.

### Beispiel 72A

### 2-Methyl-5-[2-(trifluormethyl)phenyl]-1,3,4-thiadiazol

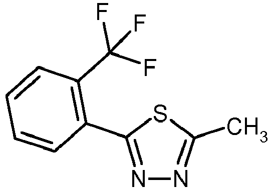

Analog zum Verfahren in Beispiel 67A wurden 500 mg (1.95 mmol) 2-Brom-5-methyl-1,3,4-thiadiazol umgesetzt. Es wurden 331 mg (36% d. Th.) der Zielverbindung in 75%-iger Reinheit erhalten.
LC/MS [Methode 4]: Rₜ = 0.93 min; MS [ESIpos]: m/z = 245 (M+H)⁺.

### Beispiel 73A

### 2-(2-Chlorphenyl)-5-methyl-1,3,4-thiadiazol

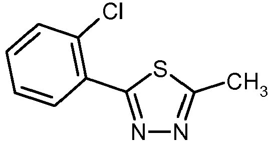

Analog zum Verfahren in Beispiel 44A wurden 315 mg (1.76 mmol) 2-Brom-5-methyl-1,3,4-thiadiazol umgesetzt. Es wurden 155 mg (36% d. Th.) der Zielverbindung erhalten.
LC/MS [Methode 4]: Rₜ = 0.92 min; MS [ESIpos]: m/z = 211 (M+H)⁺
¹H-NMR (400 MHz, DMSO-d₆): δ = 2.82 (s, 3H), 7.52-7.62 (m, 2H), 7.70 (dd, 1H), 8.10 (dd, 1H).

### Beispiel 74A

### 2-(2,3-Dichlorphenyl)-5-methyl-1,3,4-thiadiazol

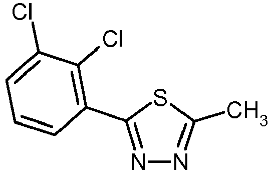

Analog zum Verfahren in Beispiel 44A wurden 310 mg (1.73 mmol) 2-Brom-5-methyl-1,3,4-thiadiazol umgesetzt. Es wurden 91 mg (21% d. Th.) der Zielverbindung erhalten.
LC/MS [Methode 6]: Rₜ = 2.13 min; MS [ESIpos]: m/z = 245 und 247 (M+H)⁺.

### Beispiel 75A

### 5-(2-Chlorphenyl)-2-methyl-1,3-thiazol

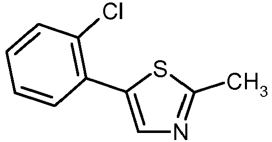

Analog zum Verfahren in Beispiel 67A wurden 756 mg (2.92 mmol) 5-Brom-2-methyl-1,3-thiazol-Hydrobromid umgesetzt. Hierbei wurden als Base 2.49 g (11.68 mmol) Kaliumphosphat eingesetzt. Es wurden 181 mg (30% d. Th.) der Zielverbindung erhalten.
LC/MS [Methode 2]: Rₜ = 2.21 min; MS [ESIpos]: m/z = 210 (M+H)⁺.

### Beispiel 76A

### 2-Methyl-5-[2-(trifluormethyl)phenyl]-1,3-thiazol

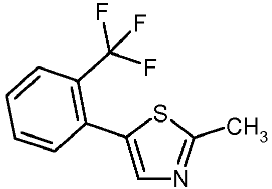

Analog zum Verfahren in Beispiel 44A wurden 430 mg (1.66 mmol) 5-Brom-2-methyl-1,3-thiazol-Hydrobromid umgesetzt. Hierbei wurden als Base 1.41 g (6.64 mmol) Kaliumphosphat eingesetzt. Es wurden 52 mg (13% d. Th.) der Zielverbindung erhalten.
LC/MS [Methode 4]: Rₜ = 1.08 min; MS [ESIpos]: m/z = 244 (M+H)⁺.

### Beispiel 77A

### 5-(3-Chlor-2-fluorphenyl)-2-methyl-1,3-thiazol

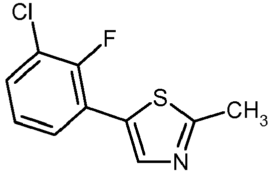

Analog zum Verfahren in Beispiel 44A wurden 586 mg (2.26 mmol) 5-Brom-2-methyl-1,3-thiazol-Hydrobromid umgesetzt. Hierbei wurden als Base 1.92 g (9.05 mmol) Kaliumphosphat eingesetzt. Es wurden 147 mg (29% d. Th.) der Zielverbindung erhalten.
LC/MS [Methode 3]: Rₜ = 1.27 min; MS [ESIpos]: m/z = 228 (M+H)⁺.

### Beispiel 78A

### 5-[2-Fluor-3-(trifluormethyl)phenyl]-2-methyl-1,3-thiazol

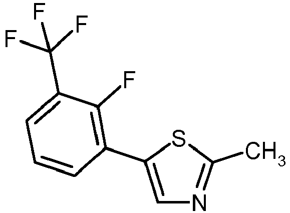

Analog zum Verfahren in Beispiel 44A wurden 350 mg (1.35 mmol) 5-Brom-2-methyl-1,3-thiazol-Hydrobromid umgesetzt. Hierbei wurden als Base 1.15 g (5.41 mmol) Kaliumphosphat eingesetzt. Die Reaktionszeit betrug 2 h. Es wurden 89 mg (25% d. Th.) der Zielverbindung erhalten.
LC/MS [Methode 4]: Rₜ = 1.12 min; MS [ESIpos]: m/z = 262 (M+H)⁺.

### Beispiel 79A

### 5-(2-Chlorphenyl)-2-methyl-4-(trifluormethyl)-1,3-thiazol

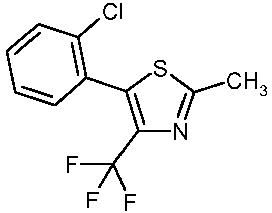

Analog zum Verfahren in Beispiel 44A wurden 635 mg (1.94 mmol) 5-Brom-2-methyl-4-(trifluormethyl)-1,3-thiazol-Hydrobromid umgesetzt. Hierbei wurden als Base 1.15 g (5.41 mmol) Kaliumphosphat eingesetzt. Es wurden 142 mg (26% d. Th.) der Zielverbindung erhalten.
LC/MS [Methode 3]: Rₜ = 1.34 min; MS [ESIpos]: m/z = 278 (M+H)⁺.

### Beispiel 80A

### 4-(Brommethyl)-2-[2-(trifluormethyl)phenyl]pyrimidin

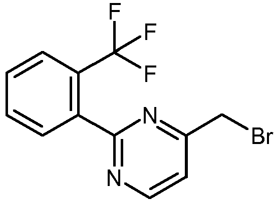

247 mg (1.04 mmol) der Verbindung aus Beispiel 67A wurden zusammen mit 185 mg (1.04 mmol) *N*-Bromsuccinimid und 17 mg (0.10 mmol) 2,2'-Azobis-2-methylpropannitril in 3 ml Tetrachlorkohlenstoff 18 h unter Rückfluss erhitzt. Zur Aufarbeitung wurde auf RT abgekühlt und mit 10 ml Dichlormethan versetzt. Es wurde mit 5 ml Wasser gewaschen und die wässrige Phase zweimal mit je 5 ml Dichlormethan rückextrahiert. Die vereinigten organischen Phasen wurden über Magnesiumsulfat getrocknet, filtriert und im Vakuum eingeengt. Das Rohprodukt wurde kurz im Hochvakuum getrocknet und ohne weitere Reinigung weiter umgesetzt. Es wurden 303 mg an Produkt erhalten, welches die Zielverbindung in 20%-iger Reinheit enthielt (entsprechend 20% d. Th.). Hauptkomponente des Rohproduktes war nicht umgesetztes Ausgangsmaterial (Beispiel 67A).
LC/MS [Methode 4]: Rₜ = 1.02 min; MS [ESIpos]: m/z = 317 und 319 (M+H)⁺.

### Beispiel 81A

### 4-(Brommethyl)-2-(2-chlorphenyl)pyrimidin

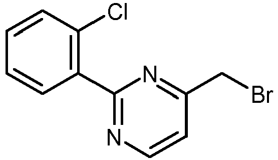

Analog zum Verfahren in Beispiel 80A wurden 200 mg (0.98 mmol) der Verbindung aus Beispiel 68A umgesetzt. Es wurden 259 mg (19% d. Th.) der Zielverbindung in ca. 20%-iger Reinheit erhalten. Hauptkomponente des Rohproduktes war nicht umgesetztes Ausgangsmaterial (Beispiel 68A).
LC/MS [Methode 4]: Rₜ = 1.00 min; MS [ESIpos]: m/z = 283 und 285 (M+H)⁺.

### Beispiel 82A

### 4-(Brommethyl)-6-[2-(trifluormethyl)phenyl]pyrimidin

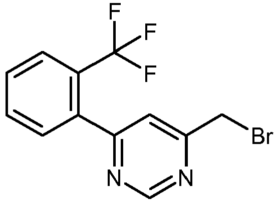

Analog zum Verfahren in Beispiel 80A wurden 332 mg (1.39 mmol) der Verbindung aus Beispiel 69A umgesetzt. Das Rohprodukt wurde chromatographisch gereinigt [Methode 19]. Es wurden 37 mg (8% d. Th.) der Zielverbindung erhalten.
LC/MS [Methode 3]: Rₜ = 1.19 min; MS [ESIpos]: m/z = 317 und 319 (M+H)⁺.

### Beispiel 83A

### 4-(Brommethyl)-6-[2-chlorphenyl]pyrimidin

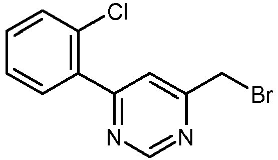

Analog zum Verfahren in Beispiel 80A wurden 191 mg (0.93 mmol) der Verbindung aus Beispiel 70A umgesetzt. Das Rohprodukt wurde chromatographisch gereinigt [Methode 19]. Es wurden 27 mg (10% d. Th.) der Zielverbindung erhalten.
LC/MS [Methode 3]: Rₜ = 1.15 min; MS [ESIpos]: m/z = 283 und 285 (M+H)⁺.

### Beispiel 84A

### 4-(Brommethyl)-6-(2,3-dichlorphenyl)pyrimidin

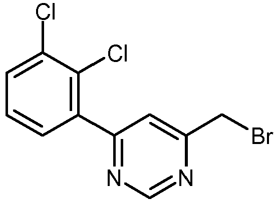

Analog zum Verfahren in Beispiel 80A wurden 185 mg (0.93 mmol) der Verbindung aus Beispiel 71A umgesetzt. Das Rohprodukt wurde chromatographisch gereinigt [Methode 19]. Es wurden 23 mg (9% d. Th.) der Zielverbindung erhalten.
LC/MS [Methode 6]: Rₜ = 2.32 min; MS [ESIpos]: m/z = 317, 319 und 321 (M+H)⁺.

### Beispiel 85A

### 2-(Brommethyl)-5-[2-(trifluormethyl)phenyl]-1,3,4-thiadiazol

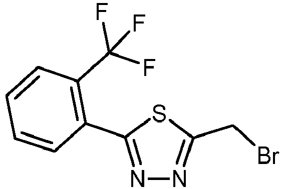

172 mg (0.70 mmol) der Verbindung aus Beispiel 72A wurden zusammen mit 251 mg (1.41 mmol) *N*-Bromsuccinimid und 12 mg (0.07 mmol) 2,2'-Azobis-2-methylpropannitril in 5 ml Tetrachlorkohlenstoff 8 h unter Rückfluss erhitzt. Zur Aufarbeitung wurde auf RT abgekühlt und mit 10 ml Dichlormethan versetzt. Es wurde mit 5 ml Wasser gewaschen und die wässrige Phase zweimal mit je 5 ml Dichlormethan rückextrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 33 mg (15% d. Th.) der Zielverbindung erhalten.
LC/MS [Methode 4]: Rₜ = 1.05 min; MS [ESIpos]: m/z = 323 und 325 (M+H)⁺.

### Beispiel 86A

### 2-(Brommethyl)-5-(2-chlorphenyl)-1,3,4-thiadiazol

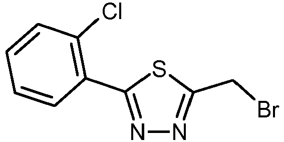

Analog zum Verfahren in Beispiel 85A wurden 185 mg (0.88 mmol) der Verbindung aus Beispiel 73A umgesetzt. Das Rohprodukt wurde chromatographisch gereinigt [Methode 19]. Es wurden 31 mg (12% d. Th.) der Zielverbindung erhalten.
LC/MS [Methode 6]: Rₜ = 2.21 min; MS [ESIpos]: m/z = 289 und 291 (M+H)⁺.

### Beispiel 87A

### 2-(Brommethyl)-5-(2,3-dichlorphenyl)-1,3,4-thiadiazol

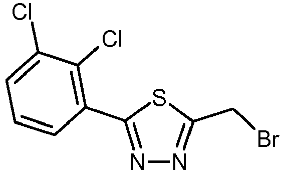

Analog zum Verfahren in Beispiel 85A wurden 91 mg (0.37 mmol) der Verbindung aus Beispiel 74A umgesetzt. Das Rohprodukt wurde chromatographisch gereinigt [Methode 19]. Es wurden 38 mg (32% d. Th.) der Zielverbindung erhalten.
LC/MS [Methode 3]: Rₜ = 1.30 min; MS [ESIpos]: m/z = 323, 325 und 327 (M+H)⁺.

### Beispiel 88A

### 2-(Brommethyl)-5-(2-chlorphenyl)-1,3-thiazol

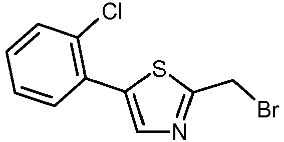

180 mg (0.86 mmol) der Verbindung aus Beispiel 75A wurden zusammen mit 229 mg (1.29 mmol) *N*-Bromsuccinimid und 14 mg (0.09 mmol) 2,2'-Azobis-2-methylpropannitril in 5 ml Tetrachlorkohlenstoff 8 h unter Rückfluss erhitzt. Zur Aufarbeitung wurde auf RT abgekühlt und mit 10 ml Dichlormethan versetzt. Es wurde mit 5 ml Wasser gewaschen und die wässrige Phase zweimal mit je 5 ml Dichlormethan rückextrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 66 mg (27% d. Th.) der Zielverbindung erhalten.
LC/MS [Methode 2]: Rₜ = 2.42 min; MS [ESIpos]: m/z = 288 und 290 (M+H)⁺.

### Beispiel 89A

### 2-(Brommethyl)-5-[2-(trifluormethyl)phenyl]-1,3-thiazol

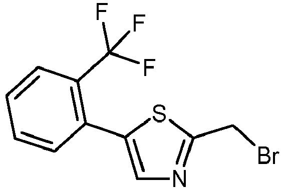

Analog zum Verfahren in Beispiel 88A wurden 110 mg (0.45 mmol) der Verbindung aus Beispiel 76A umgesetzt. Das Rohprodukt wurde chromatographisch gereinigt [Methode 19]. Es wurden 40 mg (27% d. Th.) der Zielverbindung erhalten.
LC/MS [Methode 4]: Rₜ = 1.17 min; MS [ESIpos]: m/z = 322 und 324 (M+H)⁺.

### Beispiel 90A

### 2-(Brommethyl)-5-(3-chlor-2-fluorphenyl)-1,3-thiazol

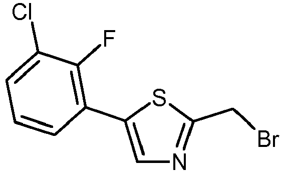

Analog zum Verfahren in Beispiel 88A wurden 142 mg (0.62 mmol) der Verbindung aus Beispiel 77A umgesetzt. Das Rohprodukt wurde chromatographisch gereinigt [Methode 19]. Es wurden 70 mg (37% d. Th.) der Zielverbindung erhalten.
LC/MS [Methode 4]: Rₜ = 1.21 min; MS [ESIpos]: m/z = 306 und 308 (M+H)⁺.

### Beispiel 91A

### 2-(Brommethyl)-5-[2-fluor-3-(trifluormethyl)phenyl]-1,3-thiazol

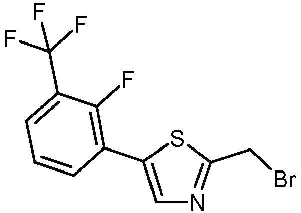

Analog zum Verfahren in Beispiel 88A wurden 120 mg (0.46 mmol) der Verbindung aus Beispiel 78A umgesetzt. Das Rohprodukt wurde chromatographisch gereinigt [Methode 19]. Es wurden 60 mg (38% d. Th.) der Zielverbindung erhalten.
LC/MS [Methode 4]: Rₜ = 1.20 min; MS [ESIpos]: m/z = 340 und 342 (M+H)⁺.

### Beispiel 92A

### 2-(Brommethyl)-5-(2-chlorphenyl)-4-(trifluormethyl)-1,3-thiazol

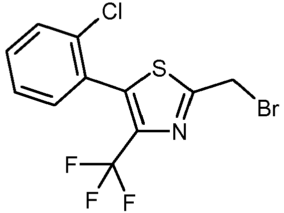

Analog zum Verfahren in Beispiel 88A wurden 140 mg (0.50 mmol) der Verbindung aus Beispiel 79A umgesetzt. Das Rohprodukt wurde chromatographisch gereinigt [Methode 19]. Es wurden 49 mg (27% d. Th.) der Zielverbindung erhalten.
LC/MS [Methode 4]: Rₜ = 1.27 min; MS [ESIpos]: m/z = 356 und 358 (M+H)⁺.

### Beispiel 93A

### 5-(4-Chlorphenyl)-4-cyclopropyl-2-(1H-1,2,4-triazol-5-ylsulfonyl)-2,4-dihydro-3H-1,2,4-triazol-3-on

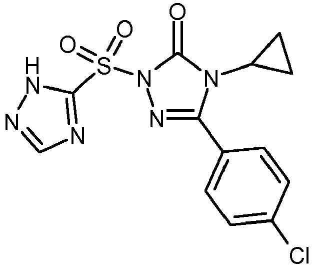

300 mg (1.27 mmol) 5-(4-Chlorphenyl)-4-cyclopropyl-2,4-dihydro-3*H*-1,2,4-triazol-3-on [Herstellung gemäß WO 2007/134862 Beispiel 36A] wurden in 10 ml THF gelöst und bei -78°C mit 143 mg (1.27 mmol) Kalium-tert.-butylat versetzt. Das Reaktionsgemisch wurde innerhalb von 30 min auf RT erwärmt und bei dieser Temperatur weitere 20 min gerührt. Anschließend wurde das Gemisch erneut auf -78°C gekühlt und mit 213 mg (1.27 mmol) 1*H*-1,2,4-Triazol-5-sulfonylchlorid, gelöst in 5 ml THF, versetzt. Das Reaktionsgemisch wurde innerhalb von 30 min auf RT erwärmt und bei dieser Temperatur weitere 20 h gerührt. Zur Aufarbeitung wurde mit 10 ml Wasser versetzt. Es wurde zweimal mit je 15 ml Ethylacetat extrahiert. Die vereinigten organischen Phasen wurden über Natriumsulfat getrocknet, filtriert und im Vakuum eingeengt. Das Rohprodukt wurde chromatograpisch gereinigt [Methode 19]. Es wurden 136 mg (29% d. Th.) der Zielverbindung erhalten.
LC/MS [Methode 5]: Rₜ = 1.91 min; m/z = 367 (M+H)⁺.

### Beispiel 94A

### 5-(4-Chlorphenyl)-4-(4-methoxybenzyl)-2-(1H-1,2,4-triazol-5-ylsulfonyl)-2,4-dihydro-3H-1,2,4-triazol-3-on

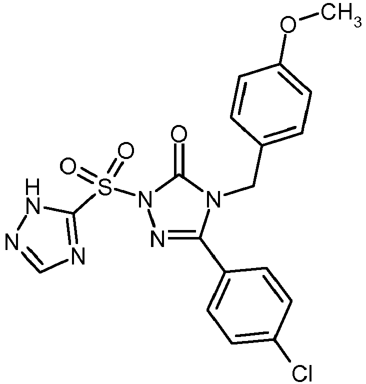

529 mg (1.68 mmol) 5-(4-Chlorphenyl)-4-(4-methoxybenzyl)-2,4-dihydro-3*H*-1,2,4-triazol-3-on [Herstellung gemäß WO 2007/134862 Beispiel 55A] wurden in 10 ml Acetonitril gelöst und nacheinander mit 1.09 g (3.35 mmol) Cäsiumcarbonat sowie 281 mg (1.68 mmol) 1*H*-1,2,4-Triazol-5-sulfonylchlorid, gelöst in 5 ml Acetonitril, versetzt. Das Reaktionsgemisch wurde 90 min bei RT gerührt. Zur Aufarbeitung wurde mit 10 g Kieselgel versetzt und das Solvens im Vakuum entfernt. Das auf Kieselgel adsorbierte Rohprodukt wurde durch Chromatographie an Kieselgel gereinigt (Elutionsmittel: zunächst Ethylacetat, dann Dichlormethan/Methanol 90:10 → 80:20). Es wurden 268 mg (32% d. Th.) der Zielverbindung erhalten.
LC/MS [Methode 5]: Rₜ = 2.21 min; m/z = 447 (M+H)⁺.

### Beispiel 95A

### 5-(4-Chlorphenyl)-2-({1-[2-(trifluormethyl)benzyl]-1H-1,2,4-triazol-5-yl}sulfonyl)-2,4-dihydro-3H-1,2,4-triazol-3-on

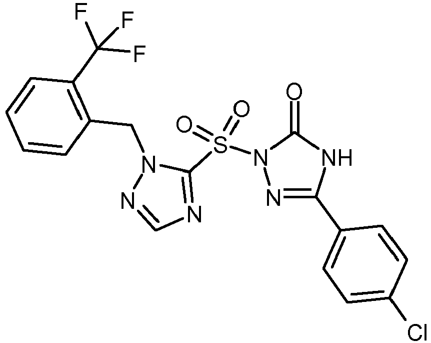

230 mg (0.38 mmol) der Verbindung aus Beispiel 116 wurden in 5 ml Acetonitril gelöst und mit 417 mg (0.76 mmol) Ammoniumcer(IV)nitrat, gelöst in 5 ml Wasser, versetzt. Anschließend wurde das Gemisch 20 h bei 70°C gerührt. Zur Aufarbeitung wurde im Vakuum eingeengt und der Rückstand in 15 ml Wasser aufgenommen und zweimal mit je 15 ml Ethylacetat extrahiert. Die vereinigten organischen Phasen wurden über Magnesiumsulfat getrocknet, filtriert und im Vakuum eingeengt. Das Rohprodukt wurde durch Chromatographie an Kieselgel gereinigt (Elutionsmittel: Dichlormethan/Methanol 99:1 → 90:10). Es wurden 130 mg (70% d. Th.) der Zielverbindung erhalten.
LC/MS [Methode 3]: Rₜ = 1.22 min; m/z = 485 (M+H)⁺
¹H-NMR (400 MHz, CDCl₃): δ = 5.59 (s, 2H), 7.38 (d, 1H), 7.44 (d, 2H), 7.45-7.55 (m, 2H), 7.70 (d, 1H), 7.81 (d, 2H), 8.12 (s, 1H).

### Beispiel 96A

### 4-Allyl-5-(4-chlorphenyl)-2-[(5-methyl-1H-imidazol-4-yl)methyl]-2,4-dihydro-3H-1,2,4-triazol-3-on

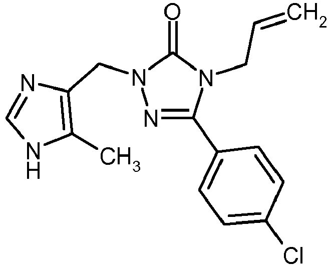

100 mg (0.89 mmol) 4-Hydroxymethyl-5-methyl-1*H*-imidazol, 252 mg (1.07 mmol) der Verbindung aus Beispiel 12A sowie 370 mg (2.68 mmol) Kaliumcarbonat wurden in 4.5 ml DMF und 4.5 ml Wasser gelöst und 75 min im Mikrowellenofen bei 200°C gerührt. Nach Abkühlen auf RT wurde zur Aufarbeitung mit 10 ml Wasser verdünnt und das Gemisch zweimal mit je 15 ml Ethylacetat extrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: zunächst Cyclohexan/Ethylacetat 1:1, dann Dichlormethan/Methanol 10:1). Man erhielt 153 mg (52% d. Th.) der Zielverbindung in 73%-iger Reinheit.
LC/MS [Methode 3]: Rₜ = 0.82 min; MS [ESIpos]: m/z = 330 (M+H)⁺.

### Beispiel 97A

### 5-(4-Chlorphenyl)-4-cyclopropyl-2-[(5-methyl-1H-imidazol-4-yl)methyl]-2,4-dihydro-3H-1,2,4-triazol-3-on

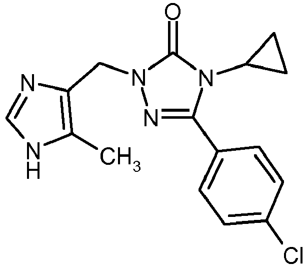

Analog zur Verbindung in Beispiel 96A wurden 181 mg (1.62 mmol) 4-Hydroxymethyl-5-methyl-1*H*-imidazol, 381 mg (1.62 mmol) 5-(4-Chlorphenyl)-4-cyclopropyl-2,4-dihydro-3*H*-1,2,4-triazol-3-on [Herstellung gemäß WO 2007/134862 Beispiel 36A] sowie 670 mg (4.85 mmol) Kaliumcarbonat miteinander umgesetzt. Man erhielt 180 mg (47% d. Th.) der Zielverbindung in 76%-iger Reinheit.
LC/MS [Methode 1]: Rₜ = 0.82 min; MS [ESIpos]: m/z = 330 (M+H)⁺.

### Beispiel 98A

### Ethyl-brom[2-(trifluormethyl)phenyl]acetat

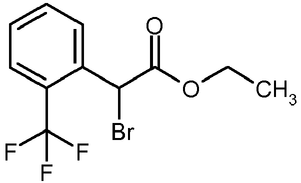

585 mg (3.89 mmol) Natriumbromat wurden in 2 ml Wasser vorgelegt und bei RT mit 300 mg (1.29 mmol) Ethyl-2-(trifluormethyl)phenylacetat, gelöst in 2.5 ml Ethylacetat, versetzt. Anschließend gab man langsam eine Lösung von 403 mg (3.89 mmol) Natriumhydrogensulfit in 3.8 ml Wasser hinzu. Es wurde 18 h bei RT gerührt. Das Reaktionsgemisch wurde dann mit 5 ml 10%-iger wässriger Natriumdithionit-Lösung versetzt. Es wurde mit 15 ml Ethylacetat extrahiert und die organische Phase je einmal mit 5 ml 10%-iger Natriumdithionit-Lösung und 5 ml gesättigter Natriumchlorid-Lösung gewaschen. Die organische Phase wurde über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 20:1, dann 10:1). Es wurden 186 mg eines Gemisches bestehend aus der Titelverbindung und dem Ausgangsmaterial Ethyl-2-(trifluormethyl)phenylacetat erhalten (Verhältnis 16:84 nach GC/MS [Methode 20]). Dieses Gemisch wurde erneut mit 362 mg (2.40 mmol) Natriumbromat und 250 mg (2.40 mmol) Natriumhydrogensulfit der oben beschriebenen Vorschrift folgend umgesetzt. Nach Aufarbeitung wurde ein Gemisch aus 31% Titelverbindung und 69% Ethyl-2-(trifluormethyl)phenylacetat erhalten, welches ohne weitere Reinigung weiter umgesetzt wurde.
GC/MS [Methode 20]: Rₜ = 4.28 min; MS [ESIpos]: m/z = 237 (M-CO₂C₂H₅)⁺.

### Beispiel 99A

### 2-(2-Brombenzyl)-5-(4-chlorphenyl)-4-cyclopropyl-2,4-dihydro-3H-1,2,4-triazol-3-on

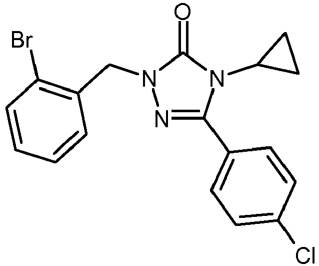

1.04 g (4.41 mmol) 5-(4-Chlorphenyl)-4-cyclopropyl-2,4-dihydro-3*H*-1,2,4-triazol-3-on [Herstellung gemäß WO 2007/134862 Beispiel 36A] und 2.16 g (6.62 mmol) Cäsiumcarbonat wurden in 35 ml Acetonitril suspendiert und mit 1.32 g (5.30 mmol) 2-Brombenzylbromid versetzt. Die Mischung wurde 18 h unter Rückfluss gerührt. Der ausgefallene Feststoff wurde danach abfiltriert und das Filtrat im Vakuum eingeengt. Der zurückbleibende Feststoff wurde in ca. 50 ml Diethylether verrührt, dann abfiltriert und mit wenig Diethylether gewaschen. Nach Trocknen im Vakuum erhielt man 1.05 g (59% d. Th.) der Zielverbindung als weißen Feststoff.
LC/MS [Methode 4]: Rₜ = 1.19 min; MS [ESIpos]: m/z = 404 und 406 (M+H)⁺
¹H-NMR (400 MHz, DMSO-d₆): δ = 0.58-0.65 (m, 2H), 0.85-0.93 (m, 2H), 3.20 (tt, 1H), 4.99 (s, 2H), 7.18-7.30 (m, 2H), 7.34-7.41 (m, 1H), 7.56-7.61 (m, 2H), 7.65 (d, 1H), 7.80 (d, 2H).

### Beispiel 100A

### 2-(5-Brom-2-fluorbenzyl)-5-(4-chlorphenyl)-4-cyclopropyl-2,4-dihydro-3H-1,2,4-triazol-3-on

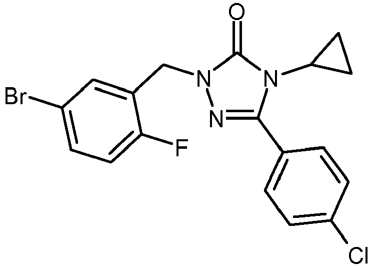

300 mg (1.27 mmol) 5-(4-Chlorphenyl)-4-cyclopropyl-2,4-dihydro-3*H*-1,2,4-triazol-3-on [Herstellung gemäß WO 2007/134862 Beispiel 36A] und 622 mg (1.91 mmol) Cäsiumcarbonat wurden in 5 ml Acetonitril suspendiert und mit 536 mg (1.40 mmol) 4-Brom-2-(brommethyl)-1-fluorbenzol versetzt. Die Mischung wurde 18 h unter Rückfluss gerührt. Der ausgefallene Feststoff wurde danach abfiltriert und das Filtrat im Vakuum auf ein Volumen von ca. 1.5 ml eingeengt. Nach Zugabe von 0.5 ml 1 N Salzsäure wurde das Gemisch direkt chromatographisch aufgereinigt [Methode 19]. Es wurden 427 mg (56% d. Th.) der Zielverbindung in einer Reinheit von 71% erhalten.
LC/MS [Methode 4]: Rₜ = 1.23 min; MS [ESIpos]: m/z = 422 und 424 (M+H)⁺.

### Beispiel 101A

### 2-(3-Brombenzyl)-5-(4-chlorphenyl)-4-(3,3,3-trifluor-2-hydroxypropyl)-2,4-dihydro-3H-1,2,4-triazol-3-on

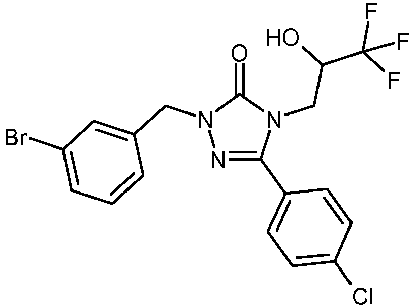

400 mg (1.30 mmol) der Verbindung aus Beispiel 4A und 635 mg (1.95 mmol) Cäsiumcarbonat wurden in 3 ml Acetonitril suspendiert und mit 357 mg (1.43 mmol) 3-Brombenzylbromid versetzt. Die Mischung wurde 20 h unter Rückfluss gerührt. Der ausgefallene Feststoff wurde danach abfiltriert, das Filtrat im Vakuum auf ein Volumen von ca. 1.5 ml eingeengt und direkt chromatographisch aufgereinigt [Methode 19]. Es wurden 507 mg (82% d. Th.) der Zielverbindung erhalten.
LC/MS [Methode 4]: Rₜ = 1.21 min; MS [ESIpos]: m/z = 476 und 478 (M+H)⁺.

### Beispiel 102A

### 2-(3-Brombenzyl)-5-(4-chlorphenyl)-4-[(2S)-3,3,3-trifluor-2-hydroxypropyl]-2,4-dihydro-3H-1,2,4-triazol-3-on

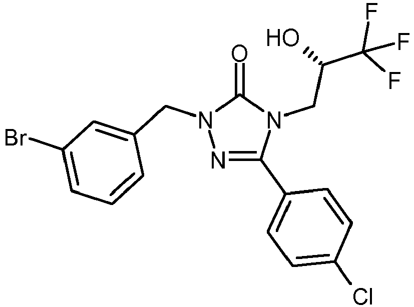

Analog zur Herstellung von Beispiel 101A wurden 80 mg (0.26 mmol) der Verbindung aus Beispiel 5A umgesetzt. Man erhielt 95 mg (76% d. Th.) der Zielverbindung.
LC/MS [Methode 4]: Rₜ = 1.21 min; MS [ESIpos]: m/z = 476 und 478 (M+H)⁺.

### Beispiel 103A

### 2-(3-Brom-5-fluorbenzyl)-5-(4-chlorphenyl)-4-[(2S)-3,3,3-trifluor-2-hydroxypropyl]-2,4-dihydro-3H-1,2,4-triazol-3-on

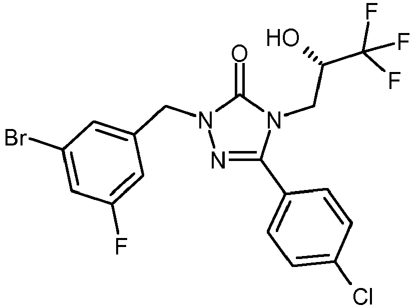

Analog zur Herstellung von Beispiel 101A wurden 219 mg (0.71 mmol) der Verbindung aus Beispiel 5A mit 191 mg (0.71 mmol) 1-Brom-3-(brommethyl)-5-fluorbenzol umgesetzt. Man erhielt 181 mg (51% d. Th.) der Zielverbindung.
LC/MS [Methode 4]: Rₜ = 1.24 min; MS [ESIpos]: m/z = 494 und 496 (M+H)⁺.

### Beispiel 104A

### Methyl-4-brom-2-{[3-(4-chlorphenyl)-5-oxo-4-(3,3,3-trifluor-2-hydroxypropyl)-4,5-dihydro-1H-1,2,4-triazol-1-yl]methyl}benzoat

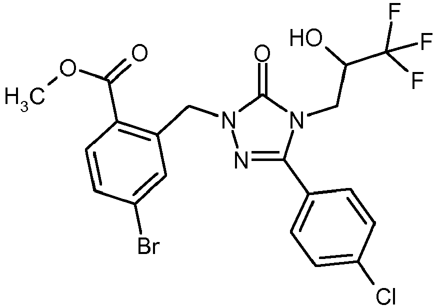

450 mg (1.30 mmol) der Verbindung aus Beispiel 4A und 715 mg (2.19 mmol) Cäsiumcarbonat wurden in 6 ml Acetonitril suspendiert und mit 708 mg (1.61 mmol) Methyl-4-brom-2-(brommethyl)benzoat versetzt. Die Mischung wurde 20 h unter Rückfluss gerührt. Der ausgefallene Feststoff wurde abfiltriert und das Filtrat im Vakuum auf ein Volumen von ca. 1.5 ml eingeengt. Nach Zugabe von 1 ml 1 N Salzsäure wurde das Gemisch direkt chromatographisch aufgereinigt [Methode 19]. Es wurden 545 mg (64% d. Th.) der Zielverbindung in einer Reinheit von 92% erhalten.
LC/MS [Methode 4]: Rₜ = 1.24 min; MS [ESIpos]: m/z = 534 und 536 (M+H)⁺
¹H-NMR (400 MHz, DMSO-d₆): δ = 3.81-3.90 (m, 4H), 4.01 (dd, 1H), 4.24-4.34 (m, 1H), 5.29-5.40 (m, 2H), 6.89 (d, 1H), 7.42 (d, 1H), 7.64 (d, 2H), 7.69 (dd, 1H), 7.74 (d, 2H), 7.84 (d, 1H).

### Beispiel 105A

### Methyl-4-brom-2-({3-(4-chlorphenyl)-5-oxo-4-[(2S)-3,3,3-trifluor-2-hydroxypropyl]-4,5-dihydro-1H-1,2,4-triazol-1-yl}methyl)benzoat

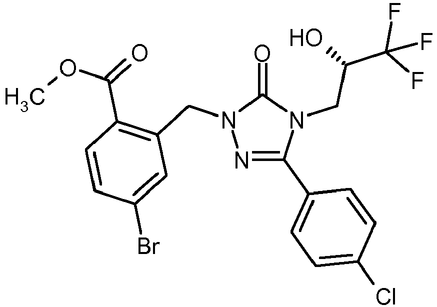

515 mg (1.67 mmol) der Verbindung aus Beispiel 5A und 818 mg (2.51 mmol) Cäsiumcarbonat wurden in 10 ml Acetonitril suspendiert und mit 810 mg (1.84 mmol) Methyl-4-brom-2-(brommethyl)benzoat versetzt. Die Mischung wurde 3 h unter Rückfluss gerührt. Nach Abkühlen auf RT wurde mit 15 ml Wasser versetzt und dreimal mit je 10 ml Ethylacetat extrahiert. Die vereinigten organischen Phasen wurden über Natriumsulfat getrocknet, filtriert und im Vakuum eingeengt. Der Rückstand wurde chromatographisch gereinigt [Methode 19]. Es wurden 455 mg (51% d. Th.) der Zielverbindung erhalten.
LC/MS [Methode 4]: Rₜ = 1.21 min; MS [ESIpos]: m/z = 534 und 536 (M+H)⁺.

### Beispiel 106A

### Methyl-5-methyl-2'-(trifluormethyl)biphenyl-2-carboxylat

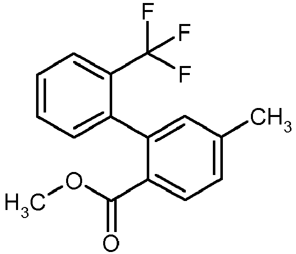

Unter Argon wurden 500 mg (2.18 mmol) Methyl-2-brom-4-methylbenzoat zusammen mit 655 mg (3.27 mmol) 2-(Trifluormethyl)phenylboronsäure in 10 ml Toluol gelöst und nacheinander mit 86 mg (0.22 mmol) 2-Dicyclohexylphosphino-2'-(*N*,*N*-dimethylamino)biphenyl, 100 mg (0.11 mmol) Tris(dibenzylidenaceton)dipalladium sowie 927 mg (4.37 mmol) Kaliumphosphat versetzt. Das Gemisch wurde auf 110°C erhitzt und 20 h bei dieser Temperatur gerührt. Zur Aufarbeitung ließ man auf RT abkühlen und verdünnte das Reaktionsgemisch mit 20 ml Ethylacetat und 20 ml Wasser. Nach Trennen der Phasen wurde die wässrige Phase noch zweimal mit je 20 ml Ethylacetat extrahiert. Die vereinigten organischen Phasen wurden über Magnesiumsulfat getrocknet, filtriert und im Vakuum eingeengt. Der Rückstand wurde durch Chromatographie an Kieselgel gereinigt (Elutionsmittel: zunächst Cyclohexan/Ethylacetat 30:1, dann 20:1). Es wurden 597 mg (86% d. Th.) der Zielverbindung erhalten.
GC/MS [Methode 20]: Rₜ = 5.55 min; MS [EIpos]: m/z = 294 (M)⁺.

### Beispiel 107A

### Methyl-2'-chlor-5-methylbiphenyl-2-carboxylat

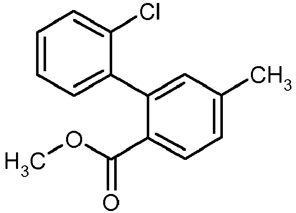

Analog zur Herstellung von Beispiel 107A wurden 500 mg (2.18 mmol) Methyl-2-brom-4-methylbenzoat mit 512 mg (3.27 mmol) 2-Chlorphenylboronsäure umgesetzt. Es wurden 275 mg (48% d. Th.) der Zielverbindung erhalten.
LC/MS [Methode 4]: Rₜ = 1.22 min; MS [ESIpos]: m/z = 261 (M+H)⁺.

### Beispiel 108A

### Methyl-5-(brommethyl)-2'-(trifluormethyl)biphenyl-2-carboxylat

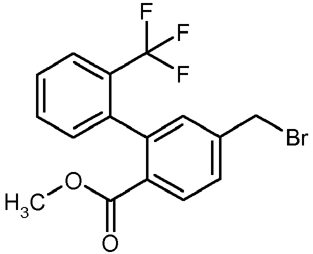

590 mg (2.01 mmol) der Verbindung aus Beispiel 107A wurden mit 357 mg (2.01 mmol) *N*-Bromsuccinimid und 33 mg (0.20 mmol) 2,2'-Azobis-2-methylpropannitril in 8 ml Tetrachlorkohlenstoff 16 h unter Rückfluss erhitzt. Nach Abkühlen auf RT wurde mit 10 ml Dichlormethan verdünnt und mit 10 ml Wasser gewaschen. Die organische Phase wurde über Magnesiumsulfat getrocknet, filtriert und im Vakuum eingeengt. Das Rohprodukt wurde chromatographisch aufgereinigt [Methode 19]. Es wurden 346 mg (30% d. Th.) der Zielverbindung in 64%-iger Reinheit erhalten, die sofort weiter umgesetzt wurden.
LC/MS [Methode 4]: Rₜ = 1.27 min; MS [DCI]: m/z = 390 und 392 (M+NH₄)⁺.

### Beispiel 109A

### Methyl-5-(brommethyl)-2'-chlorbiphenyl-2-carboxylat

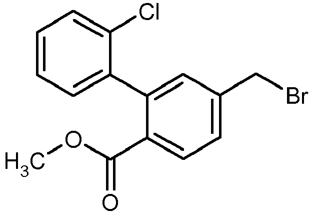

Analog zur Herstellung von Beispiel 109A wurden 270 mg (1.04 mmol) der Verbindung aus Beispiel 108A mit *N*-Bromsuccinimid umgesetzt. Es wurden 232 mg (66% d. Th.) der Zielverbindung erhalten, die sofort weiter umgesetzt wurden.
LC/MS [Methode 4]: Rₜ = 1.22 min; MS [DCI]: m/z = 356 und 358 (M+NH₄)⁺.

### Beispiel 110A

### Dimethyl-2'-chlorbiphenyl-3,5-dicarboxylat

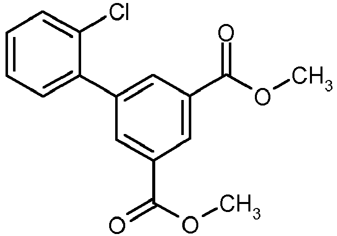

Unter Argon wurden 500 mg (1.83 mmol) Dimethyl-5-bromisophthalat zusammen mit 429 mg (2.75 mmol) 2-Chlorphenylboronsäure in 8 ml Toluol gelöst und nacheinander mit 72 mg (0.18 mmol) 2-Dicyclohexylphosphino-2'-(*N,N-*dimethylamino)biphenyl, 84 mg (0.09 mmol) Tris(dibenzyliden-aceton)dipalladium sowie 777 mg (3.66 mmol) Kaliumphosphat versetzt. Das Gemisch wurde auf 110°C erhitzt und 20 h bei dieser Temperatur gerührt. Zur Aufarbeitung ließ man auf RT abkühlen und verdünnte das Reaktionsgemisch mit 20 ml Ethylacetat. Es wurde vom Feststoff abgesaugt und der Rückstand dreimal mit je 10 ml Ethylacetat gewaschen. Die vereinigten Filtrate wurden zweimal mit je 10 ml Wasser gewaschen. Die organische Phase wurde über Magnesiumsulfat getrocknet, filtriert und im Vakuum eingeengt. Der Rückstand wurde chromatographisch gereinigt [Methode 19]. Es wurden 305 mg (55% d. Th.) der Zielverbindung erhalten.
LC/MS [Methode 4]: Rₜ = 1.24 min; MS [EIpos]: m/z = 305 (M+H)⁺
¹H-NMR (400 MHz, DMSO-d₆): δ = 3.92 (s, 6H), 7.46-7.55 (m, 3H), 7.60-7.68 (m, 1H), 8.23 (d, 2H), 8.52 (t, 1H).

### Beispiel 111A

### Methyl-2'-chlor-5-(hydroxymethyl)biphenyl-3-carboxylat

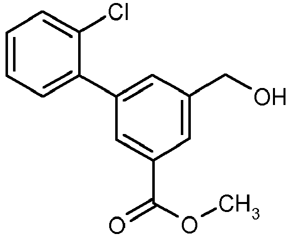

305 mg (1.00 mmol) der Verbindung aus Beispiel 110A wurden in 6 ml THF gelöst und bei -10°C mit 0.5 ml (0.50 mmol) einer 1 M Lithiumaluminiumhydrid-Lösung in THF versetzt. Anschließend wurde 1 h bei RT gerührt. Zur Aufarbeitung wurde bei RT mit 3 ml gesättigter wässriger Natriumkaliumtartrat-Lösung versetzt und mit 15 ml Ethylacetat extrahiert. Die organische Phase wurde einmal mit 10 ml gesättigter Natriumkaliumtartrat-Lösung gewaschen, über Magnesiumsulfat getrocknet, filtriert und im Vakuum eingeengt. Der Rückstand wurde chromatographisch gereinigt [Methode 19]. Es wurden 189 mg (68% d. Th.) der Zielverbindung erhalten.
LC/MS [Methode 4]: Rₜ = 1.03 min; MS [ESIpos]: m/z = 277 (M+H)⁺
¹H-NMR (400 MHz, DMSO-d₆): δ = 3.88 (s, 3H), 4.63 (d, 2H), 5.43 (t, 1H), 7.43-7.47 (m, 3H), 7.58-7.62 (m, 1H), 7.62-7.64 (m, 1H), 7.84-7.87 (m, 1H), 7.97-8.00 (m, 1H).

### Beispiel 112A

### Methyl-5-(brommethyl)-2'-chlorbiphenyl-3-carboxylat

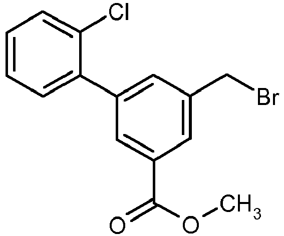

187 mg (0.68 mmol) der Verbindung aus Beispiel 111A und 266 mg (1.01 mmol) Triphenylphosphin wurden in 6 ml THF gelöst und bei RT mit 336 mg (1.01 mmol) Tetrabromkohlenstoff versetzt. Anschließend wurde 16 h bei RT gerührt. Zur Aufarbeitung wurde über 20 g Kieselgur filtriert, mit Ethylacetat nachgewaschen und das Filtrat im Vakuum eingeengt. Der Rückstand wurde chromatographisch an Kieselgel gereinigt (Elutionsmittel: Cyclohexan/Ethylacetat 7:3). Es wurden 275 mg (>100% d. Th.) der Zielverbindung erhalten, welche sofort weiter umgesetzt wurden.
¹H-NMR (400 MHz, DMSO-d₆): δ = 3.89 (s, 3H), 4.86 (s, 2H), 7.44-7.50 (m, 3H), 7.58-7.64 (m, 1H), 7.80-7.82 (m, 1H), 7.90-7.92 (m, 1H), 8.08-8.11 (m, 1H).

### Beispiel 113A und Beispiel 114A

### Methyl-5-brom-2'-chlorbiphenyl-3-carboxylat und Methyl-2,2"-dichlor-1,1':3',1"-terphenyl-5'-carboxylat

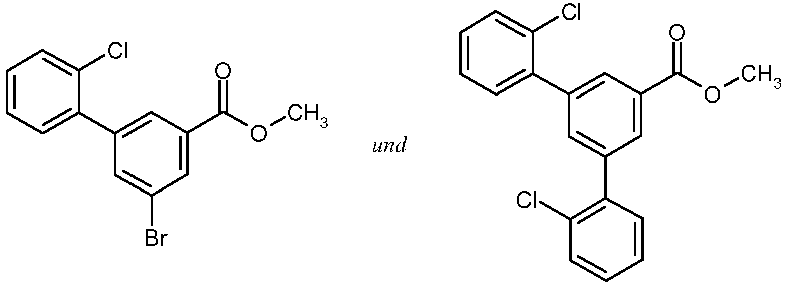

300 mg (1.02 mmol) Methyl-3,5-dibrombenzoat wurden unter Argon in 6 ml Dioxan mit 60 mg (0.05 mmol) Tetrakis(triphenylphosphin)palladium(0) versetzt. Das Gemisch wurde auf 110°C erhitzt und nacheinander mit 1.0 ml (2.00 mmol) 2 M wässriger Natriumcarbonat-Lösung und 239 mg (1.53 mmol) 2-Chlorphenylboronsäure, gelöst in 1 ml Dioxan, versetzt. Anschließend wurde 1 h bei 110°C gerührt. Zur Aufarbeitung ließ man auf RT abkühlen und verdünnte das Reaktionsgemisch mit 20 ml Ethylacetat und 20 ml Wasser. Nach Trennen der Phasen wurde die wässrige Phase noch zweimal mit je 20 ml Ethylacetat extrahiert. Die vereinigten organischen Phasen wurden über Magnesiumsulfat getrocknet, filtriert und im Vakuum eingeengt. Der Rückstand wurde durch präparative HPLC [Methode 19] in die Komponenten aufgetrennt. Es wurden 142 mg (43% d. Th.) Methyl-5-brom-2'-chlorbiphenyl-3-carboxylat (Beispiel 113A) sowie 166 mg (46% d. Th.) Methyl-2,2"-dichlor-1,1':3',1"-terphenyl-5'-carboxylat (Beispiel 114A) als Reaktionsprodukte erhalten.

### Beispiel 113A:

GC/MS [Methode 20]: Rₜ = 7.50 min; MS [ESIpos]: m/z = 324 und 326 (M)⁺.

### Beispiel 114A:

GC/MS [Methode 20]: Rₜ = 10.26 min; MS [ESIpos]: m/z = 356 und 358 (M)⁺.

### Beispiel 115A

### (5-Brom-2'-chlorbiphenyl-3-yl)methanol

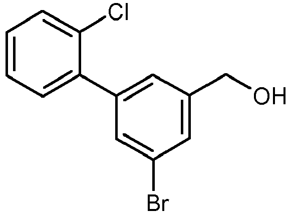

170 mg (0.52 mmol) der Verbindung aus Beispiel 113A wurden in 6 ml THF gelöst und bei -10°C mit 0.37 ml (0.37 mmol) einer 1 M Lithiumaluminiumhydrid-Lösung in THF versetzt. Anschließend wurde das Gemisch 1 h bei RT gerührt. Zur Aufarbeitung wurde bei RT mit 4 ml gesättigter wässriger Natriumkaliumtartrat-Lösung versetzt und mit 15 ml Ethylacetat extrahiert. Die organische Phase wurde einmal mit 10 ml gesättigter Natriumkaliumtartrat-Lösung gewaschen, über Magnesiumsulfat getrocknet, filtriert und im Vakuum eingeengt. Es wurden 177 mg (>100% d. Th.) der Zielverbindung erhalten.
LC/MS [Methode 2]: Rₜ = 2.39 min.
GC/MS [Methode 20]: Rₜ = 7.67 min; MS [ESIpos]: m/z = 296 und 298 (M)⁺.

### Beispiel 116A

### (2,2"-Dichlor-1,1':3', 1 "-terphenyl-5'-yl)methanol

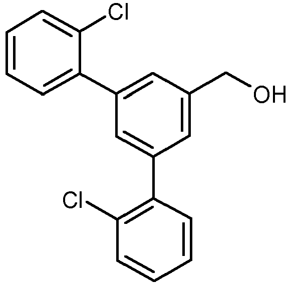

Analog zur Herstellung von Beispiel 115A wurden 311 mg (0.87 mmol) der Verbindung aus Beispiel 114A umgesetzt. Es wurden 283 mg (91% d. Th.) der Zielverbindung erhalten.
LC/MS [Methode 2]: Rₜ = 2.63 min.
MS [DCI]: m/z = 346 (M+NH₄)⁺.

### Beispiel 117A

### 3'-Brom-5'-(brommethyl)-2-chlorbiphenyl

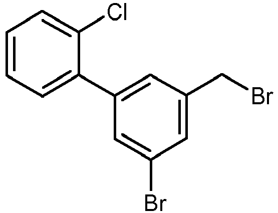

177 mg (0.60 mmol) der Verbindung aus Beispiel 115A und 187 mg (0.71 mmol) Triphenylphosphin wurden in 4 ml THF gelöst und bei RT mit 237 mg (0.71 mmol) Tetrabromkohlenstoff versetzt. Anschließend wurde das Gemisch 16 h bei RT gerührt. Zur Aufarbeitung wurde über 20 g Kieselgur filtriert und das Filtrat im Vakuum eingeengt. Der Rückstand wurde chromatographisch gereinigt [Methode 19]. Es wurden in 79% Reinheit 129 mg (60% d. Th.) der Zielverbindung erhalten, welche sofort weiter umgesetzt wurden.
LC/MS [Methode 4]: Rₜ = 1.41 min.

### Beispiel 118A

### 5'-(Brommethyl)-2,2"-dichlor-1,1':3',1 "-terphenyl

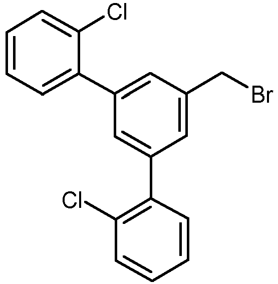

Analog zur Herstellung von Beispiel 117A wurden 280 mg (0.85 mmol) der Verbindung aus Beispiel 116A umgesetzt. Die Reinigung des Rohproduktes erfolgte durch Chromatographie an Kieselgel (Elutionsmittel: Cyclohexan/Ethylacetat 10:1). Es wurden 300 mg (76% d. Th.) der Zielverbindung erhalten.
GC/MS [Methode 20]: Rₜ = 10.64 min; MS [ESIpos]: m/z = 390, 392 und 394 (M)⁺
MS [DCI]: m/z = 408, 410 und 412 (M+NH₄)⁺.

### Beispiel 119A

### 2-[(3-Bromphenyl)sulfonyl]-5-(4-chlorphenyl)-4-[(2S)-3,3,3-trifluor-2-hydroxypropyl]-2,4-dihydro-3H-1,2,4-triazol-3-on

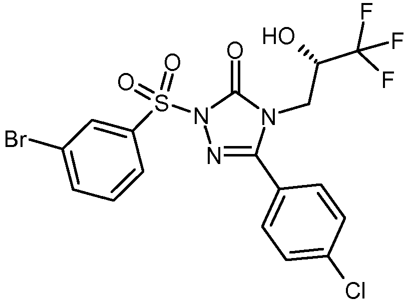

360 mg (1.17 mmol) der Verbindung aus Beispiel 5A wurden in 10 ml THF gelöst und bei 0°C mit 94 mg (2.34 mmol) Natriumhydrid (60%-ige Dispersion in Mineralöl) versetzt. Nach 20 min fügte man 299 mg (1.17 mmol) 3-Brombenzolsulfonylchlorid hinzu und rührte 1 h bei 0°C. Zur Aufarbeitung wurden 10 ml Wasser hinzugefügt und das Gemisch zweimal mit je 15 ml Ethylacetat extrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 8:1, dann 1:1). Es wurden 181 mg (27% d. Th.) der Zielverbindung erhalten.
LC/MS [Methode 3]: Rₜ = 1.33 min; MS [ESIpos]: m/z = 526 und 528 (M+H)⁺.

### Beispiel 120A

### 2-[(3-Bromphenyl)sulfonyl]-5-(4-chlorphenyl)-4-[(1E)-3,3,3-trifluorprop-1-en-1-yl]-2,4-dihydro-3H-1,2,4-triazol-3-on

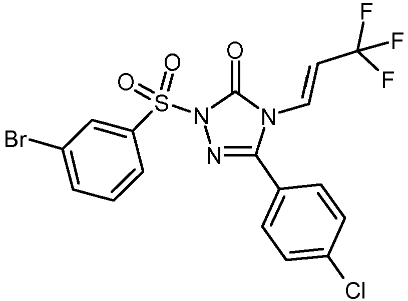

500 mg (1.63 mmol) der Verbindung aus Beispiel 5A wurden in 10 ml Acetonitril gelöst und mit 449 mg (3.25 mmol) Kaliumcarbonat sowie 415 mg (1.63 mmol) 3-Brombenzolsulfonylchlorid versetzt. Das Gemisch wurde 2 h unter Rückfluss erhitzt. Zur Aufarbeitung wurden 10 ml Wasser hinzugefügt und das Gemisch zweimal mit je 15 ml Ethylacetat extrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: Cyclohexan/ Ethylacetat 8:1 → 5:1 → 1:1). Es wurden 285 mg (34% d. Th.) der Zielverbindung erhalten.
LC/MS [Methode 4]: Rₜ = 1.31 min; MS [ESIpos]: m/z = 508 und 510 (M+H)⁺
¹H-NMR (400 MHz, CDCl₃): δ = 6.72 (dq, 1H), 6.89-6.95 (m, 1H), 7.62-7.66 (m, 2H), 7.67-7.73 (m, 3H), 8.05-8.11 (m, 2H), 8.14 (t, 1H).

### Beispiel 121A

### 5-(4-Chlorphenyl)-4-cyclopropyl-2-(hydroxymethyl)-2,4-dihydro-3H-1,2,4-triazol-3-on

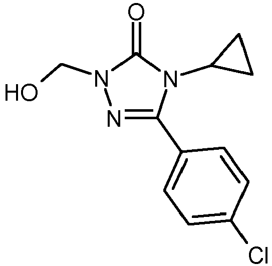

1000 mg (4.24 mmol) 5-(4-Chlorphenyl)-4-cyclopropyl-2,4-dihydro-3*H-*1,2,4-triazol-3-on [Herstellung gemäß WO 2007/134862 Beispiel 36A] wurden mit 7 ml einer 37%-igen Lösung von Formaldehyd in Wasser versetzt und 20 h bei RT gerührt. Der ausgefallene Feststoff wurde abgesaugt und mit Wasser gewaschen. Nach Trocknen im Hochvakuum erhielt man 878 mg (62% d. Th.) der Zielverbindung.
¹H-NMR (400 MHz, CDCl₃): δ = 0.71-0.78 (m, 2H), 0.98-1.06 (m, 2H), 2.98 (m, 1H), 5.34 (s, 2H), 7.46 (d, 2H), 7.69 (d, 2H).

### Beispiel 122A

### 2-(Chlormethyl)-5-(4-chlorphenyl)-4-cyclopropyl-2,4-dihydro-3H-1,2,4-triazol-3-on

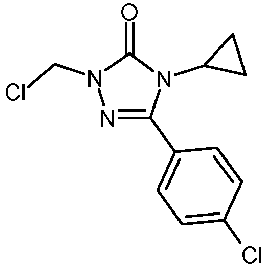

875 mg (3.29 mmol) der Verbindung aus Beispiel 121A wurden in 3 ml Dichlormethan suspendiert und mit einem Tropfen DMF sowie 288 µl (3.95 mmol) Thionylchlorid versetzt. Es wurde 3 h bei RT gerührt. Zur Aufarbeitung wurde mit 5 ml gesättigter wässriger NatriumhydrogencarbonatLösung versetzt. Es wurde einmal mit 10 ml *tert*.-Butylmethylether extrahiert. Die organische Phase wurde einmal mit 5 ml Wasser gewaschen, über Natriumsulfat getrocknet, filtriert und im Vakuum eingeengt. Nach Trocknen des Rückstands im Hochvakuum erhielt man 803 mg (86% d. Th.) der Zielverbindung.
¹H-NMR (400 MHz, CDCl₃): δ = 0.74-0.80 (m, 2H), 1.00-1.06 (m, 2H), 2.98 (m, 1H), 5.66 (s, 2H), 7.48 (d, 2H), 7.72 (d, 2H).

### Ausführungsbeispiele:

### Beispiel 1

### 5-(5-Chlorthiophen-2-yl)-4-(2-fluorbenzyl)-2-({3-[2-(trifluormethyl)phenyl]-1,2,4-oxadiazol-5-yl}-methyl)-2,4-dihydro-3H-1,2,4-triazol-3-on

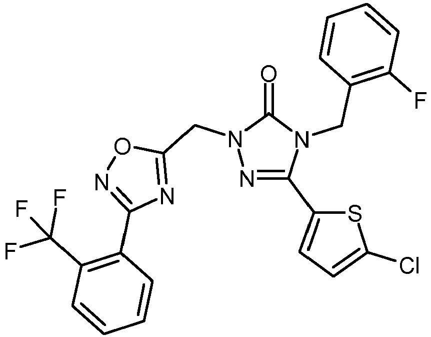

Zu einer Lösung von 56 mg (0.15 mmol) [3-(5-Chlor-2-thienyl)-4-(2-fluorbenzyl)-5-oxo-4,5-di-hydro-1*H-*1,2,4-triazol-1-yl]essigsäure [Herstellung gemäß WO 2007/134862 Beispiel 154A] und 32 µl (0.18 mmol) *N,N*-Diisopropylethylamin in 1.5 ml trockenem DMF gab man unter Argon 95 mg (0.18 mmol) Benzotriazol-1-yloxy-tris(pyrrolidino)phosphonium-Hexafluorphosphat. Nach 20 min Rühren wurden 34 mg (0.17 mmol) *N'*-Hydroxy-2-(trifluormethyl)benzolcarboximidamid hinzugefügt. Anschließend wurde das Gemisch 16 h bei RT gerührt. Zur Aufarbeitung wurden 10 ml Wasser zugesetzt und dreimal mit je 10 ml Ethylacetat extrahiert. Die vereinigten organischen Phasen wurden mit je 10 ml Wasser und gesättigter Kochsalz-Lösung gewaschen, durch Extrelut filtriert und im Vakuum eingeengt. Der Rückstand wurde in 2 ml DMF gelöst und 15 min im Mikrowellenofen bei 250°C gerührt. Nach dem Abkühlen entfernte man das Solvens am Rotationsverdampfer unter vermindertem Druck und chromatographierte das Rohprodukt an Kieselgel (Eluent: Cyclohexan/Ethylacetat 4:1). Es wurden so 60 mg (72% d. Th.) der Zielverbindung als gelbes Harz erhalten.
LC/MS [Methode 9]: Rₜ = 4.25 min; MS [ESIpos]: m/z = 536 (M+H)⁺
¹H-NMR (400 MHz, CDCl₃): δ = 5.15 (s, 2H), 5.42 (s, 2H), 6.35 (d, 2H), 6.93 (d, 2H), 7.01-7.29 (m, 3H), 7.23-7.34 (m, 1H), 7.60-7.70 (m, 2H), 7.76-7.89 (m, 2H).

### Beispiel 2

### 2-{[3-(2-Chlorbenzyl)-1,2,4-oxadiazol-5-yl]methyl}-5-(4-chlorphenyl)-4-[(2S)-3,3,3-trifluor-2-hydroxypropyl]-2,4-dihydro-3H-1,2,4-triazol-3-on

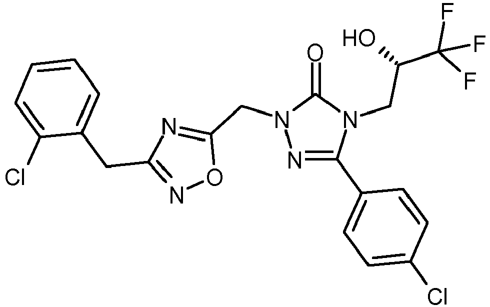

50 mg (0.13 mmol) der Verbindung aus Beispiel 7A wurden in 2 ml Toluol gelöst, mit 51 mg (0.28 mmol) (1*Z*)-2-(2-Chlorphenyl)-*N'*-hydroxyethanimidamid sowie 38 mg (0.28 mmol) Kaliumcarbonat versetzt und 6 h unter Rückfluss erhitzt. Zur Aufarbeitung wurde mit 10 ml Wasser versetzt und zweimal mit je 10 ml Ethylacetat extrahiert. Die vereinigten organischen Phasen wurden einmal mit 10 ml Wasser sowie 10 ml gesättigter Natriumchlorid-Lösung gewaschen, über Natriumsulfat getrocknet, filtriert und im Vakuum eingeengt. Das Rohprodukt wurde chromatographisch gereinigt [Methode 19]. Es wurden 40 mg (59% d. Th.) der Zielverbindung erhalten.
LC/MS [Methode 4]: Rₜ = 1.19 min; MS [ESIpos]: m/z = 514 (M+H)⁺
¹H-NMR (400 MHz, CDCl₃): δ = 3.97 (dd, 1H), 4.04 (dd, 1H), 4.22 (s, 2H), 4.47-4.59 (m, 1H), 4.64 (br. s, 1H), 5.25 (d, 1H), 5.32 (d, 1H), 7.19-7.29 (m, 3H), 7.35-7.42 (m, 1H), 7.46-7.51 (m, 2H), 7.54-7.60 (m, 2H).

### Beispiel 3

### 5-(4-Chlorphenyl)-2-{[3-(2-methylbenzyl)-1,2,4-oxadiazol-5-yl]methyl}-4-[(2S)-3,3,3-trifluor-2-hydroxypropyl]-2,4-dihydro-3H-1,2,4-triazol-3-on

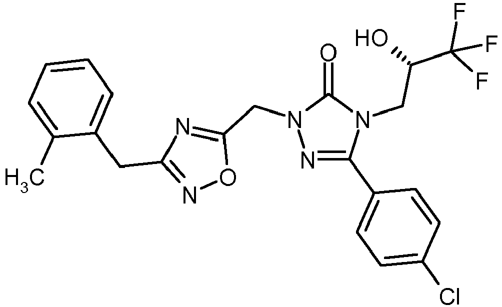

Analog zur Herstellung der Verbindung in Beispiel 2 wurden ausgehend von 50 mg (0.13 mmol) der Verbindung aus Beispiel 7A 45 mg (69% d. Th.) der Titelverbindung erhalten.
LC/MS [Methode 3]: Rₜ = 1.38 min; MS [ESIpos]: m/z = 494 (M+H)⁺
¹H-NMR (400 MHz, CDCl₃): δ = 2.33 (s, 3H), 3.97 (dd, 1H), 4.01-4.08 (m, 3H), 4.44 (d, 1H), 4.47-4.54 (m, 1H), 5.23 (d, 1H), 5.30 (d, 1H), 7.13-7.22 (m, 4H), 7.49 (d, 2H), 7.55 (d, 2H).

### Beispiel 4

### 5-(4-Chlorphenyl)-4-[(2S)-3,3,3-trifluor-2-hydroxypropyl]-2-({3-[3-(trifluormethyl)benzyl]-1,2,4-oxadiazol-5-yl}methyl)-2,4-dihydro-3H-1,2,4-triazol-3-on

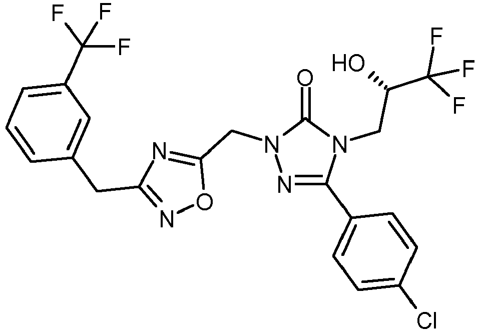

Analog zur Herstellung der Verbindung in Beispiel 2 wurden ausgehend von 50 mg (0.13 mmol) der Verbindung aus Beispiel 7A 24 mg (34% d. Th.) der Titelverbindung erhalten.
LC/MS [Methode 3]: Rₜ = 1.43 min; MS [ESIpos]: m/z = 548 (M+H)⁺
¹H-NMR (400 MHz, CDCl₃): δ = 3.99 (dd, 1H), 4.06 (dd, 1H), 4.14 (s, 2H), 4.45-4.57 (m, 2H), 5.23-5.34 (m, 2H), 7.41-7.60 (m, 8H).

### Beispiel 5

### 5-(4-Chlorphenyl)-2-{[3-(2-methylphenyl)-1,2,4-oxadiazol-5-yl]methyl}-4-[(2S)-3,3,3-trifluor-2-hydroxypropyl]-2,4-dihydro-3H-1,2,4-triazol-3-on

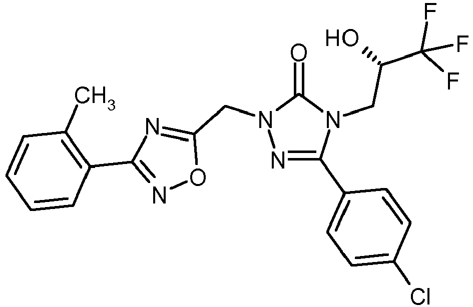

Analog zur Herstellung der Verbindung in Beispiel 2 wurden ausgehend von 50 mg (0.13 mmol) der Verbindung aus Beispiel 7A nach einer Reaktionszeit von 20 h 47 mg (74% d. Th.) der Titelverbindung erhalten.
LC/MS [Methode 4]: Rₜ = 1.22 min; MS [ESIpos]: m/z = 480 (M+H)⁺
¹H-NMR (400 MHz, CDCl₃): δ = 2.60 (s, 3H), 3.98-4.06 (m, 1H), 4.06-4.12 (m, 1H), 4.50 (d, 1H), 4.56 (d, 1H), 5.34-5.44 (m, 2H), 7.31 (d, 2H), 7.36-7.42 (m, 1H), 7.47-7.53 (m, 2H), 7.55-7.61 (m, 2H), 7.94 (d, 1H).

### Beispiel 6

### 5-(4-Chlorphenyl)-2-{[3-(2-chlorphenyl)-1,2,4-oxadiazol-5-yl]methyl}-4-[(2S)-3,3,3-trifluor-2-hydroxypropyl]-2,4-dihydro-3H-1,2,4-triazol-3-on

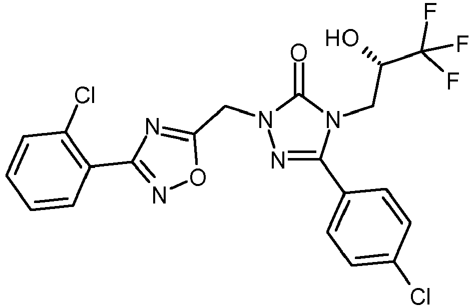

Analog zur Herstellung der Verbindung in Beispiel 2 wurden ausgehend von 50 mg (0.13 mmol) der Verbindung aus Beispiel 7A nach einer Reaktionszeit von 20 h 52 mg (79% d. Th.) der Titelverbindung erhalten.
LC/MS [Methode 4]: Rₜ = 1.17 min; MS [ESIpos]: m/z = 500 und 502 (M+H)⁺
¹H-NMR (400 MHz, CDCl₃): δ = 3.95-4.15 (m, 2H), 4.51-4.62 (m, 1H), 5.33-5.48 (m, 2H), 7.34-7.56 (m, 5H), 7.56-7.64 (m, 2H), 7.89 (d, 1H).

### Beispiel 7

### 5-(4-Chlorphenyl)-2-{[3-(2,6-difluorphenyl)-1,2,4-oxadiazol-5-yl]methyl}-4-[(2S)-3,3,3-trifluor-2-hydroxypropyl]-2,4-dihydro-3H-1,2,4-triazol-3-on

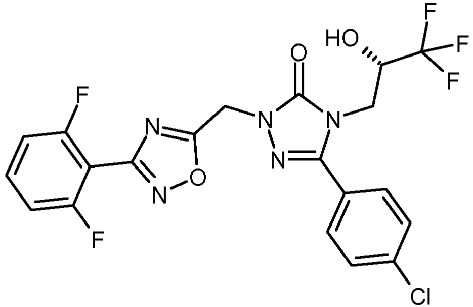

Analog zur Herstellung der Verbindung in Beispiel 2 wurden ausgehend von 50 mg (0.13 mmol) der Verbindung aus Beispiel 7A nach einer Reaktionszeit von 2 h 46 mg (70% d. Th.) der Titelverbindung erhalten.
LC/MS [Methode 4]: Rₜ = 1.13 min; MS [ESIpos]: m/z = 502 (M+H)⁺
¹H-NMR (400 MHz, CDCl₃): δ = 3.99 (dd, 1H), 4.08 (dd, 1H), 4.53-4.62 (m, 1H), 4.66 (d, 1H), 5.36-5.49 (m, 2H), 7.01-7.10 (m, 2H), 7.44-7.54 (m, 3H), 7.58-7.64 (m, 2H).

### Beispiel 8

### 5-(4-Chlorphenyl)-2-{[3-(2,3-difluorphenyl)-1,2,4-oxadiazol-5-yl]methyl}-4-[(2S)-3,3,3-trifluor-2-hydroxypropyl]-2,4-dihydro-3H-1,2,4-triazol-3-on

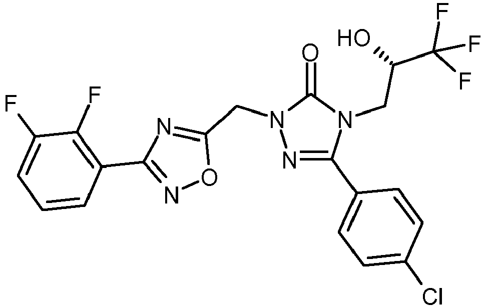

Analog zur Herstellung der Verbindung in Beispiel 2 wurden ausgehend von 50 mg (0.13 mmol) der Verbindung aus Beispiel 7A nach einer Reaktionszeit von 2 h 47 mg (71% d. Th.) der Titelverbindung erhalten.
LC/MS [Methode 4]: Rₜ = 1.18 min; MS [ESIpos]: m/z = 502 (M+H)⁺
¹H-NMR (400 MHz, CDCl₃): δ = 4.03 (dd, 1H), 4.07-4.13 (m, 1H), 4.38 (d, 1H), 4.52-4.59 (m, 1H), 5.35-5.47 (m, 2H), 7.17-7.25 (m, 1H), 7.34 (q, 1H), 7.51 (d, 2H), 7.58 (d, 2H), 7.79 (t, 1H).

### Beispiel 9

### 5-(4-Chlorphenyl)-4-[(2S)-3,3,3-trifluor-2-hydroxypropyl]-2-({3-[2-(trifluormethyl)phenyl]-1,2,4-oxadiazol-5-yl}methyl)-2,4-dihydro-3H-1,2,4-triazol-3-on

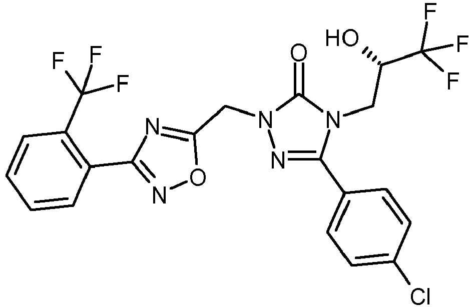

Analog zur Herstellung der Verbindung in Beispiel 2 wurden ausgehend von 50 mg (0.13 mmol) der Verbindung aus Beispiel 7A 50 mg (71% d. Th.) der Titelverbindung erhalten.
LC/MS [Methode 3]: Rₜ = 1.39 min; MS [ESIpos]: m/z = 534 (M+H)⁺
¹H-NMR (400 MHz, CDCl₃): δ = 4.01 (dd, 1H), 4.08 (dd, 1H), 4.47-4.51 (m, 1H), 4.52-4.61 (m, 1H), 5.36-5.47 (m, 2H), 7.50 (d, 2H), 7.59 (d, 2H), 7.63-7.69 (m, 2H), 7.76-7.87 (m, 2H).

### Beispiel 10

### 5-(4-Chlorphenyl)-4-[(2S)-3,3,3-trifluor-2-hydroxypropyl]-2-({3-[3-(trifluormethyl)phenyl]-1,2,4-oxadiazol-5-yl}methyl)-2,4-dihydro-3H-1,2,4-triazol-3-on

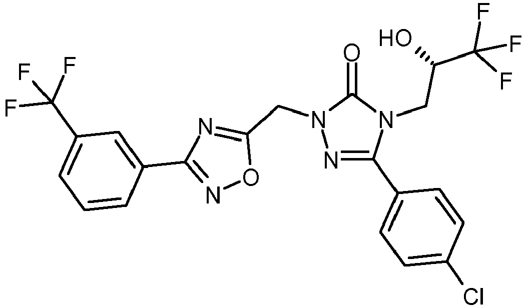

Analog zur Herstellung der Verbindung in Beispiel 2 wurden ausgehend von 50 mg (0.13 mmol) der Verbindung aus Beispiel 7A nach einer Reaktionszeit von 2 h 44 mg (62% d. Th.) der Titelverbindung erhalten.
LC/MS [Methode 4]: Rₜ = 1.25 min; MS [ESIpos]: m/z = 534 (M+H)⁺
¹H-NMR (400 MHz, CDCl₃): δ = 4.05 (dd, 1H), 4.08-4.14 (m, 1H), 4.37 (d, 1H), 4.52-4.58 (m, 1H), 5.35-5.45 (m, 2H), 7.50 (d, 2H), 7.56-7.61 (m, 2H), 7.63 (d, 1H), 7.77 (d, 1H), 8.26 (d, 1H), 8.34 (s, 1H).

### Beispiel 11

### 5-(4-Chlorphenyl)-4-[(2S)-3,3,3-trifluor-2-hydroxypropyl]-2-({3-[3-(trifluormethoxy)phenyl]-1,2,4-oxadiazol-5-yl}methyl)-2,4-dihydro-3H-1,2,4-triazol-3-on

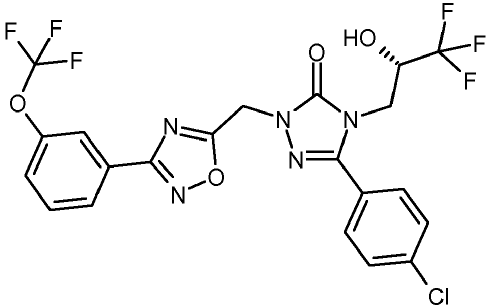

Analog zur Herstellung der Verbindung in Beispiel 2 wurden ausgehend von 50 mg (0.13 mmol) der Verbindung aus Beispiel 7A nach einer Reaktionszeit von 2 h 53 mg (73% d. Th.) der Titelverbindung erhalten.
LC/MS [Methode 4]: Rₜ = 1.27 min; MS [ESIpos]: m/z = 550 (M+H)⁺
¹H-NMR (400 MHz, CDCl₃): δ = 4.04 (dd, 1H), 4.08-4.12 (m, 1H), 4.47 (d, 1H), 4.52-4.59 (m, 1H), 5.34-5.44 (m, 2H), 7.37 (d, 1H), 7.46-7.55 (m, 3H), 7.56-7.61 (m, 2H), 7.93 (s, 1H), 8.01 (d, 1 H).

### Beispiel 12

### 5-(4-Chlorphenyl)-2-{[3-(2,3-dichlorphenyl)-1,2,4-oxadiazol-5-yl]methyl}-4-[(2S)-3,3,3-trifluor-2-hydroxypropyl]-2,4-dihydro-3H-1,2,4-triazol-3-on

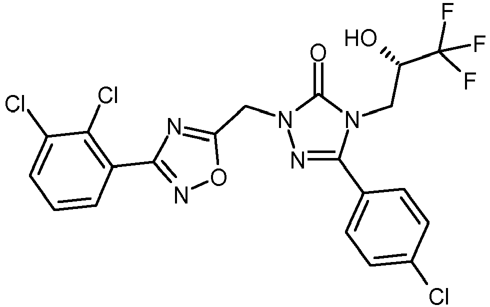

Analog zur Herstellung der Verbindung in Beispiel 2 wurden ausgehend von 50 mg (0.13 mmol) der Verbindung aus Beispiel 7A nach einer Reaktionszeit von 20 h 51 mg (72% d. Th.) der Titelverbindung erhalten.
LC/MS [Methode 4]: Rₜ = 1.26 min; MS [ESIpos]: m/z = 534 und 536 (M+H)⁺
¹H-NMR (400 MHz, CDCl₃): δ = 3.86 (dd, 1H), 3.99-4.06 (m, 1H), 4.26-4.32 (m, 1H), 5.58 (s, 2H), 6.94 (d, 1H), 7.57 (t, 1H), 7.65 (d, 2H), 7.78 (d, 2H), 7.86 (d, 1H), 7.91 (d, 1H).

### Beispiel 13

### 5-(4-Chlorphenyl)-4-[(2S)-3,3,3-trifluor-2-hydroxypropyl]-2-({3-[2-(trifluormethoxy)phenyl]-1,2,4-oxadiazol-5-yl}methyl)-2,4-dihydro-3H-1,2,4-triazol-3-on

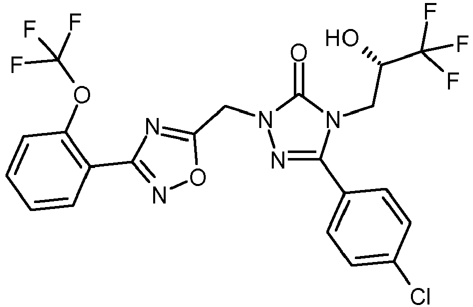

Analog zur Herstellung der Verbindung in Beispiel 2 wurden ausgehend von 50 mg (0.13 mmol) der Verbindung aus Beispiel 7A nach einer Reaktionszeit von 20 h 37 mg (52% d. Th.) der Titelverbindung erhalten.
LC/MS [Methode 4]: Rₜ = 1.25 min; MS [ESIpos]: m/z = 550 (M+H)⁺
¹H-NMR (400 MHz, CDCl₃): δ = 3.87 (dd, 1H), 4.02 (dd, 1H), 4.25-4.34 (m, 1H), 5.57 (s, 2H), 6.94 (d, 1H), 7.60-7.67 (m, 4H), 7.73-7.80 (m, 3H), 8.09 (dd, 1H).

### Beispiel 14

### 5-(5-Chlorthiophen-2-yl)-4-(2-fluorbenzyl)-2-({5-[3-(trifluormethyl)benzyl]-1,2,4-oxadiazol-3-yl}-methyl)-2,4-dihydro-3H-1,2,4-triazol-3-on

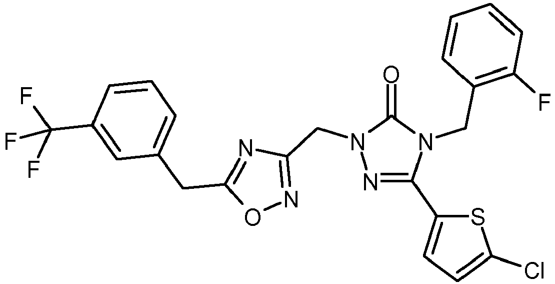

Zu einer Lösung von 29 mg (0.14 mmol) 3-(Trifluormethyl)phenylessigsäure und 27 µl (0.16 mmol) *N,N-*Diisopropylethylamin in 1.3 ml trockenem DMF gab man unter Argon 81 mg (0.16 mmol) Benzotriazol-1-yloxy-tris(pyrrolidino)phosphonium-Hexafluorphosphat. Nach 30 min Rühren fügte man 50 mg (0.13 mmol) der Verbindung aus Beispiel 25A hinzu und rührte das Gemisch für 18 h bei RT. Es wurden dann 2 ml Wasser zugesetzt und die Mischung dreimal mit je 5 ml Ethylacetat extrahiert. Die vereinigten organischen Phasen wurden mit jeweils 5 ml Wasser und Natriumchlorid-Lösung gewaschen, durch Extrelut filtriert und unter vermindertem Druck eingeengt. Der Rückstand wurde in 2 ml DMF gelöst und 15 min im Mikrowellenofen bei 250°C gerührt. Nach dem Abkühlen entfernte man das Solvens am Rotationsverdampfer unter reduziertem Druck und reinigte das Rohprodukt durch Chromatographie [Methode 15]. Es wurden so 25 mg (34% d. Th.) der Zielverbindung als dunkelgelbes Harz erhalten.
LC/MS [Methode 9]: Rₜ = 4.28 min; MS [ESIpos]: m/z = 550 (M+H)⁺
¹H-NMR (400 MHz, CDCl₃): δ = 5.12 (s, 2H), 5.20 (s, 2H), 6.32 (d, 2H), 6.90 (d, 2H), 7.04-7.19 (m, 3H), 7.22-7.34 (m, 1H), 7.43-7.55 (m, 2H), 7.55-7.62 (m, 2H).

### Beispiel 15

### 5-(5-Chlorthiophen-2-yl)-4-(2-fluorbenzyl)-2-({5-[2-(trifluormethyl)phenyl]-1,2,4-oxadiazol-3-yl}-methyl)-2,4-dihydro-3H-1,2,4-triazol-3-on

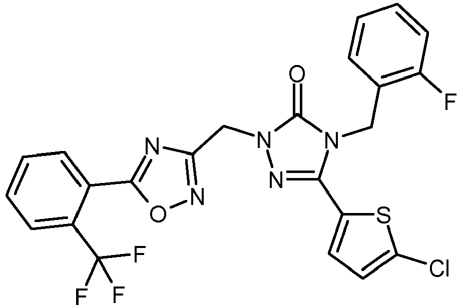

Analog zur Herstellung der Verbindung in Beispiel 14 wurden ausgehend von 50 mg (0.13 mmol) der Verbindung aus Beispiel 25A 35 mg (49% d. Th.) der Titelverbindung erhalten.
MS [ESIpos]: m/z = 536 (M+H)⁺
¹H-NMR (400 MHz, CDCl₃): δ = 5.16 (s, 2H), 5.35 (s, 2H), 6.33 (d, 2H), 6.92 (d, 2H), 7.04-7.13 (m, 2H), 7.13-7.20 (m, 1H), 7.23-7.33 (m, 1H), 7.68-7.78 (m, 2H), 7.83-7.92 (m, 1H), 7.96-8.05 (m, 1H).

### Beispiel 16

### 5-(5-Chlorthiophen-2-yl)-4-(2-fluorbenzyl)-2-({5-[2-(trifluormethyl)benzyl]-1,2,4-oxadiazol-3-yl}-methyl)-2,4-dihydro-3H-1,2,4-triazol-3-on

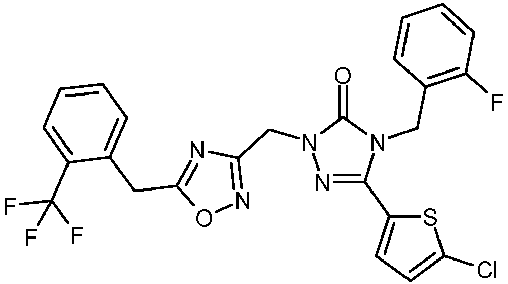

Analog zur Herstellung der Verbindung in Beispiel 14 wurden ausgehend von 50 mg (0.13 mmol) der Verbindung aus Beispiel 25A 15 mg (20% d. Th.) der Titelverbindung erhalten.
MS [CIpos]: m/z = 550 (M+H)⁺
¹H-NMR (400 MHz, CDCl₃): δ = 4.93 (s, 2H), 5.14 (s, 2H), 5.20 (s, 2H), 6.82 (d, 2H), 6.90 (d, 2H), 7.04-7.18 (m, 3H), 7.25-7.35 (m, 1H), 7.35-7.50 (m, 2H), 7.50-7.59 (m, 1H), 7.66-7.75 (m, 1 H).

### Beispiel 17

### 5-(4-Chlorphenyl)-2-{[5-(2-chlorphenyl)-1,3,4-oxadiazol-2-yl]methyl}-4-[(2S)-3,3,3-trifluor-2-hydroxypropyl]-2,4-dihydro-3H-1,2,4-triazol-3-on

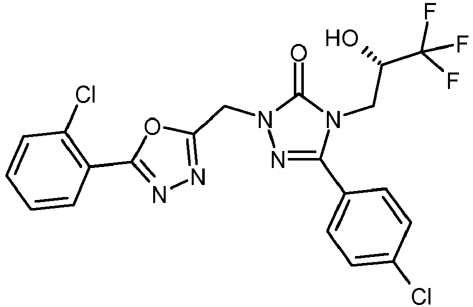

85 mg (0.28 mmol) der Verbindung aus Beispiel 5A wurden in 5 ml Acetonitril gelöst und mit 180 mg (0.55 mmol) Cäsiumcarbonat sowie 66 mg (0.29 mmol) 2-(Chlormethyl)-5-(2-chlorphenyl)-1,3,4-oxadiazol versetzt. Die Mischung wurde 1 h bei 80°C gerührt. Zur Aufarbeitung wurde auf RT abgekühlt und mit 10 ml Wasser versetzt. Es wurde zweimal mit je 15 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 66 mg (48% d. Th.) der Zielverbindung erhalten.
LC/MS [Methode 4]: Rₜ = 1.12 min; MS [ESIpos]: m/z = 500 (M+H)⁺
¹H-NMR (400 MHz, DMSO-d₆): δ = 3.85 (dd, 1H), 4.01 (dd, 1H), 4.25-4.31 (m, 1H), 5.41-5.51 (m, 2H), 6.90 (d, 1H), 7.55-7.60 (m, 1H), 7.61-7.69 (m, 3H), 7.70-7.78 (m, 3H), 7.94 (dd, 1H).

### Beispiel 18

### 5-(4-Chlorphenyl)-2-{[5-(2-chlorphenyl)-1,3,4-thiadiazol-2-yl]methyl}-4-[(2S)-3,3,3-trifluor-2-hydroxypropyl]-2,4-dihydro-3H-1,2,4-triazol-3-on

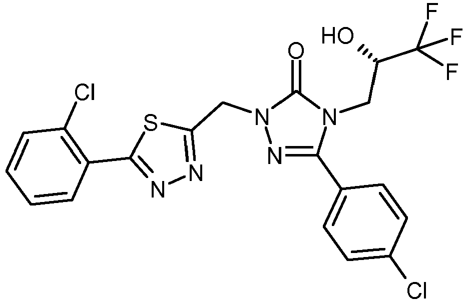

30 mg (0.10 mmol) der Verbindung aus Beispiel 5A wurden in 3 ml Acetonitril gelöst und mit 48 mg (0.15 mmol) Cäsiumcarbonat sowie 28 mg (0.10 mmol) der Verbindung aus Beispiel 86A versetzt. Die Mischung wurde 8 h bei 70°C gerührt. Zur Aufarbeitung wurde auf RT abgekühlt, mit 5 ml Methanol verdünnt und filtriert. Das Filtrat wurde im Vakuum eingeengt und das Rohprodukt anschließend chromatographisch gereinigt [Methode 19]. Es wurden 11 mg (22% d. Th.) der Zielverbindung erhalten.
LC/MS [Methode 6]: Rₜ = 2.48 min; MS [ESIpos]: m/z = 516 (M+H)⁺
¹H-NMR (400 MHz, DMSO-d₆): δ = 3.85 (dd, 1H), 4.01 (dd, 1H), 4.24-4.35 (m, 1H), 5.56-5.65 (m, 2H), 6.92 (s, 1H), 7.53-7.59 (m, 1H), 7.59-7.67 (m, 3H), 7.72 (dd, 1H), 7.77 (d, 2H), 8.14 (dd, 1 H).

### Beispiel 19

### 5-(4-Chlorphenyl)-2-{[5-(2,3-dichlorphenyl)-1,3,4-thiadiazol-2-yl]methyl}-4-[(2S)-3,3,3-trifluor-2-hydroxypropyl]-2,4-dihydro-3H-1,2,4-triazol-3-on

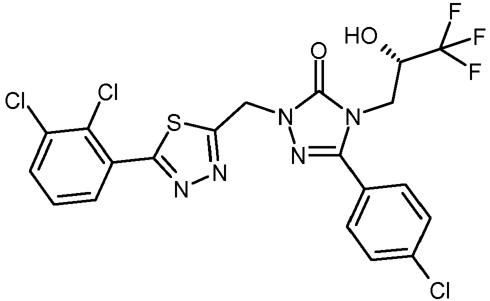

Analog zur Herstellung von Beispiel 18 wurden 36 mg (0.12 mmol) der Verbindung aus Beispiel 5A mit 38 mg (0.12 mmol) der Verbindung aus Beispiel 87A umgesetzt. Es wurden 33 mg (48% d. Th.) der Zielverbindung erhalten.
LC/MS [Methode 4]: Rₜ = 1.25 min; MS [ESIpos]: m/z = 550 und 552 (M+H)⁺
¹H-NMR (400 MHz, DMSO-d₆): δ = 3.85 (dd, 1H), 4.01 (dd, 1H), 4.25-4.35 (m, 1H), 5.56-5.66 (m, 2H), 6.94 (br. s, 1H), 7.57 (t, 1H), 7.64 (d, 2H), 7.78 (d, 2H), 7.90 (dd, 1H), 8.05 (dd, 1H).

### Beispiel 20

### 5-(4-Chlorphenyl)-4-[(2S)-3,3,3-trifluor-2-hydroxypropyl]-2-({5-[2-(trifluormethyl)phenyl]-1,3,4-thiadiazol-2-yl}methyl)-2,4-dihydro-3H-1,2,4-triazol-3-on

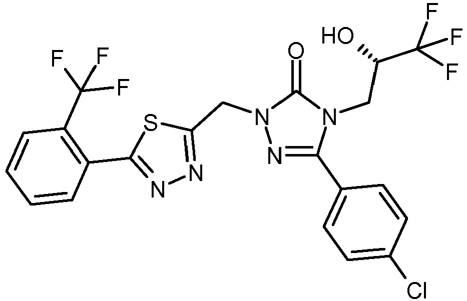

Analog zur Herstellung von Beispiel 18 wurden 33 mg (0.11 mmol) der Verbindung aus Beispiel 5A mit 35 mg (0.11 mmol) der Verbindung aus Beispiel 85A umgesetzt. Es wurden 16 mg (27% d. Th.) der Zielverbindung erhalten.
LC/MS [Methode 3]: Rₜ = 1.33 min; MS [ESIpos]: m/z = 550 (M+H)⁺
¹H-NMR (400 MHz, DMSO-d₆): δ = 3.86 (dd, 1H), 4.02 (dd, 1H), 4.25-4.35 (m, 1H), 5.56-5.65 (m, 2H), 6.92 (br. s, 1H), 7.62-7.67 (m, 2H), 7.75-7.88 (m, 5H), 7.97-8.01 (m, 1H).

### Beispiel 21

### 5-(4-Chlorphenyl)-4-cyclopropyl-2-({5-[3-(trifluormethyl)phenyl]-4H-1,2,4-triazol-3-yl}methyl)-2,4-dihydro-3H-1,2,4-triazol-3-on

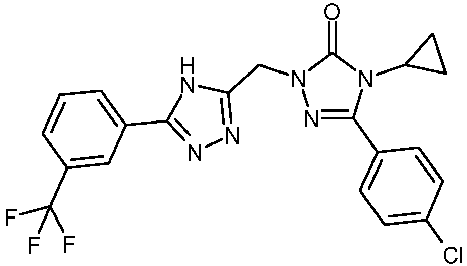

100 mg (0.33 mmol) der Verbindung aus Beispiel 21A wurden in 1.6 ml DMF gelöst, mit 109 mg (0.49 mmol) 3-Trifluormethylbenzamidin-Hydrochlorid versetzt und 45 min im Mikrowellenofen bei 150°C gerührt. Der Ansatz wurde nach dem Abkühlen am Rotationsverdampfer unter vermindertem Druck eingeengt und der verbliebene Rückstand chromatographisch gereinigt [Methode 19]. Man erhielt 70 mg (47% d. Th.) der Zielverbindung als farblosen Feststoff.
LC/MS [Methode 7]: Rₜ = 2.33 min; MS [ESIpos]: m/z = 461 (M+H)⁺
¹H-NMR (400 MHz, CDCl₃): δ = 0.74-0.84 (m, 2H), 0.99-1.10 (m, 2H), 2.96-3.06 (m, 1H), 5.28 (s, 2H), 7.45 (d, 2H), 7.53 (t, 1H), 7.64 (d, 1H), 7.68 (d, 2H), 8.25 (d, 1H), 8.36 (s, 1H).

### Beispiel 22

### 5-(4-Chlorphenyl)-4-cyclopropyl-2-({5-[2-(trifluormethyl)benzyl]-4H-1,2,4-triazol-3-yl}methyl)-2,4-dihydro-3H-1,2,4-triazol-3-on

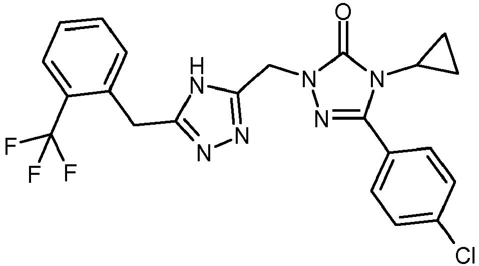

Analog zur Herstellung der Verbindung in Beispiel 21 wurden ausgehend von 100 mg (0.33 mmol) der Verbindung aus Beispiel 21A 54 mg (35% d. Th.) der Titelverbindung erhalten.
MS [ESIpos]: m/z = 475 (M+H)⁺
¹H-NMR (400 MHz, CDCl₃): δ = 0.70-0.80 (m, 2H), 0.96-1.06 (m, 2H), 2.92-3.03 (m, 1H), 4.30 (s, 2H), 5.15 (s, 2H), 7.30-7.40 (m, 2H), 7.40-7.51 (m, 3H), 7.61-7.71 (m, 3H), 10.80 (br. s, 1H).

### Beispiel 23

### 5-(4-Chlorphenyl)-4-cyclopropyl-2-({5-[3-(trifluormethyl)benzyl]-4H-1,2,4-triazol-3-yl}methyl)-2,4-dihydro-3H-1,2,4-triazol-3-on

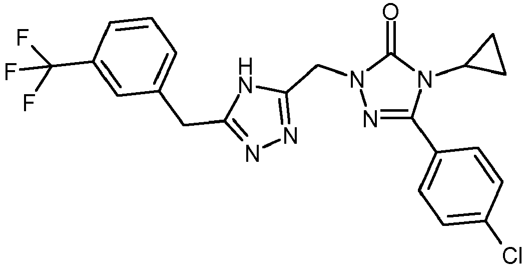

Analog zur Herstellung der Verbindung in Beispiel 21 wurden ausgehend von 75 mg (0.24 mmol) der Verbindung aus Beispiel 21A 55 mg (48% d. Th.) der Titelverbindung erhalten.
LC/MS [Methode 5]: Rₜ = 2.42 min; MS [ESIpos]: m/z = 475 (M+H)⁺
¹H-NMR (400 MHz, CDCl₃): δ = 0.70-0.79 (m, 2H), 0.96-1.06 (m, 2H), 2.93-3.03 (m, 1H), 4.14 (s, 2H), 5.15 (s, 2H), 7.38-7.51 (m, 5H), 7.56 (s, 1H), 7.62-7.70 (m, 2H).

### Beispiel 24

### 5-(4-Chlorphenyl)-4-cyclopropyl-2-{[5-(2,6-dichlorbenzyl)-4H-1,2,4-triazol-3-yl]methyl}-2,4-dihydro-3H-1,2,4-triazol-3-on

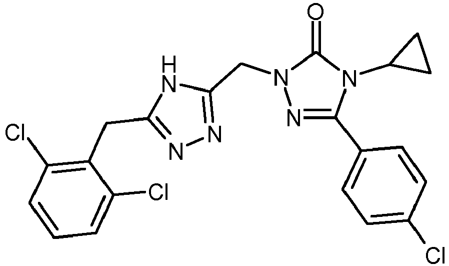

58 mg (0.24 mmol) 2-(2,6-Dichlorphenyl)ethanimidamid-Hydrochlorid wurden in 1 ml trockenem Methanol vorgelegt, mit 66 µl (0.24 mmol) einer 25%-igen methanolischen Natriummethylat-Lösung versetzt und 1 h bei RT gerührt. Anschließend gab man 50 mg (0.16 mmol) der Verbindung aus Beispiel 21A, gelöst in 0.6 ml Methanol, hinzu. Der Ansatz wurde über Nacht bei Raumtemperatur gerührt. Der ausgefallene farblose Feststoff wurde abgesaugt, mit wenig Methanol gewaschen und im Hochvakuum getrocknet. Der Feststoff wurde dann in Xylol suspendiert und 4 h unter Rückfluss gerührt. Nach dem Abkühlen dampfte man das Gemisch im Vakuum ein und reinigte den Rückstand durch Chromatographie [Methode 19]. Es wurden 30 mg (39% d. Th.) der Zielverbindung als farbloser Feststoff erhalten.
LC/MS [Methode 5]: Rₜ = 2.38 min; MS [ESIpos]: m/z = 476 (M+H)⁺
¹H-NMR (400 MHz, CDCl₃): δ = 0.70-0.80 (m, 2H), 0.96-1.06 (m, 2H), 2.93-3.04 (m, 1H), 4.46 (s, 2H), 5.13 (s, 2H), 7.19 (t, 1H), 7.35 (d, 2H), 7.44 (d, 2H), 7.67 (d, 2H), 11.00 (br. s, 1H).

### Beispiel 25

### 5-(5-Chlorthiophen-2-yl)-4-(2-fluorbenzyl)-2-({5-[3-(trifluormethyl)phenyl]-4H-1,2,4-triazol-3-yl}-methyl)-2,4-dihydro-3H-1,2,4-triazol-3-on

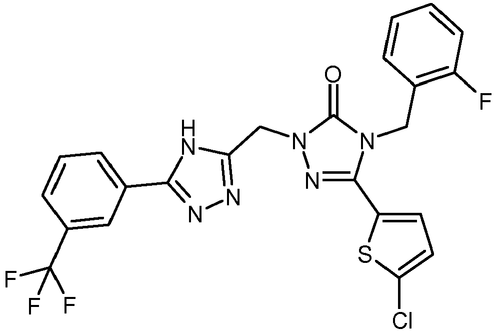

100 mg (0.26 mmol) der Verbindung aus Beispiel 22A wurden in 1.0 ml DMF gelöst, mit 88 mg (0.39 mmol) 3-(Trifluormethyl)benzolcarboximidamid-Hydrochlorid versetzt und 30 min im Mikrowellenofen bei 220°C gerührt. Der Ansatz wurde nach dem Abkühlen am Rotationsverdampfer unter vermindertem Druck eingeengt und der Rückstand chromatographisch gereinigt [Methode 19]. Man erhielt 71 mg (51% d. Th.) der Zielverbindung als farbloses Harz.
MS [ESIpos]: m/z = 535 (M+H)⁺
¹H-NMR (400 MHz, CDCl₃): δ = 5.14 (s, 2H), 5.34 (s, 2H), 6.85 (s, 1H), 6.95 (s, 1H), 7.04-7.16 (m, 3H), 7.28-7.37 (m, 1H), 7.50-7.60 (m, 1H), 7.61-7.70 (m, 1H), 8.20-8.30 (m, 1H), 8.35 (s, 1H), 12.00 (br. s, 1H).

### Beispiel 26

### 5-(5-Chlorthiophen-2-yl)-4-(2-fluorbenzyl)-2-({5-[2-(trifluormethyl)benzyl]-4H-1,2,4-triazol-3-yl}-methyl)-2,4-dihydro-3H-1,2,4-triazol-3-on

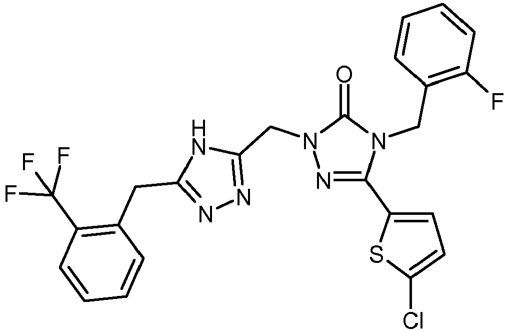

Analog zur Herstellung der Verbindung in Beispiel 25 wurden ausgehend von 75 mg (0.20 mmol) der Verbindung aus Beispiel 22A 19 mg (18% d. Th.) der Titelverbindung erhalten.
LC/MS [Methode 5]: Rₜ = 2.62 min; MS [ESIpos]: m/z = 549 (M+H)⁺
¹H-NMR (400 MHz, CDCl₃): δ = 4.32 (s, 2H), 5.11 (s, 2H), 5.21 (s, 2H), 6.82 (d, 1H), 6.90 (d, 1H), 7.03-7.16 (m, 3H), 7.25-7.32 (m, 1H), 7.32-7.44 (m, 2H), 7.45-7.52 (m, 1H), 7.68 (d, 1H), 10.70 (br. s, 1H).

### Beispiel 27

### 5-(5-Chlorthiophen-2-yl)-4-(2-fluorbenzyl)-2-({5-[3-(trifluormethyl)benzyl]-4H-1,2,4-triazol-3-yl}-methyl)-2,4-dihydro-3H-1,2,4-triazol-3-on

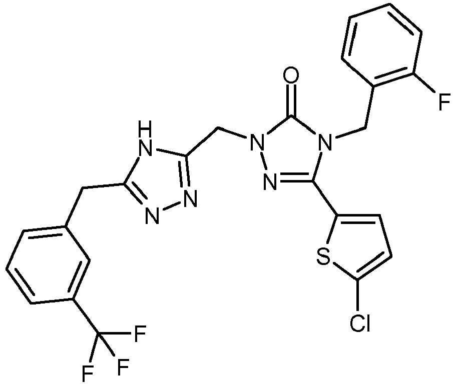

Analog zur Herstellung der Verbindung in Beispiel 25 wurden ausgehend von 75 mg (0.20 mmol) der Verbindung aus Beispiel 22A 29 mg (27% d. Th.) der Titelverbindung erhalten.
MS [ESIpos]: m/z = 549 (M+H)⁺
¹H-NMR (400 MHz, CDCl₃): δ = 4.15 (s, 2H), 5.11 (s, 2H), 5.22 (s, 2H), 6.84 (d, 1H), 6.91 (d, 1H), 7.03-7.15 (m, 3H), 7.25-7.34 (m, 1H), 7.44 (d, 1H), 7.48-7.53 (m, 2H), 7.58 (s, 1H), 11.10 (br. s, 1H).

### Beispiel 28

### Methyl-3-{[3-(4-chlorphenyl)-5-oxo-1-({5-[2-(trifluormethyl)benzyl]-4H-1,2,4-triazol-3-yl}methyl)-1,5-dihydro-4H-1,2,4-triazol-4-yl]methyl}benzolcarboxylat

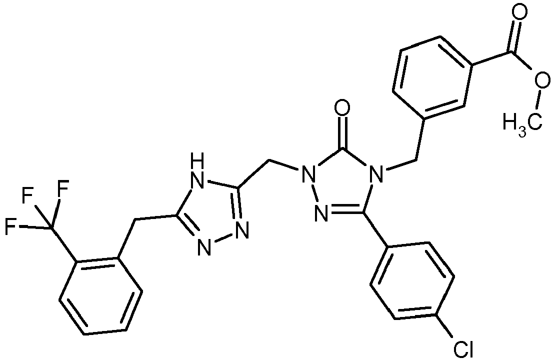

77 mg (0.27 mmol) 2-[2-(Trifluormethyl)phenyl]ethanimidamid-Hydrobromid wurden in 1 ml trockenem Methanol gelöst, mit 74 µl (0.27 mmol) einer 25%-igen methanolischen Natriummethylat-Lösung versetzt und 30 min gerührt. Dann gab man 75 mg (0.18 mmol) der Verbindung aus Beispiel 16A hinzu und rührte zunächst 16 h bei RT und anschließend 5 h unter Rückfluss. Das Gemisch wurde danach direkt chromatographisch aufgereinigt [Methode 19]. Man erhielt 67 mg (64% d. Th.) der Zielverbindung als farblosen Schaum.
LC/MS [Methode 5]: Rₜ = 2.59 min; MS [ESIpos]: m/z = 583 (M+H)⁺
¹H-NMR (400 MHz, CDCl₃): δ = 3.88 (s, 3H), 4.31 (s, 2H), 5.00 (s, 2H), 5.25 (s, 2H), 7.29-7.44 (m, 8H), 7.49 (d, 1H), 7.58 (d, 1H), 7.90 (s, 1H), 7.95 (d, 1H).

### Beispiel 29

### Methyl-3-{[3-(4-chlorphenyl)-1-{[5-(2,6-dichlorbenzyl)-4H-1,2,4-triazol-3-yl]methyl}-5-oxo-1,5-dihydro-4H-1,2,4-triazol-4-yl]methyl}benzolcarboxylat

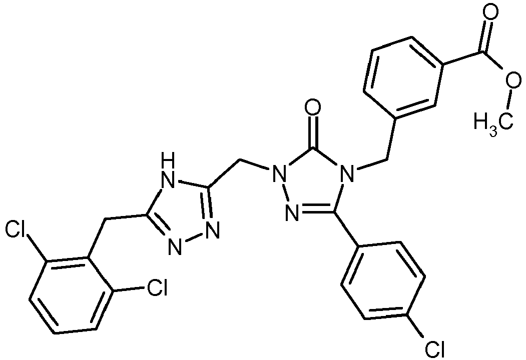

Analog zur Herstellung der Verbindung in Beispiel 28 wurden ausgehend von 80 mg (0.19 mmol) der Verbindung aus Beispiel 16A 67 mg (60% d. Th.) der Titelverbindung erhalten.
MS [ESIpos]: m/z = 583 und 585 (M+H)⁺
¹H-NMR (400 MHz, CDCl₃): δ = 3.90 (s, 3H), 4.49 (s, 2H), 5.00 (s, 2H), 5.24 (s, 2H), 7.19 (t, 1H), 7.28-7.44 (m, 8H), 7.88-8.00 (m, 2H), 11.20 (br. s, 1H).

### Beispiel 30

### 5-(4-Chlorphenyl)-4-(3,3,3-trifluor-2-hydroxypropyl)-2-({5-[2-(trifluormethyl)benzyl]-4H-1,2,4-triazol-3-yl}methyl)-2,4-dihydro-3H-1,2,4-triazol-3-on

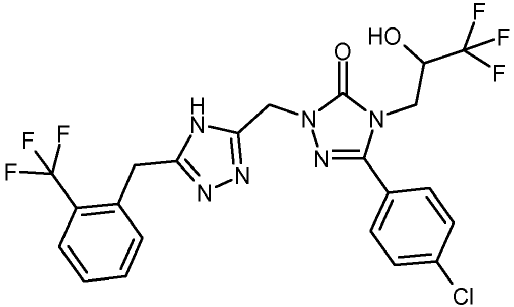

370 mg (0.97 mmol) der Verbindung aus Beispiel 18A wurden in 5 ml DMF gelöst, mit 349 mg (1.46 mmol) 2-[2-(Trifluormethyl)phenyl]ethanimidamid-Hydrochlorid versetzt und 90 min im Mikrowellenofen bei 200°C gerührt. Nach dem Abkühlen wurde der Ansatz mit 5 ml Methanol verdünnt und direkt chromatographisch gereinigt [Methode 19]. Es wurden 170 mg (32% d. Th.) der Zielverbindung erhalten.
LC/MS [Methode 3]: Rₜ = 1.24 min; MS [ESIpos]: m/z = 547 (M+H)⁺
¹H-NMR (400 MHz, CDCl₃): δ = 3.87-4.00 (m, 2H), 4.21 (s, 2H), 4.56-4.62 (m, 1H), 5.03-5.21 (m, 2H), 5.70 (br. s, 1H), 7.31 (d, 1H), 7.38 (t, 1H), 7.44 (d, 2H), 7.49 (t, 1H), 7.59 (s, 2H), 7.67 (d, 1H), 11.48 (br. s, 1H).

### Beispiel 31

### 3-{[3-(4-Chlorphenyl)-5-oxo-1-({5-[2-(trifluormethyl)benzyl]-4H-1,2,4-triazol-3-yl}methyl)-1,5-dihydro-4H-1,2,4-triazol-4-yl]methyl}benzolcarbonsäure

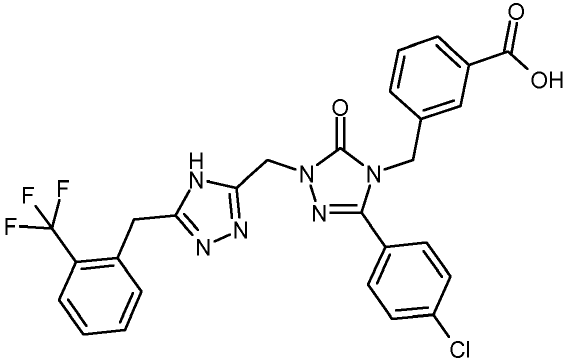

62 mg (0.11 mmol) der Verbindung aus Beispiel 28 wurden in 1 ml Ethanol suspendiert und mit 213 µl (0.21 mmol) 1 M Natronlauge versetzt. Es wurde 4 h bei 50°C gerührt. Nach dem Abkühlen auf RT neutralisierte man mit 215 µl 1 M Salzsäure, engte im Vakuum ein und reinigte den Rückstand durch Chromatographie [Methode 19]. Es wurden 37 mg (61% d. Th.) der Zielverbindung als farbloser Schaum erhalten.
MS [ESIpos]: m/z = 569 (M+H)⁺
¹H-NMR (400 MHz, DMSO-d₆): δ = 4.04-4.35 (m, 2H), 4.90-5.25 (m, 4H), 7.25-7.38 (m, 1H), 7.38-7.54 (m, 7H), 7.54-7.65 (m, 1H), 7.65-7.78 (m, 2H), 7.80 (d, 1H), 13.02 (br. s, 1H), 13.65-13.93 (br. s, 1H).

### Beispiel 32

### 3-{[3-(4-Chlorphenyl)-1-{[5-(2,6-dichlorbenzyl)-4H-1,2,4-triazol-3-yl]methyl}-5-oxo-1,5-dihydro-4H-1,2,4-triazol-4-yl]methyl}benzolcarbonsäure

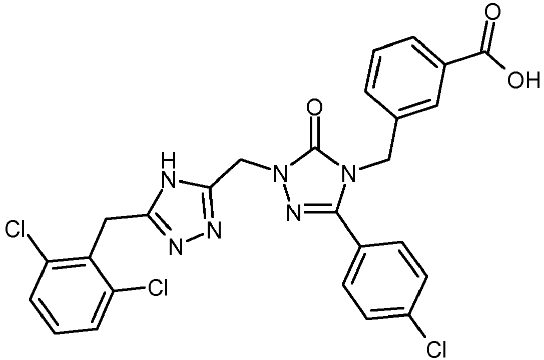

Analog zur Herstellung der Verbindung in Beispiel 31 wurden ausgehend von 60 mg (0.10 mmol) der Verbindung aus Beispiel 29 40 mg (68% d. Th.) der Titelverbindung erhalten.
MS [ESIpos]: m/z = 569 (M+H)⁺
¹H-NMR (400 MHz, DMSO-d₆): δ = 4.16-4.44 (m, 2H), 4.85-5.25 (m, 4H), 7.24-7.40 (m, 2H), 7.43 (t, 1H), 7.45-7.58 (m, 6H), 7.70 (s, 1H), 7.82 (d, 1H), 13.03 (br. s, 1H), 13.70 (br. s, 1H).

### Beispiel 33

### 5-(4-Chlorphenyl)-4-cyclopropyl-2-[(5-{2-[3-(trifluormethyl)phenyl]propan-2-yl}-4H-1,2,4-triazol-3-yl)methyl]-2,4-dihydro-3H-1,2,4-triazol-3-on

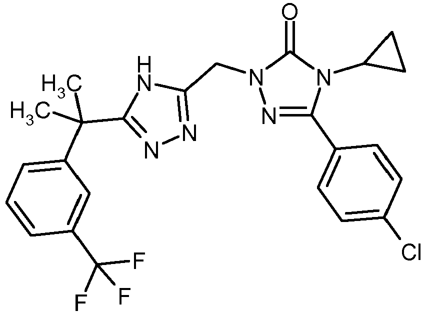

42 mg (0.14 mmol) der Verbindung aus Beispiel 21A wurden in 1.2 ml DMF gelöst, mit 40 mg (0.15 mmol) 2-Methyl-2-[3-(trifluormethyl)phenyl]propanimidamid-Hydrochlorid und 9 mg (0.16 mmol) Natriummethylat versetzt und 2 h im Mikrowellenreaktor bei 180°C gerührt. Nach dem Abkühlen auf RT wurde die Mischung direkt durch präparative HPLC aufgereinigt [Methode 19]. Man erhielt 2 mg (3% d. Th.) der Zielverbindung als farblosen Schaum.
LC/MS [Methode 1]: Rₜ = 2.06 min; MS [ESIpos]: m/z = 503 (M+H)⁺
¹H-NMR (400 MHz, CDCl₃): δ = 0.75-0.79 (m, 2H), 1.00-1.05 (m, 2H), 1.79 (s, 6H), 2.96-3.02 (m, 1H), 5.16 (s, 2H), 7.38-7.43 (q, 1H), 7.45 (d, 2H), 7.46-7.49 (m, 2H), 7.59 (s, 1H), 7.67 (d, 2H).

### Beispiel 34

### 5-(4-Chlorphenyl)-4-cyclopropyl-2-({5-[1-(2-fluorphenyl)-1-methylethyl]-4H-1,2,4-triazol-3-yl}-methyl)-2,4-dihydro-3H-1,2,4-triazol-3-on

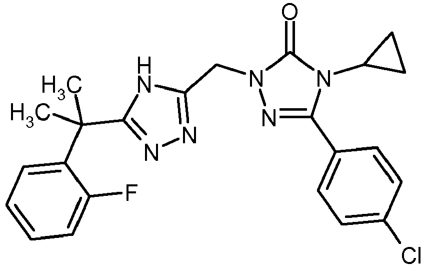

60 mg (0.20 mmol) der Verbindung aus Beispiel 21A wurden in 1 ml DMF gelöst und mit 63 mg (0.29 mmol) 2-(2-Fluorphenyl)-2-methylpropanimidamid-Hydrochlorid sowie 17 mg (0.31 mmol) Natriummethylat versetzt. Die Suspension wurde 8 h bei 150°C gerührt. Anschließend wurde die Umsetzung durch 45 min Rühren im Mikrowellenreaktor bei 200°C vervollständigt. Die Suspension wurde mit ca. 1 ml Methanol verdünnt, filtriert und das Filtrat durch präparative HPLC aufgereinigt [Methode 19]. Man erhielt 13 mg (15% d. Th.) der Titelverbindung.
LC/MS [Methode 5]: Rₜ = 2.30 min; MS [ESIpos]: m/z = 453 (M+H)⁺
¹H-NMR (400 MHz, CDCl₃): δ = 0.75 (m, 2H), 0.95 (m, 2H), 1.88 (s, 6H), 2.99 (m, 1H), 5.32 (s, 2H), 6.93-7.00 (m, 1H), 7.14-7.21 (m, 1H), 7.38-7.49 (m, 4H), 7.62-7.72 (m, 2H).

### Beispiel 35

### 5-(4-Chlorphenyl)-4-cyclopropyl-2-[(5-{2-[3-(trifluormethyl)phenyl]ethyl}-4H-1,2,4-triazol-3-yl)-methyl]-2,4-dihydro-3H-1,2,4-triazol-3-on

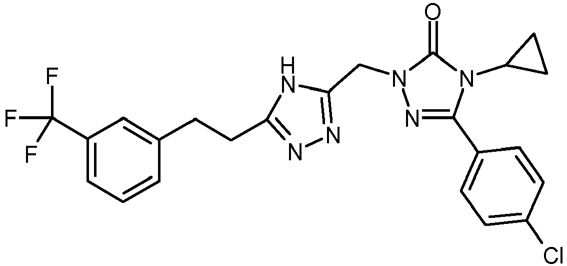

50 mg (0.16 mmol) der Verbindung aus Beispiel 21A wurden in 1 ml DMF aufgenommen, mit 62 mg (0.24 mmol) 3-[3-(Trifluormethyl)phenyl]propanimidamid-Hydrochlorid versetzt und 1 h im Mikrowellenofen bei 180°C gerührt. Nach dem Abkühlen wurde das Reaktionsgemisch mit Methanol verdünnt und die Lösung direkt durch präparative HPLC [Methode 19] aufgetrennt. Man erhielt 11 mg (14% d. Th.) der Titelverbindung.
LC/MS [Methode 1]: Rₜ = 1.96 min; MS [ESIpos]: m/z = 489 (M+H)⁺
¹H-NMR (400 MHz, CDCl₃): δ = 0.71-0.81 (m, 2H), 0.98-1.06 (m, 2H), 2.99 (m, 1H), 3.03-3.09 (m, 2H), 3.10-3.16 (m, 2H), 5.16 (s, 2H), 7.37 (d, 2H), 7.41-7.48 (m, 4H), 7.68 (d, 2H).

### Beispiel 36

### 5-(4-Chlorphenyl)-4-cyclopropyl-2-{[5-(2-ethoxybenzyl)-4H-1,2,4-triazol-3-yl]methyl}-2,4-dihydro-3H-1,2,4-triazol-3-on

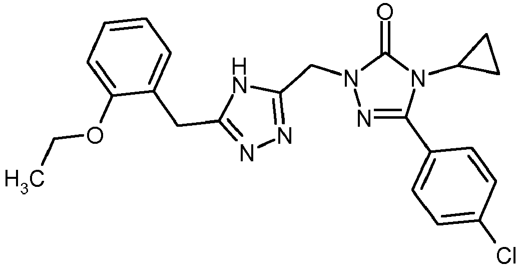

50 mg (0.16 mmol) der Verbindung aus Beispiel 21A wurden in 1 ml DMF aufgenommen, mit 52 mg (0.24 mmol) 2-(2-Ethoxyphenyl)ethanimidamid-Hydrochlorid versetzt und 1 h im Mikrowellenofen bei 200°C gerührt. Nach dem Abkühlen wurde der Ansatz mit ca. 1 ml Methanol verdünnt und die Lösung direkt durch präparative HPLC [Methode 19] aufgetrennt. Es wurden 21 mg (28% d. Th.) der Titelverbindung erhalten.
LC/MS [Methode 3]: Rₜ = 1.14 min; MS [ESIpos]: m/z = 451 (M+H)⁺
¹H-NMR (400 MHz, CDCl₃): δ = 0.75-0.79 (m, 2H), 0.97-1.02 (m, 2H), 1.44 (t, 3H), 2.97 (m, 1H), 4.09-4.14 (m, 4H), 5.10 (s, 2H), 6.91 (t, 2H), 7.25 (t, 2H), 7.41 (2d, 2H), 7.67 (d, 2H).

### Beispiel 37

### 5-(4-Chlorphenyl)-4-cyclopropyl-2-{[5-(3-fluorbenzyl)-4H-1,2,4-triazol-3-yl]methyl}-2,4-dihydro-3H-1,2,4-triazol-3-on

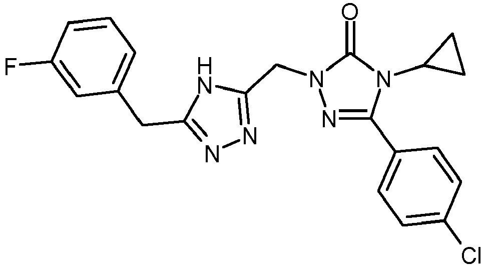

Analog zur Herstellung der Verbindung in Beispiel 36 wurden ausgehend von 50 mg (0.16 mmol) der Verbindung aus Beispiel 21A 11 mg (16% d. Th.) der Titelverbindung erhalten.
LC/MS [Methode 5]: Rₜ = 2.16 min; MS [ESIpos]: m/z = 425 (M+H)⁺
¹H-NMR (400 MHz, CDCl₃): δ = 0.75-0.79 (m, 2H), 0.99-1.05 (m, 2H), 2.98 (m, 1H), 4.10 (s, 2H), 5.15 (s, 2H), 6.93 (m, 1H), 7.00 (d, 1H), 7.07 (d, 1H), 7.23-7.30 (m, 1H), 7.44 (d, 2H), 7.67 (d, 2H).

### Beispiel 38

### 5-(4-Chlorphenyl)-4-cyclopropyl-2-{[5-(2-methoxybenzyl)-4H-1,2,4-triazol-3-yl]methyl}-2,4-dihydro-3H-1,2,4-triazol-3-on

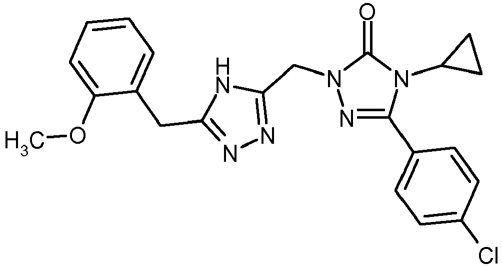

Analog zur Herstellung der Verbindung in Beispiel 36 wurden ausgehend von 50 mg (0.16 mmol) der Verbindung aus Beispiel 21A 11 mg (16% d. Th.) der Titelverbindung erhalten.
LC/MS [Methode 3]: Rₜ = 1.09 min; MS [ESIpos]: m/z = 437 (M+H)⁺.

### Beispiel 39

### 5-(4-Chlorphenyl)-4-cyclopropyl-2-{[5-(3-methylbenzyl)-4H-1,2,4-triazol-3-yl]methyl}-2,4-dihydro-3H-1,2,4-triazol-3-on

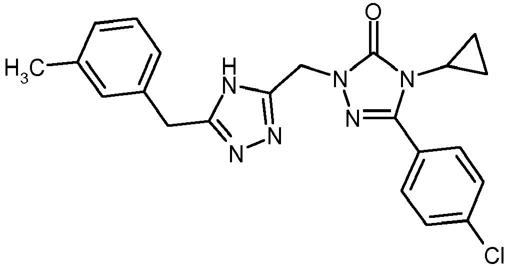

Analog zur Herstellung der Verbindung in Beispiel 36 wurden ausgehend von 50 mg (0.16 mmol) der Verbindung aus Beispiel 21A 11 mg (16% d. Th.) der Titelverbindung erhalten.
LC/MS [Methode 3]: Rₜ = 1.13 min; MS [ESIpos]: m/z = 421 (M+H)^{+ 1}H-NMR (400 MHz, CDCl₃): δ = 0.73-0.79 (m, 2H), 0.97-1.04 (m, 2H), 2.31 (s, 3H), 2.93-3.01 (m, 1H), 4.07 (s, 2H), 5.13 (s, 2H), 7.04-7.11 (m, 3H), 7.18-7.23 (m, 1H), 7.43 (d, 2H), 7.67 (d, 2H).

### Beispiel 40

### 2-{[5-(2-Chlorbenzyl)-4H-1,2,4-triazol-3-yl]methyl}-5-(4-chlorphenyl)-4-cyclopropyl-2,4-dihydro-3H-1,2,4-triazol-3-on

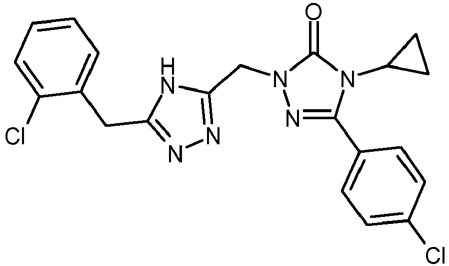

Analog zur Herstellung der Verbindung in Beispiel 36 wurden ausgehend von 50 mg (0.16 mmol) der Verbindung aus Beispiel 21A 22 mg (31% d. Th.) der Titelverbindung erhalten.
LC/MS [Methode 3]: Rₜ = 1.13 min; MS [ESIpos]: m/z = 441 (M+H)⁺
¹H-NMR (400 MHz, CDCl₃): δ = 0.74-0.79 (m, 2H), 0.99-1.04 (m, 2H), 2.98 (m, 1H), 4.25 (s, 2H), 5.14 (s, 2H), 7.21-7.23 (m, 2H), 7.30-7.32 (m, 1H), 7.38-7.40 (m, 1H), 7.44 (d, 2H), 7.67 (d, 2H).

### Beispiel 41

### 5-(4-Chlorphenyl)-4-cyclopropyl-2-{[5-(2-fluorbenzyl)-4H-1,2,4-triazol-3-yl]methyl}-2,4-dihydro-3H-1,2,4-triazol-3-on

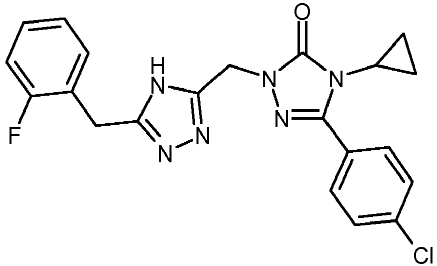

Analog zur Herstellung der Verbindung in Beispiel 36 wurden ausgehend von 50 mg (0.16 mmol) der Verbindung aus Beispiel 21A 11 mg (16% d. Th.) der Titelverbindung erhalten.
LC/MS [Methode 5]: Rₜ = 2.07 min; MS [ESIpos]: m/z = 425 (M+H)⁺
¹H-NMR (400 MHz, CDCl₃): δ = 0.74-0.79 (m, 2H), 0.98-1.05 (m, 2H), 2.98 (m, 1H), 4.14 (s, 2H), 5.14 (s, 2H), 7.03-7.12 (m, 2H), 7.21-7.31 (m, 2H), 7.44 (d, 2H), 7.67 (d, 2H).

### Beispiel 42

### 5-(4-Chlorphenyl)-2-{[5-(2,6-dichlorbenzyl)-4H-1,2,4-triazol-3-yl]methyl}-4-(4-methoxybenzyl)-2,4-dihydro-3H-1,2,4-triazol-3-on

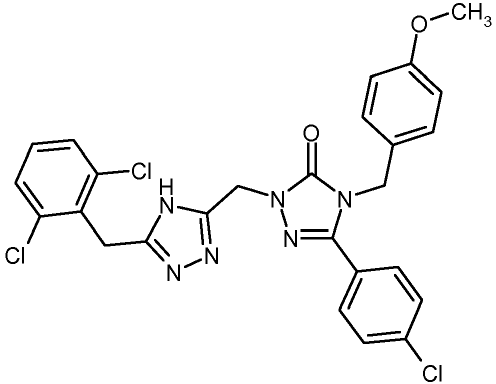

Analog zur Herstellung der Verbindung in Beispiel 36 wurden ausgehend von 70 mg (0.18 mmol) der Verbindung aus Beispiel 17A 36 mg (36% d. Th.) der Titelverbindung erhalten.
LC/MS [Methode 3]: Rₜ = 1.30 min; MS [ESIpos]: m/z = 557 (M+H)⁺
¹H-NMR (400 MHz, CDCl₃): δ = 3.78 (s, 3H), 4.47 (s, 2H), 4.86 (s, 2H), 5.22 (s, 2H), 6.82 (d, 2H), 7.06 (d, 2H), 7.18 (t, 1H), 7.33-7.39 (m, 6H).

### Beispiel 43

### 5-(4-Chlorphenyl)-2-{[5-(2,6-dichlorbenzyl)-4H-1,2,4-triazol-3-yl]methyl}-4-[(2R)-3,3,3-trifluor-2-hydroxypropyl]-2,4-dihydro-3H-1,2,4-triazol-3-on

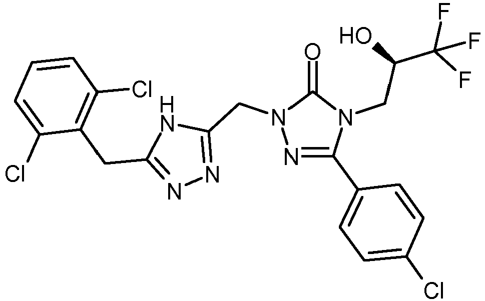

Analog zur Herstellung der Verbindung in Beispiel 36 wurden ausgehend von 70 mg (0.18 mmol) der Verbindung aus Beispiel 20A 21 mg (21% d. Th.) der Titelverbindung erhalten.
LC/MS [Methode 3]: Rₜ = 1.23 min; MS [ESIpos]: m/z = 548 (M+H)⁺
¹H-NMR (400 MHz, CDCl₃): δ = 3.86-3.99 (m, 2H), 4.36-4.45 (m, 2H), 4.56-4.66 (m, 1H), 5.07 (d, 1H), 5.18 (d, 1H), 7.20 (t, 1H), 7.35 (d, 2H), 7.44 (d, 2H), 7.58 (d, 2H).

### Beispiel 44

### 5-(4-Chlorphenyl)-4-[(2R)-3,3,3-trifluor-2-hydroxypropyl]-2-({5-[3-(trifluormethyl)benzyl]-4H-1,2,4-triazol-3-yl}methyl)-2,4-dihydro-3H-1,2,4-triazol-3-on

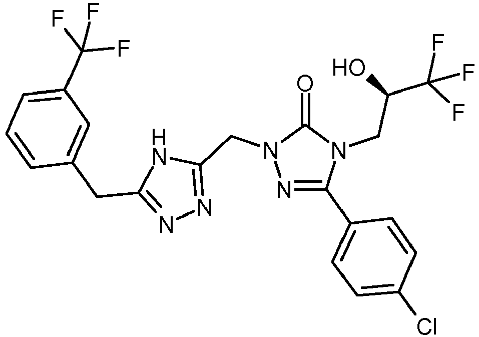

Analog zur Herstellung der Verbindung in Beispiel 36 wurden ausgehend von 70 mg (0.18 mmol) der Verbindung aus Beispiel 20A 21 mg (21% d. Th.) der Titelverbindung erhalten.
LC/MS [Methode 5]: Rₜ = 2.43 min; MS [ESIpos]: m/z = 547 (M+H)⁺
¹H-NMR (400 MHz, CDCl₃): δ = 3.86-4.00 (m, 2H), 4.05 (s, 2H), 4.56-4.66 (m, 1H), 5.08 (d, 1H), 5.21 (d, 1H), 7.38-7.47 (m, 4H), 7.50-7.55 (m, 2H), 7.61 (d, 2H).

### Beispiel 45

### 5-(4-Chlorphenyl)-4-[(2R)-3,3,3-trifluor-2-hydroxypropyl]-2-[(5-{2-[3-(trifluormethyl)phenyl]-ethyl}-4H-1,2,4-triazol-3-yl)methyl]-2,4-dihydro-3H-1,2,4-triazol-3-on

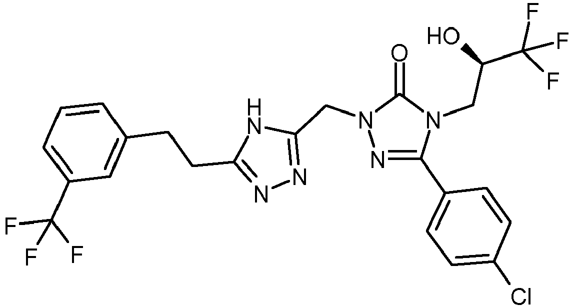

Analog zur Herstellung der Verbindung in Beispiel 36 wurden ausgehend von 70 mg (0.18 mmol) der Verbindung aus Beispiel 20A 18 mg (17% d. Th.) der Titelverbindung erhalten.
LC/MS [Methode 5]: Rₜ = 2.49 min; MS [ESIpos]: m/z = 561 (M+H)⁺
¹H-NMR (400 MHz, CDCl₃): δ = 2.91-2.99 (m, 2H), 3.02-3.10 (m, 2H), 3.87-4.01 (m, 2H), 4.59-4.69 (m, 1H), 5.09 (d, 1H), 5.25 (d, 1H), 7.32 (d, 1H), 7.38 (m, 1H), 7.41-7.49 (m, 2H), 7.44 (d, 2H), 7.63 (d, 2H).

### Beispiel 46

### 2-{[5-(2-Chlorbenzyl)-4H-1,2,4-triazol-3-yl]methyl}-5-(4-chlorphenyl)-4-[(2R)-3,3,3-trifluor-2-hydroxypropyl]-2,4-dihydro-3H-1,2,4-triazol-3-on

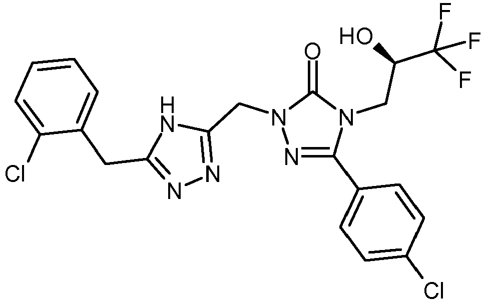

Analog zur Herstellung der Verbindung in Beispiel 36 wurden ausgehend von 70 mg (0.18 mmol) der Verbindung aus Beispiel 20A 17 mg (18% d. Th.) der Titelverbindung erhalten.
LC/MS [Methode 5]: Rₜ = 2.30 min; MS [ESIpos]: m/z = 513 (M+H)⁺
¹H-NMR (400 MHz, CDCl₃): δ = 3.83-3.97 (m, 2H), 4.04-4.16 (m, 2H), 4.59-4.70 (m, 1H), 5.03 (d, 1H), 5.14 (d, 1H), 7.19-7.25 (m, 3H), 7.36-7.40 (m, 1H), 7.42 (d, 2H), 7.59 (d, 2H).

### Beispiel 47

### 5-(4-Chlorphenyl)-2-{[5-(2-methoxybenzyl)-4H-1,2,4-triazol-3-yl]methyl}-4-[(2R)-3,3,3-trifluor-2-hydroxypropyl]-2,4-dihydro-3H-1,2,4-triazol-3-on

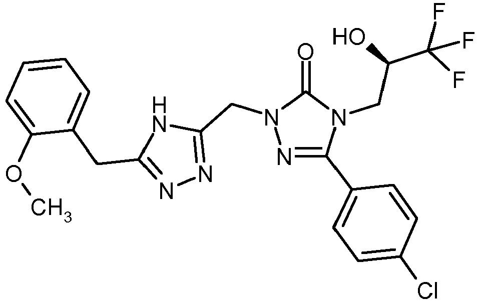

Analog zur Herstellung der Verbindung in Beispiel 36 wurden ausgehend von 70 mg (0.18 mmol) der Verbindung aus Beispiel 20A 16 mg (17% d. Th.) der Titelverbindung erhalten.
LC/MS [Methode 5]: Rₜ = 2.25 min; MS [ESIpos]: m/z = 509 (M+H)⁺
¹H-NMR (400 MHz, CDCl₃): δ = 3.86 (s, 3H), 3.83-3.99 (m, 2H), 4.01 (s, 2H), 4.63-4.71 (m, 1H), 5.01 (d, 1H), 5.19 (d, 1H), 6.02 (d, 1H), 6.88-6.95 (m, 2H), 7.13-7.19 (m, 1H), 7.25-7.29 (m, 1H), 7.43 (d, 2H), 7.61 (d, 2H), 11.03-11.15 (br. s, 1H).

### Beispiel 48

### 5-(4-Chlorphenyl)-2-{[5-(2-fluorbenzyl)-4H-1,2,4-triazol-3-yl]methyl}-4-[(2R)-3,3,3-trifluor-2-hydroxypropyl]-2,4-dihydro-3H-1,2,4-triazol-3-on

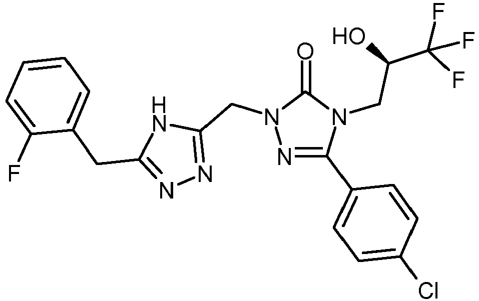

Analog zur Herstellung der Verbindung in Beispiel 36 wurden ausgehend von 100 mg (0.26 mmol) der Verbindung aus Beispiel 20A 34 mg (26% d. Th.) der Titelverbindung erhalten.
LC/MS [Methode 5]: Rₜ = 2.24 min; MS [ESIpos]: m/z = 497 (M+H)⁺
¹H-NMR (400 MHz, CDCl₃): δ = 3.83-3.98 (m, 2H), 3.97-4.03 (m, 2H), 4.59-4.71 (m, 1H), 5.02 (d, 1H), 5.15 (d, 1H), 6.00 (br. s, 1H), 7.02-7.12 (m, 2H), 7.15-7.21 (m, 1H), 7.22-7.30 (m, 1H), 7.42 (d, 2H), 7.60 (d, 2H), 11.87 (br. s, 1H).

### Beispiel 49

### 2-{[5-(2-Chlorbenzyl)-4H-1,2,4-triazol-3-yl]methyl}-5-(4-chlorphenyl)-4-[(2S)-3,3,3-trifluor-2-hydroxypropyl]-2,4-dihydro-3H-1,2,4-triazol-3-on

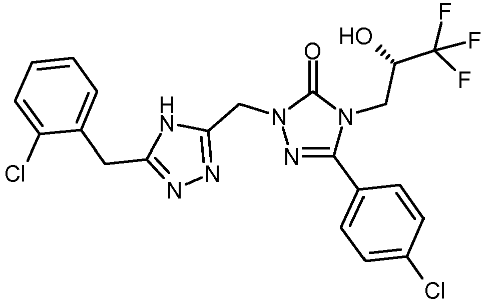

Analog zur Herstellung der Verbindung in Beispiel 36 wurden ausgehend von 138 mg (0.36 mmol) der Verbindung aus Beispiel 19A 35 mg (19% d. Th.) der Titelverbindung erhalten.
LC/MS [Methode 3]: Rₜ = 1.20 min; MS [ESIpos]: m/z = 513 und 515 (M+H)⁺
¹H-NMR (400 MHz, CDCl₃): δ = 3.89 (dd, 1H), 3.96 (dd, 1H), 4.07-4.18 (m, 2H), 4.58-4.69 (m, 1H), 5.05 + 5.16 (2d, 2H), 5.90 (br. s, 1H), 7.20-7.25 (m, 3H), 7.36-7.41 (m, 1H), 7.43 (d, 2H), 7.57-7.61 (m, 2H), 11.65 (br. s, 1H).

### Beispiel 50

### 5-(4-Chlorphenyl)-2-{[5-(2-fluorbenzyl)-4H-1,2,4-triazol-3-yl]methyl}-4-[(2S)-3,3,3-trifluor-2-hydroxypropyl]-2,4-dihydro-3H-1,2,4-triazol-3-on

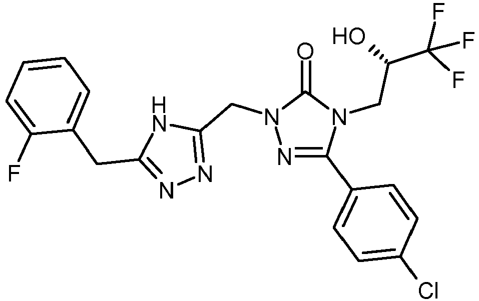

Analog zur Herstellung der Verbindung in Beispiel 36 wurden ausgehend von 124 mg (0.33 mmol) der Verbindung aus Beispiel 19A 37 mg (23% d. Th.) der Titelverbindung erhalten.
LC/MS [Methode 3]: Rₜ = 1.16 min; MS [ESIpos]: m/z = 497 (M+H)⁺
¹H-NMR (400 MHz, CDCl₃): δ = 3.89 (dd, 1H), 3.97 (dd, 1H), 3.98-4.08 (m, 2H), 4.61-4.70 (m, 1H), 5.04 + 5.16 (2d, 2H), 5.86 (br. s, 1H), 7.03-7.31 (m, 4H), 7.43 (d, 2H), 7.59 (d, 2H), 11.65 (br. s, 1H).

### Beispiel 51

### 5-(4-Chlorphenyl)-2-{[5-(2,6-dichlorbenzyl)-4H-1,2,4-triazol-3-yl]methyl}-4-[(2S)-3,3,3-trifluor-2-hydroxypropyl]-2,4-dihydro-3H-1,2,4-triazol-3-on

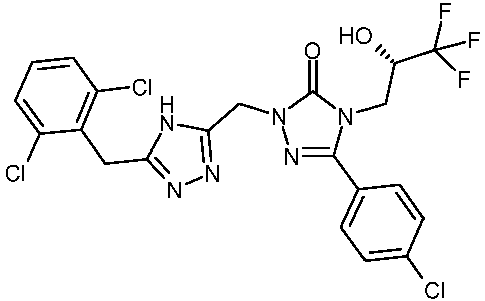

Analog zur Herstellung der Verbindung in Beispiel 36 wurden ausgehend von 106 mg (0.28 mmol) der Verbindung aus Beispiel 19A 49 mg (32% d. Th.) der Titelverbindung erhalten.
LC/MS [Methode 4]: Rₜ = 1.08 min; MS [ESIpos]: m/z = 547 und 549 (M+H)⁺
¹H-NMR (400 MHz, CDCl₃): δ = 3.88 (dd, 1H), 3.96 (dd, 1H), 4.34-4.46 (m, 2H), 4.57-4.67 (m, 1H), 5.03-5.22 (m, 2H), 7.16-7.23 (m, 1H), 7.35 (d, 2H), 7.44 (d, 2H), 7.60 (d, 2H).

### Beispiel 52

### 5-(4-Chlorphenyl)-4-[(2S)-3,3,3-trifluor-2-hydroxypropyl]-2-({5-[3-(trifluormethoxy)benzyl]-4H-1,2,4-triazol-3-yl}methyl)-2,4-dihydro-3H-1,2,4-triazol-3-on

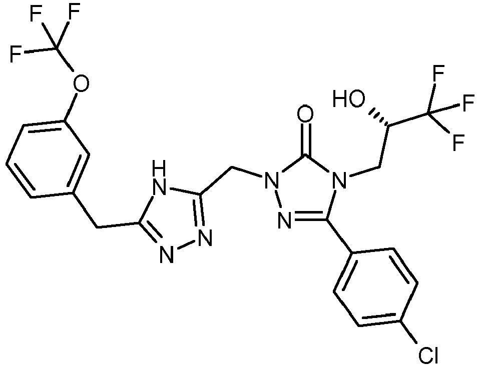

Analog zur Herstellung der Verbindung in Beispiel 36 wurden ausgehend von 120 mg (0.32 mmol) der Verbindung aus Beispiel 19A 83 mg (47% d. Th.) der Titelverbindung erhalten.
LC/MS [Methode 2]: Rₜ = 2.39 min; MS [ESIpos]: m/z = 563 (M+H)⁺
¹H-NMR (400 MHz, CDCl₃): δ = 3.86-3.96 (m, 2H), 3.95-4.05 (m, 2H), 4.57-4.67 (m, 1H), 5.04-5.24 (m, 2H), 7.09-7.17 (m, 3H), 7.30-7.36 (m, 1H), 7.45 (d, 2H), 7.60 (d, 2H).

### Beispiel 53

### 5-(4-Chlorphenyl)-4-[(2S)-3,3,3-trifluor-2-hydroxypropyl]-2-({5-[2-(trifluormethyl)benzyl]-4H-1,2,4-triazol-3-yl}methyl)-2,4-dihydro-3H-1,2,4-triazol-3-on

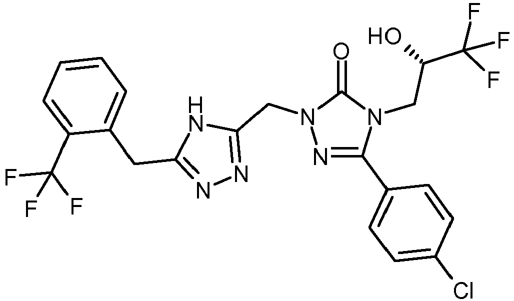

Analog zur Herstellung der Verbindung in Beispiel 36 wurden 1190 mg (3.13 mmol) der Verbindung aus Beispiel 19A umgesetzt. Zur Aufarbeitung wurde das Reaktionsgemisch mit 25 ml Wasser verdünnt und zweimal mit je 25 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 932 mg (54% d. Th.) der Titelverbindung erhalten.
LC/MS [Methode 4]: Rₜ = 1.08 min; MS [ESIpos]: m/z = 547 (M+H)⁺
¹H-NMR (400 MHz, CDCl₃): δ = 3.83-3.96 (m, 2H), 4.11-4.22 (m, 2H), 4.56-4.68 (m, 1H), 5.00-5.18 (m, 2H), 5.88 (br. s, 1H), 7.25-7.29 (m, 1H), 7.33-7.52 (m, 4H), 7.58 (d, 2H), 7.67 (d, 1H), 11.89 (br. s, 1H).

### Beispiel 54

### 5-(4-Chlorphenyl)-2-{[5-(2-chlorphenyl)-4H-1,2,4-triazol-3-yl]methyl}-4-[(2S)-3,3,3-trifluor-2-hydroxypropyl]-2,4-dihydro-3H-1,2,4-triazol-3-on

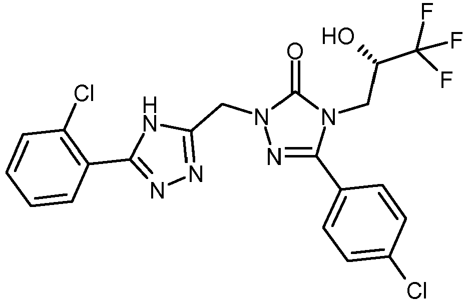

1200 mg (3.16 mmol) der Verbindung aus 19A wurden in 15 ml DMF aufgenommen, mit 906 mg (4.74 mmol) 2-Chlorbenzolcarboximidamid-Hydrochlorid versetzt und 1 h im Mikrowellenofen bei 220°C gerührt. Nach dem Abkühlen wurde der Ansatz mit 20 ml 1 N Salzsäure versetzt und zweimal mit je 25 ml Ethylacetat extrahiert. Die vereinigten organischen Phasen wurden über Natriumsulfat getrocknet, filtriert und im Vakuum eingeengt. Die weitere Reinigung erfolgte durch Chromatographie an Kieselgel (Elutionsmittel: Cyclohexan/Ethylacetat 2:1, dann 1:1). Es wurden 395 mg (80% d. Th.) der Titelverbindung mit einer Reinheit von 80% erhalten.
LC/MS [Methode 4]: Rₜ = 1.02 min; MS [ESIpos]: m/z = 499 und 501 (M+H)⁺
¹H-NMR (400 MHz, DMSO-d₆): δ = 3.85 (dd, 1H), 4.00 (dd, 1H), 4.25-4.37 (m, 1H), 5.00-5.30 (m, 2H), 6.92 (d, 1H), 7.40-7.67 (m, 5H), 7.71-7.81 (m, 3H), 14.27 (br. s, 1H).

### Beispiel 55

### Methyl-(3-{[3-(4-chlorphenyl)-5-oxo-4-(3,3,3-trifluor-2-hydroxypropyl)-4,5-dihydro-1H-1,2,4-triazol-1-yl]methyl}-5-[2-(trifluormethyl)benzyl]-1H-1,2,4-triazol-1-yl)acetat und Methyl-(5-{[3-(4-chlorphenyl)-5-oxo-4-(3,3,3-trifluor-2-hydroxypropyl)-4,5-dihydro-1H-1,2,4-triazol-1-yl]methyl}-3-[2-(trifluormethyl)benzyl]-1H-1,2,4-triazol-1-yl)acetat (Gemisch der Regioisomeren)

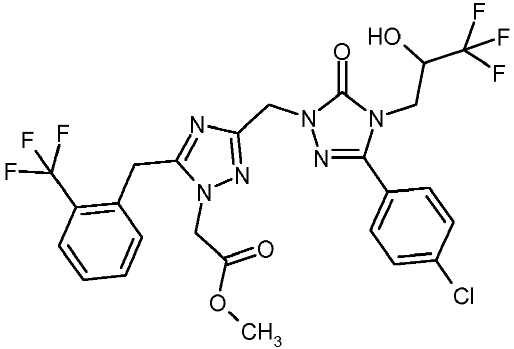

*und*

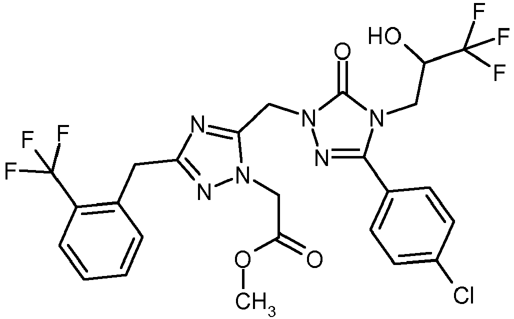

350 mg (0.57 mmol) der Verbindung aus Beispiel 30 wurden in 9 ml DMF gelöst und mit 27 mg (0.68 mmol) Natriumhydrid (60%-ig in Paraffin) versetzt. Es wurde 10 min bei RT gerührt. Anschließend gab man 68 mg (0.63 mmol) Chloressigsäuremethylester hinzu und rührte 30 min bei 40°C. Zur Aufarbeitung wurde das Gemisch mit 10 ml Wasser versetzt und zweimal mit je 15 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]; eine Trennung der Regioisomeren gelang hierbei nicht. Es wurden 310 mg (88% d. Th.) eines Gemisches der regioisomeren Titelverbindungen erhalten, das als solches weiter umgesetzt wurde (siehe Beispiele 58 und 59).
LC/MS [Methode 5]: Rₜ = 2.52 min; MS [ESIpos]: m/z = 619 (M+H)⁺ und Rₜ = 2.60 min; MS [ESIpos]: m/z = 619 (M+H)⁺.

### Beispiel 56 und Beispiel 57

### Methyl-[5-(2-chlorphenyl)-3-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]acetat (Regioisomer 1) und Methyl-[3-(2-chlorphenyl)-5-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]acetat (Regioisomer 2)

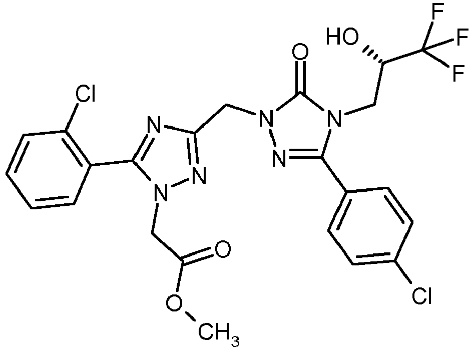

*und*

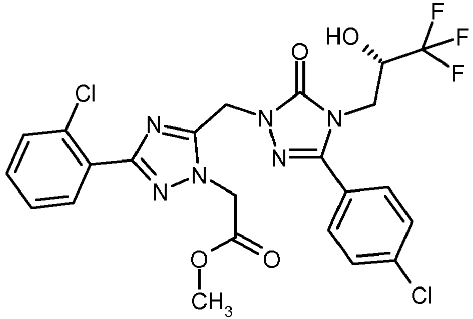

395 mg (0.79 mmol) der Verbindung aus Beispiel 54 wurden in 10 ml DMF gelöst und mit 38 mg (0.95 mmol) Natriumhydrid (60%-ig in Paraffin) versetzt. Es wurde 10 min bei RT gerührt. Anschließend gab man 94 mg (0.87 mmol) Chloressigsäuremethylester hinzu und rührte 30 min bei 40°C. Nach Abkühlen auf RT wurde das Reaktionsgemisch direkt, ohne weitere Aufarbeitung, unter vollständiger Trennung der Regioisomeren chromatographisch aufgereinigt [Methode 19]. Es wurden 71 mg (16% d. Th.) des Regioisomeren 1 (Beispiel 56) sowie 210 mg (46% d. Th.) des Regioisomeren 2 (Beispiel 57) erhalten.

### Beispiel 56:

LC/MS [Methode 4]: Rₜ = 1.09 min; MS [ESIpos]: m/z = 571 und 573 (M+H)⁺
¹H-NMR (400 MHz, CDCl₃): δ = 3.69 (s, 3H), 3.94 (dd, 1H), 4.05 (dd, 1H), 4.55-4.64 (m, 1H), 4.80 (s, 2H), 5.14-5.33 (m, 2H), 5.36 (d, 1H), 7.34-7.40 (m, 1H), 7.42-7.52 (m, 5H), 7.54-7.60 (m, 2H).

### Beispiel 57:

LC/MS [Methode 4]: Rₜ = 1.15 min; MS [ESIpos]: m/z = 571 und 573 (M+H)⁺
¹H-NMR (400 MHz, CDCl₃): δ = 3.74 (s, 3H), 3.92 (dd, 1H), 3.99 (dd, 1H), 4.55-4.65 (m, 1H), 4.89 (d, 1H), 5.16-5.39 (m, 4H), 7.28-7.34 (m, 2H), 7.42-7.50 (m, 3H), 7.59-7.64 (m, 2H), 7.85-7.91 (m, 1H).

### Beispiel 58 und Beispiel 59

### (3-{[3-(4-Chlorphenyl)-5-oxo-4-(3,3,3-trifluor-2-hydroxypropyl)-4,5-dihydro-1H-1,2,4-triazol-1-yl]methyl}-5-[2-(trifluormethyl)benzyl]-1H-1,2,4-triazol-1-yl)essigsäure (Regioisomer 1) und (5-{[3-(4-Chlorphenyl)-5-oxo-4-(3,3,3-trifluor-2-hydroxypropyl)-4,5-dihydro-1H-1,2,4-triazol-1-yl]methyl}-3-[2-(trifluormethyl)benzyl]-1H-1,2,4-triazol-1-yl)essigsäure (Regioisomer 2)

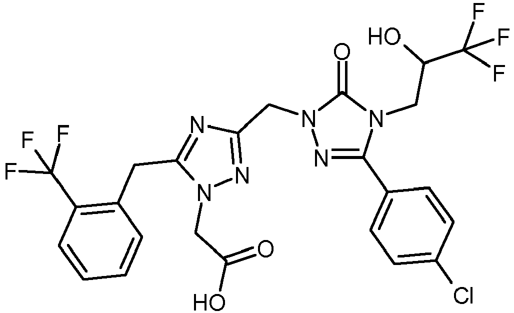

*und*

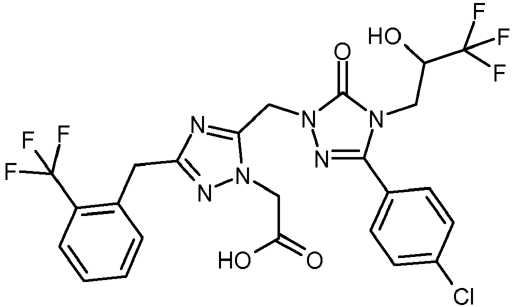

310 mg (0.50 mmol) der Verbindung aus Beispiel 55 (als Regioisomeren-Gemisch) wurden in 5 ml Methanol gelöst und mit 0.85 ml (0.85 mmol) einer 1 N wässrigen Lithiumhydroxid-Lösung versetzt. Es wurde 45 min bei RT gerührt. Anschließend wurde das Gemisch unter vermindertem Druck vom Solvens befreit, mit 10 ml Wasser aufgenommen und mit 0.85 ml (0.85 mmol) 1 N Salzäure neutralisiert. Das Gemisch wurde zweimal mit je 15 ml Ethylacetat extrahiert. Die vereinigten organischen Phasen wurden über Natriumsulfat getrocknet, filtriert und im Vakuum eingeengt. Das Rohprodukt wurde chromatographisch unter Trennung der Regioisomeren gereinigt [Methode 16]. Es wurden 81 mg (27% d. Th.) des Regioisomeren 1 (Beispiel 58) sowie 83 mg (27% d. Th.) des Regioisomeren 2 (Beispiel 59) erhalten.

### Beispiel 58:

LC/MS [Methode 2]: Rₜ = 2.27 min; MS [ESIpos]: m/z = 605 (M+H)⁺
¹H-NMR (400 MHz, DMSO-d₆): δ = 3.81 (dd, 1H), 3.97 (dd, 1H), 4.26 (s, 3H), 4.86-4.97 (m, 2H), 5.06 (s, 2H), 6.89 (d, 1H), 7.32 (d, 1H), 7.45-7.52 (m, 1H), 7.56-7.65 (m, 3H), 7.69-7.76 (m, 3H), 13.35 (br. s, 1H).

### Beispiel 59:

LC/MS [Methode 2]: Rₜ = 2.37 min; MS [ESIpos]: m/z = 605 (M+H)⁺
¹H-NMR (400 MHz, DMSO-d₆): δ = 3.79 (dd, 1H), 3.93 (dd, 1H), 4.12 (s, 2H), 4.24-4.30 (m, 1H), 5.07-5.22 (m, 4H), 6.89 (d, 1H), 7.40-7.48 (m, 2H), 7.55-7.66 (m, 3H), 7.67-7.75 (m, 3H), 13.26 (br. s, 1H).

### Beispiel 60

### [5-(2-Chlorphenyl)-3-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]essigsäure

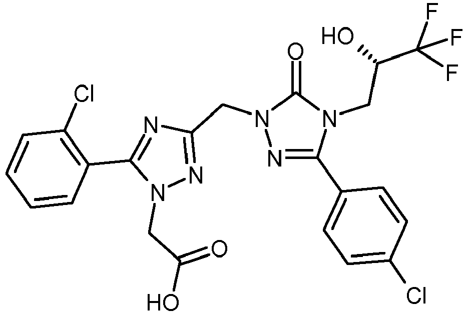

65 mg (0.11 mmol) der Verbindung aus Beispiel 56 wurden in 5 ml Methanol gelöst und mit 0.26 ml (0.26 mmol) einer 1 N wässrigen Lithiumhydroxid-Lösung versetzt. Es wurde 30 min bei RT gerührt. Anschließend wurde das Gemisch unter vermindertem Druck vom Solvens befreit, mit 10 ml Wasser aufgenommen und mit 0.85 ml (0.85 mmol) 1 N Salzäure neutralisiert. Das Gemisch wurde zweimal mit je 15 ml Ethylacetat extrahiert. Die vereinigten organischen Phasen wurden über Natriumsulfat getrocknet, filtriert und im Vakuum eingeengt. Es wurden 57 mg (76% d. Th.) der Titelverbindung in 84% Reinheit erhalten.
LC/MS [Methode 4]: Rₜ = 0.95 min; MS [ESIpos]: m/z = 557 und 559 (M+H)⁺
¹H-NMR (400 MHz, DMSO-d₆): δ = 3.85 (dd, 1H), 3.96-4.03 (m, 1H), 4.24-4.36 (m, 1H), 4.86 (s, 2H), 5.03-5.14 (m, 2H), 6.90 (d, 1H), 7.47-7.52 (m, 2H), 7.55-7.68 (m, 4H), 7.76 (d, 2H), 13.30 (br. s, 1H).

### Beispiel 61

### [3-(2-Chlorphenyl)-5-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]essigsäure

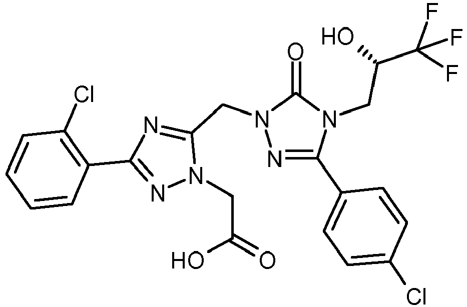

205 mg (0.36 mmol) der Verbindung aus Beispiel 57 wurden analog zur Herstellung der Verbindung in Beispiel 60 umgesetzt. Es wurden 198 mg (94% d. Th.) der Titelverbindung in 93% Reinheit erhalten.
LC/MS [Methode 2]: Rₜ = 2.20 min; MS [ESIpos]: m/z = 557 und 559 (M+H)⁺
¹H-NMR (400 MHz, DMSO-d₆): δ = 3.81 (dd, 1H), 3.96 (dd, 1H), 4.24-4.38 (m, 1H), 5.20-5.37 (m, 4H), 6.90 (br. s, 1H), 7.42-7.47 (m, 2H), 7.55-7.58 (m, 1H), 7.62 (d, 2H), 7.74 (d, 2H), 7.82-7.87 (m, 1H), 13.38 (br. s, 1H).

### Beispiel 62

### 2-(3-{[3-(4-Chlorphenyl)-5-oxo-4-(3,3,3-trifluor-2-hydroxypropyl)-4,5-dihydro-1H-1,2,4-triazol-1-yl]methyl}-5-[2-(trifluormethyl)benzyl]-1H-1,2,4-triazol-1-yl)acetamid (Racemat)

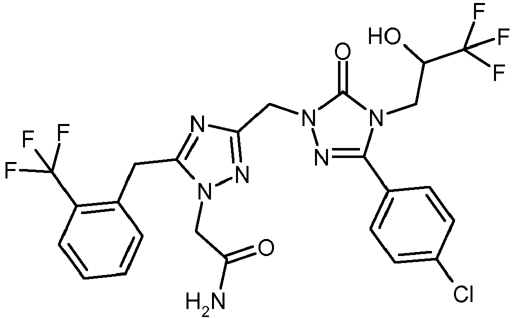

85 mg (0.14 mmol) der Verbindung aus Beispiel 58 wurden in 2.5 ml DMF vorgelegt und mit 23 mg (0.17 mmol) HOBt und 35 mg (0.18 mmol) EDC versetzt. Nach 20 min Rühren bei RT wurden 0.3 ml (5.65 mmol) Ammoniak-Lösung (32%-ig in Wasser) hinzugegeben und der Ansatz 16 h bei Raumtemperatur gerührt. Anschließend wurde die Reaktionslösung im Vakuum von überschüssigem Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 58 mg (69% d. Th.) der Zielverbindung erhalten.
LC/MS [Methode 5]: Rₜ = 2.22 min; MS [ESIpos]: m/z = 604 (M+H)⁺.

Durch präparative HPLC an chiraler Phase [Methode 17] wurde das Racemat aus Beispiel 62 (58 mg) in die Enantiomere aufgetrennt. Es wurden 27 mg des zuerst eluierenden Enantiomers 1 (Beispiel 63) sowie 29 mg des später eluierenden Enantiomers 2 (Beispiel 64) erhalten:

### Beispiel 63

### 2-(3-{[3-(4-Chlorphenyl)-5-oxo-4-(3,3,3-trifluor-2-hydroxypropyl)-4,5-dihydro-1H-1,2,4-triazol-1-yl]methyl}-5-[2-(trifluormethyl)benzyl]-1H-1,2,4-triazol-1-yl)acetamid (Enantiomer 1)

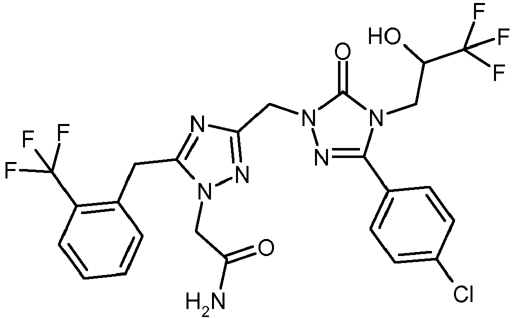

Zuerst eluierendes Enantiomer aus der Racemat-Trennung von Beispiel 62.
Chirale HPLC [Methode 18]: Rₜ = 6.57 min.
¹H-NMR (400 MHz, DMSO-d₆): δ = 3.81 (dd, 1H), 3.97 (dd, 1H), 4.22-4.33 (m, 3H), 4.83 (s, 2H), 4.85-4.95 (m, 2H), 6.89 (d, 1H), 7.31-7.38 (m, 2H), 7.45-7.50 (m, 1H), 7.55-7.64 (m, 3H), 7.65-7.76 (m, 4H).

### Beispiel 64

### 2-(3-{[3-(4-Chlorphenyl)-5-oxo-4-(3,3,3-trifluor-2-hydroxypropyl)-4,5-dihydro-1H-1,2,4-triazol-1-yl]methyl}-5-[2-(trifluormethyl)benzyl]-1H-1,2,4-triazol-1-yl)acetamid (Enantiomer 2)

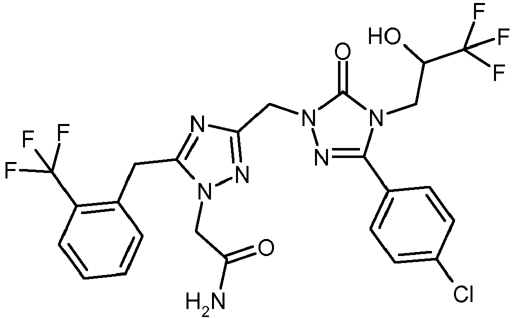

Zuletzt eluierendes Enantiomer aus der Racemat-Trennung von Beispiel 62.
Chirale HPLC [Methode 18]: Rₜ = 7.70 min.
¹H-NMR (400 MHz, DMSO-d₆): δ = 3.81 (dd, 1H), 3.97 (dd, 1H), 4.22-4.33 (m, 3H), 4.83 (s, 2H), 4.85-4.95 (m, 2H), 6.89 (d, 1H), 7.31-7.38 (m, 2H), 7.45-7.50 (m, 1H), 7.55-7.64 (m, 3H), 7.65-7.76 (m, 4H).

### Beispiel 65

### 2-(5-{[3-(4-Chlorphenyl)-5-oxo-4-(3,3,3-trifluor-2-hydroxypropyl)-4,5-dihydro-1H-1,2,4-triazol-1-yl]methyl}-3-[2-(trifluormethyl)benzyl]-1H-1,2,4-triazol-1-yl)acetamid (Racemat)

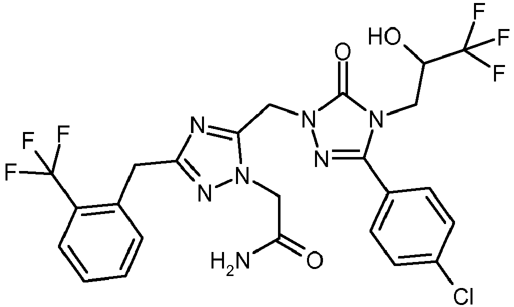

95 mg (0.16 mmol) der Verbindung aus Beispiel 59 wurden in 2.5 ml DMF vorgelegt und mit 25 mg (0.19 mmol) HOBt und 39 mg (0.20 mmol) EDC versetzt. Nach 20 min Rühren bei RT wurden 0.3 ml (5.65 mmol) Ammoniak-Lösung (32%-ig in Wasser) hinzugegeben und der Ansatz 16 h bei Raumtemperatur gerührt. Anschließend wurde die Reaktionslösung im Vakuum von überschüssigem Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 58 mg (61% d. Th.) der Zielverbindung erhalten.
LC/MS [Methode 5]: Rₜ = 2.32 min; MS [ESIpos]: m/z = 604 (M+H)⁺.

Durch präparative HPLC an chiraler Phase [Methode 12] wurde das Racemat aus Beispiel 65 (58 mg) in die Enantiomere aufgetrennt. Es wurden 28 mg des zuerst eluierenden Enantiomers 1 (Beispiel 66) sowie 28 mg des später eluierenden Enantiomers 2 (Beispiel 67) erhalten:

### Beispiel 66

### 2-(5-{[3-(4-Chlorphenyl)-5-oxo-4-(3,3,3-trifluor-2-hydroxypropyl)-4,5-dihydro-1H-1,2,4-triazol-1-yl]methyl}-3-[2-(trifluormethyl)benzyl]-1H-1,2,4-triazol-1-yl)acetamid (Enantiomer 1)

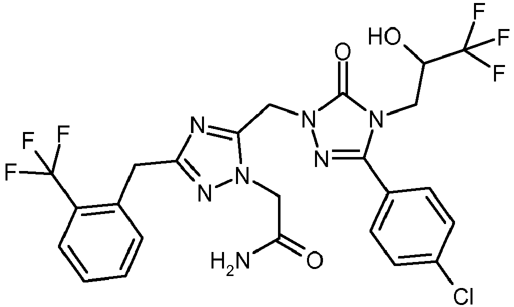

Zuerst eluierendes Enantiomer aus der Racemat-Trennung von Beispiel 65.
Chirale HPLC [Methode 13]: Rₜ = 4.78 min.
¹H-NMR (400 MHz, DMSO-d₆): δ = 3.79 (dd, 1H), 3.94 (dd, 1H), 4.10 (s, 2H), 4.24-4.35 (m, 1H), 4.93 (s, 2H), 5.09-5.19 (m, 2H), 6.87 (d, 1H), 7.34 (s, 1H), 7.40-7.48 (m, 2H), 7.55-7.75 (m, 7H).

### Beispiel 67

### 2-(5-{[3-(4-Chlorphenyl)-5-oxo-4-(3,3,3-trifluor-2-hydroxypropyl)-4,5-dihydro-1H-1,2,4-triazol-1-yl]methyl}-3-[2-(trifluormethyl)benzyl]-1H-1,2,4-triazol-1-yl)acetamid (Enantiomer 2)

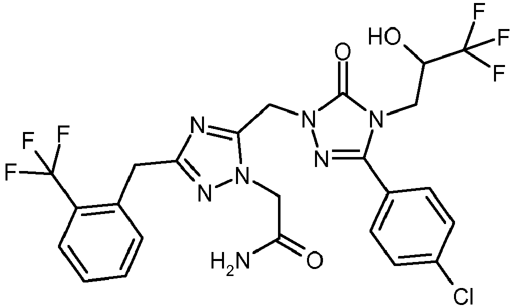

Zuletzt eluierendes Enantiomer aus der Racemat-Trennung von Beispiel 65.
Chirale HPLC [Methode 13]: Rₜ = 6.35 min.
¹H-NMR (400 MHz, DMSO-d₆): δ = 3.79 (dd, 1H), 3.94 (dd, 1H), 4.10 (s, 2H), 4.24-4.35 (m, 1H), 4.93 (s, 2H), 5.09-5.19 (m, 2H), 6.87 (d, 1H), 7.34 (s, 1H), 7.40-7.48 (m, 2H), 7.55-7.75 (m, 7H).

### Beispiel 68

### 2-[5-(2-Chlorphenyl)-3-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]acetamid

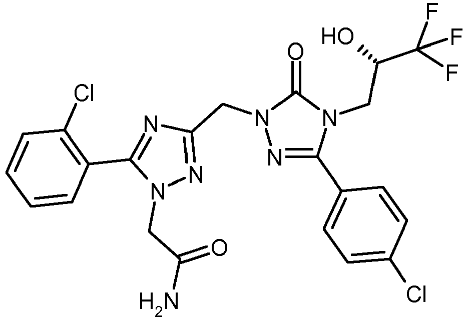

54 mg (0.10 mmol) der Verbindung aus Beispiel 60 wurden in 4 ml DMF vorgelegt und mit 19 mg (0.13 mmol) HOBt und 24 mg (0.03 mmol) EDC versetzt. Nach 10 min Rühren bei RT wurden 0.1 ml (1.93 mmol) Ammoniak-Lösung (32%-ig in Wasser) hinzugegeben und der Ansatz 16 h bei Raumtemperatur gerührt. Anschließend wurde die Reaktionslösung im Vakuum von überschüssigem Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 27 mg (46% d. Th.) der Zielverbindung erhalten.
LC/MS [Methode 3]: Rₜ = 1.08 min; MS [ESIpos]: m/z = 556 und 558 (M+H)⁺
¹H-NMR (400 MHz, DMSO-d₆): δ = 3.85 (dd, 1H), 4.00 (dd, 1H), 4.25-4.37 (m, 1H), 4.64 (s, 2H), 5.07 (s, 2H), 6.91 (d, 1H), 7.24 (br. s, 1H), 7.46-7.67 (m, 7H), 7.76 (d, 2H).

### Beispiel 69

### 2-[3-(2-Chlorphenyl)-5-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,4-triazol-1-yl]acetamid

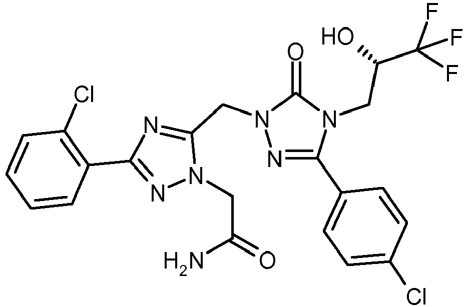

100 mg (0.18 mmol) der Verbindung aus Beispiel 61 wurden analog zur Herstellung der Verbindung in Beispiel 68 umgesetzt. Es wurden 67 mg (67% d. Th.) der Titelverbindung erhalten.
LC/MS [Methode 3]: Rₜ = 1.14 min; MS [ESIpos]: m/z = 556 und 558 (M+H)⁺
¹H-NMR (400 MHz, DMSO-d₆): δ = 3.81 (dd, 1H), 3.96 (dd, 1H), 4.28-4.34 (m, 1H), 5.04-5.14 (m, 2H), 5.22-5.32 (m, 2H), 6.86 (d, 1H), 7.39-7.46 (m, 2H), 7.52-7.57 (m, 1H), 7.62 (d, 2H), 7.72-7.78 (m, 3H), 7.83-7.88 (m, 1H), 7.95 (s, 1H).

### Beispiel 70

### 5-(4-Chlorphenyl)-2-{[5-(2-chlorphenyl)-4H-1,2,4-triazol-3-yl]methyl}-4-(3,3,3-trifluorpropyl)-2,4-dihydro-3H-1,2,4-triazol-3-on

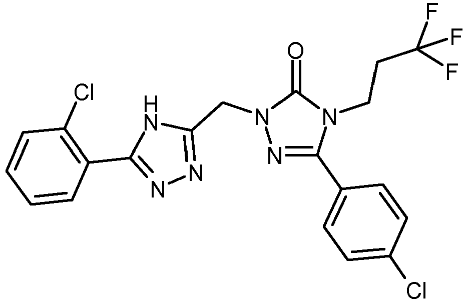

50 mg (0.14 mmol) der Verbindung aus Beispiel 23A wurden analog zur Herstellung der Verbindung in Beispiel 54 umgesetzt. Es wurden 8 mg (11% d. Th.) der Titelverbindung erhalten.
LC/MS [Methode 4]: Rₜ = 1.06 min; MS [ESIpos]: m/z = 483 (M+H)⁺
¹H-NMR (400 MHz, DMSO-d₆): δ = 2.59-2.69 (m, 2H), 4.00 (t, 2H), 5.13 (s, 2H), 7.42-7.51 (m, 2H), 7.56-7.71 (m, 5H), 7.75-7.81 (m, 1H), 14.25 (br. s, 1H).

### Beispiel 71

### 5-(4-Chlorphenyl)-2-{[5-(2,6-dichlorbenzyl)-4H-1,2,4-triazol-3-yl]methyl}-4-(3,3,3-trifluorpropyl)-2,4-dihydro-3H-1,2,4-triazol-3-on

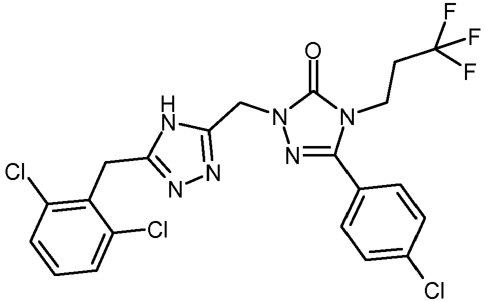

50 mg (0.14 mmol) der Verbindung aus Beispiel 23A wurden analog zur Herstellung der Verbindung in Beispiel 54 umgesetzt. Es wurden 18 mg (25% d. Th.) der Titelverbindung erhalten.
LC/MS [Methode 4]: Rₜ = 1.10 min; MS [ESIpos]: m/z = 531 und 533 (M+H)⁺
¹H-NMR (400 MHz, DMSO-d₆): δ = 2.55-2.69 (m, 2H), 3.97 (t, 2H), 4.23-4.34 (m, 2H), 4.95 (s, 2H), 7.31-7.37 (m, 1H), 7.48 (d, 2H), 7.60-7.68 (m, 4H), 13.70 (br. s, 1H).

### Beispiel 72

### 2-{[5-(2-Chlorbenzyl)-4H-1,2,4-triazol-3-yl]methyl}-5-(4-chlorphenyl)-4-(3,3,3-trifluorpropyl)-2,4-dihydro-3H-1,2,4-triazol-3-on

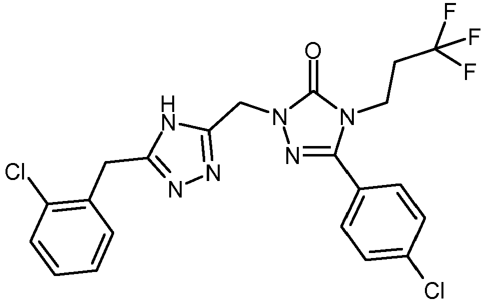

48 mg (0.13 mmol) der Verbindung aus Beispiel 23A wurden analog zur Herstellung der Verbindung in Beispiel 54 umgesetzt. Es wurden 22 mg (33% d. Th.) der Titelverbindung erhalten.
LC/MS [Methode 4]: Rₜ = 1.07 min; MS [ESIpos]: m/z = 497 und 499 (M+H)⁺
¹H-NMR (400 MHz, DMSO-d₆): δ = 2.56-2.69 (m, 2H), 3.98 (t, 2H), 4.12 (s, 2H), 4.97 (s, 2H), 7.25-7.34 (m, 3H), 7.40-7.46 (m, 1H), 7.59-7.68 (m, 5H).

### Beispiel 73

### 5-(4-Chlorphenyl)-2-{[5-(2-chlorphenyl)-2-thienyl]methyl}-4-[(2S)-3,3,3-trifluor-2-hydroxypropyl]-2,4-dihydro-3H-1,2,4-triazol-3-on

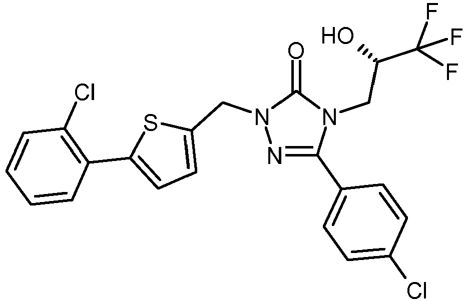

85 mg (0.28 mmol) der Verbindung aus Beispiel 5A und 134 mg (0.41 mmol) Cäsiumcarbonat wurden in 5 ml Acetonitril suspendiert und mit 113 mg (0.28 mmol) der Verbindung aus Beispiel 56A versetzt. Die Mischung wurde 20 h unter Rückfluss gerührt. Zur Aufarbeitung wurde auf RT abgekühlt, das Acetonitril im Vakuum entfernt und der Rückstand mit 10 ml Wasser versetzt. Es wurde zweimal mit je 10 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 4 mg (3% d. Th.) der Zielverbindung erhalten.
LC/MS [Methode 4]: Rₜ = 1.32 min; MS [ESIpos]: m/z = 514 und 516 (M+H)⁺
¹H-NMR (400 MHz, DMSO-d₆): δ = 3.83 (dd, 1H), 4.00 (dd, 1H), 4.22-4.35 (m, 1H), 5.16-5.26 (m, 2H), 6.91 (br. s, 1H), 7.16 (d, 1H), 7.32 (d, 1H), 7.34-7.44 (m, 2H), 7.53-7.65 (m, 4H), 7.76 (d, 2H).

### Beispiel 74

### 5-(4-Chlorphenyl)-2-{[5-(2,3-dichlorphenyl)-2-thienyl]methyl}-4-[(2S)-3,3,3-trifluor-2-hydroxypropyl]-2,4-dihydro-3H-1,2,4-triazol-3-on

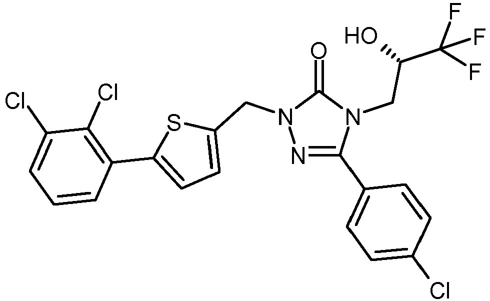

67 mg (0.22 mmol) der Verbindung aus Beispiel 5A und 142 mg (0.44 mmol) Cäsiumcarbonat wurden in 4 ml Acetonitril suspendiert und mit 70 mg (0.28 mmol) der Verbindung aus Beispiel 57A versetzt. Die Mischung wurde 2 h unter Rückfluss gerührt. Zur Aufarbeitung wurde auf RT abgekühlt, das Acetonitril im Vakuum entfernt und der Rückstand mit 10 ml Wasser versetzt. Es wurde zweimal mit je 10 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 29 mg (24% d. Th.) der Zielverbindung erhalten.
LC/MS [Methode 4]: Rₜ = 1.37 min; MS [ESIpos]: m/z = 548 und 550 (M+H)⁺
¹H-NMR (400 MHz, DMSO-d₆): δ = 3.83 (dd, 1H), 3.99 (dd, 1H), 4.24-4.34 (m, 1H), 5.18-5.27 (m, 2H), 6.90 (d, 1H), 7.17 (d, 1H), 7.33 (d, 1H), 7.42 (t, 1H), 7.57 (dd, 1H), 7.61-7.67 (m, 3H), 7.74-7.79 (m, 2H).

### Beispiel 75

### 5-(4-Chlorphenyl)-2-{[5-(2,3-difluorphenyl)-2-thienyl]methyl}-4-[(2S)-3,3,3-trifluor-2-hydroxypropyl]-2,4-dihydro-3H-1,2,4-triazol-3-on

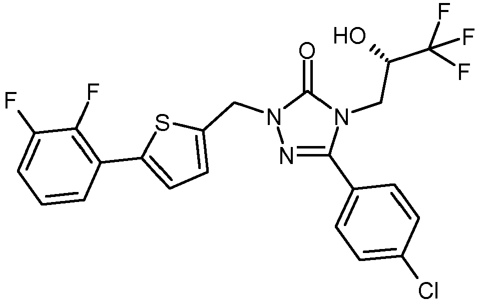

84 mg (0.19 mmol) der Verbindung aus Beispiel 30A sowie 45 mg (0.29 mmol) 2,3-Difluorphenylboronsäure wurden unter Argonatmosphäre in 2 ml Toluol gelöst. Anschließend fügte man 9 mg (0.01 mmol) Tris(dibenzylidenaceton)dipalladium, 8 mg (0.02 mmol) 2-Dicyclohexylphosphino-2'-(*N,N*-dimethylamino)biphenyl sowie 81 mg (0.38 mmol) Kaliumphosphat hinzu und erhitzte das Gemisch unter Argon für 14 h auf 110°C. Zur Aufarbeitung wurde bei RT mit 10 ml Ethylacetat und 10 ml Wasser verdünnt, die organische Phase abgetrennt und die wässrige Phase noch zweimal mit je 10 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 25 mg (24% d. Th.) der Zielverbindung erhalten.
LC/MS [Methode 2]: Rₜ = 2.72 min; MS [ESIpos]: m/z = 516 (M+H)⁺
¹H-NMR (400 MHz, DMSO-d₆): δ = 3.78-4.07 (m, 2H), 4.23-4.39 (m, 1H), 5.10-5.26 (m, 2H), 6.86-6.95 (m, 1H), 6.99-7.05 (m, 1H), 7.17-7.59 (m, 4H), 7.60-7.66 (m, 1H), 7.73-7.80 (m, 2H), 7.83-7.90 (m, 1H).

### Beispiel 76

### 5-(4-Chlorphenyl)-4-[(2S)-3,3,3-trifluor-2-hydroxypropyl]-2-({2-[2-(trifluormethyl)phenyl]-1,3-thiazol-5-yl}methyl)-2,4-dihydro-3H-1,2,4-triazol-3-on

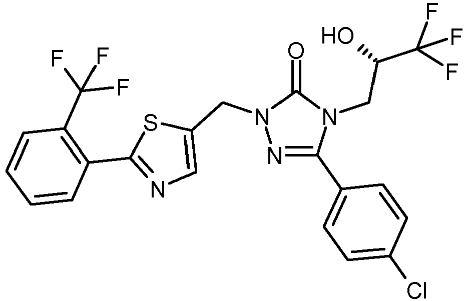

62 mg (0.14 mmol) der Verbindung aus Beispiel 31A sowie 40 mg (0.21 mmol) 2-(Trifluormethyl)-phenylboronsäure wurden unter Argonatmosphäre in 2 ml Toluol gelöst. Anschließend fügte man 6.5 mg (0.007 mmol) Tris(dibenzylidenaceton)dipalladium, 5.6 mg (0.014 mmol) 2-Dicyclohexylphosphino-2'-(N*,N-*dimethylamino)biphenyl sowie 60 mg (0.28 mmol) Kaliumphosphat hinzu und erhitzte das Gemisch unter Argon für 48 h auf 110°C. Zur Aufarbeitung wurde bei RT mit 10 ml Ethylacetat und 10 ml Wasser verdünnt, die organische Phase abgetrennt und die wässrige Phase noch zweimal mit je 10 ml Ethylacetat extrahiert. Die vereinigten organischen Phasen wurden über Natriumsulfat getrocknet, filtriert und im Vakuum eingeengt. Das Rohprodukt wurde chromatographisch gereinigt [Methode 19]. Es wurden 10 mg (13% d. Th.) der Zielverbindung erhalten.
LC/MS [Methode 2]: Rₜ = 2.54 min; MS [ESIpos]: m/z = 549 (M+H)⁺
¹H-NMR (400 MHz, DMSO-d₆): δ = 3.83 (dd, 1H), 4.00 (dd, 1H), 4.22-4.34 (m, 1H), 5.29-5.38 (m, 2H), 6.90 (d, 1H), 7.61-7.67 (m, 2H), 7.71-7.83 (m, 5H), 7.93 (d, 1H), 7.96 (s, 1H).

### Beispiel 77

### 5-(4-Chlorphenyl)-4-[(2S)-3,3,3-trifluor-2-hydroxypropyl]-2-({5-[2-(trifluormethyl)phenyl]-1,3-thiazol-2-yl}methyl)-2,4-dihydro-3H-1,2,4-triazol-3-on

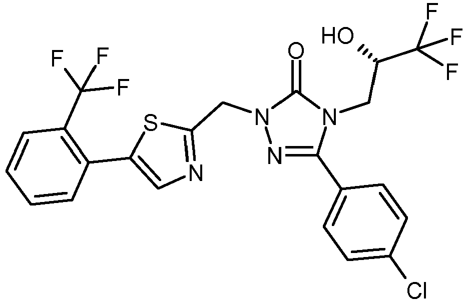

40 mg (0.13 mmol) der Verbindung aus Beispiel 5A wurden in 7 ml Acetonitril gelöst und mit 66 mg (0.20 mmol) Cäsiumcarbonat sowie 42 mg (0.13 mmol) der Verbindung aus Beispiel 89A versetzt. Das Gemisch wurde 1 h bei 80°C gerührt. Zur Aufarbeitung wurde auf RT abgekühlt, mit 5 ml Methanol verdünnt und filtriert. Das Filtrat wurde im Vakuum eingeengt und das Rohprodukt anschließend chromatographisch gereinigt [Methode 19]. Es wurden 47 mg (66% d. Th.) der Zielverbindung erhalten.
LC/MS [Methode 3]: Rₜ = 2.48 min; MS [ESIpos]: m/z = 549 (M+H)⁺
¹H-NMR (400 MHz, DMSO-d₆): δ = 3.86 (dd, 1H), 4.02 (dd, 1H), 4.24-4.34 (m, 1H), 5.35-5.45 (m, 2H), 6.92 (s, 1H), 7.58-7.66 (m, 3H), 7.66-7.80 (m, 5H), 7.90 (d, 1H).

### Beispiel 78

### 5-(4-Chlorphenyl)-2-{[5-(2-chlorphenyl)-1,3-thiazol-2-yl]methyl}-4-[(2S)-3,3,3-trifluor-2-hydroxypropyl]-2,4-dihydro-3H-1,2,4-triazol-3-on

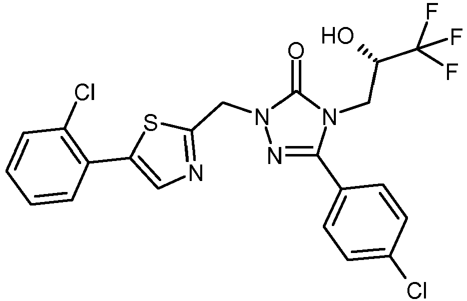

70 mg (0.23 mmol) der Verbindung aus Beispiel 5A wurden mit 66 mg (0.23 mmol) der Verbindung aus Beispiel 88A analog zur Herstellung der Verbindung in Beispiel 77 umgesetzt. Es wurden 65 mg (55% d. Th.) der Titelverbindung erhalten.
LC/MS [Methode 4]: Rₜ = 1.23 min; MS [ESIpos]: m/z = 515 und 517 (M+H)⁺
¹H-NMR (400 MHz, DMSO-d₆): δ = 3.86 (dd, 1H), 4.02 (dd, 1H), 4.24-4.35 (m, 1H), 5.39 (s, 2H), 6.92 (br. s, 1H), 7.44 (dd, 2H), 7.58-7.72 (m, 4H), 7.78 (d, 2H), 8.06 (s, 1H).

### Beispiel 79

### 2-{[5-(3-Chlor-2-fluorphenyl)-1,3-thiazol-2-yl]methyl}-5-(4-chlorphenyl)-4-[(2S)-3,3,3-trifluor-2-hydroxypropyl]-2,4-dihydro-3H-1,2,4-triazol-3-on

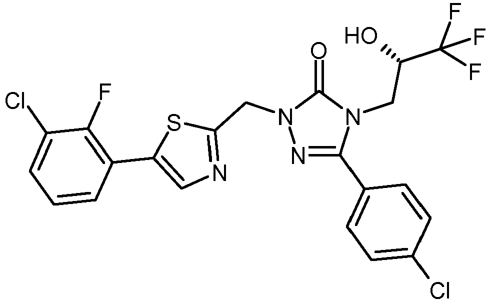

70 mg (0.23 mmol) der Verbindung aus Beispiel 5A wurden mit 70 mg (0.23 mmol) der Verbindung aus Beispiel 90A analog zur Herstellung der Verbindung in Beispiel 77 umgesetzt. Es wurden 90 mg (72% d. Th.) der Titelverbindung erhalten.
LC/MS [Methode 3]: Rₜ = 1.44 min; MS [ESIpos]: m/z = 533 und 535 (M+H)⁺
¹H-NMR (400 MHz, DMSO-d₆): δ = 3.86 (dd, 1H), 4.02 (dd, 1H), 4.25-4.35 (m, 1H), 5.37-5.43 (m, 2H), 6.92 (s, 1H), 7.30-7.36 (m, 1H), 7.60-7.67 (m, 3H), 7.75-7.82 (m, 3H), 8.27 (s, 1H).

### Beispiel 80

### 5-(4-Chlorphenyl)-2-({5-[2-fluor-3-(trifluormethyl)phenyl]-1,3-thiazol-2-yl}methyl)-4-[(2S)-3,3,3-trifluor-2-hydroxypropyl]-2,4-dihydro-3H-1,2,4-triazol-3-on

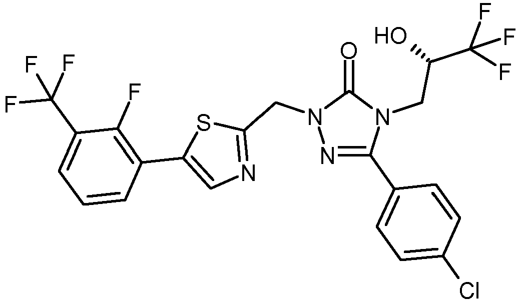

55 mg (0.18 mmol) der Verbindung aus Beispiel 5A wurden mit 61 mg (0.18 mmol) der Verbindung aus Beispiel 91A analog zur Herstellung der Verbindung in Beispiel 77 umgesetzt. Es wurden 55 mg (52% d. Th.) der Titelverbindung erhalten.
LC/MS [Methode 4]: Rₜ = 1.26 min; MS [ESIpos]: m/z = 567 (M+H)⁺
¹H-NMR (400 MHz, DMSO-d₆): δ = 3.86 (dd, 1H), 4.02 (dd, 1H), 4.25-4.35 (m, 1H), 5.41 (s, 2H), 6.92 (s, 1H), 7.52 (t, 1H), 7.64 (d, 2H), 7.75-7.84 (m, 3H), 8.17 (t, 1H), 8.33 (s, 1H).

### Beispiel 81

### 5-(4-Chlorphenyl)-2-{[5-(2-chlorphenyl)-4-(trifluormethyl)-1,3-thiazol-2-yl]methyl}-4-[(2S)-3,3,3-trifluor-2-hydroxypropyl]-2,4-dihydro-3H-1,2,4-triazol-3-on

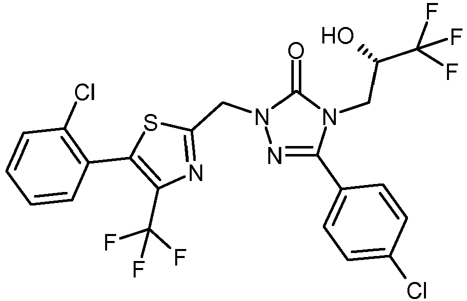

40 mg (0.13 mmol) der Verbindung aus Beispiel 5A wurden mit 46 mg (0.13 mmol) der Verbindung aus Beispiel 92A analog zur Herstellung der Verbindung in Beispiel 77 umgesetzt. Es wurden 58 mg (76% d. Th.) der Titelverbindung erhalten.
LC/MS [Methode 4]: Rₜ = 1.32 min; MS [ESIpos]: m/z = 583 und 585 (M+H)⁺
¹H-NMR (400 MHz, DMSO-d₆): δ = 3.86 (dd, 1H), 4.02 (dd, 1H), 4.24-4.34 (m, 1H), 5.44-5.49 (m, 2H), 6.90 (br. s, 1H), 7.44-7.50 (m, 1H), 7.53-7.60 (m, 2H), 7.61-7.68 (m, 3H), 7.75-7.80 (m, 2H).

### Beispiel 82

### 5-(4-Chlorphenyl)-2-{[2-(2-chlorphenyl)-1,3-oxazol-5-yl]methyl}-4-[(2S)-3,3,3-trifluor-2-hydroxypropyl]-2,4-dihydro-3H-1,2,4-triazol-3-on

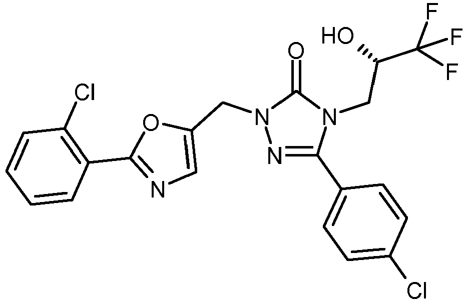

113 mg (0.37 mmol) der Verbindung aus Beispiel 5A wurden mit 100 mg (0.37 mmol) der Verbindung aus Beispiel 54A analog zur Herstellung der Verbindung in Beispiel 77 umgesetzt. Es wurden 23 mg (12% d. Th.) der Titelverbindung erhalten.
LC/MS [Methode 6]: Rₜ = 2.44 min; MS [ESIpos]: m/z = 499 (M+H)⁺
¹H-NMR (400 MHz, DMSO-d₆): δ = 3.82 (dd, 1H), 3.98 (dd, 1H), 4.25-4.33 (m, 1H), 5.15-5.25 (m, 2H), 6.88 (d, 1H), 7.41 (s, 1H), 7.48-7.57 (m, 2H), 7.60-7.66 (m, 3H), 7.72-7.76 (m, 2H), 7.92 (dd, 1H).

### Beispiel 83

### 5-(4-Chlorphenyl)-2-{[2-(2,3-dichlorphenyl)-1,3-oxazol-5-yl]methyl}-4-[(2S)-3,3,3-trifluor-2-hydroxypropyl]-2,4-dihydro-3H-1,2,4-triazol-3-on

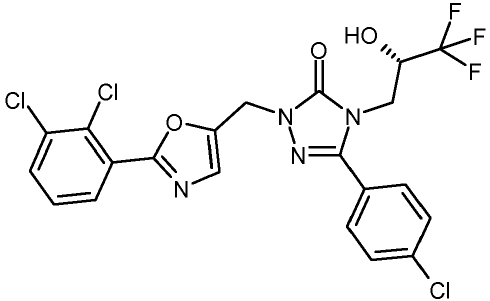

49 mg (0.16 mmol) der Verbindung aus Beispiel 5A wurden mit 49 mg (0.16 mmol) der Verbindung aus Beispiel 55A analog zur Herstellung der Verbindung in Beispiel 77 umgesetzt. Es wurden 35 mg (40% d. Th.) der Titelverbindung erhalten.
LC/MS [Methode 4]: Rₜ = 1.23 min; MS [ESIpos]: m/z = 533 und 535 (M+H)⁺
¹H-NMR (400 MHz, DMSO-d₆): δ = 3.82 (dd, 1H), 3.98 (dd, 1H), 4.22-4.34 (m, 1H), 5.17-5.26 (m, 2H), 6.89 (s, 1H), 7.45 (s, 1H), 7.53 (t, 1H), 7.61-7.65 (m, 2H), 7.72-7.76 (m, 2H), 7.84 (dd, 1H), 7.88 (dd, 1H).

### Beispiel 84

### 5-(4-Chlorphenyl)-4-cyclopropyl-2-({5-[3-(trifluormethyl)benzyl]-1,3-thiazol-2-yl}methyl)-2,4-dihydro-3H-1,2,4-triazol-3-on

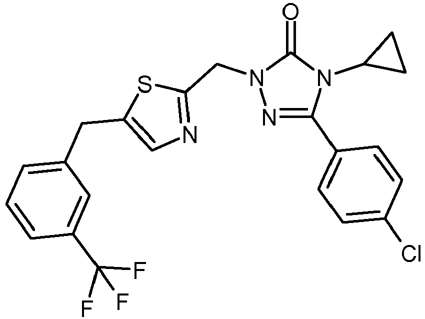

52 mg (0.11 mmol) der Verbindung aus Beispiel 27A und 47 mg (0.12 mmol) 4-Methoxyphenyldithiophosphonsäureanhydrid (Lawesson-Reagens) wurden in 1 ml THF gelöst und 16 h bei 70°C gerührt. Nach dem Abkühlen auf RT verteilte man den Ansatz zwischen 10 ml *tert.*-Butylmethylether und 10 ml Wasser. Die organische Phase wurde abgetrennt, mit je 10 ml Wasser und gesättigter Natriumchlorid-Lösung gewaschen, über Natriumsulfat getrocknet, filtriert und im Vakuum eingeengt. Der Rückstand wurde durch präparative HPLC gereinigt [Methode 19]. Es wurden 50 mg (97% d. Th.) der Titelverbindung als farbloser Feststoff erhalten.
MS [ESIpos]: m/z = 491 (M+H)⁺
¹H-NMR (400 MHz, CDCl₃): δ = 0.71-0.81 (m, 2H), 0.97-1.06 (m, 2H), 2.94-3.03 (m, 1H), 4.18 (s, 2H), 5.25 (s, 2H), 7.35-7.55 (m, 6H), 7.69 (d, 2H).

### Beispiel 85

### 5-(4-Chlorphenyl)-4-cyclopropyl-2-({5-[3-(trifluormethyl)benzyl]-1H-imidazol-2-yl}methyl)-2,4-dihydro-3H-1,2,4-triazol-3-on

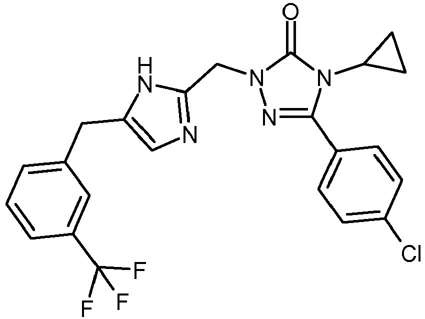

50 mg (0.10 mmol) der Verbindung aus Beispiel 27A wurden in 1 ml DMF gelöst, mit 23 mg (0.30 mmol) Ammoniumacetat vermischt und 15 min bei 200°C im Mikrowellenofen gerührt. Man gab nach Abkühlen weitere 30 mg (0.39 mmol) Ammoniumacetat hinzu und rührte erneut für 30 min im Mikrowellenofen bei 200°C. Nach Abkühlen auf RT verteilte man den Ansatz zwischen 10 ml Ethylacetat und 10 ml Wasser. Die organische Phase wurde abgetrennt, mit je 10 ml Wasser und gesättigter Natriumchlorid-Lösung gewaschen, über Natriumsulfat getrocknet, filtriert und im Vakuum eingeengt. Der Rückstand wurde durch präparative HPLC gereinigt [Methode 19]. Man erhielt 17 mg (35% d. Th.) der Zielverbindung als gelbliches Harz.
MS [ESIpos]: m/z = 474 (M+H)⁺
¹H-NMR (400 MHz, CDCl₃): δ = 0.65-0.82 (br. m, 2H), 0.97-1.09 (m, 2H), 2.92-3.03 (m, 1H), 3.98 (s, 2H), 5.10 (s, 2H), 7.33-7.58 (m, 6H), 7.68 (d, 2H), 10.19 (s, 1H).

### Beispiel 86

### 5-(4-Chlorphenyl)-4-(3,3,3-trifluor-2-hydroxypropyl)-2-({5-[2-(trifluormethyl)phenyl]pyridin-3-yl}-methyl)-2,4-dihydro-3H-1,2,4-triazol-3-on

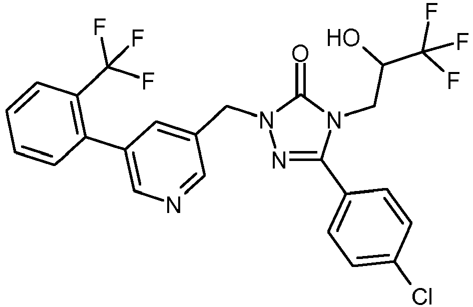

72 mg (0.15 mmol) der Verbindung aus Beispiel 28A und 43 mg (0.23 mmol) 2-(Trifluormethyl)-phenylboronsäure wurden in 2 ml Dioxan gelöst. Durch diese Lösung leitete man für 10 min einen Argonstrom und fügte dann 8.7 mg (0.008 mmol) Tetrakis(triphenylphosphin)palladium(0) unter Argon hinzu. Das Gemisch wurde zum Sieden erhitzt und unter Argon mit 0.15 ml (0.30 mmol) einer 2 N wässrigen Natriumcarbonat-Lösung versetzt. Es wurde 20 h unter Rückfluss gerührt. Das Gemisch wurde nach Abkühlen auf RT mit 10 ml Wasser verdünnt und zweimal mit je 15 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 36 mg (44% d. Th.) der Zielverbindung erhalten.
LC/MS [Methode 4]: Rₜ = 1.18 min; MS [ESIpos]: m/z = 543 (M+H)⁺
¹H-NMR (400 MHz, DMSO-d₆): δ = 3.83 (dd, 1H), 3.99 (dd, 1H), 4.23-4.35 (m, 1H), 5.09-5.20 (m, 2H), 6.90 (d, 1H), 7.47 (d, 1H), 7.59-7.65 (m, 2H), 7.65-7.81 (m, 5H), 7.89 (d, 1H), 8.49 (d, 1H), 8.62 (d, 1H).

### Beispiel 87

### 5-(4-Chlorphenyl)-2-{[5-(2-chlorphenyl)pyridin-3-yl]methyl}-4-(3,3,3-trifluor-2-hydroxypropyl)-2,4-dihydro-3H-1,2,4-triazol-3-on

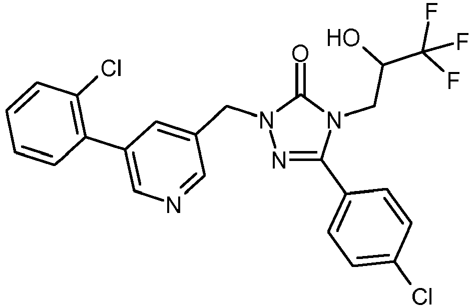

Analog zur Herstellung der Verbindung in Beispiel 86 wurden 72 mg (0.15 mmol) der Verbindung aus Beispiel 28A und 59 mg (0.23 mmol) 2-Chlorphenylboronsäure miteinander umgesetzt. Es wurden 49 mg (64% d. Th.) der Zielverbindung erhalten.
LC/MS [Methode 4]: Rₜ = 1.16 min; MS [ESIpos]: m/z = 509 und 511 (M+H)⁺
¹H-NMR (400 MHz, DMSO-d₆): δ = 3.83 (dd, 1H), 3.99 (dd, 1H), 4.24-4.36 (m, 1H), 5.10-5.20 (m, 2H), 6.90 (d, 1H), 7.44-7.50 (m, 3H), 7.58-7.65 (m, 3H), 7.75 (d, 2H), 7.86 (t, 1H), 8.57-8.62 (m, 2H).

### Beispiel 88

### 5-(4-Chlorphenyl)-2-{[5-(2,3-dichlorphenyl)pyridin-3-yl]methyl}-4-[(2S)-3,3,3-trifluor-2-hydroxypropyl]-2,4-dihydro-3H-1,2,4-triazol-3-on

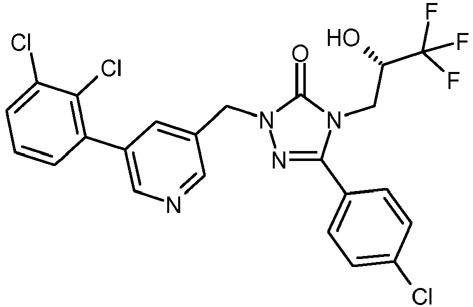

Analog zur Herstellung der Verbindung in Beispiel 86 wurden 34 mg (0.07 mmol) der Verbindung aus Beispiel 29A und 20 mg (0.10 mmol) 2,3-Dichlorphenylboronsäure miteinander umgesetzt. Es wurden 26 mg (69% d. Th.) der Zielverbindung erhalten.
LC/MS [Methode 4]: Rₜ = 1.21 min; MS [ESIpos]: m/z = 543 und 545 (M+H)⁺
¹H-NMR (400 MHz, DMSO-d₆): δ = 3.83 (dd, 1H), 3.99 (dd, 1H), 4.26-4.34 (m, 1H), 5.10-5.20 (m, 2H), 6.90 (d, 1H), 7.42-7.53 (m, 2H), 7.60-7.65 (m, 2H), 7.71-7.78 (m, 3H), 7.86 (t, 1H), 8.61 (dd, 2H).

### Beispiel 89

### 5-(4-Chlorphenyl)-4-[(2S)-3,3,3-trifluor-2-hydroxypropyl]-2-({2-[2-(trifluormethyl)phenyl]pyridin-4-yl}methyl)-2,4-dihydro-3H-1,2,4-triazol-3-on

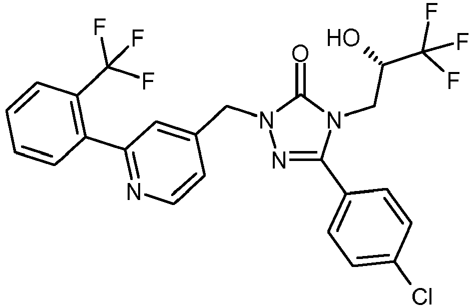

145 mg (0.46 mmol) der Verbindung aus Beispiel 64A wurden in 3 ml Acetonitril gelöst und mit 141 mg (0.46 mmol) der Verbindung aus Beispiel 5A sowie 224 mg (0.69 mmol) Cäsiumcarbonat versetzt. Das Gemisch wurde 16 h bei 60°C gerührt. Nach Abkühlen auf RT wurde vom Feststoff filtriert und mit etwas Acetonitril nachgewaschen. Das Filtrat wurde im Vakuum auf ein Volumen von ca. 2 ml reduziert, mit 0.1 ml 1 N Salzsäure versetzt und direkt chromatographisch aufgereinigt [Methode 19]. Es wurden 49 mg (20% d. Th.) der Zielverbindung erhalten.
LC/MS [Methode 4]: Rₜ = 1.15 min; MS [ESIpos]: m/z = 543 (M+H)⁺
¹H-NMR (400 MHz, DMSO-d₆): δ = 3.85 (dd, 1H), 4.01 (dd, 2H), 4.28-4.35 (m, 1H), 5.09-5.20 (m, 2H), 6.90 (d, 1H), 7.32 (dd, 1H), 7.41 (s, 1H), 7.52 (d, 1H), 7.61-7.66 (m, 2H), 7.68 (d, 1H), 7.73-7.79 (m, 3H), 7.85 (d, 1H), 8.63 (d, 1H).

### Beispiel 90

### 5-(4-Chlorphenyl)-2-{[2-(2-chlorphenyl)pyridin-4-yl]methyl}-4-[(2S)-3,3,3-trifluor-2-hydroxypropyl]-2,4-dihydro-3H-1,2,4-triazol-3-on

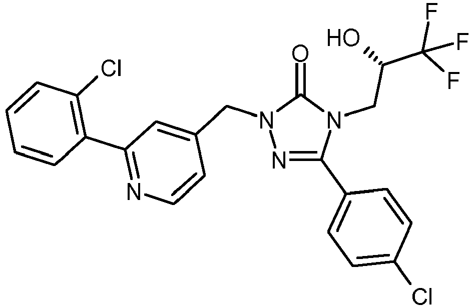

Analog zur Herstellung der Verbindung in Beispiel 89 wurden 261 mg (0.85 mmol) der Verbindung aus Beispiel 5A und 240 mg (0.85 mmol) der Verbindung aus Beispiel 65A miteinander umgesetzt. Es wurden 249 mg (54% d. Th.) der Zielverbindung erhalten.
LC/MS [Methode 3]: Rₜ = 1.28 min; MS [ESIpos]: m/z = 509 und 511 (M+H)⁺
¹H-NMR (400 MHz, DMSO-d₆): δ = 3.87 (dd, 1H), 4.01 (dd, 1H), 4.26-4.39 (m, 1H), 5.10-5.20 (m, 2H), 6.90 (d, 1H), 7.30 (d, 1H), 7.41-7.50 (m, 2H), 7.55-7.60 (m, 3H), 7.64 (d, 2H), 7.77 (d, 2H), 8.68 (d, 1H).

### Beispiel 91

### 5-(4-Chlorphenyl)-2-{[2-(2,3-dichlorphenyl)pyridin-4-yl]methyl}-4-[(2S)-3,3,3-trifluor-2-hydroxypropyl]-2,4-dihydro-3H-1,2,4-triazol-3-on

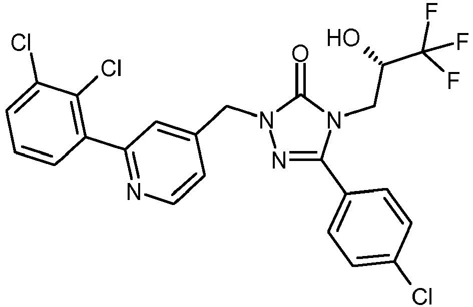

81 mg (0.26 mmol) der Verbindung aus Beispiel 66A wurden in 3 ml Acetonitril gelöst und mit 79 mg (0.26 mmol) der Verbindung aus Beispiel 5A sowie 125 mg (0.38 mmol) Cäsiumcarbonat versetzt. Das Gemisch wurde 2 h bei 65°C und weitere 16 h bei RT gerührt. Es wurde dann vom Feststoff abfiltriert und dieser mit etwas Acetonitril nachgewaschen. Das Filtrat wurde im Vakuum eingeengt und direkt chromatographisch gereinigt [Methode 19]. Es wurden 79 mg (56% d. Th.) der Zielverbindung erhalten.
LC/MS [Methode 4]: Rₜ = 1.21 min; MS [ESIpos]: m/z = 543 und 545 (M+H)⁺
¹H-NMR (400 MHz, DMSO-d₆): δ = 3.85 (dd, 1H), 4.01 (dd, 1H), 4.28-4.35 (m, 1H), 5.10-5.21 (m, 2H), 6.91 (d, 1H), 7.34 (dd, 1H), 7.45-7.52 (m, 2H), 7.57 (s, 1H), 7.61-7.66 (m, 2H), 7.71-7.79 (m, 3H), 8.67 (d, 1H).

### Beispiel 92

### 5-(4-Chlorphenyl)-4-[(2S)-3,3,3-trifluor-2-hydroxypropyl]-2-({2-[2-(trifluormethyl)phenyl]-pyrimidin-4-yl}methyl)-2,4-dihydro-3H-1,2,4-triazol-3-on

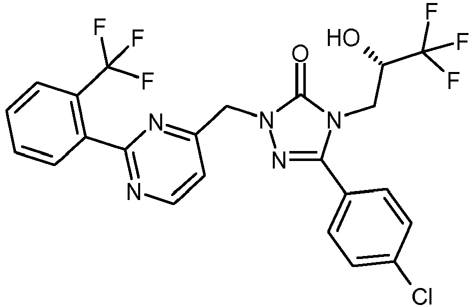

303 mg (0.19 mmol) der Verbindung aus Beispiel 80A (Reinheit ca. 20%) wurden in 2 ml Acetonitril gelöst und mit 65 mg (0.21 mmol) der Verbindung aus Beispiel 5A sowie 93 mg (0.29 mmol) Cäsiumcarbonat versetzt. Das Gemisch wurde 2.5 h bei 60°C und weitere 96 h bei RT gerührt. Es wurde dann vom Feststoff abfiltriert und dieser mit etwas Acetonitril nachgewaschen. Das Filtrat wurde im Vakuum eingeengt und direkt chromatographisch gereinigt [Methode 19]. Es wurden 19 mg der Zielverbindung erhalten, die einer weiteren chromatographischen Reinigung an Kieselgel unterworfen wurden (Elutionsmittel: Cyclohexan/Ethylacetat 7:3, dann 1:1). Es wurden 8 mg (7% d. Th.) der Zielverbindung erhalten.
LC/MS [Methode 3]: Rₜ = 1.32 min; MS [ESIpos]: m/z = 544 (M+H)⁺
¹H-NMR (400 MHz, DMSO-d₆): δ = 3.86 (dd, 1H), 4.01 (dd, 1H), 4.31-4.35 (m, 1H), 5.13-5.26 (m, 2H), 6.93 (d, 1H), 7.39 (d, 1H), 7.61-7.67 (m, 2H), 7.69-7.83 (m, 5H), 7.88 (d, 1H), 8.93 (d, 1 H).

### Beispiel 93

### 5-(4-Chlorphenyl)-2-{[2-(2-chlorphenyl)pyrimidin-4-yl]methyl}-4-[(2S)-3,3,3-trifluor-2-hydroxypropyl]-2,4-dihydro-3H-1,2,4-triazol-3-on

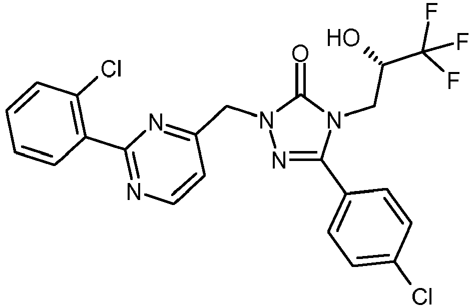

259 mg (0.18 mmol) der Verbindung aus Beispiel 81A (Reinheit ca. 20%) wurden in 2 ml Acetonitril gelöst und mit 62 mg (0.21 mmol) der Verbindung aus Beispiel 5A sowie 89 mg (0.27 mmol) Cäsiumcarbonat versetzt. Das Gemisch wurde 2.5 h bei 60°C und weitere 96 h bei RT gerührt. Es wurde dann vom Feststoff abfiltriert und dieser mit etwas Acetonitril nachgewaschen. Das Filtrat wurde im Vakuum eingeengt und direkt chromatographisch gereinigt [Methode 19]. Es wurden 31 mg der Zielverbindung erhalten, die einer weiteren chromatographischen Reinigung an Kieselgel unterworfen wurden (Elutionsmittel: Cyclohexan/Ethylacetat 7:3, dann 1:1). Es wurden 23 mg (22% d. Th.) der Zielverbindung erhalten.
LC/MS [Methode 4]: Rₜ = 1.13 min; MS [ESIpos]: m/z = 510 und 512 (M+H)⁺
¹H-NMR (400 MHz, DMSO-d₆): δ = 3.87 (dd, 1H), 4.02 (dd, 1H), 4.28-4.38 (m, 1H), 5.16-5.26 (m, 2H), 6.93 (d, 1H), 7.36 (d, 1H), 7.44-7.54 (m, 2H), 7.56-7.60 (m, 1H), 7.61-7.66 (m, 2H), 7.71-7.79 (m, 3H), 8.94 (d, 1H).

### Beispiel 94

### 5-(4-Chlorphenyl)-4-[(2S)-3,3,3-trifluor-2-hydroxypropyl]-2-({6-[2-(trifluormethyl)phenyl]-pyrimidin-4-yl}methyl)-2,4-dihydro-3H-1,2,4-triazol-3-on

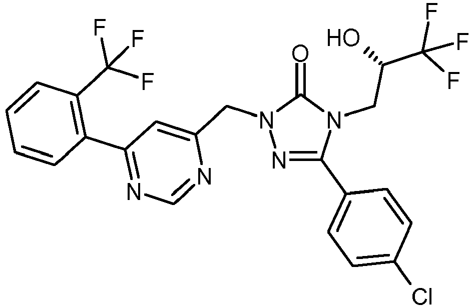

37 mg (0.12 mmol) der Verbindung aus Beispiel 82A wurden in 2 ml Acetonitril gelöst und mit 39 mg (0.13 mmol) der Verbindung aus Beispiel 5A sowie 57 mg (0.18 mmol) Cäsiumcarbonat versetzt. Das Gemisch wurde 2.5 h bei 60°C gerührt. Nach Abkühlen auf RT wurde vom Feststoff abfiltriert und dieser mit etwas Acetonitril nachgewaschen. Das Filtrat wurde im Vakuum eingeengt und direkt chromatographisch aufgereinigt [Methode 19]. Das erhaltene Produkt wurde einer weiteren chromatographischen Reinigung an Kieselgel unterworfen (Elutionsmittel: Cyclohexan/ Ethylacetat 4:1, dann 3:2). Es wurden 28 mg (41% d. Th.) der Zielverbindung erhalten.
LC/MS [Methode 6]: Rₜ = 2.34 min; MS [ESIpos]: m/z = 544 (M+H)⁺
¹H-NMR (400 MHz, DMSO-d₆): δ = 3.86 (dd, 1H), 4.01 (dd, 1H), 4.27-4.34 (m, 1H), 5.17-5.29 (m, 2H), 6.91 (d, 1H), 7.55 (s, 1H), 7.59 (d, 1H), 7.63 (d, 2H), 7.72-7.78 (m, 3H), 7.80-7.85 (m, 1H), 7.91 (d, 1H), 9.24 (d, 1H).

### Beispiel 95

### 5-(4-Chlorphenyl)-2-{[6-(2-chlorphenyl)pyrimidin-4-yl]methyl}-4-[(2S)-3,3,3-trifluor-2-hydroxypropyl]-2,4-dihydro-3H-1,2,4-triazol-3-on

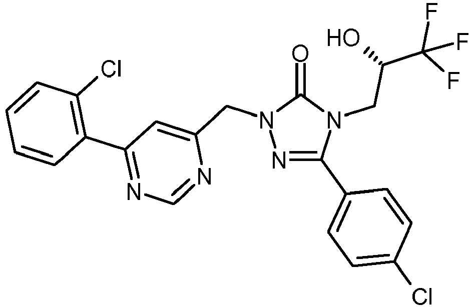

Analog zur Herstellung der Verbindung in Beispiel 94 wurden 27 mg (0.10 mmol) der Verbindung aus Beispiel 83A und 32 mg (0.11 mmol) der Verbindung aus Beispiel 5A miteinander umgesetzt. Es wurden 29 mg (54% d. Th.) der Zielverbindung erhalten.
LC/MS [Methode 4]: Rₜ = 1.15 min; MS [ESIpos]: m/z = 510 und 512 (M+H)⁺
¹H-NMR (400 MHz, DMSO-d₆): δ = 3.86 (dd, 1H), 4.02 (dd, 1H), 4.27-4.35 (m, 1H), 5.19-5.29 (m, 2H), 6.91 (d, 1H), 7.48-7.59 (m, 2H), 7.60-7.69 (m, 4H), 7.72 (d, 1H), 7.74-7.80 (m, 2H), 9.27 (d, 1H).

### Beispiel 96

### 5-(4-Chlorphenyl)-2-{[6-(2,3-dichlorphenyl)pyrimidin-4-yl]methyl}-4-[(2S)-3,3,3-trifluor-2-hydroxypropyl]-2,4-dihydro-3H-1,2,4-triazol-3-on

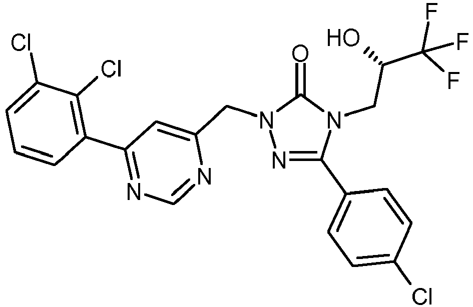

Analog zur Herstellung der Verbindung in Beispiel 94 wurden 23 mg (0.07 mmol) der Verbindung aus Beispiel 84A und 24 mg (0.08 mmol) der Verbindung aus Beispiel 5A miteinander umgesetzt. Es wurden 29 mg (66% d. Th.) der Zielverbindung erhalten.
LC/MS [Methode 6]: Rₜ = 2.54 min; MS [ESIpos]: m/z = 544 und 546 (M+H)⁺
¹H-NMR (400 MHz, DMSO-d₆): δ = 3.86 (dd, 1H), 4.02 (dd, 1H), 4.26-4.38 (m, 1H), 5.18-5.30 (m, 2H), 6.92 (s, 1H), 7.51-7.56 (m, 1H), 7.57-7.60 (m, 1H), 7.61-7.65 (m, 2H), 7.71 (d, 1H), 7.74-7.79 (m, 2H), 7.81 (dd, 1H), 9.29 (d, 1H).

### Beispiel 97

### 5-(4-Chlorphenyl)-4-(3,3,3-trifluor-2-hydroxypropyl)-2-({1-[3-(trifluormethyl)benzyl]-1H-1,2,3-triazol-4-yl}methyl)-2,4-dihydro-3H-1,2,4-triazol-3-on

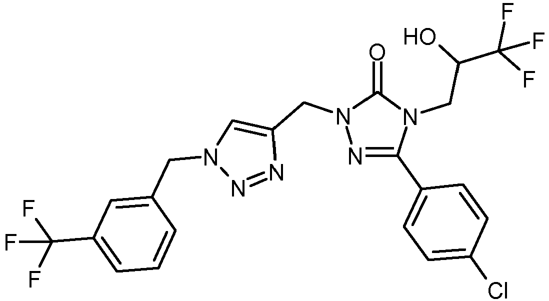

29 mg (0.12 mmol) 3-(Trifluormethyl)benzylbromid wurden in 1 ml Acetonitril vorgelegt und mit 8 mg (0.12 mmol) Natriumazid versetzt. Das Gemisch wurde 1 h bei RT gerührt. Anschließend wurden 0.24 mg (0.012 mmol) Kupfer(II)acetat-Monohydrat sowie 50 mg (0.14 mmol) der Verbindung aus Beispiel 13A hinzugegeben. Die resultierende Mischung wurde 11 Tage bei Raumtemperatur gerührt. Das Reaktionsgemisch wurde dann durch etwas Kieselgel filtriert, das Produkt mit ca. 10 ml Ethylacetat eluiert und das Filtrat im Vakuum eingeengt. Das Rohprodukt wurde chromatographisch gereinigt [Methode 19]. Es wurden 52 mg (65% d. Th.) der Zielverbindung erhalten.
LC/MS [Methode 4]: Rₜ = 1.13 min; MS [ESIpos]: m/z = 547 (M+H)⁺
¹H-NMR (400 MHz, CDCl₃): δ = 3.91-3.99 (m, 1H), 3.98-4.05 (m, 1H), 4.46-4.56 (m, 1H), 5.15 (q, 2H), 5.34 (d, 1H), 5.51-5.61 (m, 2H), 7.41-7.59 (m, 8H), 7.63 (d, 1H).

### Beispiel 98

### 5-(4-Chlorphenyl)-4-(3,3,3-trifluor-2-hydroxypropyl)-2-({1-[2-(trifluormethyl)benzyl]-1H-1,2,3-triazol-4-yl}methyl)-2,4-dihydro-3H-1,2,4-triazol-3-on

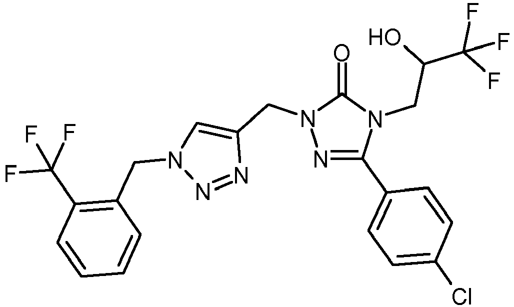

Analog zur Herstellung der Verbindung in Beispiel 97 wurden ausgehend von 50 mg (0.14 mmol) der Verbindung aus Beispiel 13A 54 mg (68% d. Th.) der Titelverbindung erhalten.
LC/MS [Methode 4]: Rₜ = 1.13 min; MS [ESIpos]: m/z = 547 (M+H)⁺
¹H-NMR (400 MHz, CDCl₃): δ = 3.91-3.99 (m, 1H), 3.99-4.06 (m, 1H), 4.46-4.57 (m, 1H), 5.16 (q, 2H), 5.31 (d, 1H), 5.72 (s, 2H), 7.20 (d, 1H), 7.42-7.58 (m, 7H), 7.72 (d, 1H).

### Beispiel 99

### 5-(4-Chlorphenyl)-4-[(2S)-3,3,3-trifluor-2-hydroxypropyl]-2-({1-[2-(trifluormethyl)phenyl]-1H-1,2,3-triazol-4-yl}methyl)-2,4-dihydro-3H-1,2,4-triazol-3-on

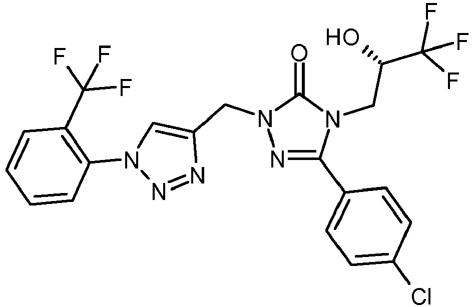

20 mg (0.31 mmol) Natriumazid wurden in 1 ml Methanol vorgelegt und mit 58 mg (0.31 mmol) 2-(Trifluormethyl)phenylboronsäure sowie 6 mg (0.03 mmol) Kupfer(II)acetat-Monohydrat versetzt. Es wurde 18 h bei RT gerührt. Das Gemisch wurde dann mit 0.9 ml Wasser, 30 mg (0.15 mmol) L-Ascorbinsäure-Natriumsalz sowie 117 mg (0.34 mmol) der Verbindung aus Beispiel 14A versetzt. Es wurde weitere 18 h bei RT gerührt. Zur Aufarbeitung verdünnte man mit 10 ml Wasser sowie 10 ml Ethylacetat und versetzte das Gemisch unter Rühren mit 5 ml 0.1 N Natronlauge. Nach Trennung der Phasen wurde die wässrige Phase noch dreimal mit je 10 ml Ethylacetat extrahiert. Die vereinigten organischen Phasen wurden über Natriumsulfat getrocknet, filtriert und im Vakuum eingeengt. Das Rohprodukt wurde durch Chromatographie an Kieselgel gereinigt (Elutionsmittel: Cyclohexan/Ethylacetat 5:1, dann 1:2). Es wurden 117 mg (71% d. Th.) der Zielverbindung erhalten.
LC/MS [Methode 3]: Rₜ = 1.26 min; MS [ESIpos]: m/z = 533 (M+H)⁺
¹H-NMR (400 MHz, DMSO-d₆): δ = 3.83 (dd, 1H), 3.99 (dd, 1H), 4.28-4.34 (m, 1H), 5.12-5.23 (m, 2H), 6.91 (d, 1H), 7.63 (d, 2H), 7.68-7.79 (m, 3H), 7.81-7.96 (m, 2H), 8.03 (d, 1H), 8.51 (s, 1 H).

### Beispiel 100

### 5-(4-Chlorphenyl)-4-[(2S)-3,3,3-trifluor-2-hydroxypropyl]-2-({1-[3-(trifluormethyl)phenyl]-1H-1,2,3-triazol-4-yl}methyl)-2,4-dihydro-3H-1,2,4-triazol-3-on

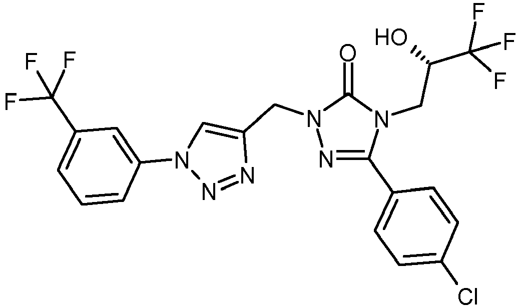

Analog zur Herstellung der Verbindung in Beispiel 99 wurden ausgehend von 117 mg (0.34 mmol) der Verbindung aus Beispiel 14A 138 mg (84% d. Th.) der Titelverbindung erhalten.
LC/MS [Methode 3]: Rₜ = 1.33 min; MS [ESIpos]: m/z = 533 (M+H)⁺
¹H-NMR (400 MHz, DMSO-d₆): δ = 3.83 (dd, 1H), 3.99 (dd, 1H), 4.27-4.38 (m, 1H), 5.13-5.22 (m, 2H), 6.91 (d, 1H), 7.59-7.65 (m, 2H), 7.73-7.79 (m, 2H), 7.81-7.90 (m, 2H), 8.24-8.32 (m, 2H), 9.02 (s, 1H).

### Beispiel 101

### 5-(4-Chlorphenyl)-2-{[1-(2-chlorphenyl)-1H-1,2,3-triazol-4-yl]methyl}-4-[(2S)-3,3,3-trifluor-2-hydroxypropyl]-2,4-dihydro-3H-1,2,4-triazol-3-on

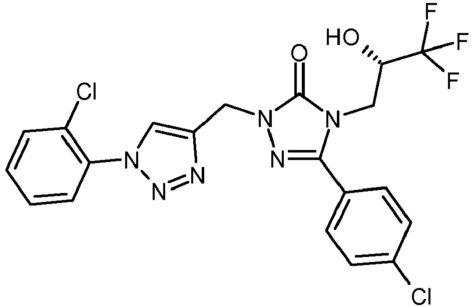

Analog zur Herstellung der Verbindung in Beispiel 99 wurden ausgehend von 75 mg (0.22 mmol) der Verbindung aus Beispiel 14A 56 mg (55% d. Th.) der Titelverbindung erhalten.
LC/MS [Methode 4]: Rₜ = 1.09 min; MS [ESIpos]: m/z = 499 und 501 (M+H)⁺
¹H-NMR (400 MHz, DMSO-d₆): δ = 3.83 (dd, 1H), 3.99 (dd, 1H), 4.28-4.35 (m, 1H), 5.13-5.22 (m, 2H), 6.91 (d, 1H), 7.55-7.70 (m, 5H), 7.74-7.80 (m, 3H), 8.54 (s, 1H).

### Beispiel 102

### 5-(4-Chlorphenyl)-2-{[1-(2,3-dichlorphenyl)-1H-1,2,3-triazol-4-yl]methyl}-4-[(2S)-3,3,3-trifluor-2-hydroxypropyl]-2,4-dihydro-3H-1,2,4-triazol-3-on

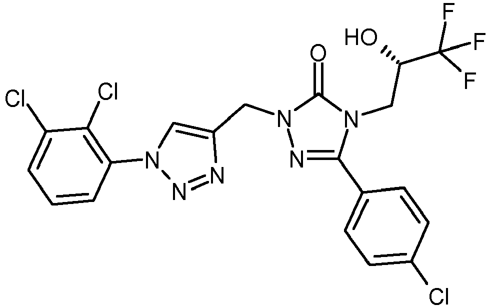

124 mg (0.36 mmol) der Verbindung aus Beispiel 14A wurden in 2 ml Acetonitril gelöst und mit 0.6 mg (0.003 mmol) Kupfer(II)acetat-Monohydrat sowie 56 mg (0.30 mmol) 1-Azido-2,3-dichlorbenzol versetzt. Es wurde 2 h bei 50°C gerührt. Zur Aufarbeitung ließ man auf RT abkühlen und filtrierte das Rohgemisch durch wenig Kieselgel. Es wurde mit Ethylacetat eluiert und die erhaltene Lösung im Vakuum eingeengt. Das Rohprodukt wurde anschließend durch Chromatographie an Kieselgel gereinigt (Elutionsmittel: Cyclohexan/Ethylacetat 1:1). Es wurden 61 mg (31% d. Th.) der Zielverbindung erhalten.
LC/MS [Methode 4]: Rₜ = 1.13 min; MS [ESIpos]: m/z = 533 und 535 (M+H)⁺
¹H-NMR (400 MHz, DMSO-d₆): δ = 3.83 (dd, 1H), 3.99 (dd, 1H), 4.29-4.34 (m, 1H), 5.13-5.23 (m, 2H), 6.91 (d, 1H), 7.58-7.66 (m, 3H), 7.69 (dd, 1H), 7.77 (d, 2H), 7.92 (dd, 1H), 8.59 (s, 1H).

### Beispiel 103

### Methyl-(2-chlorphenyl)[4-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,3-triazol-1-yl]acetat

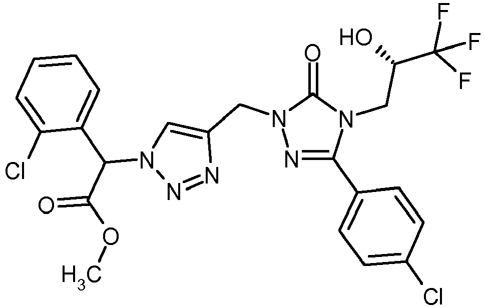

361 mg (1.37 mmol) Methyl-brom(2-chlorphenyl)acetat wurden in 10 ml Acetonitril vorgelegt und mit 89 mg (1.37 mmol) Natriumazid versetzt. Das Gemisch wurde 1 h bei RT gerührt. Anschließend wurden 2.7 mg (0.14 mmol) Kupfer(II)acetat-Monohydrat sowie 569 mg (1.64 mmol) der Verbindung aus Beispiel 14A hinzugegeben. Die resultierende Mischung wurde 48 h bei 50°C gerührt. Das Reaktionsgemisch wurde dann durch etwas Kieselgel filtriert, das Produkt mit ca. 10 ml Ethylacetat eluiert und das Filtrat im Vakuum eingeengt. Das Rohprodukt wurde durch Chromatographie an Kieselgel gereinigt (Elutionsmittel: Cyclohexan/Ethylacetat 8:1 → 6:1 → 4:1 → 2:1 → 1:1). Es wurden 496 mg (53% d. Th.) der Zielverbindung erhalten.
LC/MS [Methode 4]: Rₜ = 1.13 min; MS [ESIpos]: m/z = 571 und 573 (M+H)⁺
¹H-NMR (400 MHz, DMSO-d₆): δ = 3.76 (s, 3H), 3.81 (dd, 1H), 3.96 (dd, 1H), 4.25-4.32 (m, 1H), 5.02-5.12 (m, 2H), 6.88 (dd, 1H), 7.12 (s, 1H), 7.39-7.51 (m, 3H), 7.57 (d, 1H), 7.62 (d, 2H), 7.72 (d, 2H), 8.25 (d, 1H).

### Beispiel 104

### (2-Chlorphenyl)[4-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,3-triazol-1-yl]essigsäure

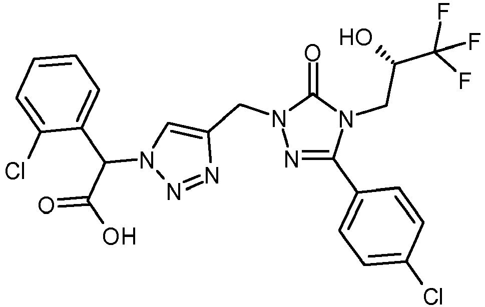

49 mg (0.09 mmol) der Verbindung aus Beispiel 103 wurden in 2 ml Methanol gelöst und mit 193 µl (0.19 mmol) einer 1 N wässrigen Lithiumhydroxid-Lösung versetzt. Es wurde 30 min bei RT gerührt. Anschließend entfernte man das Solvens im Vakuum, löste den Rückstand in 5 ml Wasser und extrahierte einmal mit 5 ml Ethylacetat. Die organische Phase wurde verworfen. Die wässrige Phase wurde mit 0.2 ml 1 N Salzsäure angesäuert und zweimal mit je 5 ml Ethylacetat extrahiert. Die vereinigten organischen Phasen wurden über Natriumsulfat getrocknet, filtriert und im Vakuum eingeengt. Es wurden 40 mg (83% d. Th.) der Zielverbindung erhalten.
LC/MS [Methode 4]: Rₜ = 0.95 min; MS [ESIpos]: m/z = 557 und 559 (M+H)⁺
¹H-NMR (400 MHz, DMSO-d₆): δ = 3.80 (dd, 1H), 3.96 (dd, 1H), 4.26-4.31 (m, 1H), 5.03-5.10 (m, 2H), 6.90 (dd, 1H), 6.94 (s, 1H), 7.42-7.51 (m, 3H), 7.54-7.58 (m, 1H), 7.59-7.65 (m, 2H), 7.73 (d, 2H), 8.22 (d, 1H).

### Beispiel 105

### 2-(2-Chlorphenyl)-2-[4-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,3-triazol-1-yl]acetamid

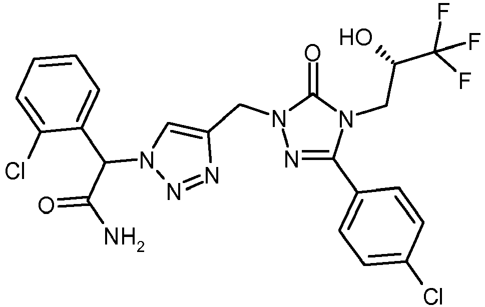

25 mg (0.045 mmol) der Verbindung aus Beispiel 104 wurden in 1 ml DMF vorgelegt und mit 9 mg (0.058 mmol) HOBt und 11 mg (0.058 mmol) EDC versetzt. Nach 10 min Rühren bei RT wurden 0.5 ml (0.90 mmol) Ammoniak-Lösung (35%-ig in Wasser) hinzugegeben und der Ansatz 16 h bei RT gerührt. Das Reaktionsgemisch wurde anschließend direkt, ohne weitere Aufarbeitung, chromatographisch gereinigt [Methode 19]. Es wurden 10 mg (40% d. Th.) der Zielverbindung erhalten.
LC/MS [Methode 4]: Rₜ = 0.99 min; MS [ESIpos]: m/z = 556 und 558 (M+H)⁺
¹H-NMR (400 MHz, DMSO-d₆): δ = 3.75-3.85 (m, 1H), 3.91-4.00 (m, 1H), 4.23-4.31 (m, 1H), 5.03 (s, 2H), 6.76 (s, 1H), 6.90 (d, 1H), 7.47 (br. s, 3H), 7.55 (m, 1H), 7.59-7.65 (m, 2H), 7.71 (d, 3H), 7.85 (m, 1H), 8.08 (br. s, 1H).

### Beispiel 106

### 5-(4-Chlorphenyl)-2-({1-[1-(2-chlorphenyl)-2-hydroxyethyl]-1H-1,2,3-triazol-4-yl}methyl)-4-[(2S)-3,3,3-trifluor-2-hydroxypropyl]-2,4-dihydro-3H-1,2,4-triazol-3-on

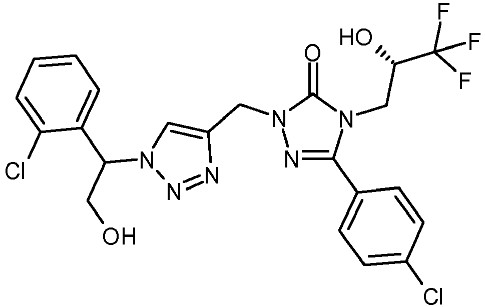

48 mg (0.084 mmol) der Verbindung aus Beispiel 103 wurden in 2 ml THF gelöst und bei -10°C mit 88 µl (0.088 mmol) einer 1 M Lithiumaluminiumhydrid-Lösung in THF versetzt. Nach vollständiger Zugabe wurde 1 h bei RT gerührt. Zur Aufarbeitung wurde bei RT mit 2 ml gesättigter wässriger Natriumkaliumtartrat-Lösung versetzt und mit 5 ml Ethylacetat extrahiert. Die organische Phase wurde einmal mit 5 ml gesättigter Kochsalz-Lösung gewaschen, über Magnesiumsulfat getrocknet, filtriert und im Vakuum eingeengt. Das Rohprodukt wurde chromatographisch gereinigt [Methode 19]. Es wurden 8 mg (18% d. Th.) der Zielverbindung erhalten.
LC/MS [Methode 2]: Rₜ = 2.20 min; MS [ESIpos]: m/z = 543 und 545 (M+H)⁺
¹H-NMR (400 MHz, DMSO-d₆): δ = 3.81 (dd, 1H), 3.93-4.04 (m, 2H), 4.21-4.32 (m, 1H), 5.02-5.12 (m, 2H), 5.45 (dd, 1H), 6.14 (dd, 1H), 6.90 (d, 1H), 7.32-7.41 (m, 3H), 7.50-7.54 (m, 1H), 7.60-7.65 (m, 2H), 7.71-7.76 (m, 2H), 8.34 (s, 1H).

### Beispiel 107

### Ethyl-[4-({3-(4-chlorphenyl)-5-oxo-4-[(2S)-3,3,3-trifluor-2-hydroxypropyl]-4,5-dihydro-1H-1,2,4-triazol-1-yl}methyl)-1H-1,2,3-triazol-1-yl][2-(trifluormethyl)phenyl]acetat

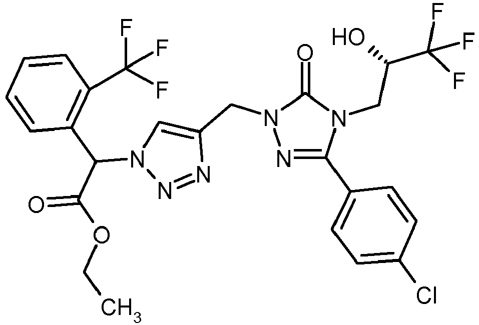

158 mg (0.15 mmol) der Verbindung aus Beispiel 98A (Reinheit ca. 30%) wurden in 2 ml Acetonitril vorgelegt und mit 9.9 mg (0.15 mmol) Natriumazid versetzt. Das Gemisch wurde 1 h bei RT gerührt. Anschließend wurden 0.3 mg (0.002 mmol) Kupfer(II)acetat-Monohydrat sowie 63 mg (0.18 mmol) der Verbindung aus Beispiel 14A hinzugegeben. Die resultierende Mischung wurde 20 h bei 50°C gerührt. Das Reaktionsgemisch wurde dann durch etwas Kieselgel filtriert, das Produkt mit ca. 10 ml Ethylacetat eluiert und das Filtrat im Vakuum eingeengt. Das Rohprodukt wurde chromatographisch gereinigt [Methode 19]. Es wurden 9 mg (8% d. Th.) der Zielverbindung erhalten.
LC/MS [Methode 3]: Rₜ = 1.36 min; MS [ESIpos]: m/z = 619 (M+H)⁺
¹H-NMR (400 MHz, DMSO-d₆): δ = 1.13 (t, 3H), 3.81 (dd, 1H), 3.96 (dd, 1H), 4.17-4.31 (m, 3H), 5.01-5.11 (m, 2H), 6.89 (dd, 1H), 6.93 (d, 1H), 7.59-7.75 (m, 6H), 7.77-7.88 (m, 2H), 8.29 (d, 1H).

### Beispiel 108

### 5-(4-Chlorphenyl)-4-cyclopropyl-2-({4-[2-(trifluormethyl)phenyl]-1H-1,2,3-triazol-1-yl}methyl)-2,4-dihydro-3H-1,2,4-triazol-3-on

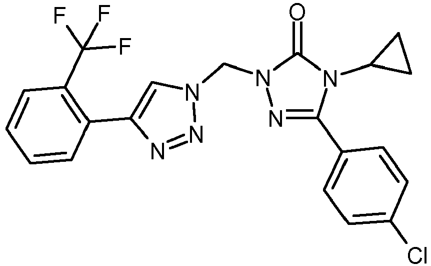

60 mg (0.21 mmol) der Verbindung aus Beispiel 122A wurden in 2 ml Acetonitril gelöst, mit 14 mg (0.21 mmol) Natriumazid versetzt und 1 h bei RT gerührt. Anschließend wurden 0.4 mg (0.002 mmol) Kupfer(II)acetat-Monohydrat sowie 43 mg (0.25 mmol) 1-Ethinyl-2-(trifluormethyl)benzol hinzugegeben. Die resultierende Mischung wurde 20 h bei RT gerührt. Zur Aufarbeitung versetzte man mit 10 ml Ethylacetat und wusch zweimal mit je 5 ml Wasser. Die organische Phase wurde über Natriumsulfat getrocknet, filtriert und im Vakuum eingeengt. Das Rohprodukt wurde chromatographisch gereinigt [Methode 19]. Es wurden 48 mg (45% d. Th.) der Zielverbindung erhalten.
LC/MS [Methode 4]: Rₜ = 1.15 min; MS [ESIpos]: m/z = 461 (M+H)⁺
¹H-NMR (400 MHz, DMSO-d₆): δ = 0.57-0.63 (m, 2H), 0.85-0.92 (m, 2H), 3.19 (m, 1H), 6.44 (s, 2H), 7.58-7.68 (m, 3H), 7.74-7.84 (m, 4H), 7.87 (d, 1H), 8.42 (s, 1H).

### Beispiel 109

### 5-(4-Chlorphenyl)-4-cyclopropyl-2-({4-[3-(trifluormethyl)phenyl]-1H-1,2,3-triazol-1-yl}methyl)-2,4-dihydro-3H-1,2,4-triazol-3-on

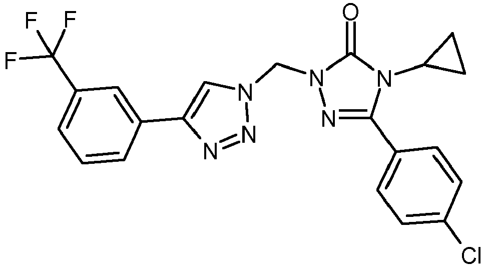

Analog zur Herstellung der Verbindung in Beispiel 108 wurden ausgehend von 60 mg (0.21 mmol) der Verbindung aus Beispiel 122A 21 mg (21% d. Th.) der Titelverbindung erhalten.
LC/MS [Methode 4]: Rₜ = 1.19 min; MS [ESIpos]: m/z = 461 (M+H)⁺
¹H-NMR (400 MHz, DMSO-d₆): δ = 0.59-0.64 (m, 2H), 0.85-0.92 (m, 2H), 3.18 (m, 1H), 6.39 (s, 2H), 7.57-7.62 (m, 2H), 7.66-7.73 (m, 2H), 7.78-7.83 (m, 2H), 8.19-8.26 (m, 2H), 8.91 (s, 1H).

### Beispiel 110

### 5-(4-Chlorphenyl)-2-{[1-(2-chlorphenyl)-1H-imidazol-4-yl]methyl}-4-[(2S)-3,3,3-trifluor-2-hydroxypropyl]-2,4-dihydro-3H-1,2,4-triazol-3-on

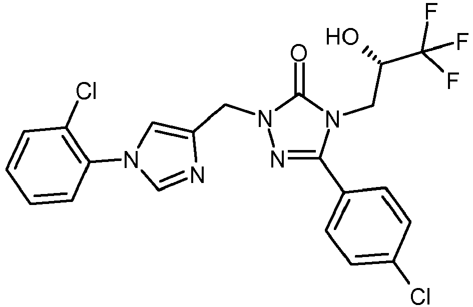

19 mg (0.06 mmol) der Verbindung aus Beispiel 5A wurden in 5 ml Acetonitril gelöst und mit 17 mg (0.13 mmol) Kaliumcarbonat sowie 17 mg (0.06 mmol) der Verbindung aus Beispiel 42A versetzt. Es wurde 2 h bei 65°C und anschließend 20 h bei RT gerührt. Zur Aufarbeitung versetzte man mit 5 ml Wasser und extrahierte zweimal mit je 10 ml Ethylacetat. Die organische Phase wurde einmal mit 5 ml gesättigter Kochsalz-Lösung gewaschen, über Natriumsulfat getrocknet, filtriert und im Vakuum eingeengt. Das Rohprodukt wurde chromatographisch gereinigt [Methode 19]. Es wurden 4 mg (12% d. Th.) der Zielverbindung erhalten.
LC/MS [Methode 4]: Rₜ = 1.04 min; MS [ESIpos]: m/z = 498 und 500 (M+H)⁺
¹H-NMR (400 MHz, DMSO-d₆): δ = 3.83 (dd, 1H), 3.99 (dd, 1H), 4.27-4.37 (m, 1H), 4.89-4.96 (m, 2H), 6.92 (br. s, 1H), 7.37 (s, 1H), 7.47-7.57 (m, 3H), 7.62 (d, 2H), 7.67-7.72 (m, 1H), 7.76 (d, 2H), 7.87 (s, 1H).

### Beispiel 111

### 5-(4-Chlorphenyl)-2-{[1-(2-chlorphenyl)-1H-pyrazol-4-yl]methyl}-4-[(2S)-3,3,3-trifluor-2-hydroxypropyl]-2,4-dihydro-3H-1,2,4-triazol-3-on

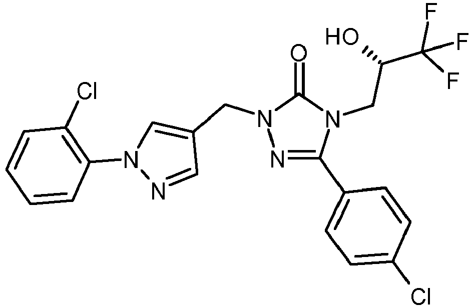

27 mg (0.09 mmol) der Verbindung aus Beispiel 5A wurden in 2 ml Acetonitril gelöst und mit 58 mg (0.18 mmol) Cäsiumcarbonat sowie 24 mg (0.09 mmol) der Verbindung aus Beispiel 43A versetzt. Es wurde 2 h bei 65°C und anschließend 20 h bei RT gerührt. Zur Aufarbeitung versetzte man mit 5 ml Wasser und extrahierte zweimal mit je 10 ml Ethylacetat. Die organische Phase wurde einmal mit 5 ml gesättigter Kochsalz-Lösung gewaschen, über Natriumsulfat getrocknet, filtriert und im Vakuum eingeengt. Das Rohprodukt wurde chromatographisch gereinigt [Methode 19]. Es wurden 25 mg (56% d. Th.) der Zielverbindung erhalten.
LC/MS [Methode 4]: Rₜ = 1.11 min; MS [ESIpos]: m/z = 498 und 500 (M+H)⁺
¹H-NMR (400 MHz, DMSO-d₆): δ = 3.82 (dd, 1H), 3.98 (dd, 1H), 4.31 (d, 1H), 4.92-5.01 (m, 2H), 6.90 (d, 1H), 7.45-7.52 (m, 2H), 7.55-7.70 (m, 4H), 7.73-7.79 (m, 3H), 8.16 (s, 1H).

### Beispiel 112

### 4-Allyl-5-(4-chlorphenyl)-2-{[1-(2,6-dichlorbenzyl)-1H-imidazol-5-yl]methyl}-2,4-dihydro-3H-1,2,4-triazol-3-on

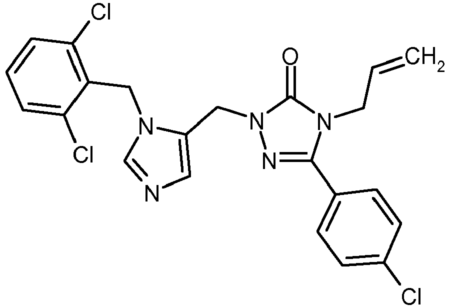

41 mg (0.17 mmol) der Verbindung aus Beispiel 12A wurden in 2 ml DMF gelöst und mit 48 mg (0.17 mmol) der Verbindung aus Beispiel 40A sowie 85 mg (0.26 mmol) Cäsiumcarbonat versetzt. Das Gemisch wurde 16 h bei 80°C gerührt. Nach Abkühlen auf RT wurde das Reaktionsgemisch mit 1 ml Methanol verdünnt und direkt chromatographisch aufgereinigt [Methode 19]. Es wurden 4 mg (5% d. Th.) der Zielverbindung erhalten.
LC/MS [Methode 5]: Rₜ = 2.01 min; MS [ESIpos]: m/z = 474 und 476 (M+H)⁺
¹H-NMR (400 MHz, CDCl₃): δ = 4.35 (d, 2H), 4.98 (s, 2H), 5.09 (d, 1H), 5.23 (d, 1H), 5.29-5.42 (m, 2H), 5.83-5.96 (m, 1H), 7.07 (s, 1H), 7.22-7.29 (m, 1H), 7.34-7.43 (m, 4H), 7.50-7.61 (m, 3H).

### Beispiel 113

### 4-Allyl-5-(4-chlorphenyl)-2-{[1-(2,6-dichlorbenzyl)-4-nitro-1H-imidazol-2-yl]methyl}-2,4-dihydro-3H-1,2,4-triazol-3-on

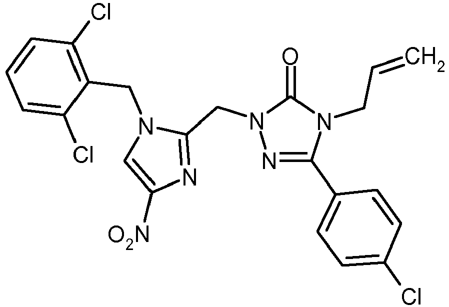

110 mg (0.47 mmol) der Verbindung aus Beispiel 12A wurden in 10 ml DMF gelöst und mit 150 mg (0.47 mmol) der Verbindung aus Beispiel 41A sowie 229 mg (0.70 mmol) Cäsiumcarbonat versetzt. Das Gemisch wurde 16 h bei 80°C gerührt. Nach Abkühlen auf RT wurde das Reaktionsgemisch auf ca. 25 ml Eiswasser gegeben und 10 min verrührt. Dabei fiel ein Niederschlag aus, der abgesaugt und mit Wasser gewaschen wurde. Der Feststoff wurde im Hochvakuum getrocknet. Es wurden 120 mg (44% d. Th.) der Zielverbindung mit einer Reinheit von 90% erhalten.
LC/MS [Methode 3]: Rₜ = 1.35 min; MS [ESIpos]: m/z = 519 und 521 (M+H)⁺
¹H-NMR (400 MHz, DMSO-d₆): δ = 4.31-4.45 (m, 2H), 5.03 (d, 1H), 5.16 (d, 1H), 5.32 (s, 2H), 5.63 (s, 2H), 5.80-5.92 (m, 1H), 7.50-7.56 (m, 1H), 7.57-7.67 (m, 6H), 7.77 (s, 1H).

### Beispiel 114

### 5-(4-Chlorphenyl)-4-cyclopropyl-2-({1-[2-(trifluormethyl)benzyl]-1H-1,2,4-triazol-5-yl}sulfonyl)-2,4-dihydro-3H-1,2,4-triazol-3-on

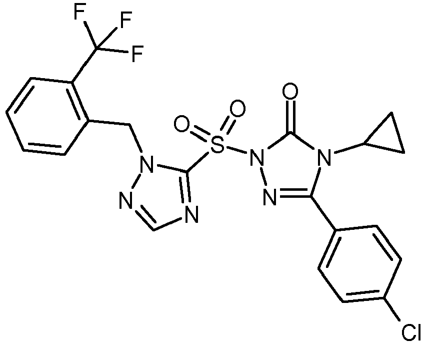

37 mg (0.10 mmol) der Verbindung aus Beispiel 93A wurden in 3 ml Dichlormethan gelöst und mit 21 µl (0.13 mmol) *N,N*-Diisopropylethylamin versetzt. Anschließend wurden 30 mg (0.13 mmol) 2-(Trifluormethyl)benzylbromid, gelöst in 1 ml Dichlormethan, hinzugefügt und das Gemisch 48 h bei RT gerührt. Zur Aufarbeitung wurde im Vakuum eingeengt und das Rohprodukt chromatographisch gereinigt [Methode 19]. Es wurden 33 mg (63% d. Th.) der Zielverbindung erhalten.
LC/MS [Methode 5]: Rₜ = 2.57 min; MS [ESIpos]: m/z = 525 (M+H)⁺
¹H-NMR (400 MHz, CDCl₃): δ = 0.72-0.78 (m, 2H), 0.97-1.04 (m, 2H), 2.94 (tt, 1H), 5.60 (s, 2H), 7.40 (d, 1H), 7.46 (d, 2H), 7.48-7.60 (m, 2H), 7.69-7.75 (m, 3H), 8.11 (s, 1H).

### Beispiel 115

### 5-(4-Chlorphenyl)-4-cyclopropyl-2-{[1-(2,6-dichlorbenzyl)-1H-1,2,4-triazol-5-yl]sulfonyl}-2,4-dihydro-3H-1,2,4-triazol-3-on

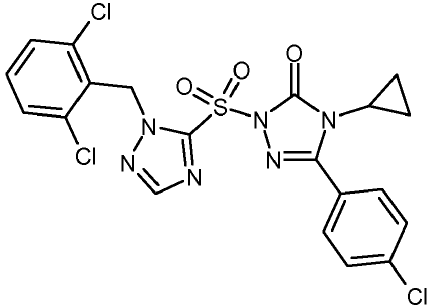

37 mg (0.10 mmol) der Verbindung aus Beispiel 93A wurden in 3 ml Dichlormethan gelöst und mit 21 µl (0.13 mmol) *N,N-*Diisopropylethylamin versetzt. Anschließend wurden 30 mg (0.13 mmol) 2,6-Dichlorbenzylbromid, gelöst in 1 ml Dichlormethan, hinzugefügt und das Gemisch 20 h bei RT gerührt. Zur Aufarbeitung wurde im Vakuum eingeengt und das Rohprodukt chromatographisch gereinigt [Methode 19]. Es wurden 39 mg (74% d. Th.) der Zielverbindung erhalten.
LC/MS [Methode 2]: Rₜ = 2.41 min; MS [ESIpos]: m/z = 525 und 527 (M+H)⁺
¹H-NMR (400 MHz, CDCl₃): δ = 0.73-0.79 (m, 2H), 0.98-1.05 (m, 2H), 2.95 (tt, 1H), 5.72 (s, 2H), 7.30-7.36 (m, 1H), 7.37-7.43 (m, 2H), 7.46 (d, 2H), 7.72 (d, 2H), 8.10 (s, 1H).

### Beispiel 116

### 5-(4-Chlorphenyl)-4-(4-methoxybenzyl)-2-({1-[2-(trifluormethyl)benzyl]-1H-1,2,4-triazol-5-yl}-sulfonyl)-2,4-dihydro-3H-1,2,4-triazol-3-on

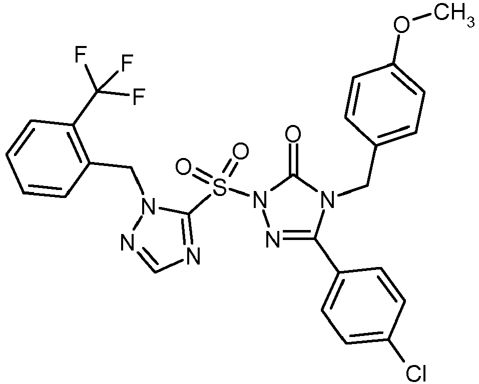

334 mg (0.75 mmol) der Verbindung aus Beispiel 94A wurden in 5 ml Dichlormethan gelöst und mit 154 µl (0.93 mmol) *N,N-*Diisopropylethylamin versetzt. Anschließend wurden 223 mg (0.93 mmol) 2-(Trifluormethyl)benzylbromid, gelöst in 0.5 ml Dichlormethan, hinzugefügt und das Gemisch 20 h bei RT gerührt. Zur Aufarbeitung wurde im Vakuum eingeengt und das Rohprodukt chromatographisch gereinigt [Methode 19]. Es wurden 245 mg (54% d. Th.) der Zielverbindung erhalten.
LC/MS [Methode 3]: Rₜ = 1.44 min; MS [ESIpos]: m/z = 605 (M+H)⁺
¹H-NMR (400 MHz, CDCl₃): δ = 3.77 (s, 3H), 4.81 (s, 2H), 5.61 (s, 2H), 6.78 (d, 2H), 7.03 (d, 2H), 7.35-7.43 (m, 5H), 7.48-7.58 (m, 2H), 7.74 (d, 1H), 8.12 (s, 1H).

### Beispiel 117

### 5-(4-Chlorphenyl)-4-(3,3,3-trifluor-2-hydroxypropyl)-2-({1-[2-(trifluormethyl)benzyl]-1H-1,2,4-triazol-5-yl}sulfonyl)-2,4-dihydro-3H-1,2,4-triazol-3-on

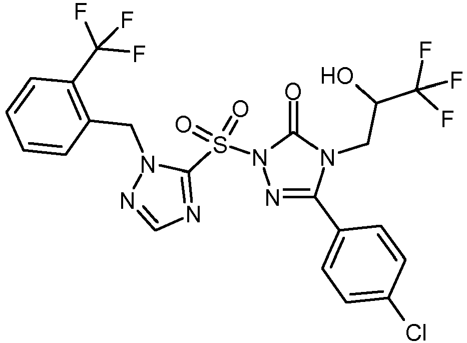

50 mg (0.10 mmol) der Verbindung aus Beispiel 95A wurden zusammen mit 50 mg (0.16 mmol) Cäsiumcarbonat in 0.5 ml DMF gelöst und mit 30 mg (0.16 mmol) 3-Brom-1,1,1-trifluorpropan-2-ol versetzt. Anschließend wurde das Gemisch 8 h bei 75°C gerührt. Zur Aufarbeitung wurde die Reaktionsmischung mit 0.5 ml Acetonitril verdünnt und direkt chromatographisch aufgereinigt [Methode 19]. Es wurden 15 mg (22% d. Th.) der Zielverbindung erhalten.
LC/MS [Methode 5]: Rₜ = 2.61 min; MS [ESIpos]: m/z = 597 (M+H)⁺
¹H-NMR (400 MHz, CDCl₃): δ = 3.86 (dd, 1H), 3.95 (dd, 1H), 4.28-4.34 (m, 1H), 4.68-4.76 (m, 1H), 5.62 (s, 2H), 7.42 (d, 1H), 7.47 (d, 2H), 7.50-7.61 (m, 2H), 7.68 (d, 2H), 7.74 (d, 1H), 8.15 (s, 1 H).

### Beispiel 118

### 4-Allyl-5-(4-chlorphenyl)-2-{[1-(2,6-dichlorbenzyl)-4-methyl-1H-imidazol-5-yl]methyl}-2,4-dihydro-3H-1,2,4-triazol-3-on und 4-Allyl-5-(4-chlorphenyl)-2-{[1-(2,6-dichlorbenzyl)-5-methyl-1H-imidazol-4-yl]methyl}-2,4-dihydro-3H-1,2,4-triazol-3-on (Regioisomerengemisch)

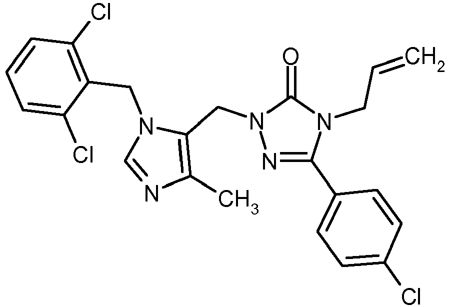

*und*

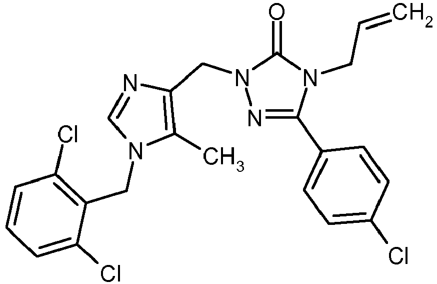

110 mg (0.33 mmol) der Verbindung aus Beispiel 96A wurden zusammen mit 88 mg (0.37 mmol) 2,6-Dichlorbenzylbromid in 5 ml DMF gelöst und mit 130 mg (0.40 mmol) Cäsiumcarbonat versetzt. Das Gemisch wurde 6 h bei 60°C gerührt. Nach Abkühlen auf RT wurde das Reaktionsgemisch mit 1 ml Methanol verdünnt und direkt chromatographisch aufgereinigt [Methode 19]. Es wurden 9 mg (6% d. Th.) eines Gemischs der regioisomeren Titelverbindungen im Verhältnis von ca. 1:1 erhalten.
LC/MS [Methode 3]: Rₜ = 1.08 min; MS [ESIpos]: m/z = 488 / 490 (M+H)⁺ und Rₜ = 1.10 min; MS [ESIpos]: m/z = 488 / 490 (M+H)⁺
¹H-NMR (400 MHz, CDCl₃): δ = 2.38 und 2.42 (2s, 3H), 4.30-4.41 (m, 2H), 4.97 und 5.49 (2s, 2H), 5.05-5.32 (m, 4H), 5.84-5.95 (m, 1H), 6.97 und 7.13 (2s, 1H), 7.24-7.47 (m, 5H), 7.49-7.58 (m, 2H).

### Beispiel 119

### 5-(4-Chlorphenyl)-4-cyclopropyl-2-({4-methyl-1-[3-(trifluormethyl)benzyl]-1H-imidazol-5-yl}-methyl)-2,4-dihydro-3H-1,2,4-triazol-3-on und 5-(4-Chlorphenyl)-4-cyclopropyl-2-({5-methyl-1-[3-(trifluormethyl)benzyl]-1H-imidazol-4-yl}-methyl)-2,4-dihydro-3H-1,2,4-triazol-3-on (Regioisomerengemisch)

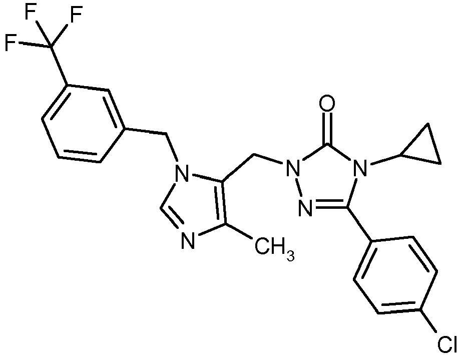

*und*

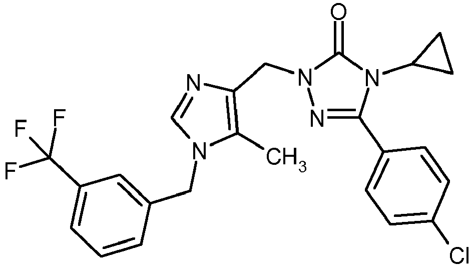

40 mg (0.12 mmol) der Verbindung aus Beispiel 97A wurden zusammen mit 88 mg (0.37 mmol) 3-(Trifluormethyl)benzylbromid in 3 ml DMF gelöst und mit 47 mg (0.15 mmol) Cäsiumcarbonat versetzt. Das Gemisch wurde 3 h bei 60°C gerührt. Nach Abkühlen auf RT wurde zur Aufarbeitung mit 5 ml Wasser verdünnt und das Gemisch dreimal mit je 10 ml Ethylacetat extrahiert. Die vereinigten organischen Phasen wurden über Natriumsulfat getrocknet, filtriert und im Vakuum eingeengt. Der Rückstand wurde in 4 ml Methanol aufgenommen und chromatographisch gereinigt [Methode 19]. Es wurden 20 mg (32% d. Th.) eines Gemischs der regioisomeren Titelverbindungen im Verhältnis von ca. 1:1.8 erhalten.
LC/MS [Methode 5]: Rₜ = 1.75 min; MS [ESIpos]: m/z = 488 (M+H)⁺ und Rₜ = 1.89 min; MS [ESIpos]: m/z = 488 (M+H)⁺
¹H-NMR (400 MHz, CDCl₃): δ = 0.53-0.59 und 0.70-0.76 (2m, 2H), 0.91-1.03 (m, 2H), 2.21 und 2.40 (2s, 3H), 2.80 und 2.94 (2tt, 1H), 4.82 und 4.93 (2s, 2H), 5.30 und 5.43 (2s, 2H), 7.11 und 7.19 (2d, 1H), 7.31-7.52 (m, 6H), 7.54-7.59 und 7.66-7.70 (2m, 2H).

### Beispiel 120

### 5-(4-Chlorphenyl)-4-cyclopropyl-2-[2-(2-methylphenoxy)benzyl]-2,4-dihydro-3H-1,2,4-triazol-3-on

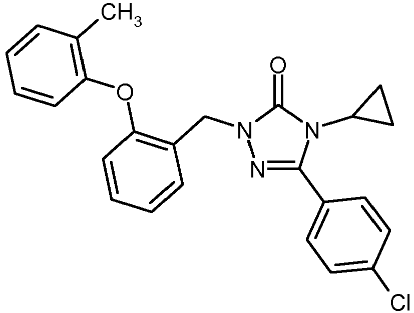

100 mg (0.25 mmol) der Verbindung aus Beispiel 99A, 53 mg (0.49 mmol) o-Kresol sowie 91 mg (0.74 mmol) 4-*N,N*-Dimethylaminopyridin wurden in 5 ml Acetonitril gelöst und mit 39 mg (0.62 mmol) Kupferpulver und 49 mg (0.62 mmol) Kupfer(II)oxid versetzt. Die Mischung wurde 16 h bei 85°C gerührt. Zur Aufarbeitung wurde das auf RT abgekühlte Gemisch über Kieselgel filtriert und der Rückstand mit etwas Ethylacetat nachgewaschen. Nach Einengen des Filtrats unter vermindertem Druck wurde das Rohprodukt chromatographisch gereinigt [Methode 19]. Es wurden 25 mg (23% d. Th.) der Zielverbindung erhalten.
LC/MS [Methode 4]: Rₜ = 1.33 min; MS [ESIpos]: m/z = 432 (M+H)⁺
¹H-NMR (400 MHz, DMSO-d₆): δ = 0.34-0.44 (m, 2H), 0.76-0.85 (m, 2H), 2.18 (s, 3H), 3.05 (tt, 1H), 5.00 (s, 2H), 6.59-6.71 (m, 2H), 7.01 (t, 1H), 7.05-7.15 (m, 2H), 7.24-7.32 (m, 2H), 7.35 (d, 1H), 7.56 (d, 2H), 7.71 (d, 2H).

### Beispiel 121

### 2-[(2'-Chlor-4-fluorbiphenyl-3-yl)methyl]-5-(4-chlorphenyl)-4-cyclopropyl-2,4-dihydro-3H-1,2,4-triazol-3-on

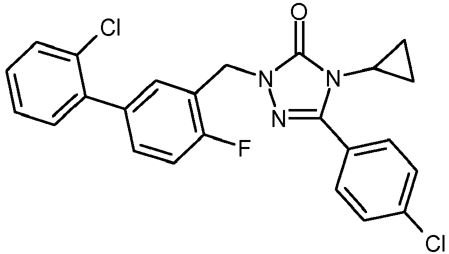

Analog zur Herstellung von Beispiel 122 wurden 89 mg (0.15 mmol) der Verbindung aus Beispiel 100A mit 59 mg (0.23 mmol) 2-Chlorphenylboronsäure umgesetzt. Es wurden 51 mg (73% d. Th.) der Zielverbindung erhalten.
LC/MS [Methode 4]: Rₜ = 1.35 min; MS [ESIpos]: m/z = 454 und 456 (M+H)⁺
¹H-NMR (400 MHz, DMSO-d₆): δ = 0.54-0.61 (m, 2H), 0.84-0.91 (m, 2H), 3.14-3.20 (m, 1H), 5.04 (s, 2H), 7.28-7.36 (m, 1H), 7.37-7.47 (m, 5H), 7.53-7.59 (m, 3H), 7.77 (d, 2H).

### Beispiel 122

### 2-[(2'-Chlorbiphenyl-3-yl)methyl]-5-(4-chlorphenyl)-4-(3,3,3-trifluor-2-hydroxypropyl)-2,4-dihydro-3H-1,2,4-triazol-3-on

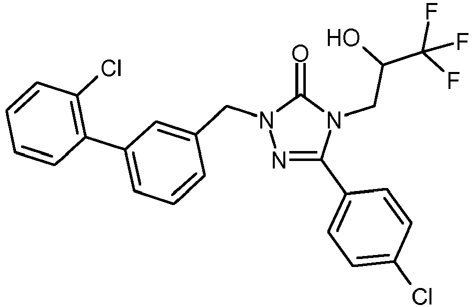

72 mg (0.15 mmol) der Verbindung aus Beispiel 101A und 59 mg (0.23 mmol) 2-Chlorphenylboronsäure wurden in 2 ml Dioxan gelöst. Durch diese Lösung leitete man für 10 min einen Argonstrom und fügte 8.7 mg (0.008 mmol) Tetrakis(triphenylphosphin)palladium(0) unter Argon hinzu. Das Gemisch wurde zum Sieden erhitzt und unter Argon mit 0.15 ml (0.30 mmol) einer 2 N wässrigen Natriumcarbonat-Lösung versetzt. Das Gemisch wurde dann 20 h unter Rückfluss gerührt. Nach Abkühlen auf RT wurde mit 10 ml Wasser verdünnt und zweimal mit je 15 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 51 mg (61% d. Th.) der Zielverbindung erhalten.
LC/MS [Methode 4]: Rₜ = 1.30 min; MS [ESIpos]: m/z = 508 (M+H)⁺
¹H-NMR (400 MHz, DMSO-d₆): δ = 3.83 (dd, 1H), 4.00 (dd, 1H), 4.26-4.34 (m, 1H), 5.01-5.13 (m, 2H), 6.88 (d, 1H), 7.32-7.50 (m, 7H), 7.54-7.59 (m, 1H), 7.62 (d, 2H), 7.74 (d, 2H).

### Beispiel 123

### 5-(4-Chlorphenyl)-4-(3,3,3-trifluor-2-hydroxypropyl)-2-{[2'-(trifluormethyl)biphenyl-3-yl]methyl}-2,4-dihydro-3H-1,2,4-triazol-3-on

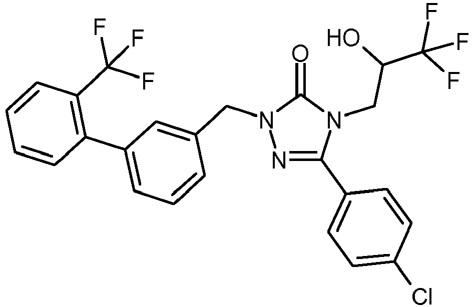

Analog zur Herstellung von Beispiel 122 wurden 72 mg (0.15 mmol) der Verbindung aus Beispiel 101A mit 43 mg (0.23 mmol) 2-(Trifluormethyl)phenylboronsäure umgesetzt. Es wurden 49 mg (58% d. Th.) der Zielverbindung erhalten.
LC/MS [Methode 4]: Rₜ = 1.31 min; MS [ESIpos]: m/z = 542 (M+H)⁺
¹H-NMR (400 MHz, DMSO-d₆): δ = 3.84 (dd, 1H), 3.99 (dd, 1H), 4.26-4.32 (m, 1H), 4.99-5.12 (m, 2H), 6.88 (d, 1H), 7.22-7.29 (m, 2H), 7.34-7.47 (m, 3H), 7.57-7.64 (m, 3H), 7.67-7.76 (m, 3H), 7.83 (d, 1H).

### Beispiel 124

### 5-(4-Chlorphenyl)-2-[(2',3'-dichlorbiphenyl-3-yl)methyl]-4-[(2S)-3,3,3-trifluor-2-hydroxypropyl]-2,4-dihydro-3H-1,2,4-triazol-3-on

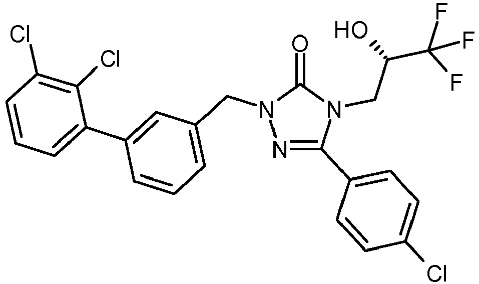

Analog zur Herstellung von Beispiel 122 wurden 94 mg (0.20 mmol) der Verbindung aus Beispiel 102A mit 56 mg (0.23 mmol) 2,3-Dichlorphenylboronsäure umgesetzt. Es wurden 48 mg (45% d. Th.) der Zielverbindung erhalten.
LC/MS [Methode 3]: Rₜ = 1.54 min; MS [ESIpos]: m/z = 542 und 544 (M+H)⁺
¹H-NMR (400 MHz, DMSO-d₆): δ = 3.84 (dd, 1H), 4.00 (dd, 1H), 4.27-4.35 (m, 1H), 5.02-5.12 (m, 2H), 6.89 (d, 1H), 7.34-7.42 (m, 4H), 7.42-7.51 (m, 2H), 7.60-7.64 (m, 2H), 7.68 (dd, 1H), 7.72-7.77 (m, 2H).

### Beispiel 125

### 5-(4-Chlorphenyl)-2-{[5-fluor-2'-(trifluormethyl)biphenyl-3-yl]methyl}-4-[(2S)-3,3,3-trifluor-2-hydroxypropyl]-2,4-dihydro-3H-1,2,4-triazol-3-on

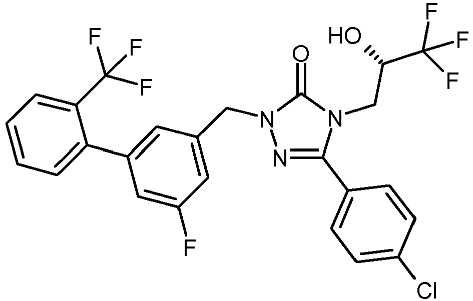

Analog zur Herstellung von Beispiel 122 wurden 59 mg (0.12 mmol) der Verbindung aus Beispiel 103A mit 36 mg (0.18 mmol) 2-(Trifluormethyl)phenylboronsäure umgesetzt. Es wurden 43 mg (64% d. Th.) der Zielverbindung erhalten.
LC/MS [Methode 4]: Rₜ = 1.34 min; MS [ESIpos]: m/z = 560 (M+H)⁺
¹H-NMR (400 MHz, DMSO-d₆): δ = 3.84 (dd, 1H), 3.99 (dd, 1H), 4.26-4.33 (m, 1H), 5.03-5.14 (m, 2H), 6.89 (d, 1H), 7.10-7.23 (m, 3H), 7.43 (d, 1H), 7.59-7.68 (m, 3H), 7.70-7.77 (m, 3H), 7.85 (d, 1H).

### Beispiel 126

### 2-[(2'-Chlor-5-fluorbiphenyl-3-yl)methyl]-5-(4-chlorphenyl)-4-[(2S)-3,3,3-trifluor-2-hydroxypropyl]-2,4-dihydro-3H-1,2,4-triazol-3-on

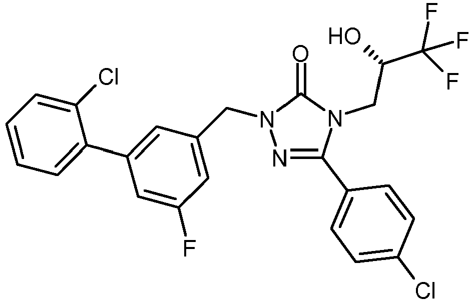

Analog zur Herstellung von Beispiel 122 wurden 59 mg (0.12 mmol) der Verbindung aus Beispiel 103A mit 28 mg (0.18 mmol) 2-Chlorphenylboronsäure umgesetzt. Es wurden 34 mg (54% d. Th.) der Zielverbindung erhalten.
LC/MS [Methode 4]: Rₜ = 1.34 min; MS [ESIpos]: m/z = 526 (M+H)⁺
¹H-NMR (400 MHz, DMSO-d₆): δ = 3.84 (dd, 1H), 4.00 (dd, 1H), 4.26-4.34 (m, 1H), 5.04-5.15 (m, 2H), 6.90 (d, 1H), 7.16-7.21 (m, 1H), 7.22-7.28 (m, 2H), 7.40-7.47 (m, 3H), 7.55-7.65 (m, 3H), 7.75 (d, 2H).

### Beispiel 127

### 2-[(5-Brom-2'-chlorbiphenyl-3-yl)methyl]-5-(4-chlorphenyl)-4-[(2S)-3,3,3-trifluor-2-hydroxypropyl]-2,4-dihydro-3H-1,2,4-triazol-3-on

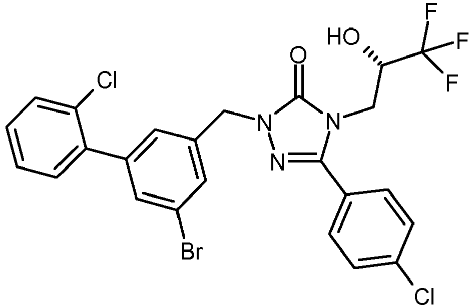

Analog zur Herstellung von Beispiel 91 wurden 109 mg (0.36 mmol) der Verbindung aus Beispiel 5A mit 128 mg (0.36 mmol) der Verbindung aus Beispiel 117A umgesetzt. Es wurden 148 mg (68% d. Th.) der Zielverbindung erhalten.
LC/MS [Methode 4]: Rₜ = 1.40 min; MS [ESIpos]: m/z = 586, 588 und 590 (M+H)⁺
¹H-NMR (400 MHz, DMSO-d₆): δ = 3.83 (dd, 1H), 3.97-4.03 (m, 1H), 4.26-4.34 (m, 1H), 5.03-5.13 (m, 2H), 6.88 (d, 1H), 7.40-7.47 (m, 4H), 7.55-7.60 (m, 3H), 7.61-7.65 (m, 2H), 7.71-7.77 (m, 2H).

### Beispiel 128

### 5-(4-Chlorphenyl)-2-[(2,2"-dichlor-1,1':3',1"-terphenyl-5'-yl)methyl]-4-[(2S)-3,3,3-trifluor-2-hydroxypropyl]-2,4-dihydro-3H-1,2,4-triazol-3-on

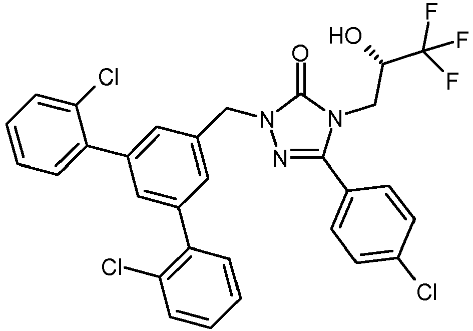

Analog zur Herstellung von Beispiel 91 wurden 105 mg (0.34 mmol) der Verbindung aus Beispiel 5A mit 135 mg (0.35 mmol) der Verbindung aus Beispiel 118A umgesetzt. Es wurden 115 mg (53% d. Th.) der Zielverbindung erhalten.
LC/MS [Methode 4]: Rₜ = 1.47 min; MS [ESIpos]: m/z = 618 und 620 (M+H)⁺
¹H-NMR (400 MHz, DMSO-d₆): δ = 3.83 (dd, 1H), 4.00 (dd, 1H), 4.26-4.32 (m, 1H), 5.08-5.20 (m, 2H), 6.86 (d, 1H), 7.39-7.52 (m, 9H), 7.55-7.65 (m, 4H), 7.74 (d, 2H).

### Beispiel 129

### Methyl-2'-chlor-3-{[3-(4-chlorphenyl)-5-oxo-4-(3,3,3-trifluor-2-hydroxypropyl)-4,5-dihydro-1H-1,2,4-triazol-1-yl]methyl}biphenyl-4-carboxylat

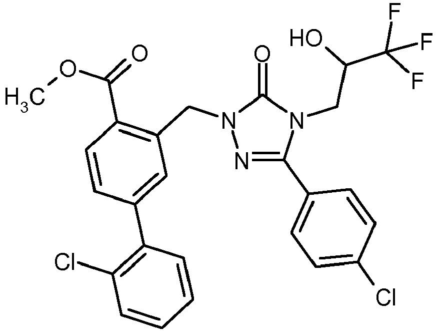

Analog zur Herstellung von Beispiel 122 wurden 265 mg (0.50 mmol) der Verbindung aus Beispiel 104A mit 194 mg (0.74 mmol) 2-Chlorphenylboronsäure umgesetzt. Es wurden 54 mg (19% d. Th.) der Zielverbindung mit einer Reinheit von 98% sowie 167 mg (49% d. Th.) mit einer Reinheit von 83% erhalten.
LC/MS [Methode 3]: Rₜ = 1.49 min; MS [ESIpos]: m/z = 566 und 568 (M+H)⁺
¹H-NMR (400 MHz, DMSO-d₆): δ = 3.84 (dd, 1H), 3.90 (s, 3H), 3.99 (dd, 1H), 4.24-4.32 (m, 1H), 5.36-5.47 (m, 2H), 6.85 (d, 1H), 7.33 (d, 1H), 7.38-7.47 (m, 3H), 7.51-7.59 (m, 2H), 7.62 (d, 2H), 7.72 (d, 2H), 8.00 (d, 1H).

### Beispiel 130

### Methyl-3-{[3-(4-chlorphenyl)-5-oxo-4-(3,3,3-trifluor-2-hydroxypropyl)-4,5-dihydro-1H-1,2,4-triazol-1-yl]methyl}-2'-(trifluormethyl)biphenyl-4-carboxylat

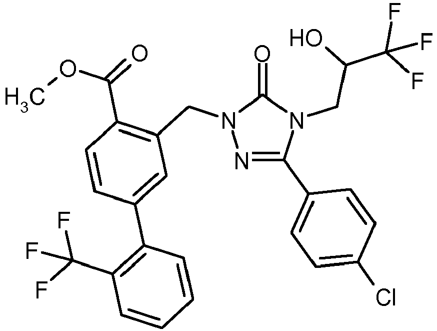

Analog zur Herstellung von Beispiel 122 wurden 265 mg (0.50 mmol) der Verbindung aus Beispiel 104A mit 149 mg (0.74 mmol) 2-(Trifluormethyl)phenylboronsäure umgesetzt. Es wurden 113 mg (38% d. Th.) der Zielverbindung mit einer Reinheit von 100% sowie 101 mg (28% d. Th.) mit einer Reinheit von 81 % erhalten.
LC/MS [Methode 3]: Rₜ = 1.50 min; MS [ESIpos]: m/z = 600 (M+H)⁺
¹H-NMR (400 MHz, DMSO-d₆): δ = 3.83 (dd, 1H), 3.90 (s, 3H), 3.97 (dd, 1H), 4.19-4.30 (m, 1H), 5.34-5.47 (m, 2H), 6.83 (d, 1H), 7.16 (s, 1H), 7.37-7.45 (m, 2H), 7.59-7.77 (m, 6H), 7.83 (d, 1H), 7.98 (d, 1H).

### Beispiel 131

### Methyl-2',3'-dichlor-3-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-4-carboxylat

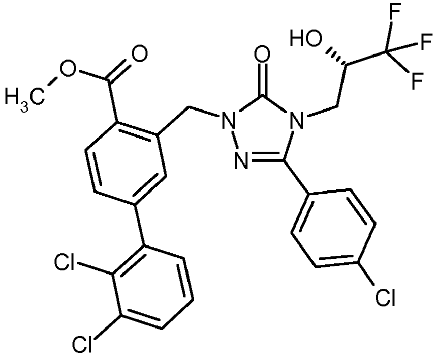

Analog zur Herstellung von Beispiel 75 wurden 455 mg (0.85 mmol) der Verbindung aus Beispiel 105A mit 244 mg (1.28 mmol) 2,3-Dichlorphenylboronsäure umgesetzt. Es wurden 347 mg (57% d. Th.) der Zielverbindung erhalten.
LC/MS [Methode 4]: Rₜ = 1.38 min; MS [ESIpos]: m/z = 600 und 602 (M+H)⁺.

### Beispiel 132

### 2'-Chlor-3-{[3-(4-chlorphenyl)-5-oxo-4-(3,3,3-trifluor-2-hydroxypropyl)-4,5-dihydro-1H-1,2,4-triazol-1-yl]methyl}biphenyl-4-carbonsäure

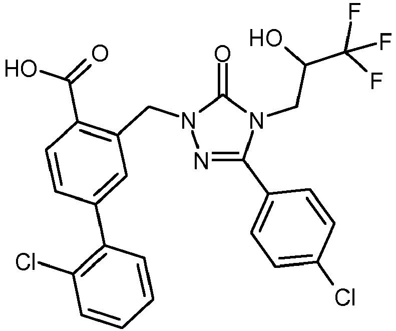

204 mg (0.36 mmol) der Verbindung aus Beispiel 129 wurden in 3 ml THF gelöst und mit 0.4 ml einer 1 N wässrigen Lithiumhydroxid-Lösung versetzt. Das Gemisch wurde 20 h bei RT gerührt. Zur Aufarbeitung entfernte man das Solvens unter vermindertem Druck, nahm den Rückstand in ca. 5 ml Wasser auf und versetzte mit 0.07 ml 6 N Salzsäure. Der ausgefallene Feststoff wurde abgesaugt und getrocknet. Die weitere Reinigung des Rohprodukts erfolgte durch Chromatographie an Kieselgel (Elutionsmittel: zunächst Cyclohexan/Ethylacetat 1:3, dann reines Ethylacetat, zuletzt Dichlormethan/Methanol 1:1). Es wurden 97 mg (47% d. Th.) der Zielverbindung erhalten.
LC/MS [Methode 4]: Rₜ = 1.20 min; MS [ESIpos]: m/z = 552 und 554 (M+H)⁺
¹H-NMR (400 MHz, DMSO-d₆): δ = 3.80 (dd, 1H), 3.99 (dd, 1H), 4.33-4.43 (m, 1H), 5.33-5.44 (m, 1H), 5.49-5.58 (m, 1H), 7.10 (s, 1H), 7.28-7.43 (m, 4H), 7.50-7.55 (m, 1H), 7.60 (d, 2H), 7.71 (d, 2H), 7.74-7.83 (m, 1H).

### Beispiel 133

### 3-{[3-(4-Chlorphenyl)-5-oxo-4-(3,3,3-trifluor-2-hydroxypropyl)-4,5-dihydro-1H-1,2,4-triazol-1-yl]-methyl}-2'-(trifluormethyl)biphenyl-4-carbonsäure

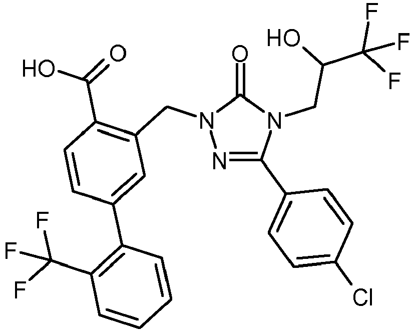

195 mg (0.36 mmol) der Verbindung aus Beispiel 130 wurden in 3 ml THF gelöst und mit 0.4 ml einer 1 N wässrigen Lithiumhydroxid-Lösung versetzt. Das Gemisch wurde 20 h bei RT gerührt. Zur Aufarbeitung entfernte man das Solvens unter vermindertem Druck, nahm den Rückstand in ca. 5 ml Wasser auf und versetzte mit 0.07 ml 6 N Salzsäure. Man ließ 15 min rühren, saugte anschließend den ausgefallenen Feststoff ab und trocknete diesen im Hochvakuum. Es wurden 146 mg (73% d. Th.) der Zielverbindung erhalten.
LC/MS [Methode 3]: Rₜ = 1.38 min; MS [ESIpos]: m/z = 586 (M+H)⁺
¹H-NMR (400 MHz, DMSO-d₆): δ = 3.78 (dd, 1H), 3.95 (dd, 1H), 4.31-4.39 (m, 1H), 5.25-5.34 (m, 1H), 5.40-5.47 (m, 1H), 7.02 (s, 1H), 7.24 (d, 1H), 7.37 (d, 1H), 7.56-7.63 (m, 2H), 7.66-7.83 (m, 6H).

### Beispiel 134

### 2',3'-Dichlor-3-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-4-carbonsäure

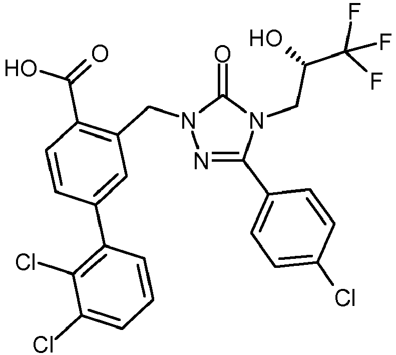

Analog zur Herstellung von Beispiel 132 wurden 347 mg (0.49 mmol) der Verbindung aus Beispiel 131 (Reinheit 84%) umgesetzt. Es wurden 243 mg (85% d. Th.) der Zielverbindung erhalten.
LC/MS [Methode 4]: Rₜ = 1.24 min; MS [ESIpos]: m/z = 586 und 588 (M+H)⁺
¹H-NMR (400 MHz, DMSO-d₆): δ = 3.84 (dd, 1H), 3.99 (dd, 1H), 4.23-4.31 (m, 1H), 5.40-5.51 (m, 2H), 6.88 (br. s, 1H), 7.24 (s, 1H), 7.33-7.37 (m, 1H), 7.45 (t, 1H), 7.50 (d, 1H), 7.62 (d, 2H), 7.69 (dd, 1H), 7.73 (d, 2H), 8.01 (d, 1H).

### Beispiel 135

### 2'-Chlor-3-{[3-(4-chlorphenyl)-5-oxo-4-(3,3,3-trifluor-2-hydroxypropyl)-4,5-dihydro-1H-1,2,4-triazol-1-yl]methyl}biphenyl-4-carboxamid

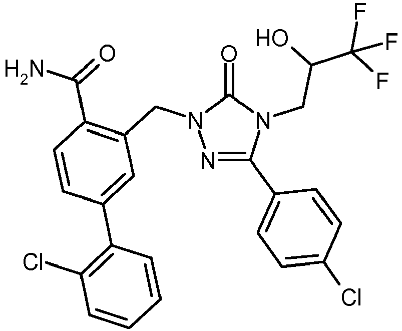

43 mg (0.08 mmol) der Verbindung aus Beispiel 132 wurden in 1 ml DMF vorgelegt und mit 14 mg (0.10 mmol) HOBt und 19 mg (0.10 mmol) EDC versetzt. Nach 10 min Rühren bei RT wurden 0.41 ml (0.20 mmol) Ammoniak-Lösung (35%-ig in Wasser) hinzugegeben und die Mischung 16 h bei RT gerührt. Anschließend wurde die Reaktionslösung im Vakuum vom überschüssigen Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch an Kieselgel gereinigt (Elutionsmittel: Cyclohexan/Ethylacetat 1:3 → 1:5). Es wurden 19 mg (42% d. Th.) der Zielverbindung erhalten.
LC/MS [Methode 4]: Rₜ = 1.14 min; MS [ESIpos]: m/z = 551 und 553 (M+H)⁺
¹H-NMR (400 MHz, DMSO-d₆): δ = 3.83 (dd, 1H), 3.98 (dd, 1H), 4.24-4.33 (m, 1H), 5.22-5.34 (m, 2H), 6.84 (d, 1H), 7.27 (s, 1H), 7.34-7.47 (m, 4H), 7.52-7.66 (m, 3H), 7.73 (d, 2H), 8.04 (s, 1H).

### Beispiel 136

### 2',3'-Dichlor-3-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-4-carboxamid

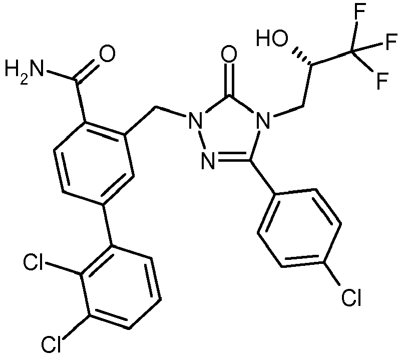

45 mg (0.07 mmol) der Verbindung aus Beispiel 134 wurden in 1 ml DMF vorgelegt und mit 13 mg (0.09 mmol) HOBt und 18 mg (0.09 mmol) EDC versetzt. Nach 10 min Rühren bei RT wurden 80 µl (1.44 mmol) Ammoniak-Lösung (35%-ig in Wasser) hinzugegeben und die Mischung 16 h bei RT gerührt. Anschließend wurde die Reaktionslösung im Vakuum vom überschüssigen Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 20 mg (46% d. Th.) der Zielverbindung erhalten.
LC/MS [Methode 3]: Rₜ = 1.36 min; MS [ESIpos]: m/z = 585 und 587 (M+H)⁺
¹H-NMR (400 MHz, DMSO-d₆): δ = 3.83 (dd, 1H), 3.98 (dd, 1H), 4.23-4.32 (m, 1H), 5.22-5.33 (m, 2H), 6.84 (d, 1H), 7.24 (s, 1H), 7.30-7.35 (m, 1H), 7.41-7.47 (m, 2H), 7.56-7.65 (m, 4H), 7.65-7.70 (m, 1H), 7.73 (d, 2H), 8.06 (s, 1H).

### Beispiel 137

### 5-(4-Chlorphenyl)-2-{[2',3'-dichlor-4-(hydroxymethyl)biphenyl-3-yl]methyl}-4-[(2S)-3,3,3-trifluor-2-hydroxypropyl]-2,4-dihydro-3H-1,2,4-triazol-3-on

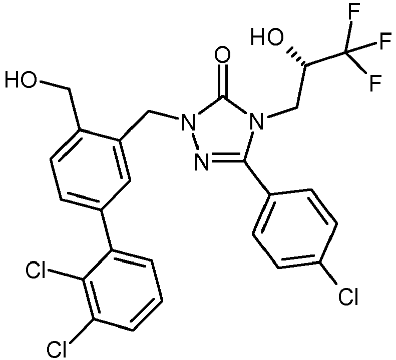

230 mg (0.39 mmol) der Verbindung aus Beispiel 134 wurden in 5 ml THF gelöst, auf 0°C abgekühlt und mit 55 µl (0.39 mmol) Triethylamin sowie 56 µl (0.43 mmol) Chlorameisensäureisobutylester versetzt. Es wurde 1 h bei 0°C gerührt. Die Suspension wurde dann über eine Seitz-Fritte in einen auf 0°C gekühlten Kolben filtriert und der Rückstand mit ca. 2 ml THF nachgewaschen. Diese Lösung wurde unter kräftigem Rühren zu einer auf 0°C gekühlten Lösung von 44 mg (1.18 mmol) Natriumborhydrid in 0.6 ml Wasser gegeben. Nach 1 h wurde mit 5 ml gesättigter wässriger Natriumhydrogencarbonat-Lösung versetzt und auf RT erwärmt. Es wurde mit 15 ml Ethylacetat extrahiert. Die organische Phase wurde nacheinander mit je 5 ml gesättigter Natriumhydrogencarbonat-Lösung und gesättigter Natriumchlorid-Lösung gewaschen. Nach Trocknen über Natriumsulfat wurde filtriert und im Vakuum eingeengt. Das Rohprodukt wurde chromatographisch gereinigt [Methode 19]. Es wurden 13 mg (6% d. Th.) der Zielverbindung erhalten.
LC/MS [Methode 3]: Rₜ = 1.44 min; MS [ESIneg]: m/z = 572 (M-H)⁻
¹H-NMR (400 MHz, DMSO-d₆): δ = 3.82 (dd, 1H), 3.98 (dd, 1H), 4.23-4.30 (m, 1H), 4.74 (d, 2H), 5.05-5.15 (m, 2H), 5.30 (t, 1H), 6.86 (d, 1H), 7.27 (d, 1H), 7.33 (dd, 1H), 7.38 (dd, 1H), 7.43 (t, 1H), 7.54 (d, 1H), 7.61 (d, 2H), 7.66 (dd, 1H), 7.70-7.74 (m, 2H).

### Beispiel 138

### [2',3'-Dichlor-3-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-4-yl]methylcarbamat

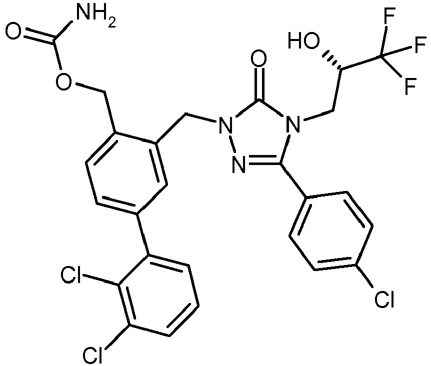

65 mg (0.11 mmol) der Verbindung aus Beispiel 137 wurden in 3 ml Dichlormethan gelöst und auf 0°C abgekühlt. Es wurden 14 µl (0.16 mmol) Chlorsulfonylisocyanat zugefügt und die Mischung 18 h bei RT gerührt. Danach wurden 1.5 ml Wasser hinzugegeben und weitere 18 h bei 60°C gerührt. Zur Aufarbeitung versetzte man mit 3 ml gesättigter wässriger Natriumhydrogencarbonat-Lösung und extrahierte zweimal mit jeweils 10 ml Ethylacetat. Die vereinigten organischen Phasen wurden über Natriumsulfat getrocknet, filtriert und im Vakuum eingeengt. Das Rohprodukt wurde chromatographisch gereinigt [Methode 19]. Es wurden 30 mg (43% d. Th.) der Zielverbindung erhalten.
LC/MS [Methode 4]: Rₜ = 1.23 min; MS [ESIneg]: m/z = 615 und 617 (M-H)⁻
¹H-NMR (400 MHz, DMSO-d₆): δ = 3.82 (dd, 1H), 3.98 (dd, 1H), 4.26-4.33 (m, 1H), 5.08-5.18 (m, 2H), 5.23 (s, 2H), 6.66 (br. s, 2H), 6.86 (d, 1H), 7.32 (d, 1H), 7.34 (dd, 1H), 7.39-7.51 (m, 3H), 7.59-7.64 (m, 2H), 7.67 (dd, 1H), 7.73 (d, 2H).

### Beispiel 139

### Methyl-5-({3-(4-chlorphenyl)-5-oxo-4-[(2S)-3,3,3-trifluor-2-hydroxypropyl]-4,5-dihydro-1H-1,2,4-triazol-1-yl}methyl)-2'-(trifluormethyl)biphenyl-2-carboxylat

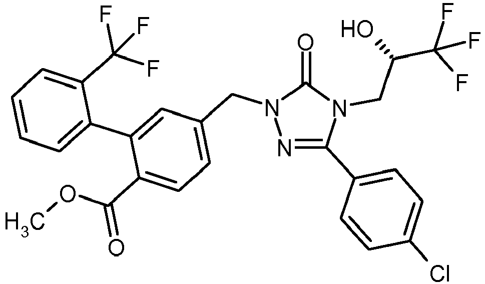

179 mg (0.58 mmol) der Verbindung aus Beispiel 5A und 285 mg (0.88 mmol) Cäsiumcarbonat wurden in 5 ml Acetonitril suspendiert und mit 340 mg (0.58 mmol) der Verbindung aus Beispiel 108A versetzt. Das Gemisch wurde 4 h unter Rückfluss gerührt. Der ausgefallene Feststoff wurde danach abfiltriert und das Filtrat im Vakuum auf ein Volumen von ca. 1.5 ml eingeengt. Nach Zugabe von 0.5 ml 1 N Salzsäure wurde das Gemisch direkt chromatographisch gereinigt [Methode 19]. Es wurden 231 mg (65% d. Th.) der Zielverbindung erhalten.
LC/MS [Methode 4]: Rₜ = 1.26 min; MS [ESIpos]: m/z = 600 (M+H)⁺
¹H-NMR (400 MHz, DMSO-d₆): δ = 3.52 (s, 3H), 3.84 (dd, 1H), 3.99 (dd, 1H), 4.25-4.32 (m, 1H), 5.03-5.18 (m, 2H), 6.88 (dd, 1H), 7.21-7.29 (m, 2H), 7.46 (d, 1H), 7.55-7.69 (m, 4H), 7.70-7.75 (m, 2H), 7.77 (d, 1H), 7.97 (d, 1H).

### Beispiel 140

### Methyl-2'-chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-2-carboxylat

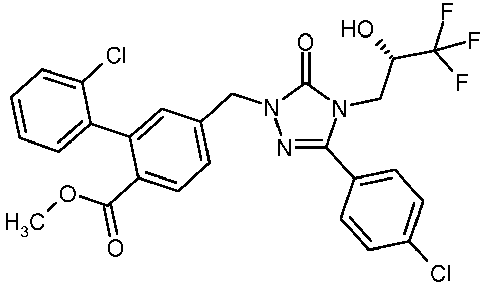

Analog zur Herstellung von Beispiel 139 wurden 208 mg (0.68 mmol) der Verbindung aus Beispiel 5A mit 230 mg (0.68 mmol) der Verbindung aus Beispiel 109A umgesetzt. Es wurden 231 mg (59% d. Th.) der Zielverbindung erhalten.
LC/MS [Methode 4]: Rₜ = 1.26 min; MS [ESIpos]: m/z = 566 und 568 (M+H)⁺
¹H-NMR (400 MHz, DMSO-d₆): δ = 3.58 (s, 3H), 3.84 (dd, 1H), 4.02 (dd, 1H), 4.26-4.33 (m, 1H), 5.07-5.18 (m, 2H), 6.88 (d, 1H), 7.25-7.31 (m, 2H), 7.36-7.52 (m, 4H), 7.62 (d, 2H), 7.74 (d, 2H), 7.93 (d, 1H).

### Beispiel 141

### 5-({3-(4-Chlorphenyl)-5-oxo-4-[(2S)-3,3,3-trifluor-2-hydroxypropyl]-4,5-dihydro-1H-1,2,4-triazol-1-yl}methyl)-2'-(trifluormethyl)biphenyl-2-carbonsäure

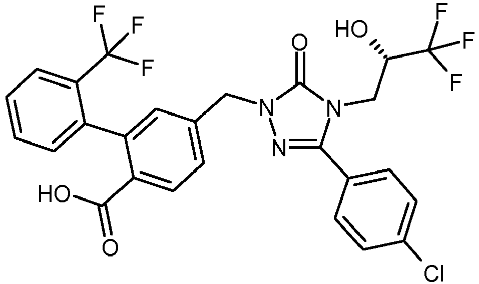

215 mg (0.36 mmol) der Verbindung aus Beispiel 139 wurden in 3 ml THF und 3 ml Methanol gelöst und mit 0.36 ml 2 N Natronlauge versetzt. Das Gemisch wurde 16 h bei 80°C gerührt. Zur Aufarbeitung wurde mit 10 ml Wasser verdünnt und zweimal mit je 10 ml Ethylacetat extrahiert. Die wässrige Phase wurde mit 1 N Salzsäure angesäuert und erneut mit 10 ml Ethylacetat extrahiert. Die organischen Phasen wurden vereinigt, über Magnesiumsulfat getrocknet, filtriert und im Vakuum eingeengt. Der Rückstand wurde im Hochvakuum getrocknet. Es wurden 222 mg (quant.) der Zielverbindung erhalten.
LC/MS [Methode 3]: Rₜ = 1.29 min; MS [ESIpos]: m/z = 586 (M+H)⁺
¹H-NMR (400 MHz, DMSO-d₆): δ = 3.84 (dd, 1H), 3.98 (dd, 1H), 4.24-4.31 (m, 1H), 5.02-5.16 (m, 2H), 6.88 (dd, 1H), 7.19 (br. s, 1H), 7.26 (d, 1H), 7.43 (d, 1H), 7.56 (t, 1H), 7.59-7.67 (m, 3H), 7.69-7.77 (m, 3H), 7.96 (d, 1H), 12.59 (br. s, 1H).

### Beispiel 142

### 2'-Chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-2-carbonsäure

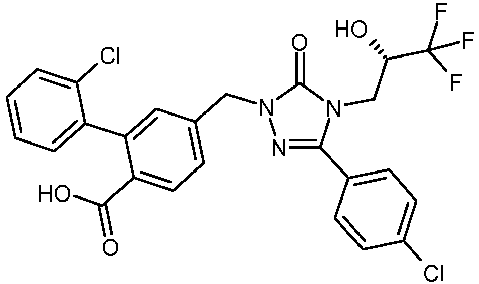

Analog zur Herstellung von Beispiel 141 wurden 218 mg (0.39 mmol) der Verbindung aus Beispiel 140 mit 2 N Natronlauge umgesetzt. Es wurden 220 mg (quant.) der Zielverbindung erhalten.
LC/MS [Methode 3]: Rₜ = 1.26 min; MS [ESIpos]: m/z = 552 und 554 (M+H)⁺
¹H-NMR (400 MHz, DMSO-d₆): δ = 3.84 (dd, 1H), 4.00 (dd, 1H), 4.24-4.35 (m, 1H), 5.05-5.16 (m, 2H), 6.89 (d, 1H), 7.24 (s, 1H), 7.25-7.30 (m, 1H), 7.34-7.39 (m, 2H), 7.42 (dd, 1H), 7.45-7.50 (m, 1H), 7.59-7.65 (m, 2H), 7.72-7.77 (m, 2H), 7.92 (d, 1H), 12.64 (br. s, 1H).

### Beispiel 143

### 5-({3-(4-Chlorphenyl)-5-oxo-4-[(2S)-3,3,3-trifluor-2-hydroxypropyl]-4,5-dihydro-1H-1,2,4-triazol-1-yl}methyl)-2'-(trifluormethyl)biphenyl-2-carboxamid

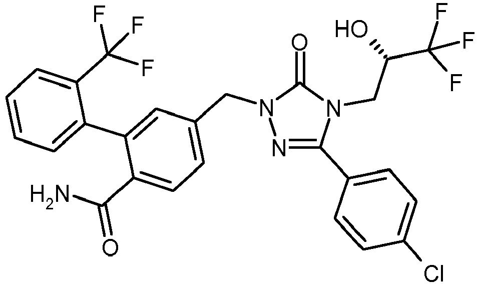

208 mg (0.36 mmol) der Verbindung aus Beispiel 141 wurden in 5 ml DMF vorgelegt und mit 62 mg (0.46 mmol) HOBt und 88 mg (0.46 mmol) EDC versetzt. Nach 10 min Rühren bei RT wurden 1.0 ml (16 mmol) Ammoniak-Lösung (33%-ig in Wasser) hinzugegeben und die Mischung 16 h bei RT gerührt. Anschließend wurde die Reaktionslösung im Vakuum vom überschüssigen Ammoniak befreit, mit ca. 3 ml Wasser versetzt und dreimal mit je 5 ml Ethylacetat extrahiert. Die vereinigten organischen Phasen wurden über Magnesiumsulfat getrocknet, filtriert und im Vakuum eingeengt. Das Rohprodukt wurde chromatographisch gereinigt [Methode 19]. Es wurden 85 mg (39% d. Th.) der Zielverbindung erhalten.
LC/MS [Methode 2]: Rₜ = 2.29 min; MS [ESIpos]: m/z = 585 (M+H)⁺
¹H-NMR (400 MHz, DMSO-d₆): δ = 3.83 (dd, 1H), 3.99 (dd, 1H), 4.24-4.31 (m, 1H), 4.98-5.12 (m, 2H), 6.87 (d, 1H), 7.08-7.18 (m, 2H), 7.29 (d, 1H), 7.37 (d, 1H), 7.50-7.65 (m, 6H), 7.67-7.76 (m, 3H).

### Beispiel 144

### 2'-Chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-2-carboxamid

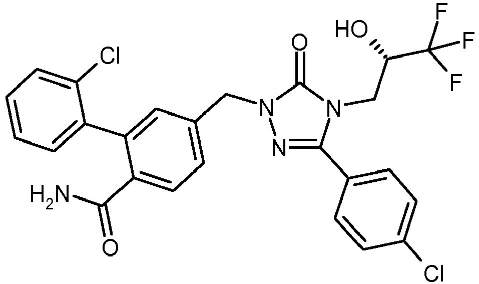

Analog zur Herstellung von Beispiel 143 wurden 210 mg (0.38 mmol) der Verbindung aus Beispiel 142 mit Ammoniak-Lösung umgesetzt. Es wurden 122 mg (54% d. Th.) der Zielverbindung erhalten.
LC/MS [Methode 4]: Rₜ = 1.06 min; MS [ESIpos]: m/z = 551 und 553 (M+H)⁺
¹H-NMR (400 MHz, DMSO-d₆): δ = 3.84 (dd, 1H), 4.00 (dd, 1H), 4.24-4.35 (m, 1H), 5.01-5.12 (m, 2H), 6.88 (d, 1H), 7.17 (s, 1H), 7.23 (d, 1H), 7.26-7.31 (m, 1H), 7.31-7.39 (m, 3H), 7.44-7.49 (m, 1H), 7.52-7.59 (m, 2H), 7.62 (d, 2H), 7.74 (d, 2H).

### Beispiel 145

### N-tert.-Butyl-5-({3-(4-chlorphenyl)-5-oxo-4-[(2S)-3,3,3-trifluor-2-hydroxypropyl]-4,5-dihydro-1H-1,2,4-triazol-1-yl}methyl)-2'-(trifluormethyl)biphenyl-2-carboxamid

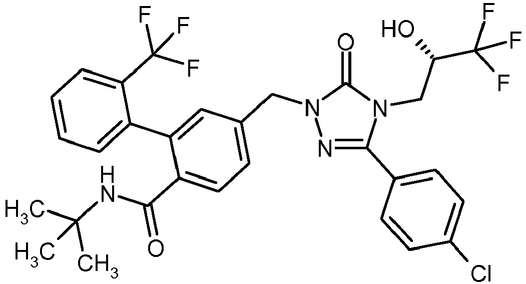

30 mg (0.05 mmol) der Verbindung aus Beispiel 141 wurden in 0.75 ml DMF vorgelegt und mit 9 mg (0.074 mmol) HOBt und 13 mg (0.07 mmol) EDC versetzt. Nach 10 min Rühren bei RT wurden 6 µl (0.06 mmol) 2-Methylpropan-2-amin hinzugegeben und die Mischung 16 h bei RT gerührt. Anschließend wurde mit 50 µl 1 N Salzsäure versetzt und das Gemisch direkt chromatographisch gereinigt [Methode 19]. Es wurden 8.2 mg (24% d. Th.) der Zielverbindung erhalten.
LC/MS [Methode 2]: Rₜ = 2.70 min; MS [ESIpos]: m/z = 641 (M+H)⁺
¹H-NMR (400 MHz, DMSO-d₆): δ = 1.08 (s, 9H), 3.84 (dd, 1H), 3.99 (dd, 1H), 4.24-4.35 (m, 1H), 4.99-5.11 (m, 2H), 6.88 (d, 1H), 7.17 (d, 1H), 7.25 (s, 1H), 7.38 (t, 2H), 7.48 (d, 1H), 7.54-7.67 (m, 4H), 7.71 (d, 2H), 7.78 (d, 1H).

### Beispiel 146

### Methyl-2'-chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-3-carboxylat

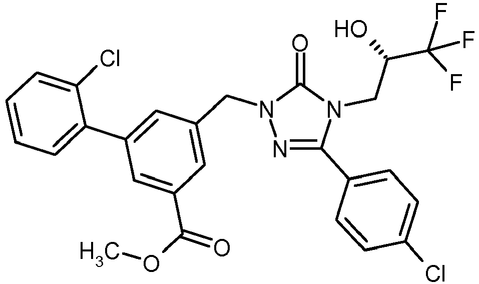

Analog zur Herstellung von Beispiel 139 wurden 248 mg (0.81 mmol) der Verbindung aus Beispiel 5A mit 274 mg (0.81 mmol) der Verbindung aus Beispiel 112A umgesetzt. Es wurden 271 mg (59% d. Th.) der Zielverbindung erhalten.
LC/MS [Methode 3]: Rₜ = 1.48 min; MS [ESIpos]: m/z = 566 und 568 (M+H)⁺
¹H-NMR (400 MHz, DMSO-d₆): δ = 3.84 (dd, 1H), 3.87 (s, 3H), 4.01 (dd, 1H), 4.23-4.33 (m, 1H), 5.10-5.21 (m, 2H), 6.87 (s, 1H), 7.42-7.48 (m, 3H), 7.58-7.65 (m, 3H), 7.68-7.76 (m, 3H), 7.93 (s, 1H), 7.99 (s, 1H).

### Beispiel 147

### 2'-Chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-3-carbonsäure

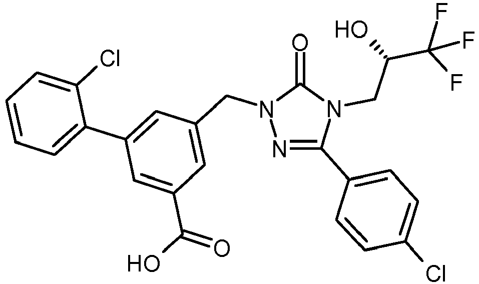

Analog zur Herstellung von Beispiel 141 wurden 244 mg (0.43 mmol) der Verbindung aus Beispiel 146 mit 2 N Natronlauge umgesetzt. Es wurden 242 mg (100% d. Th.) der Zielverbindung erhalten.
LC/MS [Methode 3]: Rₜ = 1.33 min; MS [ESIpos]: m/z = 552 und 554 (M+H)⁺
¹H-NMR (400 MHz, DMSO-d₆): δ = 3.83 (dd, 1H), 3.98-4.04 (m, 1H), 4.29-4.35 (m, 1H), 5.04-5.14 (m, 2H), 7.36-7.48 (m, 5H), 7.57 (d, 1H), 7.61 (d, 2H), 7.74 (d, 2H), 7.87 (br. s, 1H).

### Beispiel 148

### 2'-Chlor-5-({3-(4-chlorphenyl)-5-oxo-4-[(2S)-3,3,3-trifluor-2-hydroxypropyl]-4,5-dihydro-1H-1,2,4-triazol-1-yl}methyl)biphenyl-3-carboxamid

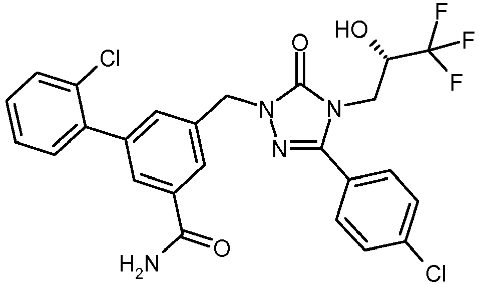

Analog zur Herstellung von Beispiel 143 wurden 55 mg (0.10 mmol) der Verbindung aus Beispiel 147 mit Ammoniak-Lösung umgesetzt. Es wurden 25 mg (43% d. Th.) der Zielverbindung erhalten.
LC/MS [Methode 4]: Rₜ = 1.12 min; MS [ESIpos]: m/z = 551 und 553 (M+H)⁺
¹H-NMR (400 MHz, DMSO-d₆): δ = 3.83 (dd, 1H), 3.96-4.03 (m, 1H), 4.26-4.33 (m, 1H), 5.05-5.16 (m, 2H), 6.52 (s, 1H), 6.89 (d, 1H), 7.41-7.49 (m, 4H), 7.55-7.65 (m, 3H), 7.74 (d, 2H), 7.88 (d, 2H), 8.06 (s, 1H).

### B. Bewertung der pharmakologischen Wirksamkeit

Die pharmakologische Wirkung der erfindungsgemäßen Verbindungen kann in folgenden Assays gezeigt werden:

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydroxymethyl)-1,3-propandiol-Hydrochlorid

### B-1. Zellulärer in vitro-Test zur Bestimmung der Vasopressin-Rezeptor-Aktivität

Die Identifizierung von Agonisten und Antagonisten der V1a- und V2-Vasopressin-Rezeptoren von Mensch und Ratte sowie die Quantifizierung der Wirksamkeit der erfindungsgemäßen Verbindungen erfolgt mit Hilfe von rekombinanten Zelllinien. Diese Zellen leiten sich ursprünglich von einer Ovarepithelzelle des Hamsters (Chinese Hamster Ovary, CHO K1, ATCC: American Type Culture Collection, Manassas, VA 20108, USA) ab. Die Testzelllinien exprimieren konstitutiv eine modifizierte Form des Calcium-sensitiven Photoproteins Aequorin, das nach Rekonstitution mit dem Co-Faktor Coelenterazin bei Erhöhung der freien Calcium-Konzentrationen Licht emittiert [Rizzuto R, Simpson AW, Brini M, Pozzan T, Nature 358, 325-327 (1992)]. Zusätzlich sind die Zellen stabil transfiziert mit den V1a- oder V2-Rezeptoren des Menschen oder der Ratte. Im Falle der Gskoppelnden V2-Rezeptoren sind die Zellen mit einem weiteren Gen, das für das promiskuitive G_{α16}-Protein kodiert [Amatruda TT, Steele DA, Slepak VZ, Simon MI, Proceedings in the National Academy of Science USA 88, 5587-5591 (1991)], entweder unabhängig oder als Fusionsgen stabil transfiziert. Die resultierenden Vasopressin-Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszens mit einem geeignetem Luminometer quantifiziert werden kann [Milligan G, Marshall F, Rees S, Trends in Pharmacological Sciences 17, 235-237 (1996)].

### Testablauf:

Die Zellen werden am Tag vor dem Test in Kulturmedium (DMEM, 10% FCS, 2 mM Glutamin, 10 mM HEPES) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (140 mM NaCl, 5 mM KCl, 1 mM MgCl₂, 2 mM CaCl₂, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Vertiefungen der Mikrotiterplatte vorgelegt, ehe der Agonist [Arg⁸]-Vasopressin hinzugegeben und das resultierende Lichtsignal sofort im Luminometer gemessen wird. Die Berechnung der IC₅₀-Werte erfolgt mit Hilfe des Computerprogramms GraphPad PRISM (Version 3.02).

In der folgenden Tabelle sind repräsentative IC₅₀-Werte für die erfindungsgemäßen Verbindungen an der mit dem humanen V1a- bzw. V2-Rezeptor transfizierten Zelllinie aufgeführt:

**Tabelle**

| **Beispiel Nr.** | **IC₅₀ hV1a [µM]** | **IC₅₀ hV2 [µM]** |
|---|---|---|
| 9 | 0.060 | 0.023 |
| 17 | 0.12 | 0.032 |
| 20 | 0.046 | 0.15 |
| 26 | 0.023 | 0.028 |
| 32 | 0.0086 | 0.0020 |
| 42 | 0.0040 | 0.011 |
| 49 | 0.019 | 0.0015 |
| 50 | 0.034 | 0.0014 |
| 54 | 0.0068 | 0.0085 |
| 60 | 0.050 | 0.012 |
| 68 | 0.012 | 0.0083 |
| 71 | 0.0042 | 0.013 |
| 74 | 0.26 | 0.040 |
| 76 | 0.017 | 0.023 |
| 78 | 0.029 | 0.023 |
| 83 | 0.23 | 0.067 |
| 84 | 9.2 | 1.1 |
| 85 | 9.3 | 1.7 |
| 87 | 0.26 | 0.012 |
| 90 | 0.088 | 0.40 |
| 93 | 0.089 | 0.10 |
| 96 | 0.12 | 0.0084 |
| 101 | 0.027 | 0.0044 |
| 110 | 0.0065 | 0.0033 |
| 111 | 0.031 | 0.0077 |
| 112 | 0.82 | 0.11 |
| 117 | 0.039 | 0.045 |
| 122 | 0.57 | 0.027 |
| 132 | 1.7 | 0.0046 |
| 135 | 0.49 | 0.0058 |

### B-2. Zellulärer in vitro-Test zum Nachweis der Wirkung von Vasopressin-V1a-Rezeptor-antagonisten auf die Regulation pro-fibrotischer Gene

Die als Kardiomyozyten-Typ beschriebene Zelllinie H9C2 (American Type Culture Collection ATCC-Nr. CRL-1446), die aus Herzgewebe der Ratte isoliert wurde, exprimiert endogen den Vasopressin-V1a-Rezeptor AVPR1A in hoher Kopienzahl, während die AVPR2-Expression nicht nachweisbar ist. Für Zell-Tests zur Hemmung der AVPR1A-abhängigen Regulation der Genexpression durch Rezeptor-Antagonisten wird wie folgt verfahren:
H9C2-Zellen werden in 1.0 ml Medium Opti-MEM (Invitrogen Corp., Carlsbad CA, USA, Kat.-Nr. 11058-021) mit 2% FCS und 1% Penicillin/Streptomycin-Lösung (Invitrogen, Kat.-Nr. 10378-016) mit einer Zelldichte von 100 000 Zellen/well in 12-well-Mikrotiterplatten für die Zellkultur ausgesät und in einem Zellinkubator (96% Luftfeuchtigkeit, 5% v/v CO₂, 37°C) gehalten. Nach 24 Stunden werden in je drei wells (Triplikate) Vehikel-Lösung (als Negativkontrolle), Vasopressin-Lösung ([Arg⁸]-Vasopressin-Acetat, Fa. Sigma, Kat.-Nr. V9879) oder Testsubstanz (gelöst in Vehikel Wasser mit 20 Vol.-% Ethanol) und Vasopressin-Lösung gegeben. Die finale Vasopressin-Konzentration in der Zellkultur ist 0.05 µM. Die Testsubstanz-Lösung wird in kleinen Volumina zur Zellkultur gegeben, so dass eine finale Konzentration von 0.1% Ethanol im Zelltest nicht überschritten wird. Nach einer Inkubationszeit von 6 Stunden wird der Kulturüberstand abgesaugt, die adhärenten Zellen werden in 250 µl RLT-Puffer (Fa. Qiagen, Ratingen, Kat.-Nr. 79216) lysiert, und die RNA wird aus diesem Lysat mit dem RNeasy-Kit (Qiagen, Kat.-Nr. 74104) isoliert. Anschließend erfolgt der DNAse-Verdau (Invitrogen, Kat.-Nr. 18068-015), die cDNA-Synthese (ImProm-II Reverse Transcription System, Fa. Promaga, Kat.-Nr. A3800) und die RTPCR (pPCR MasterMix RT-QP2X-03-075, Fa. Eurogentec, Seraing, Belgien). Alle Prozeduren erfolgen gemäß den Arbeitsprotokollen der Test-Reagenzien-Hersteller. Die Primer-Sets für die RTPCR werden anhand der mRNA-Gensequenzen (NCBI Genbank Entrez Nucleotide Data Base) mit Hilfe des Programms Primer3Plus mit 6-FAM TAMRA-markierten Sonden ausgewählt. Die RTPCR zur Bestimmung der relativen mRNA-Expression in den Zellen der verschiedenen Test-Ansätze erfolgt mit dem Applied Biosystems ABI Prism 7700 Sequence Detector im 96-well- oder 3 84-well-Mikrotiterplatten-Format gemäß der Betriebsanleitung des Gerätes. Die relative Genexpression wird durch den delta-delta Ct-Wert dargestellt [Fa. Applied Biosystems, User Bulletin No. 2 ABI Prism 7700 SDS December 11, 1997 (update 10/2001)] mit dem Bezug auf die Expressionsstärke des Gens Ribosomales Protein L-32 (Genbank Acc. No. NM_013226) und den Ct-Schwellenwert von 35.

### B-3. In vivo-Test zum Nachweis der kardiovaskulären Wirkung: Blutdruckmessung an narkotisierten Ratten ("Vasopressin-Challenge-Modell")

Bei männlichen Sprague-Dawley-Ratten (250-350 g Körpergewicht) werden in Ketamin/Xylazin/ Pentobarbital-Injektionsnarkose Polyethylenschläuche (PE-50; Intramedic^{®}), die mit Heparinhaltiger (500 I.E./ml) isotoner Natriumchlorid-Lösung vorgefüllt sind, in die *Vena jugularis* und die *Vena femoralis* eingeführt und anschließend eingebunden. Über einen venösen Zugang wird mit Hilfe einer Spritze Arg-Vasopressin injiziert, über den zweiten venösen Zugang wird die Testsubstanz appliziert. Zur Ermittlung des systolischen Blutdrucks wird ein Druckkatheter (Millar SPR-320 2F) in die *A. carotis* eingebunden. Der arterielle Katheter wird an einen Druckwandler angeschlossen, der seine Signale in einen mit einer geeigneten Registrierungssoftware ausgestatteten Messcomputer einspeist. In einem typischen Experiment werden dem Versuchstier 3-4 aufeinanderfolgende Bolusinjektionen im Abstand von 10-15 min mit einer definierten Menge Arg-Vasopressin (30 ng/kg) in isotoner Natriumchlorid-Lösung verabreicht und, nachdem der Blutdruck wieder Ausgangswerte erreicht hat, die zu testende Substanz als Bolus mit anschließender Dauerinfusion in einem geeigneten Lösungsmittel appliziert. Hiernach wird in definierten Abständen (10-15 min) erneut die gleiche Menge Arg-Vasopressin wie zu Anfang verabreicht. Anhand der Blutdruckwerte wird ermittelt, inwieweit die Testsubstanz der blutdrucksteigernden Wirkung des Arg-Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arg-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar-Ratten (300-450 g Körpergwicht) werden mit freiem Zugang zu Futter (Altromin) und Trinkwasser gehalten. Während des Versuchs werden die Tiere für 4-8 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 1-3 ml/kg Körpergewicht eines geeigneten Lösungsmittels mit Hilfe einer Schlundsonde in den Magen verabreicht. Als Kontrolle dienende Tiere erhalten nur Lösungsmittel. Kontrollen und Substanztestungen werden am selben Tag parallel durchgeführt. Kontrollgruppen und Substanzdosisgruppen bestehen aus jeweils 4-8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro kg Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich zu Lösungsmittel-Kontrollapplikationen eine gesteigerte Ausscheidung von Urin, die im wesentlichen auf einer gesteigerten Wasserausscheidung (Aquarese) beruht.

### B-5. In vivo-Test zum Nachweis der kardiovaskulären Wirkung: Hämodynamische Untersuchungen an narkotisierten Hunden

Männliche oder weibliche Mischlingshunde (Mongrels, Marshall BioResources, USA) mit einem Gewicht zwischen 20 und 30 kg werden für die operativen Eingriffe und die hämodynamischen und funktionellen Untersuchungstermine jeweils mit Pentobarbital (30 mg/kg i.v., Narcoren^{®}, Fa. Merial, Deutschland) an-narkotisiert. Alcuroniumchlorid (3 mg/Tier i.v., Alloferin^{®}, Fa. ICN Pharmaceuticals, Deutschland) dient dabei zusätzlich als Muskelrelaxans. Die Hunde werden intubiert und mit einem Sauerstoff-Raumluft-Gemisch beatmet (40/60%, etwa 5-6 L/min). Die Beatmung erfolgt mit einem Beatmungsgerät (Fa. Draeger, Sulla 808) und wird überwacht mit einem Kohlendioxid-Analysator (Fa. Engström). Die Narkose wird aufrecht erhalten durch eine ständige Infusion von Pentobarbital (50 µg/kg/min); als Analgetikum dient Fentanyl (10 µg/kg/h). Eine Alternative zu Pentobarbital besteht in der Verwendung von Isofluran (1-2 Vol.-%).

Die Hunde werden in vorbereitenden Eingriffen mit einem Herzschrittmacher ausgestattet. Zum Zeitpunkt 21 Tage vor der ersten Substanz-Testung (= Versuchsbeginn) wird ein Herzschrittmacher (Fa. Biotronik, Logos^{®}) in eine subcutane Hauttasche implantiert und über eine Schrittmacher-Elektrode, die durch die *Vena jugularis externa* unter Durchleuchtung bis in den rechten Ventrikel vorgeschoben wird, mit dem Herzen in Kontakt gebracht.

Zum gleichen Zeitpunkt der Schrittmacher-Implantation erfolgt durch retrogrades Vorschieben einer 7F-Biopsie-Zange (Fa. Cordis) über eine Schleuse (Avanti+^{®}, Fa. Cordis) in der *Arteria femoralis* und nach atraumatischer Passage der Aortenklappe eine definierte Läsion der Mitralklappe unter echokardiographischer und Durchleuchtungskontrolle. Im Anschluss werden alle Zugänge entfernt, und der Hund wacht spontan aus der Narkose auf. Nach 7 weiteren Tagen (= 14 Tage vor der ersten Substanz-Testung) wird der oben beschriebene Schrittmacher aktiviert und das Herz mit einer Frequenz von 220 Schlägen pro Minute stimuliert.

Die eigentlichen Experimente zur Substanz-Testung erfolgen 14 und 28 Tage nach Beginn der Schrittmacher-Stimulation nach folgender Instrumentierung:
- Einführung eines Blasenkatheters zur Blasenentlastung bzw. zur Messung des Urinflusses;
- Anbringung von EKG-Ableitungen an den Extremitäten zur EKG-Messung;
- Einführung eines mit Natriumchlorid-Lösung gefüllten Fluidmedic^{®} PE 300-Schlauches in die *A. femoralis,* welcher mit einem Drucksensor (Fa. Braun Melsungen, Deutschland) verbunden ist zur Messung des systemischen Blutdrucks;
- Einführung eines Millar Tip-Katheters (Typ 350 PC, Fa. Millar Instruments, Houston, USA) über den linken Vorhof oder über eine in der *A. carotis* eingebundene Schleuse zur Messung der Herz-Hämodynamik;
- Einführung eines Swan-Ganz-Katheters (CCOmbo 7.5F, Fa. Edwards, Irvine, USA) über *die V. jugularis* in die *A. pulmonalis* zur Messung von Herzzeitvolumen, Sauerstoffsättigung, Pulmonalartiendrücken und zentralvenösem Druck;
- Plazierung einer Braunüle in den *Venae cephalicae* zur Infusion von Pentobarbital, der Flüssigkeitssubstitution und zur Blutentnahme (für die Bestimmung der Substanz-Plasmaspiegel oder sonstiger klinischer Blutwerte);
- Plazierung einer Braunüle in der *Venae saphenae* zur Infusion von Fentanyl und zur Substanzapplikation;
- Infusion von Vasopressin (Fa. Sigma) in aufsteigender Dosierung bis zu einer Dosis von 4 mU/kg/min; unter dieser Dosierung erfolgt dann die Testung pharmakologischer Substanzen.

Die Primärsignale werden eventuell verstärkt (Gould-Verstärker, Fa. Gould Instrument Systems, Valley View, USA bzw. Edwards-Vigilance-Monitor, Fa. Edwards, Irvine, USA) und anschließend zur Auswertung in das Ponemah-System (DataSciences Inc., Minneapolis, USA) eingespeist. Die Signale werden kontinuierlich über den gesamten Versuchszeitraum aufgezeichnet, von dieser Software digital weiterarbeitet und über 30 Sekunden gemittelt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 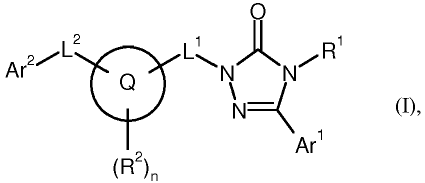 in welcher
R¹ für (C₁-C₆)-Alkyl steht, welches ein- bis dreifach, gleich oder verschieden, mit Resten ausgewählt aus der Reihe Fluor, Trifluormethyl, Oxo, Hydroxy, Methoxy, Ethoxy, (C₃-C₆)-Cycloalkyl und Phenyl substituiert sein kann,
wobei (C₃-C₆)-Cycloalkyl bis zu zweifach, gleich oder verschieden, mit Resten ausgewählt aus der Reihe Fluor, Methyl, Trifluormethyl, Ethyl und Hydroxy substituiert sein kann
und
wobei Phenyl bis zu zweifach, gleich oder verschieden, mit Resten ausgewählt aus der Reihe Fluor, Chlor, Cyano, Methyl, Difluormethyl, Trifluormethyl, Ethyl, Hydroxy, Methoxy, Trifluormethoxy, Ethoxy, Hydroxycarbonyl, Methoxycarbonyl, Ethoxycarbonyl und Aminocarbonyl substituiert sein kann,
oder für (C₂-C₆)-Alkenyl steht
oder
für (C₃-C₆)-Cycloalkyl steht, welches ein- oder zweifach, gleich oder verschieden, mit Resten ausgewählt aus der Reihe Fluor, Methyl, Trifluormethyl, Ethyl und Hydroxy substituiert sein kann,
Ar¹ für Phenyl oder Thienyl steht, welche jeweils ein- oder zweifach, gleich oder verschieden, mit Resten ausgewählt aus der Reihe Fluor, Chlor, Cyano, Methyl, Trifluormethyl, Ethyl, Hydroxy, Methoxy, Trifluormethoxy und Ethoxy substituiert sein können,
L¹ für die Gruppe -CH₂- oder -SO₂- steht,
Q für einen 5-gliedrigen Heteroaryl-Ring mit bis zu drei Ring-Heteroatomen aus der Reihe N, O und/oder S oder einen 6-gliedrigen Heteroaryl-Ring mit bis zu zwei Ring-Stickstoffatomen oder
für einen gegebenenfalls substituierten Phenyl-Ring der Formel 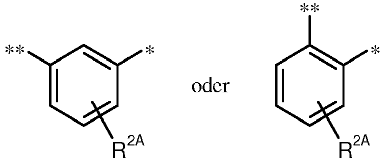 steht, worin
* die Verknüpfungsstelle mit der Gruppe L¹
und ** die Verknüpfungsstelle mit der Gruppe L² kennzeichnen,
und R^{2A} Wasserstoff, Fluor, Chlor, Brom, Methyl, Trifluormethyl, Hydroxymethyl, Carbamoyloxymethyl, Hydroxycarbonyl, Methoxycarbonyl, Ethoxycarbonyl, Aminocarbonyl, Methylaminocarbonyl oder *tert*.-Butylaminocarbonyl bedeutet,
R² für einen Substituenten ausgewählt aus der Reihe Fluor, Chlor, Brom, (C₁-C₄)-Alkyl, (C₃-C₆)-Cycloalkyl, Phenyl, (C₁-C₄)-Alkoxy, Hydroxycarbonyl, (C₁-C₄)-Alkoxycarbonyl, Aminocarbonyl und Mono-(C₁-C₄)-alkylaminocarbonyl steht,
wobei der (C₁-C₄)-Alkyl-Substituent seinerseits mit Hydroxy, (C₁-C₄)-Alkoxy, Carbamoyloxy, Hydroxycarbonyl, (C₁-C₄)-Alkoxycarbonyl oder Aminocarbonyl oder bis zu dreifach mit Fluor substituiert sein kann
und
wobei der Phenyl-Substituent seinerseits mit Fluor, Chlor, Methyl oder Trifluormethyl substituiert sein kann,
n für die Zahl 0 oder 1 steht,
L² für eine Bindung oder für eine Gruppe der Formel -(CR^{3A}R^{3B})ₚ- steht, worin
R^{3A} Wasserstoff oder Methyl bedeutet, R^{3B} Wasserstoff, (C₁-C₄)-Alkyl, Hydroxycarbonyl, (C₁-C₄)-Alkoxycarbonyl oder Aminocarbonyl bedeutet,
wobei (C₁-C₄)-Alkyl mit Hydroxy oder Carbamoyloxy substituiert sein kann,
und
p die Zahl 1 oder 2 bedeutet,
wobei im Falle, dass die Gruppe -CR^{3A}R^{3B}- zweifach auftritt, die einzelnen Bedeutungen von R^{3A} und R^{3B} jeweils gleich oder verschieden sein können,
und
Ar² für Phenyl steht, welches ein- oder zweifach, gleich oder verschieden, mit Resten ausgewählt aus der Reihe Fluor, Chlor, Cyano, Difluormethyl, Trifluormethyl, (C₁-C₄)-Alkyl, Methoxy, Difluormethoxy, Trifluormethoxy und Ethoxy substituiert sein kann,
sowie ihre Salze, Solvate und Solvate der Salze.

2. Verbindung der Formel (I) nach Anspruch 1, in welcher
R¹ für (C₁-C₄)-Alkyl steht, welches ein- oder zweifach, gleich oder verschieden, mit Resten ausgewählt aus der Reihe Fluor, Trifluormethyl, Oxo, Hydroxy und Phenyl substituiert sein kann,
wobei Phenyl seinerseits mit einem Rest ausgewählt aus der Reihe Fluor, Chlor, Methyl, Trifluormethyl, Methoxy, Hydroxycarbonyl und Methoxycarbonyl substituiert sein kann,
oder
für Allyl oder Cyclopropyl steht,
Ar¹ für Phenyl oder Thienyl steht, welche jeweils mit einem Rest ausgewählt aus der Reihe Fluor und Chlor substituiert sind,
L¹ für die Gruppe -CH₂- steht,
Q für einen Pyridyl-, Pyrimidinyl- oder gegebenenfalls substituierten Phenyl-Ring der Formel 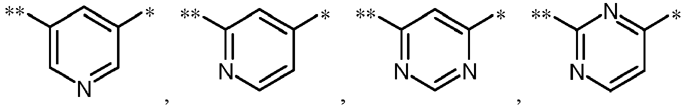 oder 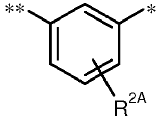 oder
für einen gegebenenfalls substituierten 5-gliedrigen Heteroaryl-Ring der Formel 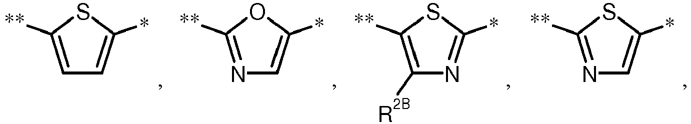 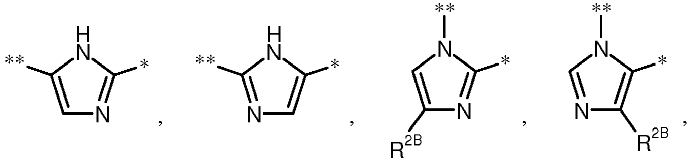 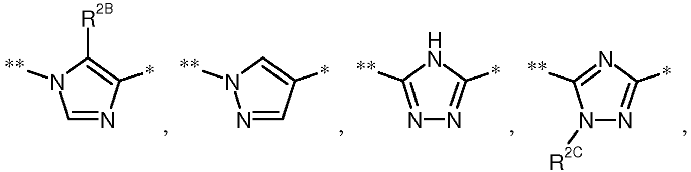 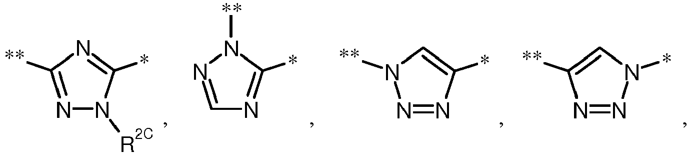 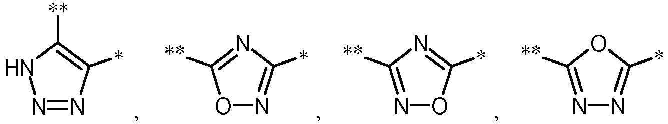 oder 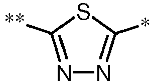 steht, worin
* die Verknüpfungsstelle mit der Gruppe L¹
und
** die Verknüpfungsstelle mit der Gruppe L² kennzeichnen,
R^{2A} Wasserstoff, Fluor, Chlor, Brom, Methyl, Trifluormethyl, Hydroxymethyl, Carbamoyloxymethyl, Hydroxycarbonyl, Methoxycarbonyl, Ethoxycarbonyl, Aminocarbonyl, Methylaminocarbonyl oder *tert*.-Butylaminocarbonyl bedeutet,
R^{2B} Wasserstoff, Methyl oder Trifluormethyl bedeutet und
R^{2C} Wasserstoff oder Methyl, welches mit Hydroxycarbonyl, Methoxycarbonyl oder Aminocarbonyl substituiert sein kann, bedeutet,
L² für eine Bindung oder die Gruppe -CH₂- steht
und
Ar² für Phenyl steht, welches ein- oder zweifach, gleich oder verschieden, mit Resten ausgewählt aus der Reihe Fluor, Chlor, Methyl, Trifluormethyl, Methoxy und Trifluormethoxy substituiert ist,
sowie ihre Salze, Solvate und Solvate der Salze.

3. Verbindung der Formel (I) nach Anspruch 1 oder 2, in welcher
R¹ für (C₁-C₄)-Alkyl, welches ein- oder zweifach, gleich oder verschieden, mit Resten ausgewählt aus der Reihe Fluor, Trifluormethyl und Hydroxy substituiert sein kann, oder für Cyclopropyl steht,
Ar¹ für *p*-Chlorphenyl steht,
L¹ für die Gruppe -CH₂- steht,
Q für einen Pyrimidinyl-Ring der Formel 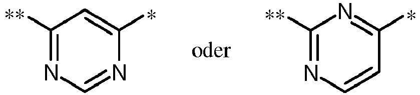 oder
für einen gegebenenfalls substituierten 5-gliedrigen Heteroaryl-Ring der Formel 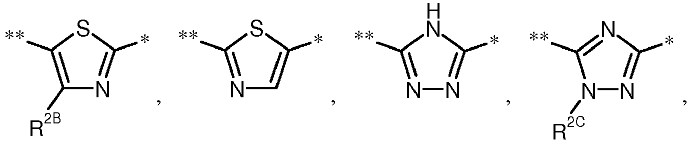 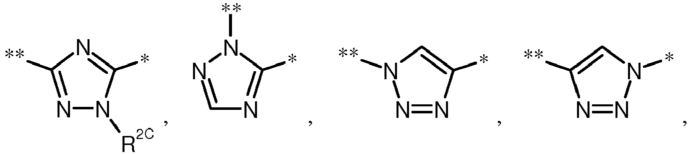 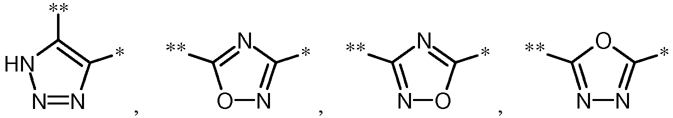 oder 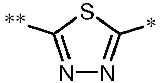 steht, worin
* die Verknüpfungsstelle mit der Gruppe L¹ und
** die Verknüpfungsstelle mit der Gruppe L² kennzeichnen,
R^{2B} Wasserstoff, Methyl oder Trifluormethyl bedeutet und
R^{2C} Wasserstoff oder Methyl, welches mit Hydroxycarbonyl, Methoxycarbonyl oder Aminocarbonyl substituiert sein kann, bedeutet,
L² für eine Bindung oder die Gruppe -CH₂- steht
und
Ar² für Phenyl steht, welches ein- oder zweifach, gleich oder verschieden, mit Resten ausgewählt aus der Reihe Fluor, Chlor, Methyl, Trifluormethyl, Methoxy und Trifluormethoxy substituiert ist,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man ein 5-Aryl-1,2,4-triazolon-Derivat der Formel (II) 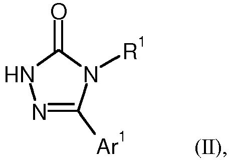 in welcher Ar¹ und R¹ die in den Ansprüchen 1 bis 3 angegebenen Bedeutungen haben,
in Gegenwart einer Base entweder
[A] mit einer Verbindung der Formel (III) 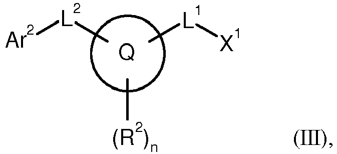 in welcher Ar², L¹, L², Q, R² und n die in den Ansprüchen 1 bis 3 angegebenen Bedeutungen haben
und
X¹ für eine Abgangsgruppe wie Chlor, Brom, Iod, Mesylat oder Tosylat steht,
zu einer Verbindung der Formel (I) umsetzt oder
[B] in einer Alternative für den Fall, dass L² in Formel (I) für eine Bindung steht und die Gruppe Ar² an ein Kohlenstoffatom des Ringes Q gebunden ist,
mit einer Verbindung der Formel (IV) 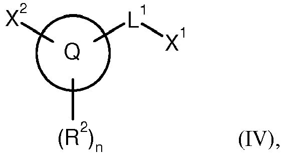 in welcher L¹, Q, R² und n die in den Ansprüchen 1 bis 3 angegebenen Bedeutungen haben,
X¹ für eine Abgangsgruppe wie Chlor, Brom, Iod, Mesylat oder Tosylat steht
und
X² für eine an ein Kohlenstoffatom des Ringes Q gebundene Abgangsgruppe wie Chlor, Brom, Iod, Mesylat oder Triflat steht,
zu einem Intermediat der Formel (V) 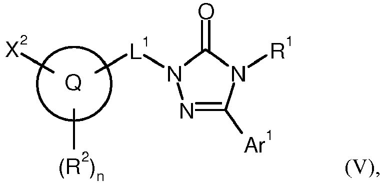 in welcher Ar¹, L¹, Q, R¹, R², X² und n die oben angegebenen Bedeutungen haben, umsetzt und dieses dann in Gegenwart eines geeigneten Übergangsmetall-Katalysators mit einer Verbindung der Formel (VI)
Ar²-M (VI),
in welcher Ar² die in den Ansprüchen 1 bis 3 angegebene Bedeutung hat
und
M für eine Gruppe der Formel -B(OR⁴)₂, -MgHal, -ZnHal oder -Sn(R⁵)₃ steht, worin
Hal Halogen, insbesondere Chlor, Brom oder Iod bedeutet,
R⁴ Wasserstoff oder (C₁-C₄)-Alkyl bedeutet oder beide Reste R⁴ miteinander verknüpft sind und zusammen eine -(CH₂)₂-, -(CH₂)₃-, -C(CH₃)₂-C(CH₃)₂- oder -CH₂-C(CH₃)₂-CH₂-Brücke bilden
und
R⁵ (C₁-C₄)-Alkyl bedeutet,
zu einer Verbindung der Formel (I-A) 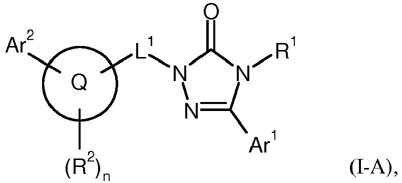 in welcher Ar¹, Ar², L¹, Q, R¹, R² und n die oben angegebenen Bedeutungen haben, kuppelt
oder
[C] in einer Alternative für den Fall, dass L² in Formel (I) für die Gruppe -(CR^{3A}R^{3B})ₚ-, wie in den Ansprüchen 1 bis 3 definiert, steht und an ein Stickstoffatom des Ringes Q gebunden ist,
mit einer Verbindung der Formel (VII) 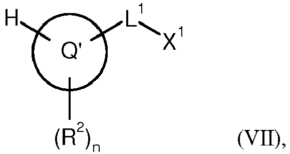 in welcher L¹, R² und n die in den Ansprüchen 1 bis 3 angegebenen Bedeutungen haben,
Q' für einen 5-gliedrigen Heteroaryl-Ring, wie in den Ansprüchen 1 bis 3 unter Q definiert, steht, welcher ein mit dem angezeigten Wasserstoffatom verbundenes, trivalentes Ring-Stickstoffatom enthält,
und
X¹ für eine Abgangsgruppe wie Chlor, Brom, Iod, Mesylat oder Tosylat steht,
zu einem Intermediat der Formel (VIII) 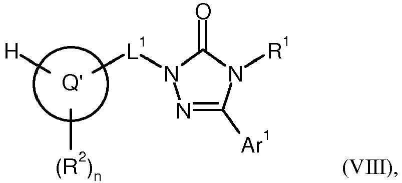 in welcher Ar¹, L¹, Q', R¹, R² und n die oben angegebenen Bedeutungen haben,
umsetzt und dieses dann in Gegenwart einer Base mit einer Verbindung der Formel (IX) 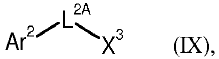 in welcher Ar² die in den Ansprüchen 1 bis 3 angegebene Bedeutung hat,
L^{2A} für die Gruppe -(CR^{3A}R^{3B})ₚ-, wie in den Ansprüchen 1 bis 3 definiert, steht
und
X³ für eine Abgangsgruppe wie Chlor, Brom, Iod, Mesylat oder Tosylat steht,
zu einer Verbindung der Formel (I-B) 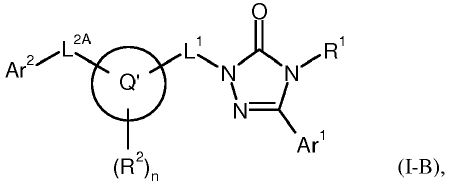 in welcher Ar¹, Ar², L¹, L^{2A}, Q', R¹, R² und n die oben angegebenen Bedeutungen haben,
N-alkyliert
und die so erhaltenen Verbindungen der Formel (I), (I-A) bzw. (I-B) gegebenenfalls in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Verwendung in der Behandlung und/oder Prävention von Krankheiten.

6. Verbindung der Formel (I), wie in einem der Ansprüche 1 bis 3 definiert, zur Verwendung in einem Verfahren zur Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

7. Arzneimittel enthaltend eine Verbindung der Formel (I), wie in einem der Ansprüche 1 bis 3 definiert, in Kombination mit einem oder mehreren inerten, nicht-toxischen, pharmazeutisch geeigneten Hilfsstoffen.

8. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus Diuretika, Angiotensin AII-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

9. Arzneimittel nach Anspruch 7 oder 8 zur Verwendung in der Behandlung und/oder Prävention von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

## Claims

1. Compound of the formula (I) 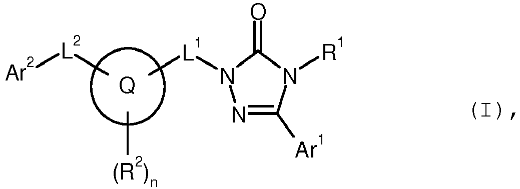 in which
R¹ represents (C₁-C₆)-alkyl which may be mono- to trisubstituted by identical or different radicals selected from the group consisting of fluorine, trifluoromethyl, oxo, hydroxyl, methoxy, ethoxy, (C₃-C₆)-cycloalkyl and phenyl,
where (C₃-C₆)-cycloalkyl may be substituted up to two times by identical or different radicals selected from the group consisting of fluorine, methyl, trifluoromethyl, ethyl and hydroxyl
and
where phenyl may be substituted up to two times by identical or different radicals selected from the group consisting of fluorine, chlorine, cyano, methyl, difluoromethyl, trifluoromethyl, ethyl, hydroxyl, methoxy, trifluoromethoxy, ethoxy, hydroxycarbonyl, methoxycarbonyl, ethoxycarbonyl and aminocarbonyl,
or
represents (C₂-C₆)-alkenyl
or
represents (C₃-C₆)-cycloalkyl which may be mono- or disubstituted by identical or different radicals selected from the group consisting of fluorine, methyl, trifluoromethyl, ethyl and hydroxyl,
Ar¹ represents phenyl or thienyl, each of which may be mono- or disubstituted by identical or different radicals selected from the group consisting of fluorine, chlorine, cyano, methyl, trifluoromethyl, ethyl, hydroxyl, methoxy, trifluoromethoxy and ethoxy,
L¹ represents the group -CH₂- or -SO₂-,
Q represents a 5-membered heteroaryl ring having up to three ring heteroatoms from the group consisting of N, O and S or a 6-membered heteroaryl ring having up to two nitrogen ring atoms or
represents an optionally substituted phenyl ring of the formula 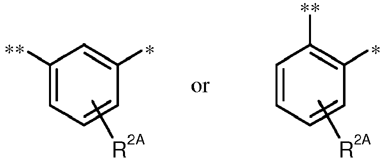 in which
* denotes the point of attachment to the group L¹
and
** denotes the point of attachment to the group L²,
and
R^{2A} represents hydrogen, fluorine, chlorine, bromine, methyl, trifluoromethyl, hydroxymethyl, carbamoyloxymethyl, hydroxycarbonyl, methoxycarbonyl, ethoxycarbonyl, aminocarbonyl, methylaminocarbonyl or *tert-*butylaminocarbonyl,
R² represents a substituent selected from the group consisting of fluorine, chlorine, bromine, (C₁-C₄)-alkyl, (C₃-C₆)-cycloalkyl, phenyl, (C₁-C₄)-alkoxy, hydroxycarbonyl, (C₁-C₄)-alkoxycarbonyl, aminocarbonyl and mono-(C₁-C₄)-alkylaminocarbonyl,
where the (C₁-C₄)-alkyl substituent for its part may be substituted by hydroxyl, (C₁-C₄)-alkoxy, carbamoyloxy, hydroxycarbonyl, (C₁-C₄)-alkoxycarbonyl or aminocarbonyl or up to three times by fluorine
and
where the phenyl substituent for its part may be substituted by fluorine, chlorine, methyl or trifluoromethyl,
n represents the number 0 or 1,
L² represents a bond or represents a group of the formula -(CR^{3A}R^{3B})ₚ- in which
R^{3A} represents hydrogen or methyl,
R^{3B} represents hydrogen, (C₁-C₄)-alkyl, hydroxycarbonyl, (C₁-C₄)-alkoxycarbonyl or aminocarbonyl,
where (C₁-C₄)-alkyl may be substituted by hydroxyl or carbamoyloxy,
and
p represents the number 1 or 2,
where in the case that the group -CR^{3A}R^{3B}- occurs twice the individual meanings of R^{3A} and R^{3B} may in each case be identical or different,
and
Ar² represents phenyl which may be mono- or disubstituted by identical or different radicals selected from the group consisting of fluorine, chlorine, cyano, difluoromethyl, trifluoromethyl, (C₁-C₄)-alkyl, methoxy, difluoromethoxy, trifluoromethoxy and ethoxy,
and salts, solvates and solvates of the salts thereof.

2. Compound of the formula (I) according to Claim 1 in which
R¹ represents (C₁-C₄)-alkyl which may be mono- or disubstituted by identical or different radicals selected from the group consisting of fluorine, trifluoromethyl, oxo, hydroxyl and phenyl, where phenyl for its part may be substituted by a radical selected from the group consisting of fluorine, chlorine, methyl, trifluoromethyl, methoxy, hydroxycarbonyl and methoxycarbonyl,
or
represents allyl or cyclopropyl,
Ar¹ represents phenyl or thienyl, each of which is substituted by a radical selected from the group consisting of fluorine and chlorine,
L¹ represents the group -CH₂-,
Q represents a pyridyl ring, a pyrimidinyl ring or an optionally substituted phenyl ring of the formula 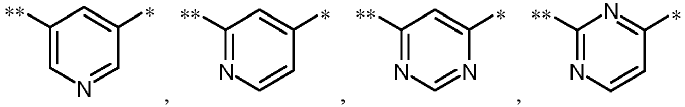 or 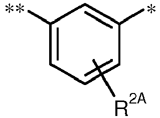 or
represents an optionally substituted 5-membered heteroaryl ring of the formula 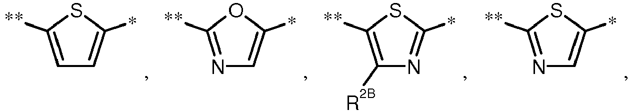 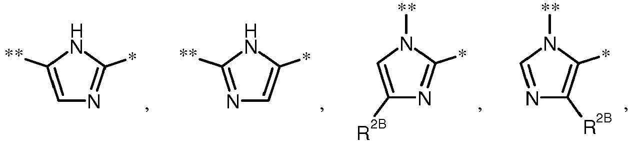 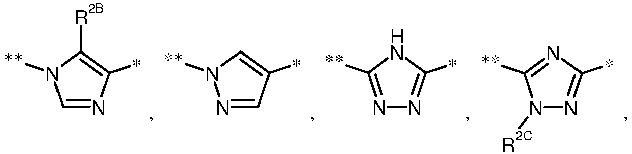 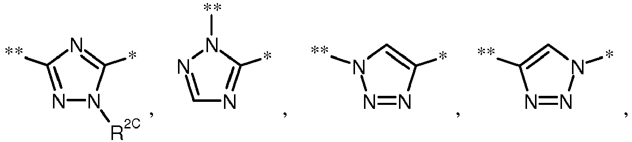 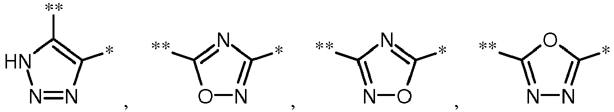 or 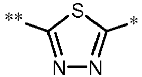 in which
* denotes the point of attachment to the group L¹
and
** denotes the point of attachment to the group L²,
R^{2A} represents hydrogen, fluorine, chlorine, bromine, methyl, trifluoromethyl, hydroxymethyl, carbamoyloxymethyl, hydroxycarbonyl, methoxycarbonyl, ethoxycarbonyl, aminocarbonyl, methylaminocarbonyl or *tert*-butylaminocarbonyl,
R^{2B} represents hydrogen, methyl or trifluoromethyl
and
R^{2C} represents hydrogen or methyl which may be substituted by hydroxycarbonyl, methoxycarbonyl or aminocarbonyl,
L² represents a bond or the group -CH₂-
and
Ar² represents phenyl which is mono- or disubstituted by identical or different radicals selected from the group consisting of fluorine, chlorine, methyl, trifluoromethyl, methoxy and trifluoromethoxy,
and salts, solvates and solvates of the salts thereof.

3. Compound of the formula (I) according to either of Claims 1 and 2 in which
R¹ represents (C₁-C₄)-alkyl which may be mono- or disubstituted by identical or different radicals selected from the group consisting of fluorine, trifluoromethyl and hydroxyl, or represents cyclopropyl,
Ar¹ represents *p*-chlorophenyl,
L¹ represents the group -CH₂-,
Q represents a pyrimidinyl ring of the formula 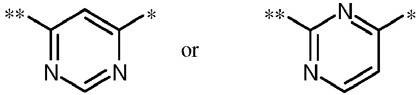 or
represents an optionally substituted 5-membered heteroaryl ring of the formula 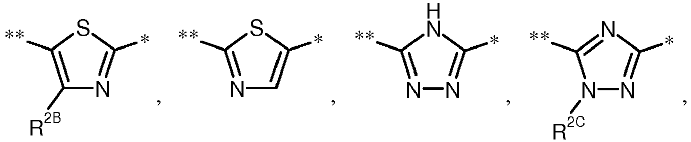 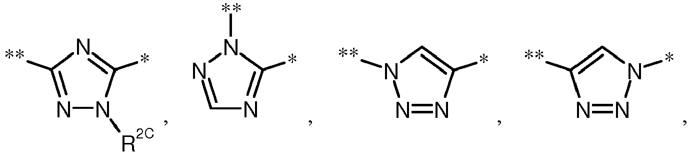 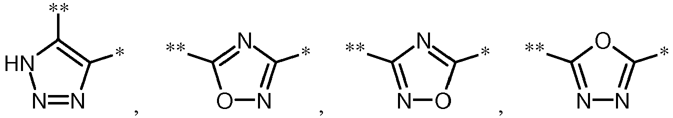 or 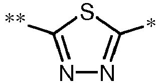 in which
* denotes the point of attachment to the group L¹ and
** denotes the point of attachment to the group L²,
R^{2B} represents hydrogen, methyl or trifluoromethyl and
R^{2C} represents hydrogen or methyl which may be substituted by hydroxycarbonyl, methoxycarbonyl or aminocarbonyl,
L² represents a bond or the group -CH₂-
and
Ar² represents phenyl which is mono- or disubstituted by identical or different radicals selected from the group consisting of fluorine, chlorine, methyl, trifluoromethyl, methoxy and trifluoromethoxy,
and salts, solvates and solvates of the salts thereof.

4. Process for preparing compounds of the formula (I) as defined in any of Claims 1 to 3, **characterized in that** a 5-aryl-1,2,4-triazolone derivative of the formula (II) 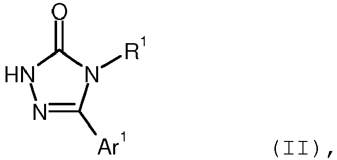 in which Ar¹ and R¹ have the meanings given in any of Claims 1 to 3
is reacted in the presence of a base either
[A] with a compound of the formula (III) 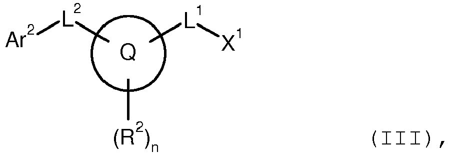 in which Ar², L¹, L², Q, R² and n have the meanings given in any of Claims 1 to 3 and
X¹ represents a leaving group such as chlorine, bromine, iodine, mesylate or tosylate,
to give a compound of the formula (I)
or
[B] in an alternative in the case that L² in formula (I) represents a bond and the group Ar² is attached to a carbon atom of ring Q with a compound of the formula (IV) 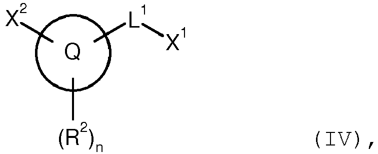 in which L¹, Q, R² and n have the meanings given in any of Claims 1 to 3,
X¹ represents a leaving group such as chlorine, bromine, iodine, mesylate or tosylate
and
X² represents a leaving group, such as chlorine, bromine, iodine, mesylate or triflate, which is attached to a carbon atom of ring Q,
to give an intermediate of the formula (V) 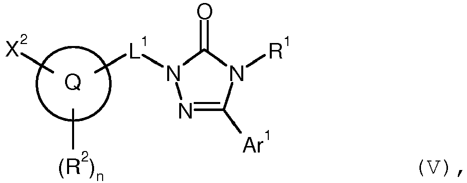 in which Ar¹, L¹, Q, R¹, R², X² and n have the meanings given above,
and this is then coupled in the presence of a suitable transition metal catalyst with a compound of the formula (VI)
**Ar²-M** (VI),
in which Ar² has the meaning given in any of Claims 1 to 3
and
M represents a group of the formula-B(OR⁴)₂, -MgHal, -ZnHal or -Sn(R⁵)₃ in which
Hal represents halogen, in particular chlorine, bromine or iodine,
R⁴ represents hydrogen or (C₁-C₄)-alkyl or both radicals R⁴ are attached to one another and together form a-(CH₂)₂-, -(CH₂)₃-, -C(CH₃)₂-C(CH₃)₂- or -CH₂-C(CH₃)₂-CH₂- bridge
and
R⁵ represents (C₁-C₄)-alkyl,
to give a compound of the formula (I-A) 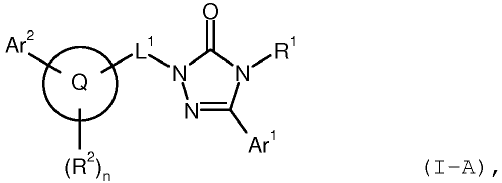 in which Ar¹, Ar², L¹, Q, R¹, R² and n have the meanings given above,
or
[C] in an alternative in the case that L² in formula (I) represents the group -(CR^{3A}R^{3B})ₚ-, as defined in any of Claims 1 to 3, and is attached to a nitrogen atom of ring Q,
with a compound of the formula (VII) 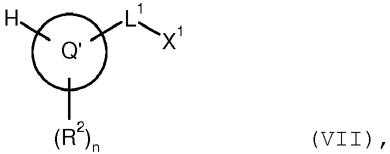 in which L¹, R² and n have the meanings given in any of Claims 1 to 3
Q' represents a 5-membered heteroaryl ring, as defined in any of Claims 1 to 3 under Q, which contains a trivalent nitrogen ring atom attached to the hydrogen atom indicated,
and
X¹ represents a leaving group such as chlorine, bromine, iodine, mesylate or tosylate,
to give an intermediate of the formula (VIII) 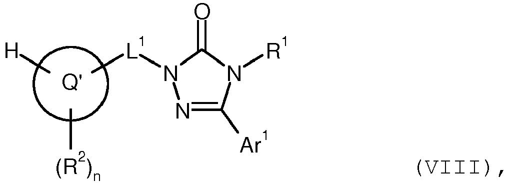 in which Ar¹, L¹, Q', R¹, R² and n have the meanings given above,
and this is then N-alkylated in the presence of a base with a compound of the formula (IX) 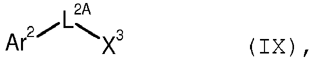 in which Ar² has the meaning given in any of Claims 1 to 4,
L^{2A} represents the group -(CR^{3A}R^{3B})ₚ-, as defined in any of Claims 1 to 3, and
X³ represents a leaving group such as chlorine, bromine, iodine, mesylate or tosylate,
to give a compound of the formula (I-B) 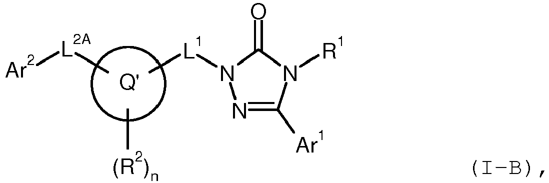 in which Ar¹, Ar², L¹, L^{2A}, Q', R¹, R² and n have the meanings given above, and the resulting compounds of the formula (I), (I-A) or (I-B) are optionally separated into their enantiomers and/or diastereomers and/or converted with the appropriate (i) solvents and/or (ii) bases or acids into their solvates, salts and/or solvates of the salts.

5. Compound of the formula (I) as defined in any of Claims 1 to 3 for use in the treatment and/or prevention of diseases.

6. Compound of the formula (I) as defined in any of Claims 1 to 3 for use in a method for the treatment and/or prevention of acute and chronic heart failure, hypervolemic and euvolemic hyponatremia, cirrhosis of the liver, ascites, edema and the syndrome of inadequate ADH secretion (SIADH).

7. Medicament comprising a compound of the formula (I) as defined in any of Claims 1 to 3 in combination with one or more inert, non-toxic, pharmaceutically suitable auxiliaries.

8. Medicament comprising a compound of the formula (I) as defined in any of Claims 1 to 3 in combination with one or more further active substances selected from the group consisting of diuretics, angiotensin AII antagonists, ACE inhibitors, beta-receptor blockers, mineralocorticoid receptor antagonists, organic nitrates, NO donators and positive-inotropic active substances.

9. Medicament according to Claim 7 or 8 for use in the treatment and/or prevention of acute and chronic heart failure, hypervolemic and euvolemic hyponatremia, cirrhosis of the liver, ascites, edema and the syndrome of inadequate ADH secretion (SIADH).

## Revendications

1. Composé de formule (I) 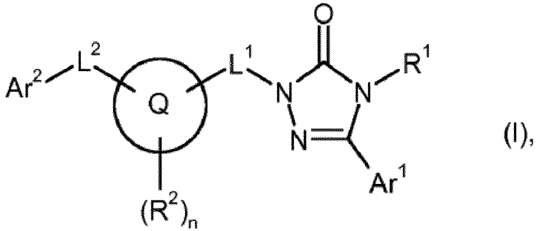 dans lequel
R¹ représente alkyle en (C₁-C₆), qui peut être substitué une à trois fois, de manière identique ou différente, avec des radicaux choisis dans la série constituée par fluor, trifluorométhyle, oxo, hydroxy, méthoxy, éthoxy, cycloalkyle en (C₃-C₆) et phényle,
le cycloalkyle en (C₃-C₆) pouvant être substitué jusqu'à deux fois, de manière identique ou différente, avec des radicaux choisis dans la série constituée par fluor, méthyle, trifluorométhyle, éthyle et hydroxy, et
le phényle pouvant être substitué jusqu'à deux fois, de manière identique ou différente, avec des radicaux choisis dans la série constituée par fluor, chlore, cyano, méthyle, difluorométhyle, trifluorométhyle, éthyle, hydroxy, méthoxy, trifluorométhoxy, éthoxy, hydroxycarbonyle, méthoxycarbonyle, éthoxycarbonyle et aminocarbonyle,
ou
alcényle en (C₂-C₆),
ou
cycloalkyle en (C₃-C₆), qui peut être substitué une ou deux fois, de manière identique ou différente, avec des radicaux choisis dans la série constituée par fluor, méthyle, trifluorométhyle, éthyle et hydroxy,
Ar¹ représente phényle ou thiényle, qui peuvent chacun être substitués une ou deux fois, de manière identique ou différente, avec des radicaux choisis dans la série constituée par fluor, chlore, cyano, méthyle, trifluorométhyle, éthyle, hydroxy, méthoxy, trifluorométhoxy et éthoxy,
L¹ représente le groupe -CH₂- ou -SO₂-,
Q représente un cycle hétéroaryle à 5 éléments contenant jusqu'à trois hétéroatomes de cycle de la série constituée par N, O et/ou S, ou un cycle hétéroaryle à 6 éléments contenant jusqu'à deux atomes d'azote de cycle, ou
un cycle phényle éventuellement substitué de formule 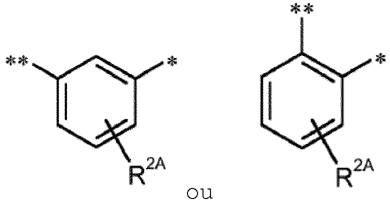 dans lesquelles
* désigne l'emplacement de liaison avec le groupe L¹,
et
** désigne l'emplacement de liaison avec le groupe L², et
R^{2A} signifie hydrogène, fluor, chlore, brome, méthyle, trifluorométhyle, hydroxyméthyle, carbamoyloxyméthyle, hydroxycarbonyle, méthoxycarbonyle, éthoxycarbonyle, aminocarbonyle, méthylaminocarbonyle ou tert.-butylaminocarbonyle,
R² représente un substituant choisi dans la série constituée par fluor, chlore, brome, alkyle en (C₁-C₄), cycloalkyle en (C₃-C₆), phényle, alcoxy en (C₁-C₄), hydroxycarbonyle, alcoxycarbonyle en (C₁-C₄), aminocarbonyle et mono-alkyle en (C₁-C₄)-aminocarbonyle,
le substituant alkyle en (C₁-C₄) pouvant lui-même être substitué avec hydroxy, alcoxy en (C₁-C₄), carbamoyloxy, hydroxycarbonyle, alcoxycarbonyle en (C₁-C₄) ou aminocarbonyle, ou jusqu'à trois fois avec fluor,
et
le substituant phényle pouvant lui-même être substitué avec fluor, chlore, méthyle ou trifluorométhyle,
n représente le nombre 0 ou 1,
L² représente une liaison ou un groupe de formule-(CR^{3A}R^{3B})ₚ-,
dans lequel
R^{3A} signifie hydrogène ou méthyle,
R^{3B} signifie hydrogène, alkyle en (C₁-C₄), hydroxycarbonyle, alcoxycarbonyle en (C₁-C₄) ou aminocarbonyle,
l'alkyle en (C₁-C₄) pouvant être substitué avec hydroxy ou carbamoyloxy,
et
p signifie le nombre 1 ou 2,
lorsque le groupe -CR^{3A}R^{3B}- est présent à deux reprises, les significations individuelles de R^{3A} et R^{3B} pouvant chacune être identiques ou différentes,
et
Ar² représente phényle, qui peut être substitué une ou deux fois, de manière identique ou différente, avec des radicaux choisis dans la série constituée par fluor, chlore, cyano, difluorométhyle, trifluorométhyle, alkyle en (C₁-C₄), méthoxy, difluorométhoxy, trifluorométhoxy et éthoxy,
ainsi que leurs sels, solvates et solvates des sels.

2. Composé de formule (I) selon la revendication 1, dans lequel
R¹ représente alkyle en (C₁-C₄), qui peut être substitué une ou deux fois, de manière identique ou différente, avec des radicaux choisis dans la série constituée par fluor, trifluorométhyle, oxo, hydroxy et phényle,
le phényle pouvant lui-même être substitué avec un radical choisi dans la série constituée par fluor, chlore, méthyle, trifluorométhyle, méthoxy hydroxycarbonyle et méthoxycarbonyle,
ou
allyle ou cyclopropyle,
Ar¹ représente phényle ou thiényle, qui peuvent chacun être substitués avec un radical choisi dans la série constituée par fluor et chlore,
L¹ représente le groupe -CH₂-,
Q représente un cycle pyridyle, pyrimidinyle ou phényle éventuellement substitué de formule 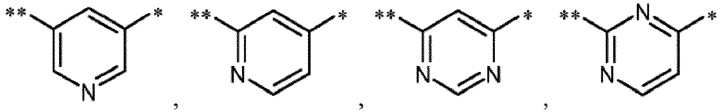 ou 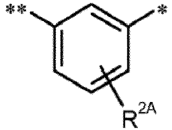
ou
un cycle hétéroaryle à 5 éléments éventuellement substitué de formule 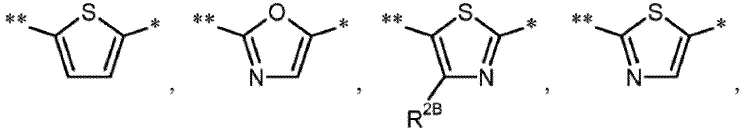 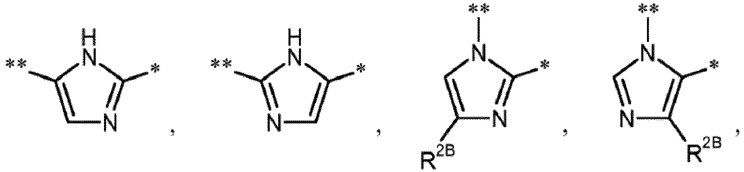 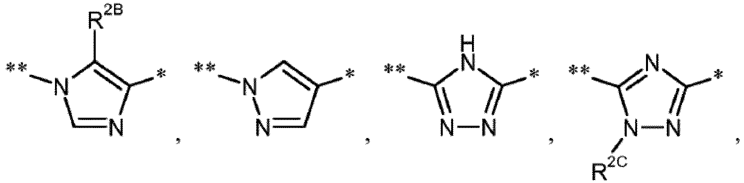 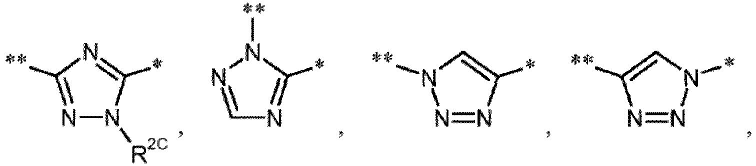 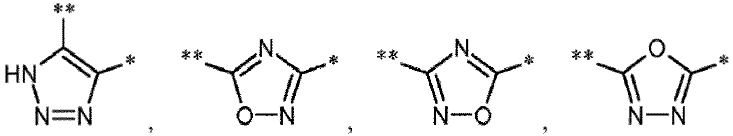 ou 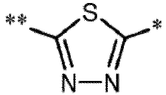 dans lesquelles
* désigne l'emplacement de liaison avec le groupe L¹, et
** désigne l'emplacement de liaison avec le groupe L²,
R^{2A} signifie hydrogène, fluor, chlore, brome, méthyle, trifluorométhyle, hydroxyméthyle, carbamoyloxyméthyle, hydroxycarbonyle, méthoxycarbonyle, éthoxycarbonyle, aminocarbonyle, méthylaminocarbonyle ou tert.-butylaminocarbonyle,
R^{2B} signifie hydrogène, méthyle ou trifluorométhyle,
et
R^{2C} signifie hydrogène ou méthyle, qui peut être substitué avec hydroxycarbonyle, méthoxycarbonyle ou aminocarbonyle,
L² représente une liaison ou un groupe -CH₂-,
et
Ar² représente phényle, qui peut être substitué une ou deux fois, de manière identique ou différente, avec des radicaux choisis dans la série constituée par fluor, chlore, méthyle, trifluorométhyle, méthoxy et trifluorométhoxy,
ainsi que leurs sels, solvates et solvates des sels.

3. Composé de formule (I) selon la revendication 1 ou 2, dans lequel
R¹ représente alkyle en (C₁-C₄), qui peut être substitué une ou deux fois, de manière identique ou différente, avec des radicaux choisis dans la série constituée par fluor, trifluorométhyle et hydroxy, ou cyclopropyle,
Ar¹ représente p-chlorophényle,
L¹ représente le groupe -CH₂-,
Q représente un cycle pyrimidinyle de formule 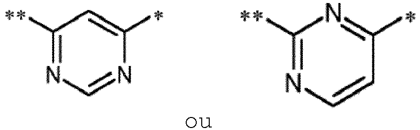 ou
un cycle hétéroaryle à 5 éléments éventuellement substitué de formule 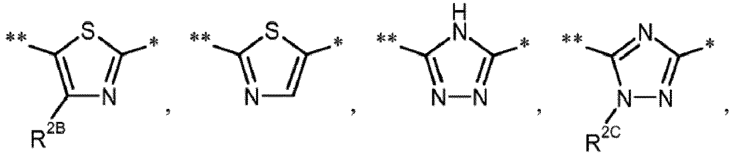 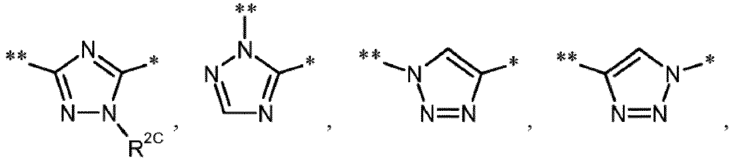 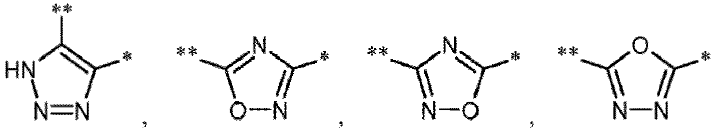 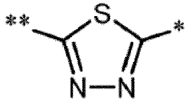 ou
dans lesquelles
* désigne l'emplacement de liaison avec le groupe L¹, et
** désigne l'emplacement de liaison avec le groupe L², R^{2B} signifie hydrogène, méthyle ou trifluorométhyle, et
R^{2C} signifie hydrogène ou méthyle, qui peut être substitué avec hydroxycarbonyle, méthoxycarbonyle ou aminocarbonyle,
L² représente une liaison ou un groupe -CH₂-,
et
Ar² représente phényle, qui peut être substitué une ou deux fois, de manière identique ou différente, avec des radicaux choisis dans la série constituée par fluor, chlore, méthyle, trifluorométhyle, méthoxy et trifluorométhoxy,
ainsi que leurs sels, solvates et solvates des sels.

4. Procédé de fabrication de composés de formule (I), tels que définis dans les revendications 1 à 3, **caractérisé en ce qu'**un dérivé de 5-aryl-1,2,4-triazolone de formule (II) 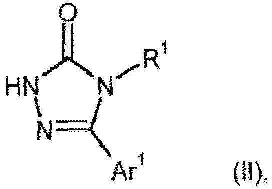 dans laquelle Ar¹ et R¹ ont les significations indiquées dans les revendications 1 à 3,
est mis en réaction en présence d'une base soit
[A] avec un composé de formule (III) 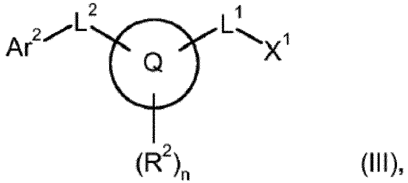
dans laquelle Ar², L¹, L², Q, R² et n ont les significations indiquées dans les revendications 1 à 3, et
X¹ représente un groupe partant tel que chlore, brome, iode, mésylate ou tosylate,
pour former un composé de formule (I),
soit
[B] selon une variante, lorsque L² dans la formule (I) représente une liaison et que le groupe Ar² est relié à un atome de carbone du cycle Q,
avec un composé de formule (IV) 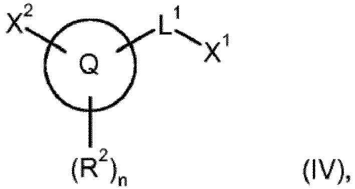
dans laquelle L¹, Q, R² et n ont les significations indiquées dans les revendications 1 à 3,
X¹ représente un groupe partant tel que chlore, brome, iode, mésylate ou tosylate,
et
X² représente un groupe partant relié à un atome de carbone du cycle Q, tel que chlore, brome, iode, mésylate ou triflate,
pour former un intermédiaire de formule (V) 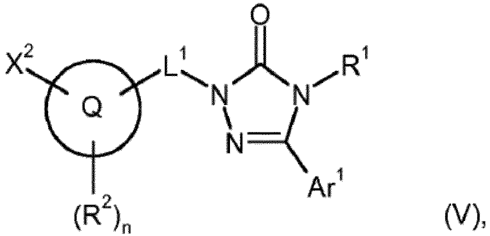
dans laquelle Ar¹, L¹, Q, R¹, R², X² et n ont les significations indiquées précédemment,
puis celui-ci est couplé en présence d'un catalyseur de métal de transition approprié avec un composé de formule (VI)
Ar²-M (VI)
dans laquelle Ar² a la signification indiquée dans les revendications 1 à 3,
et
M représente un groupe de formule -B(OR⁴)_{2'} -MgHal,-ZnHal ou -Sn(R⁵)₃, dans lesquelles
Hal signifie halogène, notamment chlore, brome ou iode,
R⁴ signifie hydrogène ou alkyle en (C₁-C₄), ou les deux radicaux R4 sont reliés l'un à l'autre et forment ensemble un pont -(CH₂)₂-, -(CH₂)₃-, -C(CH₃)₂-C(CH₃)₂- ou -CH₂-C (CH₃)₂-CH₂,
et
R⁵ signifie alkyle en (C₁-C₄),
pour former un composé de formule (I-A) 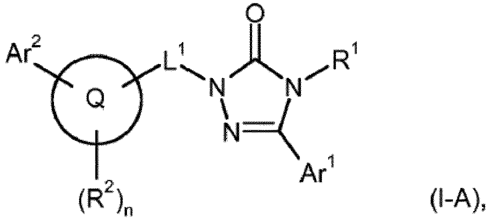 dans laquelle Ar¹, Ar², L¹, Q, R¹, R² et n ont les significations indiquées précédemment,
soit
[C] selon une variante, lorsque L² dans la formule (I) représente le groupe-(CR^{3A}R^{3B})ₚ₋, tel que défini dans les revendications 1 à 3, et est relié à un atome d'azote du cycle Q,
avec un composé de formule (VII) 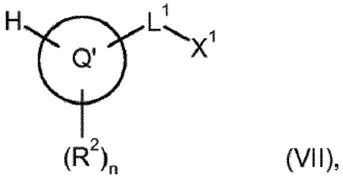 dans laquelle L¹, R² et n ont les significations indiquées dans les revendications 1 à 3,
Q' représente un cycle hétéroaryle à 5 éléments, tel que défini par Q dans les revendications 1 à 3, qui contient un atome d'azote de cycle trivalent relié avec l'atome d'hydrogène indiqué,
et
X¹ représente un groupe partant tel que chlore, brome, iode, mésylate ou tosylate,
pour former un intermédiaire de formule (VIII) 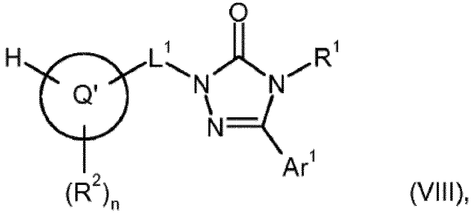 dans laquelle Ar¹, L¹, Q', R¹, R² et n ont les significations indiquées précédemment,
puis celui-ci est N-alkylé en présence d'une base avec un composé de formule (IX) 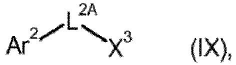
dans laquelle Ar² a la signification indiquée dans les revendications 1 à 4,
L^{2A} représente le groupe -(CR^{3A}R^{3B})ₚ-, tel que défini dans les revendications 1 à 3,
et
X³ représente un groupe partant tel que chlore, brome, iode, mésylate ou tosylate,
pour former un composé de formule (I-B) 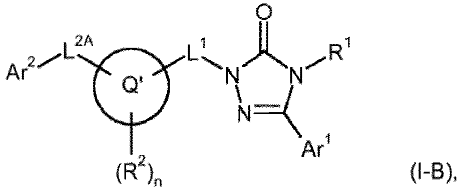 dans laquelle Ar¹, Ar², L¹, L^{2A}, Q', R¹, R² et n ont les significations indiquées précédemment,
et les composés de formule (I), (I-A) ou (I-B) ainsi obtenus sont éventuellement séparés en leurs énantiomères et/ou diastéréomères et/ou transformés avec (i) les solvants et/ou (ii) les bases ou les acides appropriés en leurs solvates, sels et/ou solvates des sels.

5. Composé de formule (I), tel que défini dans l'une quelconque des revendications 1 à 3, destiné à une utilisation dans le traitement et/ou la prévention de maladies.

6. Composé de formule (I), tel que défini dans l'une quelconque des revendications 1 à 3, destiné à une utilisation dans un procédé de traitement et/ou de prévention de l'insuffisance cardiaque aigue et chronique, de l'hyponatrémie hypervolémique et euvolémique, de la cirrhose du foie, de l'ascite, des oedèmes et du syndrome de la sécrétion inappropriée d'ADH (SIADH).

7. Médicament contenant un composé de formule (I), tel que défini dans l'une quelconque des revendications 1 à 3, en combinaison avec un ou plusieurs adjuvants inertes, non toxiques, pharmaceutiquement appropriés.

8. Médicament contenant un composé de formule (I), tel que défini dans l'une quelconque des revendications 1 à 3, en combinaison avec un ou plusieurs agents actifs supplémentaires choisis dans le groupe constitué par les diurétiques, les antagonistes d'angiotensine AII, les inhibiteurs d'ACE, les bloquants des récepteurs bêta, les antagonistes des récepteurs minéralocorticoïdes, les nitrates organiques, les donneurs de NO et les substances à effet inotrope positif.

9. Médicament selon la revendication 7 ou 8, destiné à une utilisation pour le traitement et/ou la prévention de l'insuffisance cardiaque aigue et chronique, de l'hyponatrémie hypervolémique et euvolémique, de la cirrhose du foie, de l'ascite, des oedèmes et du syndrome de la sécrétion inappropriée d'ADH (SIADH).
